# EUROPEAN PATENT SPECIFICATION

(11) **EP 3 242 132 B1**
(45) Date of publication and mention of the grant of the patent: **12.02.2020**
(21) Application number: 16168524.3
(22) Date of filing: 05.05.2016
(51) Int. Cl.: G01N 33/574

(54) **METHODS OF DETECTING CANCER**
VERFAHREN ZUR KREBSERKENNUNG
MÉTHODES DE DÉTECTION D'UN CANCER

(43) Date of publication of application: 08.11.2017
(73) Proprietor: Ascendant Diagnostics, LLC, Springdale, AR 72764 (US)
(72) Inventor: DAILY, Anna, Fayetteville, AR 72703 (US); RUTHERFORD, Lindsay, Fayetteville, AR 72701 (US); KLIMBERG, Suzanne V., Little Rock, AR 72212 (US); BECKMANN, Patricia M., Hansville, WA 98340 (US)
(74) Representative: Cooley (UK) LLP

(56) References cited:
- WO-A1-2009/097692
- WO-A1-2011/100472
- WO-A1-2014/133855
- WO-A2-2008/054764
- US-A1- 2009 035 801
- TOM GROOT KORMELINK ET AL: "Immunoglobulin free light chains are biomarkers of poor prognosis in basal-like breast cancer and are potential targets in tumor-associated inflammation", ONCOTARGET, vol. 5, no. 10, 26 March 2014 (2014-03-26) , pages 3159-3167, XP055310623, United States ISSN: 1949-2553, DOI: 10.18632/oncotarget.1868
- DILEK COLAK ET AL: "Age-Specific Gene Expression Signatures for Breast Tumors and Cross-Species Conserved Potential Cancer Progression Markers in Young Women", PLOS ONE, vol. 8, no. 5, 21 May 2013 (2013-05-21), page e63204, XP055310627, DOI: 10.1371/journal.pone.0063204
- CANCEMI PATRIZIA ET AL: "Large-scale proteomic identification of S100 proteins in breast cancer tissues", BMC CANCER, BIOMED CENTRAL, LONDON, GB, vol. 10, no. 1, 3 September 2010 (2010-09-03), page 476, XP021075298, ISSN: 1471-2407, DOI: 10.1186/1471-2407-10-476
- Michael Braun ET AL: "Down-regulation of Microfilamental Network-associated Proteins in Leukocytes of Breast Cancer Patients: Potential Application to Predictive Diagnosis", Cancer Genomics - Proteomics, 1 January 2009 (2009-01-01), page 31, XP055336710, Greece Retrieved from the Internet: URL:http://cgp.iiarjournals.org/content/6/ 1/31.full.pdf#page=1&view=FitH [retrieved on 2017-01-19]
- LEI ZHANG ET AL: "Discovery and Preclinical Validation of Salivary Transcriptomic and Proteomic Biomarkers for the Non- Invasive Detection of Breast Cancer", PLOS ONE, vol. 5, no. 12, 31 December 2010 (2010-12-31), page e15573, XP055336817,
- HAN-BYOEL LEE ET AL: "Development and Validation of a Novel Plasma Protein Signature for Breast Cancer Diagnosis by Using Multiple Reaction Monitoring-based Mass Spectrometry HAN-", ANTICANCER RESEARCH, vol. 35, 1 November 2015 (2015-11-01), pages 6271-6280, XP055336834,
- DANIEL BÖHM: "Comparison of tear protein levels in breast cancer patients and healthy controls using a de novo proteomic approach", ONCOLOGY REPORTS, 1 June 2012 (2012-06-01), XP055336860, ISSN: 1021-335X, DOI: 10.3892/or.2012.1849
- S S CROSS ET AL: "Expression of S100 proteins in normal human tissues and common cancers using tissue microarrays: S100A6, S100A8, S100A9 and S100A11 are all overexpressed in common cancers", HISTOPATHOLOGY., vol. 46, no. 3, 17 February 2005 (2005-02-17), pages 256-269, XP055336893, GB ISSN: 0309-0167, DOI: 10.1111/j.1365-2559.2005.02097.x
- GUNALDI MERAL ET AL: "Diagnostic importance of S100A9 and S100A12 in breast cancer", BIOMEDICINE AND PHARMACOTHERAPY, vol. 76, 10 November 2015 (2015-11-10), pages 52-56, XP029332870, ISSN: 0753-3322, DOI: 10.1016/J.BIOPHA.2015.10.029
- CELIS J E ET AL: "Molecular pathology of breast apocrine carcinomas: A protein expression signature specific for benign apocrine metaplasia", FEBS LETTERS, ELSEVIER, AMSTERDAM, NL, vol. 580, no. 12, 22 May 2006 (2006-05-22) , pages 2935-2944, XP028030568, ISSN: 0014-5793, DOI: 10.1016/J.FEBSLET.2006.03.080 [retrieved on 2006-05-22]
- SETH A ET AL: "GENE EXPRESSION PROFILING OF DUCTAL CARCINOMAS IN SITU AND INVASIVE BREAST TUMORS", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 23, no. 3A, 1 January 2003 (2003-01-01), pages 2043-2052, XP009055494, ISSN: 0250-7005
- FUSCO O ET AL: "90K (MAC-2 BP) gene expression in breast cancer and evidence for the production of 90K by peripheral-blood mononuclear cells", INTERNATIONAL JOURNAL OF CANCER, JOHN WILEY & SONS, INC, US, vol. 79, no. 1, 20 February 1998 (1998-02-20), pages 23-26, XP002438972, ISSN: 0020-7136, DOI: 10.1002/(SICI)1097-0215(19980220)79:1<23:: AID-IJC5>3.0.CO;2-Y
- S Iacobelli ET AL: "Prognostic value of a novel circulating serum 90K antigen in breast cancer", British Journal of Cancer, 1 January 1994 (1994-01-01), pages 172-176, XP055336934, SCOTLAND DOI: 10.1038/bjc.1994.29 Retrieved from the Internet: URL:https://www.ncbi.nlm.nih.gov/pmc/artic les/PMC1968766/pdf/brjcancer00191-0174.pdf [retrieved on 2017-01-19]
- XIANG CHEN ET AL: "Comparative Profiling of Triple-Negative Breast Carcinomas Tissue Glycoproteome by Sequential Purification of Glycoproteins and Stable Isotope Labeling", CELLULAR PHYSIOLOGY AND BIOCHEMISTRY., vol. 38, no. 1, 8 January 2016 (2016-01-08), pages 110-121, XP055336937, CH ISSN: 1015-8987, DOI: 10.1159/000438613
- KOTHS K ET AL: "CLONING AND CHARACTERIZATION OF A HUMAN MAC-2-BINDING PROTEIN, A NEW MEMBER OF THE SUPERFAMILY DEFINED BY THE MACROPHAGE SCAVENGER RECEPTOR CYSTEINE-RICH DOMAIN", JOURNAL OF BIOLOGICAL CHEMISTRY, AMERICAN SOCIETY FOR BIOCHEMISTRY AND MOLECULAR BIOLOGY, US, vol. 268, no. 19, 5 July 1993 (1993-07-05) , pages 14245-14249, XP002914299, ISSN: 0021-9258
- T. Plavina ET AL: "Increased Plasma Concentrations of Cytoskeletal and Ca2+-Binding Proteins and Their Peptides in Psoriasis Patients", CLINICAL CHEMISTRY., vol. 54, no. 11, 1 November 2008 (2008-11-01), pages 1805-1814, XP055506954, WASHINGTON, DC. ISSN: 0009-9147, DOI: 10.1373/clinchem.2008.103770

### SEQUENCE LISTING

<110> Ascendant Diagnostics, LLC
<120> Methods of Detecting Cancer
<130> ADx01
<160> 1162
<170> PatentIn version 3.5
<210> 1
   <211> 599
   <212> PRT
   <213> Homo sapiens
<400> 1
   Met Lys Leu Leu Lys Leu Thr Gly Phe Ile Phe Phe Leu Phe Phe Leu
   1               5                   10                  15
   Thr Glu Ser Leu Thr Leu Pro Thr Gln Pro Arg Asp Ile Glu Asn Phe
               20                  25                  30
   Asn Ser Thr Gln Lys Phe Ile Glu Asp Asn Ile Glu Tyr Ile Thr Ile
           35                  40                  45
   Ile Ala Phe Ala Gln Tyr Val Gln Glu Ala Thr Phe Glu Glu Met Glu
       50                  55                  60
   Lys Leu Val Lys Asp Met Val Glu Tyr Lys Asp Arg Cys Met Ala Asp
   65                  70                  75                  80
   Lys Thr Leu Pro Glu Cys Ser Lys Leu Pro Asn Asn Val Leu Gln Glu
                   85                  90                  95
   Lys Ile Cys Ala Met Glu Gly Leu Pro Gln Lys His Asn Phe Ser His
               100                 105                 110
   Cys Cys Ser Lys Val Asp Ala Gln Arg Arg Leu Cys Phe Phe Tyr Asn
           115                 120                 125
   Lys Lys Ser Asp Val Gly Phe Leu Pro Pro Phe Pro Thr Leu Asp Pro
       130                 135                 140
   Glu Glu Lys Cys Gln Ala Tyr Glu Ser Asn Arg Glu Ser Leu Leu Asn
   145                 150                 155                 160
   His Phe Leu Tyr Glu Val Ala Arg Arg Asn Pro Phe Val Phe Ala Pro
                   165                 170                 175
   Thr Leu Leu Thr Val Ala Val His Phe Glu Glu Val Ala Lys Ser Cys
               180                 185                 190
   Cys Glu Glu Gln Asn Lys Val Asn Cys Leu Gln Thr Arg Ala Ile Pro
           195                 200                 205
   Val Thr Gln Tyr Leu Lys Ala Phe Ser Ser Tyr Gln Lys His Val Cys
       210                 215                 220
   Gly Ala Leu Leu Lys Phe Gly Thr Lys Val Val His Phe Ile Tyr Ile
   225                 230                 235                 240
   Ala Ile Leu Ser Gln Lys Phe Pro Lys Ile Glu Phe Lys Glu Leu Ile
                   245                 250                 255
   Ser Leu Val Glu Asp Val Ser Ser Asn Tyr Asp Gly Cys Cys Glu Gly
               260                 265                 270
   Asp Val Val Gln Cys Ile Arg Asp Thr Ser Lys Val Met Asn His Ile
           275                 280                 285
   Cys Ser Lys Gln Asp Ser Ile Ser Ser Lys Ile Lys Glu Cys Cys Glu
       290                 295                 300
   Lys Lys Ile Pro Glu Arg Gly Gln Cys Ile Ile Asn Ser Asn Lys Asp
   305                 310                 315                 320
   Asp Arg Pro Lys Asp Leu Ser Leu Arg Glu Gly Lys Phe Thr Asp Ser
                   325                 330                 335
   Glu Asn Val Cys Gln Glu Arg Asp Ala Asp Pro Asp Thr Phe Phe Ala
               340                 345                 350
   Lys Phe Thr Phe Glu Tyr Ser Arg Arg His Pro Asp Leu Ser Ile Pro
           355                 360                 365
   Glu Leu Leu Arg Ile Val Gln Ile Tyr Lys Asp Leu Leu Arg Asn Cys
       370                 375                 380
   Cys Asn Thr Glu Asn Pro Pro Gly Cys Tyr Arg Tyr Ala Glu Asp Lys
   385                 390                 395                 400
   Phe Asn Glu Thr Thr Glu Lys Ser Leu Lys Met Val Gln Gln Glu Cys
                   405                 410                 415
   Lys His Phe Gln Asn Leu Gly Lys Asp Gly Leu Lys Tyr His Tyr Leu
               420                 425                 430
   Ile Arg Leu Thr Lys Ile Ala Pro Gln Leu Ser Thr Glu Glu Leu Val
           435                 440                 445
   Ser Leu Gly Glu Lys Met Val Thr Ala Phe Thr Thr Cys Cys Thr Leu
       450                 455                 460
   Ser Glu Glu Phe Ala Cys Val Asp Asn Leu Ala Asp Leu Val Phe Gly
   465                 470                 475                 480
   Glu Leu Cys Gly Val Asn Glu Asn Arg Thr Ile Asn Pro Ala Val Asp
                   485                 490                 495
   His Cys Cys Lys Thr Asn Phe Ala Phe Arg Arg Pro Cys Phe Glu Ser
               500                 505                 510
   Leu Lys Ala Asp Lys Thr Tyr Val Pro Pro Pro Phe Ser Gln Asp Leu
           515                 520                 525
   Phe Thr Phe His Ala Asp Met Cys Gln Ser Gln Asn Glu Glu Leu Gln
       530                 535                 540
   Arg Lys Thr Asp Arg Phe Leu Val Asn Leu Val Lys Leu Lys His Glu
   545                 550                 555                 560
   Leu Thr Asp Glu Glu Leu Gln Ser Leu Phe Thr Asn Phe Ala Asn Val
                   565                 570                 575
   Val Asp Lys Cys Cys Lys Ala Glu Ser Pro Glu Val Cys Phe Asn Glu
               580                 585                 590
   Glu Ser Pro Lys Ile Gly Asn
           595
<210> 2
   <211> 93
   <212> PRT
   <213> Homo sapiens
<400> 2
   Met Leu Thr Glu Leu Glu Lys Ala Leu Asn Ser Ile Ile Asp Val Tyr
   1               5                   10                  15
   His Lys Tyr Ser Leu Ile Lys Gly Asn Phe His Ala Val Tyr Arg Asp
               20                  25                  30
   Asp Leu Lys Lys Leu Leu Glu Thr Glu Cys Pro Gln Tyr Ile Arg Lys
           35                  40                  45
   Lys Gly Ala Asp Val Trp Phe Lys Glu Leu Asp Ile Asn Thr Asp Gly
       50                  55                  60
   Ala Val Asn Phe Gln Glu Phe Leu Ile Leu Val Ile Lys Met Gly Val
   65                  70                  75                  80
   Ala Ala His Lys Lys Ser His Glu Glu Ser His Lys Glu
                   85                  90
<210> 3
   <211> 103
   <212> PRT
   <213> Homo sapiens
<400> 3
   Met Ala Asp Glu Ile Ala Lys Ala Gln Val Ala Arg Pro Gly Gly Asp
   1               5                   10                  15
   Thr Ile Phe Gly Lys Ile Ile Arg Lys Glu Ile Pro Ala Lys Ile Ile
               20                  25                  30
   Phe Glu Asp Asp Arg Cys Leu Ala Phe His Asp Ile Ser Pro Gln Ala
           35                  40                  45
   Pro Thr His Phe Leu Val Ile Pro Lys Lys His Ile Ser Gln Ile Ser
       50                  55                  60
   Val Ala Glu Asp Asp Asp Glu Ser Val Ile Thr Lys Glu Lys Pro Glu
   65                  70                  75                  80
   Lys Pro Leu Gly Leu Gln Leu Pro Ser Cys Phe Pro Lys Leu Leu His
                   85                  90                  95
   His Phe Val Ser His Gln Gln
               100
<210> 4
   <211> 585
   <212> PRT
   <213> Homo sapiens
<400> 4
   Met Thr Pro Pro Arg Leu Phe Trp Val Trp Leu Leu Val Ala Gly Thr
   1               5                   10                  15
   Gln Gly Val Asn Asp Gly Asp Met Arg Leu Ala Asp Gly Gly Ala Thr
               20                  25                  30
   Asn Gln Gly Arg Val Glu Ile Phe Tyr Arg Gly Gln Trp Gly Thr Val
           35                  40                  45
   Cys Asp Asn Leu Trp Asp Leu Thr Asp Ala Ser Val Val Cys Arg Ala
       50                  55                  60
   Leu Gly Phe Glu Asn Ala Thr Gln Ala Leu Gly Arg Ala Ala Phe Gly
   65                  70                  75                  80
   Gln Gly Ser Gly Pro Ile Met Leu Asp Glu Val Gln Cys Thr Gly Thr
                   85                  90                  95
   Glu Ala Ser Leu Ala Asp Cys Lys Ser Leu Gly Trp Leu Lys Ser Asn
               100                 105                 110
   Cys Arg His Glu Arg Asp Ala Gly Val Val Cys Thr Asn Glu Thr Arg
           115                 120                 125
   Ser Thr His Thr Leu Asp Leu Ser Arg Glu Leu Ser Glu Ala Leu Gly
       130                 135                 140
   Gln Ile Phe Asp Ser Gln Arg Gly Cys Asp Leu Ser Ile Ser Val Asn
   145                 150                 155                 160
   Val Gln Gly Glu Asp Ala Leu Gly Phe Cys Gly His Thr Val Ile Leu
                   165                 170                 175
   Thr Ala Asn Leu Glu Ala Gln Ala Leu Trp Lys Glu Pro Gly Ser Asn
               180                 185                 190
   Val Thr Met Ser Val Asp Ala Glu Cys Val Pro Met Val Arg Asp Leu
           195                 200                 205
   Leu Arg Tyr Phe Tyr Ser Arg Arg Ile Asp Ile Thr Leu Ser Ser Val
       210                 215                 220
   Lys Cys Phe His Lys Leu Ala Ser Ala Tyr Gly Ala Arg Gln Leu Gln
   225                 230                 235                 240
   Gly Tyr Cys Ala Ser Leu Phe Ala Ile Leu Leu Pro Gln Asp Pro Ser
                   245                 250                 255
   Phe Gln Met Pro Leu Asp Leu Tyr Ala Tyr Ala Val Ala Thr Gly Asp
               260                 265                 270
   Ala Leu Leu Glu Lys Leu Cys Leu Gln Phe Leu Ala Trp Asn Phe Glu
           275                 280                 285
   Ala Leu Thr Gln Ala Glu Ala Trp Pro Ser Val Pro Thr Asp Leu Leu
   290 295 300
   Gln Leu Leu Leu Pro Arg Ser Asp Leu Ala Val Pro Ser Glu Leu Ala
   305 310 315 320
   Leu Leu Lys Ala Val Asp Thr Trp Ser Trp Gly Glu Arg Ala Ser His
   325 330 335
   Glu Glu Val Glu Gly Leu Val Glu Lys Ile Arg Phe Pro Met Met Leu
   340 345 350
   Pro Glu Glu Leu Phe Glu Leu Gln Phe Asn Leu Ser Leu Tyr Trp Ser
   355 360 365
   His Glu Ala Leu Phe Gln Lys Lys Thr Leu Gln Ala Leu Glu Phe His
   370 375 380
   Thr Val Pro Phe Gln Leu Leu Ala Arg Tyr Lys Gly Leu Asn Leu Thr
   385 390 395 400
   Glu Asp Thr Tyr Lys Pro Arg Ile Tyr Thr Ser Pro Thr Trp Ser Ala
   405 410 415
   Phe Val Thr Asp Ser Ser Trp Ser Ala Arg Lys Ser Gln Leu Val Tyr
   420 425 430
   Gln Ser Arg Arg Gly Pro Leu Val Lys Tyr Ser Ser Asp Tyr Phe Gln
   435 440 445
   Ala Pro Ser Asp Tyr Arg Tyr Tyr Pro Tyr Gln Ser Phe Gln Thr Pro
   450 455 460
   Gln His Pro Ser Phe Leu Phe Gln Asp Lys Arg Val Ser Trp Ser Leu
   465 470 475 480
   Val Tyr Leu Pro Thr Ile Gln Ser Cys Trp Asn Tyr Gly Phe Ser Cys
   485 490 495
   Ser Ser Asp Glu Leu Pro Val Leu Gly Leu Thr Lys Ser Gly Gly Ser
   500 505 510
   Asp Arg Thr Ile Ala Tyr Glu Asn Lys Ala Leu Met Leu Cys Glu Gly
   515 520 525
   Leu Phe Val Ala Asp Val Thr Asp Phe Glu Gly Trp Lys Ala Ala Ile
   530 535 540
   Pro Ser Ala Leu Asp Thr Asn Ser Ser Lys Ser Thr Ser Ser Phe Pro
   545 550 555 560
   Cys Pro Ala Gly His Phe Asn Gly Phe Arg Thr Val Ile Arg Pro Phe
   565 570 575
   Tyr Leu Thr Asn Ser Ser Gly Val Asp
   580 585
<210> 5
   <211> 353
   <212> PRT
   <213> Homo sapiens
<400> 5
   Ala Ser Pro Thr Ser Pro Lys Val Phe Pro Leu Ser Leu Cys Ser Thr
   1 5 10 15
   Gln Pro Asp Gly Asn Val Val Ile Ala Cys Leu Val Gln Gly Phe Phe
   20 25 30
   Pro Gln Glu Pro Leu Ser Val Thr Trp Ser Glu Ser Gly Gln Gly Val
   35 40 45
   Thr Ala Arg Asn Phe Pro Pro Ser Gln Asp Ala Ser Gly Asp Leu Tyr
   50 55 60
   Thr Thr Ser Ser Gln Leu Thr Leu Pro Ala Thr Gln Cys Leu Ala Gly
   65 70 75 80
   Lys Ser Val Thr Cys His Val Lys His Tyr Thr Asn Pro Ser Gln Asp
   85 90 95
   Val Thr Val Pro Cys Pro Val Pro Ser Thr Pro Pro Thr Pro Ser Pro
   100 105 110
   Ser Thr Pro Pro Thr Pro Ser Pro Ser Cys Cys His Pro Arg Leu Ser
   115 120 125
   Leu His Arg Pro Ala Leu Glu Asp Leu Leu Leu Gly Ser Glu Ala Asn
   130 135 140
   Leu Thr Cys Thr Leu Thr Gly Leu Arg Asp Ala Ser Gly Val Thr Phe
   145 150 155 160
   Thr Trp Thr Pro Ser Ser Gly Lys Ser Ala Val Gln Gly Pro Pro Glu
   165 170 175
   Arg Asp Leu Cys Gly Cys Tyr Ser Val Ser Ser Val Leu Pro Gly Cys
   180 185 190
   Ala Glu Pro Trp Asn His Gly Lys Thr Phe Thr Cys Thr Ala Ala Tyr
   195 200 205
   Pro Glu Ser Lys Thr Pro Leu Thr Ala Thr Leu Ser Lys Ser Gly Asn
   210 215 220
   Thr Phe Arg Pro Glu Val His Leu Leu Pro Pro Pro Ser Glu Glu Leu
   225 230 235 240
   Ala Leu Asn Glu Leu Val Thr Leu Thr Cys Leu Ala Arg Gly Phe Ser
   245 250 255
   Pro Lys Asp Val Leu Val Arg Trp Leu Gln Gly Ser Gln Glu Leu Pro
   260 265 270
   Arg Glu Lys Tyr Leu Thr Trp Ala Ser Arg Gln Glu Pro Ser Gln Gly
   275 280 285
   Thr Thr Thr Phe Ala Val Thr Ser Ile Leu Arg Val Ala Ala Glu Asp
   290 295 300
   Trp Lys Lys Gly Asp Thr Phe Ser Cys Met Val Gly His Glu Ala Leu
   305 310 315 320
   Pro Leu Ala Phe Thr Gln Lys Thr Ile Asp Arg Leu Ala Gly Lys Pro
   325 330 335
   Thr His Val Asn Val Ser Val Val Met Ala Glu Val Asp Gly Thr Cys
   340 345 350
   Tyr
<210> 6
   <211> 334
   <212> PRT
   <213> Homo sapiens
<400> 6
   Met Ala Thr Leu Lys Glu Lys Leu Ile Ala Pro Val Ala Glu Glu Glu
   1 5 10 15
   Ala Thr Val Pro Asn Asn Lys Ile Thr Val Val Gly Val Gly Gln Val
   20 25 30
   Gly Met Ala Cys Ala Ile Ser Ile Leu Gly Lys Ser Leu Ala Asp Glu
           35                  40                  45
   Leu Ala Leu Val Asp Val Leu Glu Asp Lys Leu Lys Gly Glu Met Met
       50                  55                  60
   Asp Leu Gln His Gly Ser Leu Phe Leu Gln Thr Pro Lys Ile Val Ala
   65                  70                  75                  80
   Asp Lys Asp Tyr Ser Val Thr Ala Asn Ser Lys Ile Val Val Val Thr
                   85                  90                  95
   Ala Gly Val Arg Gln Gln Glu Gly Glu Ser Arg Leu Asn Leu Val Gln
               100                 105                 110
   Arg Asn Val Asn Val Phe Lys Phe Ile Ile Pro Gln Ile Val Lys Tyr
           115                 120                 125
   Ser Pro Asp Cys Ile Ile Ile Val Val Ser Asn Pro Val Asp Ile Leu
       130                 135                 140
   Thr Tyr Val Thr Trp Lys Leu Ser Gly Leu Pro Lys His Arg Val Ile
   145                 150                 155                 160
   Gly Ser Gly Cys Asn Leu Asp Ser Ala Arg Phe Arg Tyr Leu Met Ala
                   165                 170                 175
   Glu Lys Leu Gly Ile His Pro Ser Ser Cys His Gly Trp Ile Leu Gly
               180                 185                 190
   Glu His Gly Asp Ser Ser Val Ala Val Trp Ser Gly Val Asn Val Ala
           195                 200                 205
   Gly Val Ser Leu Gln Glu Leu Asn Pro Glu Met Gly Thr Asp Asn Asp
       210                 215                 220
   Ser Glu Asn Trp Lys Glu Val His Lys Met Val Val Glu Ser Ala Tyr
   225                 230                 235                 240
   Glu Val Ile Lys Leu Lys Gly Tyr Thr Asn Trp Ala Ile Gly Leu Ser
                   245                 250                 255
   Val Ala Asp Leu Ile Glu Ser Met Leu Lys Asn Leu Ser Arg Ile His
               260                 265                 270
   Pro Val Ser Thr Met Val Lys Gly Met Tyr Gly Ile Glu Asn Glu Val
   275 280 285
   Phe Leu Ser Leu Pro Cys Ile Leu Asn Ala Arg Gly Leu Thr Ser Val
   290 295 300
   Ile Asn Gln Lys Leu Lys Asp Asp Glu Val Ala Gln Leu Lys Lys Ser
   305 310 315 320
   Ala Asp Thr Leu Trp Asp Ile Gln Lys Asp Leu Lys Asp Leu
   325 330
<210> 7
   <211> 332
   <212> PRT
   <213> Homo sapiens
<400> 7
<210> 8
   <211> 119
   <212> PRT
   <213> Homo sapiens
<400> 8
   Met Lys Val Leu Ile Ser Ser Leu Leu Leu Leu Leu Pro Leu Met Leu
   1               5                   10                  15
   Met Ser Met Val Ser Ser Ser Leu Asn Pro Gly Val Ala Arg Gly His
               20                  25                  30
   Arg Asp Arg Gly Gln Ala Ser Arg Arg Trp Leu Gln Glu Gly Gly Gln
           35                  40                  45
   Glu Cys Glu Cys Lys Asp Trp Phe Leu Arg Ala Pro Arg Arg Lys Phe
       50                  55                  60
   Met Thr Val Ser Gly Leu Pro Lys Lys Gln Cys Pro Cys Asp His Phe
   65                  70                  75                  80
   Lys Gly Asn Val Lys Lys Thr Arg His Gln Arg His His Arg Lys Pro
                   85                  90                  95
   Asn Lys His Ser Arg Ala Cys Gln Gln Phe Leu Lys Gln Cys Gln Leu
               100                 105                 110
   Arg Ser Phe Ala Leu Pro Leu
           115
<210> 9
   <211> 501
   <212> PRT
   <213> Homo sapiens
<400> 9
   Met Ser Ser Ser Gly Thr Pro Asp Leu Pro Val Leu Leu Thr Asp Leu
   1               5                   10                  15
   Lys Ile Gln Tyr Thr Lys Ile Phe Ile Asn Asn Glu Trp His Asp Ser
               20                  25                  30
   Val Ser Gly Lys Lys Phe Pro Val Phe Asn Pro Ala Thr Glu Glu Glu
           35                  40                  45
   Leu Cys Gln Val Glu Glu Gly Asp Lys Glu Asp Val Asp Lys Ala Val
       50                  55                  60
   Lys Ala Ala Arg Gln Ala Phe Gln Ile Gly Ser Pro Trp Arg Thr Met
   65                  70                  75                  80
   Asp Ala Ser Glu Arg Gly Arg Leu Leu Tyr Lys Leu Ala Asp Leu Ile
                   85                  90                  95
   Glu Arg Asp Arg Leu Leu Leu Ala Thr Met Glu Ser Met Asn Gly Gly
               100                 105                 110
   Lys Leu Tyr Ser Asn Ala Tyr Leu Asn Asp Leu Ala Gly Cys Ile Lys
           115                 120                 125
   Thr Leu Arg Tyr Cys Ala Gly Trp Ala Asp Lys Ile Gln Gly Arg Thr
       130                 135                 140
   Ile Pro Ile Asp Gly Asn Phe Phe Thr Tyr Thr Arg His Glu Pro Ile
   145 150 155 160
   Gly Val Cys Gly Gln Ile Ile Pro Trp Asn Phe Pro Leu Val Met Leu
   165 170 175
   Ile Trp Lys Ile Gly Pro Ala Leu Ser Cys Gly Asn Thr Val Val Val
   180 185 190
   Lys Pro Ala Glu Gln Thr Pro Leu Thr Ala Leu His Val Ala Ser Leu
   195 200 205
   Ile Lys Glu Ala Gly Phe Pro Pro Gly Val Val Asn Ile Val Pro Gly
   210 215 220
   Tyr Gly Pro Thr Ala Gly Ala Ala Ile Ser Ser His Met Asp Ile Asp
   225 230 235 240
   Lys Val Ala Phe Thr Gly Ser Thr Glu Val Gly Lys Leu Ile Lys Glu
   245 250 255
   Ala Ala Gly Lys Ser Asn Leu Lys Arg Val Thr Leu Glu Leu Gly Gly
   260 265 270
   Lys Ser Pro Cys Ile Val Leu Ala Asp Ala Asp Leu Asp Asn Ala Val
   275 280 285
   Glu Phe Ala His His Gly Val Phe Tyr His Gln Gly Gln Cys Cys Ile
   290 295 300
   Ala Ala Ser Arg Ile Phe Val Glu Glu Ser Ile Tyr Asp Glu Phe Val
   305 310 315 320
   Arg Arg Ser Val Glu Arg Ala Lys Lys Tyr Ile Leu Gly Asn Pro Leu
   325 330 335
   Thr Pro Gly Val Thr Gln Gly Pro Gln Ile Asp Lys Glu Gln Tyr Asp
   340 345 350
   Lys Ile Leu Asp Leu Ile Glu Ser Gly Lys Lys Glu Gly Ala Lys Leu
   355 360 365
   Glu Cys Gly Gly Gly Pro Trp Gly Asn Lys Gly Tyr Phe Val Gln Pro
   370 375 380
   Thr Val Phe Ser Asn Val Thr Asp Glu Met Arg Ile Ala Lys Glu Glu
   385 390 395 400
   Ile Phe Gly Pro Val Gln Gln Ile Met Lys Phe Lys Ser Leu Asp Asp
   405 410 415
   Val Ile Lys Arg Ala Asn Asn Thr Phe Tyr Gly Leu Ser Ala Gly Val
   420 425 430
   Phe Thr Lys Asp Ile Asp Lys Ala Ile Thr Ile Ser Ser Ala Leu Gln
   435 440 445
   Ala Gly Thr Val Trp Val Asn Cys Tyr Gly Val Val Ser Ala Gln Cys
   450 455 460
   Pro Phe Gly Gly Phe Lys Met Ser Gly Asn Gly Arg Glu Leu Gly Glu
   465 470 475 480
   Tyr Gly Phe His Glu Tyr Thr Glu Val Lys Thr Val Thr Val Lys Ile
   485 490 495
   Ser Gln Lys Asn Ser
   500
<210> 10
   <211> 1266
   <212> PRT
   <213> Homo sapiens
<400> 10
   Met Gly Pro Leu Met Val Leu Phe Cys Leu Leu Phe Leu Tyr Pro Gly
   1 5 10 15
   Leu Ala Asp Ser Ala Pro Ser Cys Pro Gln Asn Val Asn Ile Ser Gly
   20 25 30
   Gly Thr Phe Thr Leu Ser His Gly Trp Ala Pro Gly Ser Leu Leu Thr
   35 40 45
   Tyr Ser Cys Pro Gln Gly Leu Tyr Pro Ser Pro Ala Ser Arg Leu Cys
   50 55 60
   Lys Ser Ser Gly Gln Trp Gln Thr Pro Gly Ala Thr Arg Ser Leu Ser
   65 70 75 80
   Lys Ala Val Cys Lys Pro Gly His Cys Pro Asn Pro Gly Ile Ser Leu
   85 90 95
   Gly Ala Val Arg Thr Gly Phe Arg Phe Gly His Gly Asp Lys Val Arg
   100 105 110
   Tyr Arg Cys Ser Ser Asn Leu Val Leu Thr Gly Ser Ser Glu Arg Glu
           115                 120                 125
   Cys Gln Gly Asn Gly Val Trp Ser Gly Thr Glu Pro Ile Cys Arg Gln
       130                 135                 140
   Pro Tyr Ser Tyr Asp Phe Pro Glu Asp Val Ala Pro Ala Leu Gly Thr
   145                 150                 155                 160
   Ser Phe Ser His Met Leu Gly Ala Thr Asn Pro Thr Gln Lys Thr Lys
                   165                 170                 175
   Asp His Glu Asn Gly Thr Gly Thr Asn Thr Tyr Ala Ala Leu Asn Ser
               180                 185                 190
   Val Tyr Leu Met Met Asn Asn Gln Met Arg Leu Leu Gly Met Glu Thr
           195                 200                 205
   Met Ala Trp Gln Glu Ile Arg His Ala Ile Ile Leu Leu Thr Asp Gly
       210                 215                 220
   Lys Ser Asn Met Gly Gly Ser Pro Lys Thr Ala Val Asp His Ile Arg
   225                 230                 235                 240
   Glu Ile Leu Asn Ile Asn Gln Lys Arg Asn Asp Tyr Leu Asp Ile Tyr
                   245                 250                 255
   Ala Ile Gly Val Gly Lys Leu Asp Val Asp Trp Arg Glu Leu Asn Glu
               260                 265                 270
   Leu Gly Ser Lys Lys Asp Gly Glu Arg His Ala Phe Ile Leu Gln Asp
           275                 280                 285
   Thr Lys Ala Leu His Gln Val Phe Glu His Met Leu Asp Val Ser Lys
       290                 295                 300
   Leu Thr Asp Thr Ile Cys Gly Val Gly Asn Met Ser Ala Asn Ala Ser
   305                 310                 315                 320
   Asp Gln Glu Arg Thr Pro Trp His Val Thr Ile Lys Pro Lys Ser Gln
                   325                 330                 335
   Glu Thr Cys Arg Gly Ala Leu Ile Ser Asp Gln Trp Val Leu Thr Ala
               340                 345                 350
   Ala His Cys Phe Arg Asp Gly Asn Asp His Ser Leu Trp Arg Val Asn
           355                 360                 365
   Val Gly Asp Pro Lys Ser Gln Trp Gly Lys Glu Phe Leu Ile Glu Lys
       370                 375                 380
   Ala Val Ile Ser Pro Gly Phe Asp Val Phe Ala Lys Lys Asn Gln Gly
   385                 390                 395                 400
   Ile Leu Glu Phe Tyr Gly Asp Asp Ile Ala Leu Leu Lys Leu Ala Gln
                   405                 410                 415
   Lys Val Lys Met Ser Thr His Ala Arg Pro Ile Cys Leu Pro Cys Thr
               420                 425                 430
   Met Glu Ala Asn Leu Ala Leu Arg Arg Pro Gln Gly Ser Thr Cys Arg
           435                 440                 445
   Asp His Glu Asn Glu Leu Leu Asn Lys Gln Ser Val Pro Ala His Phe
       450                 455                 460
   Val Ala Leu Asn Gly Ser Lys Leu Asn Ile Asn Leu Lys Met Gly Val
   465                 470                 475                 480
   Glu Trp Thr Ser Cys Ala Glu Val Val Ser Gln Glu Lys Thr Met Phe
                   485                 490                 495
   Pro Asn Leu Thr Asp Val Arg Glu Val Val Thr Asp Gln Phe Leu Cys
               500                 505                 510
   Ser Gly Thr Gln Glu Asp Glu Ser Pro Cys Lys Gly Val Thr Thr Thr
           515                 520                 525
   Pro Trp Ser Leu Ala Arg Pro Gln Gly Ser Cys Ser Leu Glu Gly Val
       530                 535                 540
   Glu Ile Lys Gly Gly Ser Phe Arg Leu Leu Gln Glu Gly Gln Ala Leu
   545                 550                 555                 560
   Glu Tyr Val Cys Pro Ser Gly Phe Tyr Pro Tyr Pro Val Gln Thr Arg
                   565                 570                 575
   Thr Cys Arg Ser Thr Gly Ser Trp Ser Thr Leu Lys Thr Gln Asp Gln
               580                 585                 590
   Lys Thr Val Arg Lys Ala Glu Cys Arg Ala Ile His Cys Pro Arg Pro
           595                 600                 605
   His Asp Phe Glu Asn Gly Glu Tyr Trp Pro Arg Ser Pro Tyr Tyr Asn
       610                 615                 620
   Val Ser Asp Glu Ile Ser Phe His Cys Tyr Asp Gly Tyr Thr Leu Arg
   625 630 635 640
   Gly Ser Ala Asn Arg Thr Cys Gln Val Asn Gly Arg Trp Ser Gly Gln
   645 650 655
   Thr Ala Ile Cys Asp Asn Gly Ala Gly Tyr Cys Ser Asn Pro Gly Ile
   660 665 670
   Pro Ile Gly Thr Arg Lys Val Gly Ser Gln Tyr Arg Leu Glu Asp Ser
   675 680 685
   Val Thr Tyr His Cys Ser Arg Gly Leu Thr Leu Arg Gly Ser Gln Arg
   690 695 700
   Arg Thr Cys Gln Glu Gly Gly Ser Trp Ser Gly Thr Glu Pro Ser Cys
   705 710 715 720
   Gln Asp Ser Phe Met Tyr Asp Thr Pro Gln Glu Val Ala Glu Ala Phe
   725 730 735
   Leu Ser Ser Leu Thr Glu Thr Ile Glu Gly Val Asp Ala Glu Asp Gly
   740 745 750
   His Gly Pro Gly Glu Gln Gln Lys Arg Lys Ile Val Leu Asp Pro Ser
   755 760 765
   Gly Ser Met Asn Ile Tyr Leu Val Leu Asp Gly Ser Asp Ser Ile Gly
   770 775 780
   Ala Ser Asn Phe Thr Gly Ala Lys Lys Cys Leu Val Asn Leu Ile Glu
   785 790 795 800
   Lys Val Ala Ser Tyr Gly Val Lys Pro Arg Tyr Gly Leu Val Thr Tyr
   805 810 815
   Ala Thr Tyr Pro Lys Ile Trp Val Lys Val Ser Glu Ala Asp Ser Ser
   820 825 830
   Asn Ala Asp Trp Val Thr Lys Gln Leu Asn Glu Ile Asn Tyr Glu Asp
   835 840 845
   His Lys Leu Lys Ser Gly Thr Asn Thr Lys Lys Ala Leu Gln Ala Val
   850 855 860
   Tyr Ser Met Met Ser Trp Pro Asp Asp Val Pro Pro Glu Gly Trp Asn
   865 870 875 880
   Arg Thr Arg His Val Ile Ile Leu Met Thr Asp Gly Leu His Asn Met
   885 890 895
   Gly Gly Asp Pro Ile Thr Val Ile Asp Glu Ile Arg Asp Leu Leu Tyr
   900 905 910
   Ile Gly Lys Asp Arg Lys Asn Pro Arg Glu Asp Tyr Leu Asp Val Tyr
   915 920 925
   Val Phe Gly Val Gly Pro Leu Val Asn Gln Val Asn Ile Asn Ala Leu
   930 935 940
   Ala Ser Lys Lys Asp Asn Glu Gln His Val Phe Lys Val Lys Asp Met
   945 950 955 960
   Glu Asn Leu Glu Asp Val Phe Tyr Gln Met Ile Asp Glu Ser Gln Ser
   965 970 975
   Leu Ser Leu Cys Gly Met Val Trp Glu His Arg Lys Gly Thr Asp Tyr
   980 985 990
   His Lys Gln Pro Trp Gln Ala Lys Ile Ser Val Ile Arg Pro Ser Lys
   995 1000 1005
   Gly His Glu Ser Cys Met Gly Ala Val Val Ser Glu Tyr Phe Val
   1010 1015 1020
   Leu Thr Ala Ala His Cys Phe Thr Val Asp Asp Lys Glu His Ser
   1025 1030 1035
   Ile Lys Val Ser Val Gly Gly Glu Lys Arg Asp Leu Glu Ile Glu
   1040 1045 1050
   Val Val Leu Phe His Pro Asn Tyr Asn Ile Asn Gly Lys Lys Glu
   1055 1060 1065
   Ala Gly Ile Pro Glu Phe Tyr Asp Tyr Asp Val Ala Leu Ile Lys
   1070 1075 1080
   Leu Lys Asn Lys Leu Lys Tyr Gly Gln Thr Ile Arg Pro Ile Cys
   1085 1090 1095
   Leu Pro Cys Thr Glu Gly Thr Thr Arg Ala Leu Arg Leu Pro Pro
   1100 1105 1110
   Thr Thr Thr Cys Gln Gln Gln Lys Glu Glu Leu Leu Pro Ala Gln
   1115 1120 1125
   Asp Ile Lys Ala Leu Phe Val Ser Glu Glu Glu Lys Lys Leu Thr
   1130 1135 1140
   Arg Lys Glu Val Tyr Ile Lys Asn Gly Asp Lys Lys Gly Ser Cys
   1145 1150 1155
   Glu Arg Asp Ala Gln Tyr Ala Pro Gly Tyr Asp Lys Val Lys Asp
   1160 1165 1170
   Ile Ser Glu Val Val Thr Pro Arg Phe Leu Cys Thr Gly Gly Val
   1175 1180 1185
   Ser Pro Tyr Ala Asp Pro Asn Thr Cys Arg Gly Asp Ser Gly Gly
   1190 1195 1200
   Pro Leu Ile Val His Lys Arg Ser Arg Phe Ile Gln Val Gly Val
   1205 1210 1215
   Ile Ser Trp Gly Val Val Asp Val Cys Lys Asn Gln Lys Arg Gln
   1220 1225 1230
   Lys Gln Val Pro Ala His Ala Arg Asp Phe His Ile Asn Leu Phe
   1235 1240 1245
   Gln Val Leu Pro Trp Leu Lys Glu Lys Leu Gln Asp Glu Asp Leu
   1250 1255 1260
   Gly Phe Leu
   1265
<210> 11
   <211> 423
   <212> PRT
   <213> Homo sapiens
<400> 11
   Met Glu Arg Met Leu Pro Leu Leu Ala Leu Gly Leu Leu Ala Ala Gly
   1 5 10 15
   Phe Cys Pro Ala Val Leu Cys His Pro Asn Ser Pro Leu Asp Glu Glu
   20 25 30
   Asn Leu Thr Gln Glu Asn Gln Asp Arg Gly Thr His Val Asp Leu Gly
   35 40 45
   Leu Ala Ser Ala Asn Val Asp Phe Ala Phe Ser Leu Tyr Lys Gln Leu
       50                  55                  60
   Val Leu Lys Ala Pro Asp Lys Asn Val Ile Phe Ser Pro Leu Ser Ile
   65                  70                  75                  80
   Ser Thr Ala Leu Ala Phe Leu Ser Leu Gly Ala His Asn Thr Thr Leu
                   85                  90                  95
   Thr Glu Ile Leu Lys Gly Leu Lys Phe Asn Leu Thr Glu Thr Ser Glu
               100                 105                 110
   Ala Glu Ile His Gln Ser Phe Gln His Leu Leu Arg Thr Leu Asn Gln
           115                 120                 125
   Ser Ser Asp Glu Leu Gln Leu Ser Met Gly Asn Ala Met Phe Val Lys
       130                 135                 140
   Glu Gln Leu Ser Leu Leu Asp Arg Phe Thr Glu Asp Ala Lys Arg Leu
   145                 150                 155                 160
   Tyr Gly Ser Glu Ala Phe Ala Thr Asp Phe Gln Asp Ser Ala Ala Ala
                   165                 170                 175
   Lys Lys Leu Ile Asn Asp Tyr Val Lys Asn Gly Thr Arg Gly Lys Ile
               180                 185                 190
   Thr Asp Leu Ile Lys Asp Leu Asp Ser Gln Thr Met Met Val Leu Val
           195                 200                 205
   Asn Tyr Ile Phe Phe Lys Ala Lys Trp Glu Met Pro Phe Asp Pro Gln
       210                 215                 220
   Asp Thr His Gln Ser Arg Phe Tyr Leu Ser Lys Lys Lys Trp Val Met
   225                 230                 235                 240
   Val Pro Met Met Ser Leu His His Leu Thr Ile Pro Tyr Phe Arg Asp
                   245                 250                 255
   Glu Glu Leu Ser Cys Thr Val Val Glu Leu Lys Tyr Thr Gly Asn Ala
               260                 265                 270
   Ser Ala Leu Phe Ile Leu Pro Asp Gln Asp Lys Met Glu Glu Val Glu
           275                 280                 285
   Ala Met Leu Leu Pro Glu Thr Leu Lys Arg Trp Arg Asp Ser Leu Glu
       290                 295                 300
   Phe Arg Glu Ile Gly Glu Leu Tyr Leu Pro Lys Phe Ser Ile Ser Arg
   305                 310                 315                 320
   Asp Tyr Asn Leu Asn Asp Ile Leu Leu Gln Leu Gly Ile Glu Glu Ala
                   325                 330                 335
   Phe Thr Ser Lys Ala Asp Leu Ser Gly Ile Thr Gly Ala Arg Asn Leu
               340                 345                 350
   Ala Val Ser Gln Val Val His Lys Ala Val Leu Asp Val Phe Glu Glu
           355                 360                 365
   Gly Thr Glu Ala Ser Ala Ala Thr Ala Val Lys Ile Thr Leu Leu Ser
       370                 375                 380
   Ala Leu Val Glu Thr Arg Thr Ile Val Arg Phe Asn Arg Pro Phe Leu
   385                 390                 395                 400
   Met Ile Ile Val Pro Thr Asp Thr Gln Asn Ile Phe Phe Met Ser Lys
                   405                 410                 415
   Val Thr Asn Pro Lys Gln Ala
               420
<210> 12
   <211> 664
   <212> PRT
   <213> Homo sapiens
<400> 12
   Met Lys Thr Gly Leu Phe Phe Leu Cys Leu Leu Gly Thr Ala Ala Ala
   1               5                   10                  15
   Ile Pro Thr Asn Ala Arg Leu Leu Ser Asp His Ser Lys Pro Thr Ala
               20                  25                  30
   Glu Thr Val Ala Pro Asp Asn Thr Ala Ile Pro Ser Leu Arg Ala Glu
           35                  40                  45
   Ala Glu Glu Asn Glu Lys Glu Thr Ala Val Ser Thr Glu Asp Asp Ser
       50                  55                  60
   His His Lys Ala Glu Lys Ser Ser Val Leu Lys Ser Lys Glu Glu Ser
   65                  70                  75                  80
   His Glu Gln Ser Ala Glu Gln Gly Lys Ser Ser Ser Gln Glu Leu Gly
                   85                  90                  95
   Leu Lys Asp Gln Glu Asp Ser Asp Gly His Leu Ser Val Asn Leu Glu
               100                 105                 110
   Tyr Ala Pro Thr Glu Gly Thr Leu Asp Ile Lys Glu Asp Met Ser Glu
           115                 120                 125
   Pro Gln Glu Lys Lys Leu Ser Glu Asn Thr Asp Phe Leu Ala Pro Gly
       130                 135                 140
   Val Ser Ser Phe Thr Asp Ser Asn Gln Gln Glu Ser Ile Thr Lys Arg
   145                 150                 155                 160
   Glu Glu Asn Gln Glu Gln Pro Arg Asn Tyr Ser His His Gln Leu Asn
                   165                 170                 175
   Arg Ser Ser Lys His Ser Gln Gly Leu Arg Asp Gln Gly Asn Gln Glu
               180                 185                 190
   Gln Asp Pro Asn Ile Ser Asn Gly Glu Glu Glu Glu Glu Lys Glu Pro
           195                 200                 205
   Gly Glu Val Gly Thr His Asn Asp Asn Gln Glu Arg Lys Thr Glu Leu
       210                 215                 220
   Pro Arg Glu His Ala Asn Ser Lys Gln Glu Glu Asp Asn Thr Gln Ser
   225                 230                 235                 240
   Asp Asp Ile Leu Glu Glu Ser Asp Gln Pro Thr Gln Val Ser Lys Met
                   245                 250                 255
   Gln Glu Asp Glu Phe Asp Gln Gly Asn Gln Glu Gln Glu Asp Asn Ser
               260                 265                 270
   Asn Ala Glu Met Glu Glu Glu Asn Ala Ser Asn Val Asn Lys His Ile
           275                 280                 285
   Gln Glu Thr Glu Trp Gln Ser Gln Glu Gly Lys Thr Gly Leu Glu Ala
       290                 295                 300
   Ile Ser Asn His Lys Glu Thr Glu Glu Lys Thr Val Ser Glu Ala Leu
   305                 310                 315                 320
   Leu Met Glu Pro Thr Asp Asp Gly Asn Thr Thr Pro Arg Asn His Gly
                   325                 330                 335
   Val Asp Asp Asp Gly Asp Asp Asp Gly Asp Asp Gly Gly Thr Asp Gly
               340                 345                 350
   Pro Arg His Ser Ala Ser Asp Asp Tyr Phe Ile Pro Ser Gln Ala Phe
   355 360 365
   Leu Glu Ala Glu Arg Ala Gln Ser Ile Ala Tyr His Leu Lys Ile Glu
   370 375 380
   Glu Gln Arg Glu Lys Val His Glu Asn Glu Asn Ile Gly Thr Thr Glu
   385 390 395 400
   Pro Gly Glu His Gln Glu Ala Lys Lys Ala Glu Asn Ser Ser Asn Glu
   405 410 415
   Glu Glu Thr Ser Ser Glu Gly Asn Met Arg Val His Ala Val Asp Ser
   420 425 430
   Cys Met Ser Phe Gln Cys Lys Arg Gly His Ile Cys Lys Ala Asp Gln
   435 440 445
   Gln Gly Lys Pro His Cys Val Cys Gln Asp Pro Val Thr Cys Pro Pro
   450 455 460
   Thr Lys Pro Leu Asp Gln Val Cys Gly Thr Asp Asn Gln Thr Tyr Ala
   465 470 475 480
   Ser Ser Cys His Leu Phe Ala Thr Lys Cys Arg Leu Glu Gly Thr Lys
   485 490 495
   Lys Gly His Gln Leu Gln Leu Asp Tyr Phe Gly Ala Cys Lys Ser Ile
   500 505 510
   Pro Thr Cys Thr Asp Phe Glu Val Ile Gln Phe Pro Leu Arg Met Arg
   515 520 525
   Asp Trp Leu Lys Asn Ile Leu Met Gln Leu Tyr Glu Ala Asn Ser Glu
   530 535 540
   His Ala Gly Tyr Leu Asn Glu Lys Gln Arg Asn Lys Val Lys Lys Ile
   545 550 555 560
   Tyr Leu Asp Glu Lys Arg Leu Leu Ala Gly Asp His Pro Ile Asp Leu
   565 570 575
   Leu Leu Arg Asp Phe Lys Lys Asn Tyr His Met Tyr Val Tyr Pro Val
   580 585 590
   His Trp Gln Phe Ser Glu Leu Asp Gln His Pro Met Asp Arg Val Leu
   595 600 605
   Thr His Ser Glu Leu Ala Pro Leu Arg Ala Ser Leu Val Pro Met Glu
   610 615 620
   His Cys Ile Thr Arg Phe Phe Glu Glu Cys Asp Pro Asn Lys Asp Lys
   625 630 635 640
   His Ile Thr Leu Lys Glu Trp Gly His Cys Phe Gly Ile Lys Glu Glu
   645 650 655
   Asp Ile Asp Glu Asn Leu Leu Phe
   660
<210> 13
   <211> 115
   <212> PRT
   <213> Homo sapiens
<400> 13
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Val Met Ser Trp Val Arg Gln Ala Pro Gly Lys Gly Leu Glx Trp Val
   35 40 45
   Gly Ala Ile Glx Gly Leu Ser Val Ser Glx Ser Tyr Ala Asx Ser Val
   50 55 60
   Lys Gly Arg Phe Thr Ile Ser Arg Asp Asp Ser Lys Asn Thr Met Asn
   65 70 75 80
   Ser Leu Arg Ala Glu Asp Thr Ala Val Tyr Tyr Cys Ala Lys Gly Lys
   85 90 95
   Val Ser Ala Tyr Tyr Phe Asx Tyr Trp Gly Glx Gly Thr Leu Val Thr
   100 105 110
   Val Ser Ser
   115
<210> 14
   <211> 623
   <212> PRT
   <213> Homo sapiens
<400> 14
<210> 15
   <211> 141
   <212> PRT
   <213> Homo sapiens
<400> 15
   Met Ala Gln Tyr Leu Ser Thr Leu Leu Leu Leu Leu Ala Thr Leu Ala
   1 5 10 15
   Val Ala Leu Ala Trp Ser Pro Lys Glu Glu Asp Arg Ile Ile Pro Gly
   20 25 30
   Gly Ile Tyr Asn Ala Asp Leu Asn Asp Glu Trp Val Gln Arg Ala Leu
   35 40 45
   His Phe Ala Ile Ser Glu Tyr Asn Lys Ala Thr Lys Asp Asp Tyr Tyr
   50 55 60
   Arg Arg Pro Leu Arg Val Leu Arg Ala Arg Gln Gln Thr Val Gly Gly
   65 70 75 80
   Val Asn Tyr Phe Phe Asp Val Glu Val Gly Arg Thr Ile Cys Thr Lys
   85 90 95
   Ser Gln Pro Asn Leu Asp Thr Cys Ala Phe His Glu Gln Pro Glu Leu
               100                 105                 110
   Gln Lys Lys Gln Leu Cys Ser Phe Glu Ile Tyr Glu Val Pro Trp Glu
           115                 120                 125
   Asn Arg Arg Ser Leu Val Lys Ser Arg Cys Gln Glu Ser
       130                 135                 140
<210> 16
   <211> 911
   <212> PRT
   <213> Homo sapiens
<400> 16
<210> 17
   <211> 235
   <212> PRT
   <213> Homo sapiens
<400> 17
   Met Ala Trp Ile Pro Leu Phe Leu Gly Val Leu Ala Tyr Cys Thr Gly
   1 5 10 15
   Ser Val Ala Ser Tyr Glu Leu Thr Gln Pro Pro Ser Val Ser Val Ser
   20 25 30
   Pro Gly Gln Thr Ala Ser Ile Thr Cys Ser Gly Asp Lys Leu Gly Asp
   35 40 45
   Lys Tyr Ala Cys Trp Tyr Gln Gln Lys Pro Gly Gln Ser Pro Val Leu
   50 55 60
   Val Ile Tyr Gln Asp Ser Lys Arg Pro Ser Gly Ile Pro Glu Arg Phe
   65 70 75 80
   Ser Gly Ser Asn Ser Gly Asn Thr Ala Thr Leu Thr Ile Ser Gly Thr
   85 90 95
   Gln Ala Met Asp Glu Ala Asp Tyr Tyr Cys Gln Ala Trp Asp Ser Ser
   100 105 110
   Ala Ser Gly Pro Val Leu Val Phe Gly Thr Gly Thr Lys Val Thr Val
   115 120 125
   Leu Gly Gln Pro Lys Ala Asn Pro Thr Val Thr Leu Phe Pro Pro Ser
   130 135 140
   Ser Glu Glu Leu Gln Ala Asn Lys Ala Thr Leu Val Cys Leu Ile Ser
   145 150 155 160
   Asp Phe Tyr Pro Gly Ala Val Thr Val Ala Trp Lys Ala Asp Gly Ser
   165 170 175
   Pro Val Lys Ala Gly Val Glu Thr Thr Lys Pro Ser Lys Gln Ser Asn
   180 185 190
   Asn Lys Tyr Ala Ala Ser Ser Tyr Leu Ser Leu Thr Pro Glu Gln Trp
   195 200 205
   Lys Ser His Arg Ser Tyr Ser Cys Gln Val Thr His Glu Gly Ser Thr
   210 215 220
   Val Glu Lys Thr Val Ala Pro Thr Glu Cys Ser
   225 230 235
<210> 18
   <211> 106
   <212> PRT
   <213> Homo sapiens
<400> 18
   Gly Gln Pro Lys Ala Ala Pro Ser Val Thr Leu Phe Pro Pro Ser Ser
   1               5                   10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Ala Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Lys Ser Tyr Ser Cys Gln Val Thr His Glu Gly Ser Thr Val
                   85                  90                  95
   Glu Lys Thr Val Ala Pro Thr Glu Cys Ser
               100                 105
<210> 19
   <211> 138
   <212> PRT
   <213> Homo sapiens
<400> 19
   Met Lys Phe Thr Thr Leu Leu Phe Leu Ala Ala Val Ala Gly Ala Leu
   1               5                   10                  15
   Val Tyr Ala Glu Asp Ala Ser Ser Asp Ser Thr Gly Ala Asp Pro Ala
               20                  25                  30
   Gln Glu Ala Gly Thr Ser Lys Pro Asn Glu Glu Ile Ser Gly Pro Ala
           35                  40                  45
   Glu Pro Ala Ser Pro Pro Glu Thr Thr Thr Thr Ala Gln Glu Thr Ser
       50                  55                  60
   Ala Ala Ala Val Gln Gly Thr Ala Lys Val Thr Ser Ser Arg Gln Glu
   65                  70                  75                  80
   Leu Asn Pro Leu Lys Ser Ile Val Glu Lys Ser Ile Leu Leu Thr Glu
                   85                  90                  95
   Gln Ala Leu Ala Lys Ala Gly Lys Gly Met His Gly Gly Val Pro Gly
               100                 105                 110
   Gly Lys Gln Phe Ile Glu Asn Gly Ser Glu Phe Ala Gln Lys Leu Leu
           115                 120                 125
   Lys Lys Phe Ser Leu Leu Lys Pro Trp Ala
       130                 135
<210> 20
   <211> 206
   <212> PRT
   <213> Homo sapiens
<400> 20
   Met Ala Val Lys Lys Ile Ala Ile Phe Gly Ala Thr Gly Gln Thr Gly
   1               5                   10                  15
   Leu Thr Thr Leu Ala Gln Ala Val Gln Ala Gly Tyr Glu Val Thr Val
               20                  25                  30
   Leu Val Arg Asp Ser Ser Arg Leu Pro Ser Glu Gly Pro Arg Pro Ala
           35                  40                  45
   His Val Val Val Gly Asp Val Leu Gln Ala Ala Asp Val Asp Lys Thr
       50                  55                  60
   Val Ala Gly Gln Asp Ala Val Ile Val Leu Leu Gly Thr Arg Asn Asp
   65                  70                  75                  80
   Leu Ser Pro Thr Thr Val Met Ser Glu Gly Ala Arg Asn Ile Val Ala
                   85                  90                  95
   Ala Met Lys Ala His Gly Val Asp Lys Val Val Ala Cys Thr Ser Ala
               100                 105                 110
   Phe Leu Leu Trp Asp Pro Thr Lys Val Pro Pro Arg Leu Gln Ala Val
           115                 120                 125
   Thr Asp Asp His Ile Arg Met His Lys Val Leu Arg Glu Ser Gly Leu
       130                 135                 140
   Lys Tyr Val Ala Val Met Pro Pro His Ile Gly Asp Gln Pro Leu Thr
   145                 150                 155                 160
   Gly Ala Tyr Thr Val Thr Leu Asp Gly Arg Gly Pro Ser Arg Val Ile
                   165                 170                 175
   Ser Lys His Asp Leu Gly His Phe Met Leu Arg Cys Leu Thr Thr Asp
               180                 185                 190
   Glu Tyr Asp Gly His Ser Thr Tyr Pro Ser His Gln Tyr Gln
           195                 200                 205
<210> 21
   <211> 396
   <212> PRT
   <213> Homo sapiens
<400> 21
<210> 22
   <211> 267
   <212> PRT
   <213> Homo sapiens
<400> 22
<210> 23
   <211> 326
   <212> PRT
   <213> Homo sapiens
<400> 23
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Asn Phe Gly Thr Gln Thr
   65                  70                  75                  80
   Tyr Thr Cys Asn Val Asp His Lys Pro Ser Asn Thr Lys Val Asp Lys
                   85                  90                  95
   Thr Val Glu Arg Lys Cys Cys Val Glu Cys Pro Pro Cys Pro Ala Pro
               100                 105                 110
   Pro Val Ala Gly Pro Ser Val Phe Leu Phe Pro Pro Lys Pro Lys Asp
           115                 120                 125
   Thr Leu Met Ile Ser Arg Thr Pro Glu Val Thr Cys Val Val Val Asp
       130                 135                 140
   Val Ser His Glu Asp Pro Glu Val Gln Phe Asn Trp Tyr Val Asp Gly
   145                 150                 155                 160
   Val Glu Val His Asn Ala Lys Thr Lys Pro Arg Glu Glu Gln Phe Asn
                   165                 170                 175
   Ser Thr Phe Arg Val Val Ser Val Leu Thr Val Val His Gln Asp Trp
               180                 185                 190
   Leu Asn Gly Lys Glu Tyr Lys Cys Lys Val Ser Asn Lys Gly Leu Pro
           195                 200                 205
   Ala Pro Ile Glu Lys Thr Ile Ser Lys Thr Lys Gly Gln Pro Arg Glu
       210                 215                 220
   Pro Gln Val Tyr Thr Leu Pro Pro Ser Arg Glu Glu Met Thr Lys Asn
   225                 230                 235                 240
   Gln Val Ser Leu Thr Cys Leu Val Lys Gly Phe Tyr Pro Ser Asp Ile
                   245                 250                 255
   Ser Val Glu Trp Glu Ser Asn Gly Gln Pro Glu Asn Asn Tyr Lys Thr
               260                 265                 270
   Thr Pro Pro Met Leu Asp Ser Asp Gly Ser Phe Phe Leu Tyr Ser Lys
           275                 280                 285
   Leu Thr Val Asp Lys Ser Arg Trp Gln Gln Gly Asn Val Phe Ser Cys
       290                 295                 300
   Ser Val Met His Glu Ala Leu His Asn His Tyr Thr Gln Lys Ser Leu
   305                 310                 315                 320
   Ser Leu Ser Pro Gly Lys
                   325
<210> 24
   <211> 462
   <212> PRT
   <213> Homo sapiens
<400> 24
   Met Ala Arg Val Leu Gly Ala Pro Val Ala Leu Gly Leu Trp Ser Leu
   1               5                   10                  15
   Cys Trp Ser Leu Ala Ile Ala Thr Pro Leu Pro Pro Thr Ser Ala His
               20                  25                  30
   Gly Asn Val Ala Glu Gly Glu Thr Lys Pro Asp Pro Asp Val Thr Glu
           35                  40                  45
   Arg Cys Ser Asp Gly Trp Ser Phe Asp Ala Thr Thr Leu Asp Asp Asn
       50                  55                  60
   Gly Thr Met Leu Phe Phe Lys Gly Glu Phe Val Trp Lys Ser His Lys
   65                  70                  75                  80
   Trp Asp Arg Glu Leu Ile Ser Glu Arg Trp Lys Asn Phe Pro Ser Pro
                   85                  90                  95
   Val Asp Ala Ala Phe Arg Gln Gly His Asn Ser Val Phe Leu Ile Lys
   100 105 110
   Gly Asp Lys Val Trp Val Tyr Pro Pro Glu Lys Lys Glu Lys Gly Tyr
   115 120 125
   Pro Lys Leu Leu Gln Asp Glu Phe Pro Gly Ile Pro Ser Pro Leu Asp
   130 135 140
   Ala Ala Val Glu Cys His Arg Gly Glu Cys Gln Ala Glu Gly Val Leu
   145 150 155 160
   Phe Phe Gln Gly Asp Arg Glu Trp Phe Trp Asp Leu Ala Thr Gly Thr
   165 170 175
   Met Lys Glu Arg Ser Trp Pro Ala Val Gly Asn Cys Ser Ser Ala Leu
   180 185 190
   Arg Trp Leu Gly Arg Tyr Tyr Cys Phe Gln Gly Asn Gln Phe Leu Arg
   195 200 205
   Phe Asp Pro Val Arg Gly Glu Val Pro Pro Arg Tyr Pro Arg Asp Val
   210 215 220
   Arg Asp Tyr Phe Met Pro Cys Pro Gly Arg Gly His Gly His Arg Asn
   225 230 235 240
   Gly Thr Gly His Gly Asn Ser Thr His His Gly Pro Glu Tyr Met Arg
   245 250 255
   Cys Ser Pro His Leu Val Leu Ser Ala Leu Thr Ser Asp Asn His Gly
   260 265 270
   Ala Thr Tyr Ala Phe Ser Gly Thr His Tyr Trp Arg Leu Asp Thr Ser
   275 280 285
   Arg Asp Gly Trp His Ser Trp Pro Ile Ala His Gln Trp Pro Gln Gly
   290 295 300
   Pro Ser Ala Val Asp Ala Ala Phe Ser Trp Glu Glu Lys Leu Tyr Leu
   305 310 315 320
   Val Gln Gly Thr Gln Val Tyr Val Phe Leu Thr Lys Gly Gly Tyr Thr
   325 330 335
   Leu Val Ser Gly Tyr Pro Lys Arg Leu Glu Lys Glu Val Gly Thr Pro
   340 345 350
   His Gly Ile Ile Leu Asp Ser Val Asp Ala Ala Phe Ile Cys Pro Gly
   355 360 365
   Ser Ser Arg Leu His Ile Met Ala Gly Arg Arg Leu Trp Trp Leu Asp
   370 375 380
   Leu Lys Ser Gly Ala Gln Ala Thr Trp Thr Glu Leu Pro Trp Pro His
   385 390 395 400
   Glu Lys Val Asp Gly Ala Leu Cys Met Glu Lys Ser Leu Gly Pro Asn
   405 410 415
   Ser Cys Ser Ala Asn Gly Pro Gly Leu Tyr Leu Ile His Gly Pro Asn
   420 425 430
   Leu Tyr Cys Tyr Ser Asp Val Glu Lys Leu Asn Ala Ala Lys Ala Leu
   435 440 445
   Pro Gln Pro Gln Asn Val Thr Ser Leu Leu Gly Cys Thr His
   450 455 460
<210> 25
   <211> 583
   <212> PRT
   <213> Homo sapiens
<400> 25
   Met Ala His Val Arg Gly Leu Gln Leu Pro Gly Cys Leu Ala Leu Ala
   1 5 10 15
   Ala Leu Cys Ser Leu Val His Ser Gln His Val Phe Leu Ala Pro Gln
   20 25 30
   Gln Ala Arg Ser Leu Leu Gln Arg Val Arg Arg Ala Asn Thr Phe Leu
   35 40 45
   Glu Glu Val Arg Lys Gly Asn Leu Glu Arg Glu Cys Val Glu Glu Thr
   50 55 60
   Cys Ser Tyr Glu Glu Ala Phe Glu Ala Leu Glu Ser Ser Thr Ala Thr
   65 70 75 80
   Asp Val Phe Trp Ala Lys Tyr Thr Ala Cys Glu Thr Ala Arg Thr Pro
   85 90 95
   Arg Asp Lys Leu Ala Ala Cys Leu Glu Gly Asn Cys Ala Glu Gly Leu
   100 105 110
   Gly Thr Asn Tyr Arg Gly His Val Asn Ile Thr Arg Ser Gly Ile Glu
           115                 120                 125
   Cys Gln Leu Trp Arg Ser Arg Tyr Pro His Lys Pro Glu Ile Asn Ser
       130                 135                 140
   Thr Thr His Pro Gly Ala Asp Leu Gln Glu Asn Phe Cys Arg Asn Pro
   145                 150                 155                 160
   Asp Ser Ser Thr Thr Gly Pro Trp Cys Tyr Thr Thr Asp Pro Thr Val
                   165                 170                 175
   Arg Arg Gln Glu Cys Ser Ile Pro Val Cys Gly Gln Asp Gln Val Thr
               180                 185                 190
   Val Ala Met Thr Pro Arg Ser Glu Gly Ser Ser Val Asn Leu Ser Pro
           195                 200                 205
   Pro Leu Glu Gln Cys Val Pro Asp Arg Gly Gln Gln Tyr Gln Gly Arg
       210                 215                 220
   Leu Ala Val Thr Thr His Gly Leu Pro Cys Leu Ala Trp Ala Ser Ala
   225                 230                 235                 240
   Gln Ala Lys Ala Leu Ser Lys His Gln Asp Phe Asn Ser Ala Val Gln
                   245                 250                 255
   Leu Val Glu Asn Phe Cys Arg Asn Pro Asp Gly Asp Glu Glu Gly Val
               260                 265                 270
   Trp Cys Tyr Val Ala Gly Lys Pro Gly Asp Phe Gly Tyr Cys Asp Leu
           275                 280                 285
   Asn Tyr Cys Glu Glu Ala Val Glu Glu Glu Thr Gly Asp Gly Leu Asp
       290                 295                 300
   Glu Asp Ser Asp Arg Ala Ile Glu Gly Arg Thr Ala Thr Ser Glu Tyr
   305                 310                 315                 320
   Gln Thr Phe Phe Asn Pro Arg Thr Phe Gly Ser Gly Glu Ala Asp Cys
                   325                 330                 335
   Gly Leu Arg Pro Leu Phe Glu Lys Lys Ser Leu Glu Asp Lys Thr Glu
               340                 345                 350
   Arg Glu Leu Leu Glu Ser Tyr Ile Asp Gly Arg Ile Val Glu Gly Ser
           355                 360                 365
   Asp Ala Glu Ile Gly Met Ser Pro Trp Gln Val Met Leu Phe Arg Lys
       370                 375                 380
   Ser Pro Gln Glu Leu Leu Cys Gly Ala Ser Leu Ile Ser Asp Arg Trp
   385                 390                 395                 400
   Val Leu Thr Ala Ala His Cys Leu Leu Tyr Pro Pro Trp Asp Lys Asn
                   405                 410                 415
   Phe Thr Glu Asn Asp Leu Leu Val Arg Ile Gly Lys His Ser Arg Thr
               420                 425                 430
   Arg Tyr Glu Arg Asn Ile Glu Lys Ile Ser Met Leu Glu Lys Ile Tyr
           435                 440                 445
   Ile His Pro Ser Leu Leu Gln Ala Gly Tyr Lys Gly Arg Val Thr Gly
       450                 455                 460
   Trp Gly Asn Leu Lys Glu Thr Trp Thr Ala Asn Val Gly Lys Gly Gln
   465                 470                 475                 480
   Pro Ser Val Leu Gln Val Val Asn Leu Pro Ile Val Glu Arg Pro Val
                   485                 490                 495
   Cys Lys Asp Ser Thr Arg Ile Arg Ile Thr Asp Asn Met Phe Cys Ala
               500                 505                 510
   Gly Tyr Lys Pro Asp Glu Gly Lys Arg Gly Asp Ala Cys Glu Gly Asp
           515                 520                 525
   Ser Gly Gly Pro Phe Val Met Lys Ser Pro Phe Asn Asn Arg Trp Tyr
       530                 535                 540
   Gln Met Gly Ile Val Ser Trp Gly Glu Gly Cys Asp Arg Asp Gly Lys
   545                 550                 555                 560
   Tyr Gly Phe Tyr Thr His Val Phe Arg Leu Lys Lys Trp Ile Gln Lys
                   565                 570                 575
   Val Ile Asp Gln Phe Gly Glu
               580
<210> 26
   <211> 111
   <212> PRT
   <213> Homo sapiens
<400> 26
   Tyr Val Leu Thr Gln Pro Pro Ser Val Ser Val Ala Pro Gly Glu Thr
   1               5                   10                  15
   Ala Arg Leu Thr Cys Gly Gly Asn Asp Ile Gly Ser Glu Ser Val His
               20                  25                  30
   Trp Tyr Gln Gln Lys Pro Gly Gln Ala Pro Val Leu Val Ile Tyr Phe
           35                  40                  45
   Asp Arg Asp Arg Pro Ser Gly Ile Pro Glu Arg Phe Ser Gly Ser Asn
       50                  55                  60
   Ser Gly Asn Thr Ala Thr Leu Thr Ile Ser Arg Val Glu Ala Gly Asp
   65                  70                  75                  80
   Glu Ala Asp Tyr Tyr Cys Gln Leu Trp Asp Ser Ser Ser Glu His Val
                   85                  90                  95
   Val Phe Gly Gly Gly Thr Lys Leu Thr Val Leu Ser Gln Pro Lys
               100                 105                 110
<210> 27
   <211> 140
   <212> PRT
   <213> Homo sapiens
<400> 27
<210> 28
   <211> 175
   <212> PRT
   <213> Homo sapiens
<400> 28
   Met Glu Lys Ile Pro Val Ser Ala Phe Leu Leu Leu Val Ala Leu Ser
   1               5                   10                  15
   Tyr Thr Leu Ala Arg Asp Thr Thr Val Lys Pro Gly Ala Lys Lys Asp
               20                  25                  30
   Thr Lys Asp Ser Arg Pro Lys Leu Pro Gln Thr Leu Ser Arg Gly Trp
           35                  40                  45
   Gly Asp Gln Leu Ile Trp Thr Gln Thr Tyr Glu Glu Ala Leu Tyr Lys
       50                  55                  60
   Ser Lys Thr Ser Asn Lys Pro Leu Met Ile Ile His His Leu Asp Glu
   65                  70                  75                  80
   Cys Pro His Ser Gln Ala Leu Lys Lys Val Phe Ala Glu Asn Lys Glu
                   85                  90                  95
   Ile Gln Lys Leu Ala Glu Gln Phe Val Leu Leu Asn Leu Val Tyr Glu
               100                 105                 110
   Thr Thr Asp Lys His Leu Ser Pro Asp Gly Gln Tyr Val Pro Arg Ile
           115                 120                 125
   Met Phe Val Asp Pro Ser Leu Thr Val Arg Ala Asp Ile Thr Gly Arg
       130                 135                 140
   Tyr Ser Asn Arg Leu Tyr Ala Tyr Glu Pro Ala Asp Thr Ala Leu Leu
   145                 150                 155                 160
   Leu Asp Asn Met Lys Lys Ala Leu Lys Leu Leu Lys Thr Glu Leu
                   165                 170                 175
<210> 29
   <211> 147
   <212> PRT
   <213> Homo sapiens
<400> 29
   Met Ser Glu Glu Lys Pro Lys Glu Gly Val Lys Thr Glu Asn Asp His
   1               5                   10                  15
   Ile Asn Leu Lys Val Ala Gly Gln Asp Gly Ser Val Val Gln Phe Lys
               20                  25                  30
   Ile Lys Arg His Thr Pro Leu Ser Lys Leu Met Lys Ala Tyr Cys Glu
           35                  40                  45
   Arg Gln Gly Leu Ser Met Arg Gln Ile Arg Phe Arg Phe Asp Gly Gln
       50                  55                  60
   Pro Ile Asn Glu Thr Asp Thr Pro Ala Gln Gly Ile Ile Leu Ser Trp
   65                  70                  75                  80
   Lys Glu Leu Trp Thr Trp Lys Gln Thr Phe Phe Phe Glu Thr Glu Ser
                   85                  90                  95
   Arg Phe Val Ala Gln Ala Arg Met Gln Trp Arg Ser Leu Ser Ser Leu
               100                 105                 110
   Cys Lys Leu Cys Leu Leu Ser Ser Arg His Ser Pro Ala Ser Ala Ser
           115                 120                 125
   Gln Val Ala Gly Thr Ile Gly Ala His His His Ser Arg Leu Ile Phe
       130                 135                 140
   Leu Tyr Phe
   145
<210> 30
   <211> 347
   <212> PRT
   <213> Homo sapiens
<400> 30
   Met Ser Ser Trp Ser Arg Gln Arg Pro Lys Ser Pro Gly Gly Ile Gln
   1               5                   10                  15
   Pro His Val Ser Arg Thr Leu Phe Leu Leu Leu Leu Leu Ala Ala Ser
               20                  25                  30
   Ala Trp Gly Val Thr Leu Ser Pro Lys Asp Cys Gln Val Phe Arg Ser
           35                  40                  45
   Asp His Gly Ser Ser Ile Ser Cys Gln Pro Pro Ala Glu Ile Pro Gly
       50                  55                  60
   Tyr Leu Pro Ala Asp Thr Val His Leu Ala Val Glu Phe Phe Asn Leu
   65                  70                  75                  80
   Thr His Leu Pro Ala Asn Leu Leu Gln Gly Ala Ser Lys Leu Gln Glu
                   85                  90                  95
   Leu His Leu Ser Ser Asn Gly Leu Glu Ser Leu Ser Pro Glu Phe Leu
               100                 105                 110
   Arg Pro Val Pro Gln Leu Arg Val Leu Asp Leu Thr Arg Asn Ala Leu
           115                 120                 125
   Thr Gly Leu Pro Pro Gly Leu Phe Gln Ala Ser Ala Thr Leu Asp Thr
       130                 135                 140
   Leu Val Leu Lys Glu Asn Gln Leu Glu Val Leu Glu Val Ser Trp Leu
   145                 150                 155                 160
   His Gly Leu Lys Ala Leu Gly His Leu Asp Leu Ser Gly Asn Arg Leu
                   165                 170                 175
   Arg Lys Leu Pro Pro Gly Leu Leu Ala Asn Phe Thr Leu Leu Arg Thr
               180                 185                 190
   Leu Asp Leu Gly Glu Asn Gln Leu Glu Thr Leu Pro Pro Asp Leu Leu
           195                 200                 205
   Arg Gly Pro Leu Gln Leu Glu Arg Leu His Leu Glu Gly Asn Lys Leu
       210                 215                 220
   Gln Val Leu Gly Lys Asp Leu Leu Leu Pro Gln Pro Asp Leu Arg Tyr
   225                 230                 235                 240
   Leu Phe Leu Asn Gly Asn Lys Leu Ala Arg Val Ala Ala Gly Ala Phe
                   245                 250                 255
   Gln Gly Leu Arg Gln Leu Asp Met Leu Asp Leu Ser Asn Asn Ser Leu
               260                 265                 270
   Ala Ser Val Pro Glu Gly Leu Trp Ala Ser Leu Gly Gln Pro Asn Trp
           275                 280                 285
   Asp Met Arg Asp Gly Phe Asp Ile Ser Gly Asn Pro Trp Ile Cys Asp
       290                 295                 300
   Gln Asn Leu Ser Asp Leu Tyr Arg Trp Leu Gln Ala Gln Lys Asp Lys
   305 310 315 320
   Met Phe Ser Gln Asn Asp Thr Arg Cys Ala Gly Pro Glu Ala Val Lys
   325 330 335
   Gly Gln Thr Leu Leu Ala Val Ala Lys Ser Gln
   340 345
<210> 31
   <211> 495
   <212> PRT
   <213> Homo sapiens
<400> 31
   Met Ser Met Leu Val Val Phe Leu Leu Leu Trp Gly Val Thr Trp Gly
   1 5 10 15
   Pro Val Thr Glu Ala Ala Ile Phe Tyr Glu Thr Gln Pro Ser Leu Trp
   20 25 30
   Ala Glu Ser Glu Ser Leu Leu Lys Pro Leu Ala Asn Val Thr Leu Thr
   35 40 45
   Cys Gln Ala His Leu Glu Thr Pro Asp Phe Gln Leu Phe Lys Asn Gly
   50 55 60
   Val Ala Gln Glu Pro Val His Leu Asp Ser Pro Ala Ile Lys His Gln
   65 70 75 80
   Phe Leu Leu Thr Gly Asp Thr Gln Gly Arg Tyr Arg Cys Arg Ser Gly
   85 90 95
   Leu Ser Thr Gly Trp Thr Gln Leu Ser Lys Leu Leu Glu Leu Thr Gly
   100 105 110
   Pro Lys Ser Leu Pro Ala Pro Trp Leu Ser Met Ala Pro Val Ser Trp
   115 120 125
   Ile Thr Pro Gly Leu Lys Thr Thr Ala Val Cys Arg Gly Val Leu Arg
   130 135 140
   Gly Val Thr Phe Leu Leu Arg Arg Glu Gly Asp His Glu Phe Leu Glu
   145 150 155 160
   Val Pro Glu Ala Gln Glu Asp Val Glu Ala Thr Phe Pro Val His Gln
   165 170 175
   Pro Gly Asn Tyr Ser Cys Ser Tyr Arg Thr Asp Gly Glu Gly Ala Leu
               180                 185                 190
   Ser Glu Pro Ser Ala Thr Val Thr Ile Glu Glu Leu Ala Ala Pro Pro
           195                 200                 205
   Pro Pro Val Leu Met His His Gly Glu Ser Ser Gln Val Leu His Pro
       210                 215                 220
   Gly Asn Lys Val Thr Leu Thr Cys Val Ala Pro Leu Ser Gly Val Asp
   225                 230                 235                 240
   Phe Gln Leu Arg Arg Gly Glu Lys Glu Leu Leu Val Pro Arg Ser Ser
                   245                 250                 255
   Thr Ser Pro Asp Arg Ile Phe Phe His Leu Asn Ala Val Ala Leu Gly
               260                 265                 270
   Asp Gly Gly His Tyr Thr Cys Arg Tyr Arg Leu His Asp Asn Gln Asn
           275                 280                 285
   Gly Trp Ser Gly Asp Ser Ala Pro Val Glu Leu Ile Leu Ser Asp Glu
       290                 295                 300
   Thr Leu Pro Ala Pro Glu Phe Ser Pro Glu Pro Glu Ser Gly Arg Ala
   305                 310                 315                 320
   Leu Arg Leu Arg Cys Leu Ala Pro Leu Glu Gly Ala Arg Phe Ala Leu
                   325                 330                 335
   Val Arg Glu Asp Arg Gly Gly Arg Arg Val His Arg Phe Gln Ser Pro
               340                 345                 350
   Ala Gly Thr Glu Ala Leu Phe Glu Leu His Asn Ile Ser Val Ala Asp
           355                 360                 365
   Ser Ala Asn Tyr Ser Cys Val Tyr Val Asp Leu Lys Pro Pro Phe Gly
       370                 375                 380
   Gly Ser Ala Pro Ser Glu Arg Leu Glu Leu His Val Asp Gly Pro Pro
   385                 390                 395                 400
   Pro Arg Pro Gln Leu Arg Ala Thr Trp Ser Gly Ala Val Leu Ala Gly
                   405                 410                 415
   Arg Asp Ala Val Leu Arg Cys Glu Gly Pro Ile Pro Asp Val Thr Phe
               420                 425                 430
   Glu Leu Leu Arg Glu Gly Glu Thr Lys Ala Val Lys Thr Val Arg Thr
   435 440 445
   Pro Gly Ala Ala Ala Asn Leu Glu Leu Ile Phe Val Gly Pro Gln His
   450 455 460
   Ala Gly Asn Tyr Arg Cys Arg Tyr Arg Ser Trp Val Pro His Thr Phe
   465 470 475 480
   Glu Ser Glu Leu Ser Asp Pro Val Glu Leu Leu Val Ala Glu Ser
   485 490 495
<210> 32
   <211> 418
   <212> PRT
   <213> Homo sapiens
<400> 32
<210> 33
   <211> 146
   <212> PRT
   <213> Homo sapiens
<400> 33
   Met Ala Gly Glu Leu Thr Pro Glu Glu Glu Ala Gln Tyr Lys Lys Ala
   1 5 10 15
   Phe Ser Ala Val Asp Thr Asp Gly Asn Gly Thr Ile Asn Ala Gln Glu
   20 25 30
   Leu Gly Ala Ala Leu Lys Ala Thr Gly Lys Asn Leu Ser Glu Ala Gln
   35 40 45
   Leu Arg Lys Leu Ile Ser Glu Val Asp Ser Asp Gly Asp Gly Glu Ile
   50 55 60
   Ser Phe Gln Glu Phe Leu Thr Ala Ala Lys Lys Ala Arg Ala Gly Leu
   65 70 75 80
   Glu Asp Leu Gln Val Ala Phe Arg Ala Phe Asp Gln Asp Gly Asp Gly
   85 90 95
   His Ile Thr Val Asp Glu Leu Arg Arg Ala Met Ala Gly Leu Gly Gln
   100 105 110
   Pro Leu Pro Gln Glu Glu Leu Asp Ala Met Ile Arg Glu Ala Asp Val
   115 120 125
   Asp Gln Asp Gly Arg Val Asn Tyr Glu Glu Phe Ala Arg Met Leu Ala
   130 135 140
   Gln Glu
   145
<210> 34
   <211> 338
   <212> PRT
   <213> Homo sapiens
<400> 34
   Met Leu Ser Ala Leu Ala Arg Pro Ala Ser Ala Ala Leu Arg Arg Ser
   1 5 10 15
   Phe Ser Thr Ser Ala Gln Asn Asn Ala Lys Val Ala Val Leu Gly Ala
   20 25 30
   Ser Gly Gly Ile Gly Gln Pro Leu Ser Leu Leu Leu Lys Asn Ser Pro
   35 40 45
   Leu Val Ser Arg Leu Thr Leu Tyr Asp Ile Ala His Thr Pro Gly Val
   50 55 60
   Ala Ala Asp Leu Ser His Ile Glu Thr Lys Ala Ala Val Lys Gly Tyr
   65 70 75 80
   Leu Gly Pro Glu Gln Leu Pro Asp Cys Leu Lys Gly Cys Asp Val Val
   85 90 95
   Val Ile Pro Ala Gly Val Pro Arg Lys Pro Gly Met Thr Arg Asp Asp
   100 105 110
   Leu Phe Asn Thr Asn Ala Thr Ile Val Ala Thr Leu Thr Ala Ala Cys
   115 120 125
   Ala Gln His Cys Pro Glu Ala Met Ile Cys Val Ile Ala Asn Pro Val
   130 135 140
   Asn Ser Thr Ile Pro Ile Thr Ala Glu Val Phe Lys Lys His Gly Val
   145 150 155 160
   Tyr Asn Pro Asn Lys Ile Phe Gly Val Thr Thr Leu Asp Ile Val Arg
   165 170 175
   Ala Asn Thr Phe Val Ala Glu Leu Lys Gly Leu Asp Pro Ala Arg Val
   180 185 190
   Asn Val Pro Val Ile Gly Gly His Ala Gly Lys Thr Ile Ile Pro Leu
   195 200 205
   Ile Ser Gln Cys Thr Pro Lys Val Asp Phe Pro Gln Asp Gln Leu Thr
   210 215 220
   Ala Leu Thr Gly Arg Ile Gln Glu Ala Gly Thr Glu Val Val Lys Ala
   225 230 235 240
   Lys Ala Gly Ala Gly Ser Ala Thr Leu Ser Met Ala Tyr Ala Gly Ala
   245 250 255
   Arg Phe Val Phe Ser Leu Val Asp Ala Met Asn Gly Lys Glu Gly Val
   260 265 270
   Val Glu Cys Ser Phe Val Lys Ser Gln Glu Thr Glu Cys Thr Tyr Phe
   275 280 285
   Ser Thr Pro Leu Leu Leu Gly Lys Lys Gly Ile Glu Lys Asn Leu Gly
   290 295 300
   Ile Gly Lys Val Ser Ser Phe Glu Glu Lys Met Ile Ser Asp Ala Ile
   305 310 315 320
   Pro Glu Leu Lys Ala Ser Ile Lys Lys Gly Glu Asp Phe Val Lys Thr
   325 330 335
   Leu Lys
<210> 35
   <211> 320
   <212> PRT
   <213> Homo sapiens
<400> 35
   Met Ala Gln Val Leu Arg Gly Thr Val Thr Asp Phe Pro Gly Phe Asp
   1               5                   10                  15
   Glu Arg Ala Asp Ala Glu Thr Leu Arg Lys Ala Met Lys Gly Leu Gly
               20                  25                  30
   Thr Asp Glu Glu Ser Ile Leu Thr Leu Leu Thr Ser Arg Ser Asn Ala
           35                  40                  45
   Gln Arg Gln Glu Ile Ser Ala Ala Phe Lys Thr Leu Phe Gly Arg Asp
       50                  55                  60
   Leu Leu Asp Asp Leu Lys Ser Glu Leu Thr Gly Lys Phe Glu Lys Leu
   65                  70                  75                  80
   Ile Val Ala Leu Met Lys Pro Ser Arg Leu Tyr Asp Ala Tyr Glu Leu
                   85                  90                  95
   Lys His Ala Leu Lys Gly Ala Gly Thr Asn Glu Lys Val Leu Thr Glu
               100                 105                 110
   Ile Ile Ala Ser Arg Thr Pro Glu Glu Leu Arg Ala Ile Lys Gln Val
           115                 120                 125
   Tyr Glu Glu Glu Tyr Gly Ser Ser Leu Glu Asp Asp Val Val Gly Asp
   130 135 140
   Thr Ser Gly Tyr Tyr Gln Arg Met Leu Val Val Leu Leu Gln Ala Asn
   145 150 155 160
   Arg Asp Pro Asp Ala Gly Ile Asp Glu Ala Gln Val Glu Gln Asp Ala
   165 170 175
   Gln Ala Leu Phe Gln Ala Gly Glu Leu Lys Trp Gly Thr Asp Glu Glu
   180 185 190
   Lys Phe Ile Thr Ile Phe Gly Thr Arg Ser Val Ser His Leu Arg Lys
   195 200 205
   Val Phe Asp Lys Tyr Met Thr Ile Ser Gly Phe Gln Ile Glu Glu Thr
   210 215 220
   Ile Asp Arg Glu Thr Ser Gly Asn Leu Glu Gln Leu Leu Leu Ala Val
   225 230 235 240
   Val Lys Ser Ile Arg Ser Ile Pro Ala Tyr Leu Ala Glu Thr Leu Tyr
   245 250 255
   Tyr Ala Met Lys Gly Ala Gly Thr Asp Asp His Thr Leu Ile Arg Val
   260 265 270
   Met Val Ser Arg Ser Glu Ile Asp Leu Phe Asn Ile Arg Lys Glu Phe
   275 280 285
   Arg Lys Asn Phe Ala Thr Ser Leu Tyr Ser Met Ile Lys Gly Asp Thr
   290 295 300
   Ser Gly Asp Tyr Lys Lys Ala Leu Leu Leu Leu Cys Gly Glu Asp Asp
   305 310 315 320
<210> 36
   <211> 474
   <212> PRT
   <213> Homo sapiens
<400> 36
<210> 37
   <211> 847
   <212> PRT
   <213> Homo sapiens
<400> 37
<210> 38
   <211> 375
   <212> PRT
   <213> Homo sapiens
<400> 38
   Met Ser Thr Ala Gly Lys Val Ile Lys Cys Lys Ala Ala Val Leu Trp
   1               5                   10                  15
   Glu Leu Lys Lys Pro Phe Ser Ile Glu Glu Val Glu Val Ala Pro Pro
               20                  25                  30
   Lys Ala His Glu Val Arg Ile Lys Met Val Ala Ala Gly Ile Cys Arg
           35                  40                  45
   Ser Asp Glu His Val Val Ser Gly Asn Leu Val Thr Pro Leu Pro Val
       50                  55                  60
   Ile Leu Gly His Glu Ala Ala Gly Ile Val Glu Ser Val Gly Glu Gly
   65                  70                  75                  80
   Val Thr Thr Val Lys Pro Gly Asp Lys Val Ile Pro Leu Phe Thr Pro
                   85                  90                  95
   Gln Cys Gly Lys Cys Arg Ile Cys Lys Asn Pro Glu Ser Asn Tyr Cys
               100                 105                 110
   Leu Lys Asn Asp Leu Gly Asn Pro Arg Gly Thr Leu Gln Asp Gly Thr
           115                 120                 125
   Arg Arg Phe Thr Cys Ser Gly Lys Pro Ile His His Phe Val Gly Val
       130                 135                 140
   Ser Thr Phe Ser Gln Tyr Thr Val Val Asp Glu Asn Ala Val Ala Lys
   145                 150                 155                 160
   Ile Asp Ala Ala Ser Pro Leu Glu Lys Val Cys Leu Ile Gly Cys Gly
   165 170 175
   Phe Ser Thr Gly Tyr Gly Ser Ala Val Lys Val Ala Lys Val Thr Pro
   180 185 190
   Gly Ser Thr Cys Ala Val Phe Gly Leu Gly Gly Val Gly Leu Ser Val
   195 200 205
   Val Met Gly Cys Lys Ala Ala Gly Ala Ala Arg Ile Ile Ala Val Asp
   210 215 220
   Ile Asn Lys Asp Lys Phe Ala Lys Ala Lys Glu Leu Gly Ala Thr Glu
   225 230 235 240
   Cys Ile Asn Pro Gln Asp Tyr Lys Lys Pro Ile Gln Glu Val Leu Lys
   245 250 255
   Glu Met Thr Asp Gly Gly Val Asp Phe Ser Phe Glu Val Ile Gly Arg
   260 265 270
   Leu Asp Thr Met Met Ala Ser Leu Leu Cys Cys His Glu Ala Cys Gly
   275 280 285
   Thr Ser Val Ile Val Gly Val Pro Pro Asp Ser Gln Asn Leu Ser Ile
   290 295 300
   Asn Pro Met Leu Leu Leu Thr Gly Arg Thr Trp Lys Gly Ala Ile Phe
   305 310 315 320
   Gly Gly Phe Lys Ser Lys Glu Ser Val Pro Lys Leu Val Ala Asp Phe
   325 330 335
   Met Ala Lys Lys Phe Ser Leu Asp Ala Leu Ile Thr Asn Ile Leu Pro
   340 345 350
   Phe Glu Lys Ile Asn Glu Gly Phe Asp Leu Leu Arg Ser Gly Lys Ser
   355 360 365
   Ile Arg Thr Val Leu Thr Phe
   370 375
<210> 39
   <211> 1311
   <212> PRT
   <213> Homo sapiens
<220>
   <221> misc_feature
   <222> (1)..(1)
   <223> Xaa can be any naturally occurring amino acid
<400> 39
   Xaa Arg Glu Ile Val Thr Arg Asn Leu Ser Gln Pro Glu Ser Pro Val
   1               5                   10                  15
   Leu Leu Pro Ala Thr Glu Met Ala Ser Leu Leu Ser Leu Gln Glu Glu
               20                  25                  30
   Asn Gln Leu Leu Gln Gln Glu Leu Ser Arg Val Glu Asp Leu Leu Ala
           35                  40                  45
   Gln Ser Arg Ala Glu Arg Asp Glu Leu Ala Ile Lys Tyr Asn Ala Val
       50                  55                  60
   Ser Glu Arg Asp Thr Glu His Ser Gln Asp Leu Glu Ser Ala Leu Ile
   65                  70                  75                  80
   Arg Leu Glu Glu Glu Gln Gln Arg Ser Ala Ser Leu Ala Gln Val Asn
                   85                  90                  95
   Ala Met Leu Arg Glu Gln Leu Asp Gln Ala Gly Ser Ala Asn Gln Ala
               100                 105                 110
   Leu Ser Glu Asp Ile Arg Lys Val Thr Asn Asp Trp Thr Arg Cys Arg
           115                 120                 125
   Lys Glu Leu Glu His Arg Glu Ala Ala Trp Arg Arg Glu Glu Glu Ser
       130                 135                 140
   Phe Asn Ala Tyr Phe Ser Asn Glu His Ser Arg Leu Leu Leu Leu Trp
   145                 150                 155                 160
   Arg Gln Val Val Gly Phe Arg Arg Leu Val Ser Glu Val Lys Met Phe
                   165                 170                 175
   Thr Glu Arg Asp Leu Leu Gln Leu Gly Gly Glu Leu Ala Arg Thr Ser
               180                 185                 190
   Arg Ala Val Gln Glu Ala Gly Leu Gly Leu Ser Thr Gly Leu Arg Leu
           195                 200                 205
   Ala Glu Ser Arg Ala Glu Ala Ala Leu Glu Lys Gln Ala Leu Leu Gln
       210                 215                 220
   Ala Gln Leu Glu Glu Gln Leu Arg Asp Lys Val Leu Arg Glu Lys Asp
   225                 230                 235                 240
   Leu Ala Gln Gln Gln Met Gln Ser Asp Leu Asp Lys Ala Asp Leu Ser
                   245                 250                 255
   Ala Arg Val Thr Glu Leu Gly Leu Ala Val Lys Arg Leu Glu Lys Gln
               260                 265                 270
   Asn Leu Glu Lys Asp Gln Val Asn Lys Asp Leu Thr Glu Lys Leu Glu
           275                 280                 285
   Ala Leu Glu Ser Leu Arg Leu Gln Glu Gln Ala Ala Leu Glu Thr Glu
       290                 295                 300
   Asp Gly Glu Gly Leu Gln Gln Thr Leu Arg Asp Leu Ala Gln Ala Val
   305                 310                 315                 320
   Leu Ser Asp Ser Glu Ser Gly Val Gln Leu Ser Gly Ser Glu Arg Thr
                   325                 330                 335
   Ala Asp Ala Ser Asn Gly Ser Leu Arg Gly Leu Ser Gly Gln Arg Thr
               340                 345                 350
   Pro Ser Pro Pro Arg Arg Ser Ser Pro Gly Arg Gly Arg Ser Pro Arg
           355                 360                 365
   Arg Gly Pro Ser Pro Ala Cys Ser Asp Ser Ser Thr Leu Ala Leu Ile
       370                 375                 380
   His Ser Ala Leu His Lys Arg Gln Leu Gln Val Gln Asp Met Arg Gly
   385                 390                 395                 400
   Arg Tyr Glu Ala Ser Gln Asp Leu Leu Gly Thr Leu Arg Lys Gln Leu
                   405                 410                 415
   Ser Asp Ser Glu Ser Glu Arg Arg Ala Leu Glu Glu Gln Leu Gln Arg
               420                 425                 430
   Leu Arg Asp Lys Thr Asp Gly Ala Met Gln Ala His Glu Asp Ala Gln
           435                 440                 445
   Arg Glu Val Gln Arg Leu Arg Ser Ala Asn Glu Leu Leu Ser Arg Glu
       450                 455                 460
   Lys Ser Asn Leu Ala His Ser Leu Gln Val Ala Gln Gln Gln Ala Glu
   465                 470                 475                 480
   Glu Leu Arg Gln Glu Arg Glu Lys Leu Gln Ala Ala Gln Glu Glu Leu
   485 490 495
   Arg Arg Gln Arg Asp Arg Leu Glu Glu Glu Gln Glu Asp Ala Val Gln
   500 505 510
   Asp Gly Ala Arg Val Arg Arg Glu Leu Glu Arg Ser His Arg Gln Leu
   515 520 525
   Glu Gln Leu Glu Gly Lys Arg Ser Val Leu Ala Lys Glu Leu Val Glu
   530 535 540
   Val Arg Glu Ala Leu Ser Arg Ala Thr Leu Gln Arg Asp Met Leu Gln
   545 550 555 560
   Ala Glu Lys Ala Glu Val Ala Glu Ala Leu Thr Lys Ala Glu Ala Gly
   565 570 575
   Arg Val Glu Leu Glu Leu Ser Met Thr Lys Leu Arg Ala Glu Glu Ala
   580 585 590
   Ser Leu Gln Asp Ser Leu Ser Lys Leu Ser Ala Leu Asn Glu Ser Leu
   595 600 605
   Ala Gln Asp Lys Leu Asp Leu Asn Arg Leu Val Ala Gln Leu Glu Glu
   610 615 620
   Glu Lys Ser Ala Leu Gln Gly Arg Gln Arg Gln Ala Glu Gln Glu Ala
   625 630 635 640
   Thr Val Ala Arg Glu Glu Gln Glu Arg Leu Glu Glu Leu Arg Leu Glu
   645 650 655
   Gln Glu Val Ala Arg Gln Gly Leu Glu Gly Ser Leu Arg Val Ala Glu
   660 665 670
   Gln Ala Gln Glu Ala Leu Glu Gln Gln Leu Pro Thr Leu Arg His Glu
   675 680 685
   Arg Ser Gln Leu Gln Glu Gln Leu Ala Gln Leu Ser Arg Gln Leu Ser
   690 695 700
   Gly Arg Glu Gln Glu Leu Glu Gln Ala Arg Arg Glu Ala Gln Arg Gln
   705 710 715 720
   Val Glu Ala Leu Glu Arg Ala Ala Arg Glu Lys Glu Ala Leu Ala Lys
   725 730 735
   Glu His Ala Gly Leu Ala Val Gln Leu Val Ala Ala Glu Arg Glu Gly
               740                 745                 750
   Arg Thr Leu Ser Glu Glu Ala Thr Arg Leu Arg Leu Glu Lys Glu Ala
           755                 760                 765
   Leu Glu Gly Ser Leu Phe Glu Val Gln Arg Gln Leu Ala Gln Leu Glu
       770                 775                 780
   Ala Arg Arg Glu Gln Leu Glu Ala Glu Gly Gln Ala Leu Leu Leu Ala
   785                 790                 795                 800
   Lys Glu Thr Leu Thr Gly Glu Leu Ala Gly Leu Arg Gln Gln Ile Ile
                   805                 810                 815
   Ala Thr Gln Glu Lys Ala Ser Leu Asp Lys Glu Leu Met Ala Gln Lys
               820                 825                 830
   Leu Val Gln Ala Glu Arg Glu Ala Gln Ala Ser Leu Arg Glu Gln Arg
           835                 840                 845
   Ala Ala His Glu Glu Asp Leu Gln Arg Leu Gln Arg Glu Lys Glu Ala
       850                 855                 860
   Ala Trp Arg Glu Leu Glu Ala Glu Arg Ala Gln Leu Gln Ser Gln Leu
   865                 870                 875                 880
   Gln Arg Glu Gln Glu Glu Leu Leu Ala Arg Leu Glu Ala Glu Lys Glu
                   885                 890                 895
   Glu Leu Ser Glu Glu Ile Ala Ala Leu Gln Gln Glu Arg Asp Glu Gly
               900                 905                 910
   Leu Leu Leu Ala Glu Ser Glu Lys Gln Gln Ala Leu Ser Leu Lys Glu
           915                 920                 925
   Ser Glu Lys Thr Ala Leu Ser Glu Lys Leu Met Gly Thr Arg His Ser
       930                 935                 940
   Leu Ala Thr Ile Ser Leu Glu Met Glu Arg Gln Lys Arg Asp Ala Gln
   945                 950                 955                 960
   Ser Arg Gln Glu Gln Asp Arg Ser Thr Val Asn Ala Leu Thr Ser Glu
                   965                 970                 975
   Leu Arg Asp Leu Arg Ala Gln Arg Glu Glu Ala Ala Ala Ala His Ala
               980                 985                 990
   Gln Glu Val Arg Arg Leu Gln Glu  Gln Ala Arg Asp Leu  Gly Lys Gln
           995                 1000                 1005
   Arg Asp  Ser Cys Leu Arg Glu  Ala Glu Glu Leu Arg  Thr Gln Leu
       1010                 1015                 1020
   Arg Leu  Leu Glu Asp Ala Arg  Asp Gly Leu Arg Arg  Glu Leu Leu
       1025                 1030                 1035
   Glu Ala  Gln Arg Lys Leu Arg  Glu Ser Gln Glu Gly  Arg Glu Val
       1040                 1045                 1050
   Gln Arg  Gln Glu Ala Gly Glu  Leu Arg Arg Ser Leu  Gly Glu Gly
       1055                 1060                 1065
   Ala Lys  Glu Arg Glu Ala Leu  Arg Arg Ser Asn Glu  Glu Leu Arg
       1070                 1075                 1080
   Ser Ala  Val Lys Lys Ala Glu  Ser Glu Arg Ile Ser  Leu Lys Leu
       1085                 1090                 1095
   Ala Asn  Glu Asp Lys Glu Gln  Lys Leu Ala Leu Leu  Glu Glu Ala
       1100                 1105                 1110
   Arg Thr  Ala Val Gly Lys Glu  Ala Gly Glu Leu Arg  Thr Gly Leu
       1115                 1120                 1125
   Gln Glu  Val Glu Arg Ser Arg  Leu Glu Ala Arg Arg  Glu Leu Gln
       1130                 1135                 1140
   Glu Leu  Arg Arg Gln Met Lys  Met Leu Asp Ser Glu  Asn Thr Arg
       1145                 1150                 1155
   Leu Gly  Arg Glu Leu Ala Glu  Leu Gln Gly Arg Leu  Ala Leu Gly
       1160                 1165                 1170
   Glu Arg  Ala Glu Lys Glu Ser  Arg Arg Glu Thr Leu  Gly Leu Arg
       1175                 1180                 1185
   Gln Arg  Leu Leu Lys Gly Glu  Ala Ser Leu Glu Val  Met Arg Gln
       1190                 1195                 1200
   Glu Leu  Gln Val Ala Gln Arg  Lys Leu Gln Glu Gln  Glu Gly Glu
       1205                 1210                 1215
   Phe Arg  Thr Arg Glu Arg Arg  Leu Leu Gly Ser Leu  Glu Glu Ala
       1220                 1225                 1230
   Arg Gly Thr Glu Lys Gln Gln Leu Asp His Ala Arg Gly Leu Glu
   1235 1240 1245
   Leu Lys Leu Glu Ala Ala Arg Ala Glu Ala Ala Glu Leu Gly Leu
   1250 1255 1260
   Arg Leu Ser Ala Ala Glu Gly Arg Ala Gln Gly Leu Glu Ala Glu
   1265 1270 1275
   Leu Ala Arg Val Glu Val Gln Arg Arg Ala Ala Glu Ala Gln Leu
   1280 1285 1290
   Gly Gly Leu Arg Ser Ala Leu Arg Arg Gly Leu Gly Leu Gly Arg
   1295 1300 1305
   Ala Pro Ser
   1310
<210> 40
   <211> 154
   <212> PRT
   <213> Homo sapiens
<400> 40
<210> 41
   <211> 324
   <212> PRT
   <213> Homo sapiens
<400> 41
   Met Asp Ser Lys Tyr Gln Cys Val Lys Leu Asn Asp Gly His Phe Met
   1 5 10 15
   Pro Val Leu Gly Phe Gly Thr Tyr Ala Pro Ala Glu Val Pro Lys Ser
   20 25 30
   Lys Ala Leu Glu Ala Thr Lys Leu Ala Ile Glu Ala Gly Phe Arg His
   35 40 45
   Ile Asp Ser Ala His Leu Tyr Asn Asn Glu Glu Gln Val Gly Leu Ala
   50 55 60
   Ile Arg Ser Lys Ile Ala Asp Gly Ser Val Lys Arg Glu Asp Ile Phe
   65 70 75 80
   Tyr Thr Ser Lys Leu Trp Cys Asn Ser His Arg Pro Glu Leu Val Arg
   85 90 95
   Pro Ala Leu Glu Arg Ser Leu Lys Asn Leu Gln Leu Asp Tyr Val Asp
   100 105 110
   Leu Tyr Leu Ile His Phe Pro Val Ser Val Lys Pro Gly Glu Glu Val
   115 120 125
   Ile Pro Lys Asp Glu Asn Gly Lys Ile Leu Phe Asp Thr Val Asp Leu
   130 135 140
   Cys Ala Thr Trp Glu Val Met Glu Lys Cys Lys Asp Ala Gly Leu Ala
   145 150 155 160
   Lys Ser Ile Gly Val Ser Asn Phe Asn Arg Arg Gln Leu Glu Met Ile
   165 170 175
   Leu Asn Lys Pro Gly Leu Lys Tyr Lys Pro Val Cys Asn Gln Val Glu
   180 185 190
   Cys His Pro Tyr Phe Asn Gln Arg Lys Leu Leu Asp Phe Cys Lys Ser
   195 200 205
   Lys Asp Ile Val Leu Val Ala Tyr Ser Ala Leu Gly Ser His Arg Glu
   210 215 220
   Glu Pro Trp Val Asp Pro Asn Ser Pro Val Leu Leu Glu Asp Pro Val
   225 230 235 240
   Leu Cys Ala Leu Ala Lys Lys His Lys Arg Thr Pro Ala Leu Ile Ala
   245 250 255
   Leu Arg Tyr Gln Leu Gln Arg Gly Val Val Val Leu Ala Lys Ser Tyr
   260 265 270
   Asn Glu Gln Arg Ile Arg Gln Asn Val Gln Val Phe Glu Phe Gln Leu
   275 280 285
   Thr Ser Glu Glu Met Lys Ala Ile Asp Gly Leu Asn Arg Asn Val Arg
   290 295 300
   Tyr Leu Thr Leu Asp Met Tyr Phe Ala Ser His Pro Asn Tyr Pro Tyr
   305 310 315 320
   Ser Asp Glu Tyr
<210> 42
   <211> 114
   <212> PRT
   <213> Homo sapiens
<400> 42
   Met Ala Ser Val Ser Glu Leu Ala Cys Ile Tyr Ser Ala Leu Ile Leu
   1 5 10 15
   His Asp Asp Glu Val Thr Val Thr Glu Asp Lys Ile Asn Ala Leu Ile
   20 25 30
   Lys Ala Ala Gly Val Asn Val Glu Pro Phe Trp Pro Gly Leu Phe Ala
   35 40 45
   Lys Ala Leu Ala Asn Val Asn Ile Gly Ser Leu Ile Cys Asn Val Gly
   50 55 60
   Ala Gly Gly Pro Ala Pro Ala Ala Gly Ala Ala Pro Ala Gly Gly Pro
   65 70 75 80
   Ala Pro Ser Thr Ala Ala Ala Pro Ala Glu Glu Lys Lys Val Glu Ala
   85 90 95
   Lys Lys Glu Glu Ser Glu Glu Ser Asp Asp Asp Met Gly Phe Gly Leu
   100 105 110
   Phe Asp
<210> 43
   <211> 945
   <212> PRT
   <213> Homo sapiens
<400> 43
   Met Lys Arg Leu Thr Cys Phe Phe Ile Cys Phe Phe Leu Ser Glu Val
   1 5 10 15
   Ser Gly Phe Glu Ile Pro Ile Asn Gly Leu Ser Glu Phe Val Asp Tyr
   20 25 30
   Glu Asp Leu Val Glu Leu Ala Pro Gly Lys Phe Gln Leu Val Ala Glu
   35 40 45
   Asn Arg Arg Tyr Gln Arg Ser Leu Pro Gly Glu Ser Glu Glu Met Met
   50 55 60
   Glu Glu Val Asp Gln Val Thr Leu Tyr Ser Tyr Lys Val Gln Ser Thr
   65 70 75 80
   Ile Thr Ser Arg Met Ala Thr Thr Met Ile Gln Ser Lys Val Val Asn
   85 90 95
   Asn Ser Pro Gln Pro Gln Asn Val Val Phe Asp Val Gln Ile Pro Lys
   100 105 110
   Gly Ala Phe Ile Ser Asn Phe Ser Met Thr Val Asp Gly Lys Thr Phe
   115 120 125
   Arg Ser Ser Ile Lys Glu Lys Thr Val Gly Arg Ala Leu Tyr Ala Gln
   130 135 140
   Ala Arg Ala Lys Gly Lys Thr Ala Gly Leu Val Arg Ser Ser Ala Leu
   145 150 155 160
   Asp Met Glu Asn Phe Arg Thr Glu Val Asn Val Leu Pro Gly Ala Lys
   165 170 175
   Val Gln Phe Glu Leu His Tyr Gln Glu Val Lys Trp Arg Lys Leu Gly
               180                 185                 190
   Ser Tyr Glu His Arg Ile Tyr Leu Gln Pro Gly Arg Leu Ala Lys His
           195                 200                 205
   Leu Glu Val Asp Val Trp Val Ile Glu Pro Gln Gly Leu Arg Phe Leu
       210                 215                 220
   His Val Pro Asp Thr Phe Glu Gly His Phe Asp Gly Val Pro Val Ile
   225                 230                 235                 240
   Ser Lys Gly Gln Gln Lys Ala His Val Ser Phe Lys Pro Thr Val Ala
                   245                 250                 255
   Gln Gln Arg Ile Cys Pro Asn Cys Arg Glu Thr Ala Val Asp Gly Glu
               260                 265                 270
   Leu Val Val Leu Tyr Asp Val Lys Arg Glu Glu Lys Ala Gly Glu Leu
           275                 280                 285
   Glu Val Phe Asn Gly Tyr Phe Val His Phe Phe Ala Pro Asp Asn Leu
       290                 295                 300
   Asp Pro Ile Pro Lys Asn Ile Leu Phe Val Ile Asp Val Ser Gly Ser
   305                 310                 315                 320
   Met Trp Gly Val Lys Met Lys Gln Thr Val Glu Ala Met Lys Thr Ile
                   325                 330                 335
   Leu Asp Asp Leu Arg Ala Glu Asp His Phe Ser Val Ile Asp Phe Asn
               340                 345                 350
   Gln Asn Ile Arg Thr Trp Arg Asn Asp Leu Phe Gln Leu Gln Lys His
           355                 360                 365
   Arg Leu Gln Met Pro Arg Tyr Ile Glu Lys Ile Gln Pro Ser Gly Gly
       370                 375                 380
   Thr Asn Ile Asn Glu Ala Leu Leu Arg Ala Ile Phe Ile Leu Asn Glu
   385                 390                 395                 400
   Ala Asn Asn Leu Gly Leu Leu Asp Pro Asn Ser Val Ser Leu Ile Ile
                   405                 410                 415
   Leu Val Ser Asp Gly Asp Pro Thr Val Gly Glu Leu Lys Leu Ser Lys
   420 425 430
   Ile Gln Lys Asn Val Lys Glu Asn Ile Gln Asp Asn Ile Ser Leu Phe
   435 440 445
   Ser Leu Gly Met Gly Phe Asp Val Asp Tyr Asp Phe Leu Lys Arg Leu
   450 455 460
   Ser Asn Glu Asn His Gly Ile Ala Gln Arg Ile Tyr Gly Asn Gln Asp
   465 470 475 480
   Thr Ser Ser Gln Leu Lys Lys Phe Tyr Asn Gln Val Ser Thr Pro Leu
   485 490 495
   Leu Arg Asn Val Gln Phe Asn Tyr Pro His Thr Ser Val Thr Asp Val
   500 505 510
   Thr Gln Asn Asn Phe His Asn Tyr Phe Gly Gly Ser Glu Ile Val Val
   515 520 525
   Ala Gly Lys Phe Asp Pro Ala Lys Leu Asp Gln Ile Glu Ser Val Ile
   530 535 540
   Thr Ala Thr Ser Ala Asn Thr Gln Leu Val Leu Glu Thr Leu Ala Gln
   545 550 555 560
   Met Asp Asp Leu Gln Asp Phe Leu Ser Lys Asp Lys His Ala Asp Pro
   565 570 575
   Asp Phe Thr Arg Lys Leu Trp Ala Tyr Leu Thr Ile Asn Gln Leu Leu
   580 585 590
   Ala Glu Arg Ser Leu Ala Pro Thr Ala Ala Ala Lys Arg Arg Ile Thr
   595 600 605
   Arg Ser Ile Leu Gln Met Ser Leu Asp His His Ile Val Thr Pro Leu
   610 615 620
   Thr Ser Leu Val Ile Glu Asn Glu Ala Gly Asp Glu Arg Met Leu Ala
   625 630 635 640
   Asp Ala Pro Pro Gln Asp Pro Ser Cys Cys Ser Gly Ala Leu Tyr Tyr
   645 650 655
   Gly Ser Lys Val Val Pro Asp Ser Thr Pro Ser Trp Ala Asn Pro Ser
   660 665 670
   Pro Thr Pro Val Ile Ser Met Leu Ala Gln Gly Ser Gln Val Leu Glu
           675                 680                 685
   Ser Thr Pro Pro Pro His Val Met Arg Val Glu Asn Asp Pro His Phe
       690                 695                 700
   Ile Ile Tyr Leu Pro Lys Ser Gln Lys Asn Ile Cys Phe Asn Ile Asp
   705                 710                 715                 720
   Ser Glu Pro Gly Lys Ile Leu Asn Leu Val Ser Asp Pro Glu Ser Gly
                   725                 730                 735
   Ile Val Val Asn Gly Gln Leu Val Gly Ala Lys Lys Pro Asn Asn Gly
               740                 745                 750
   Lys Leu Ser Thr Tyr Phe Gly Lys Leu Gly Phe Tyr Phe Gln Ser Glu
           755                 760                 765
   Asp Ile Lys Ile Glu Ile Ser Thr Glu Thr Ile Thr Leu Ser His Gly
       770                 775                 780
   Ser Ser Thr Phe Ser Leu Ser Trp Ser Asp Thr Ala Gln Val Thr Asn
   785                 790                 795                 800
   Gln Arg Val Gln Ile Ser Val Lys Lys Glu Lys Val Val Thr Ile Thr
                   805                 810                 815
   Leu Asp Lys Glu Met Ser Phe Ser Val Leu Leu His Arg Val Trp Lys
               820                 825                 830
   Lys His Pro Val Asn Val Asp Phe Leu Gly Ile Tyr Ile Pro Pro Thr
           835                 840                 845
   Asn Lys Phe Ser Pro Lys Ala His Gly Leu Ile Gly Gln Phe Met Gln
       850                 855                 860
   Glu Pro Lys Ile His Ile Phe Asn Glu Arg Pro Gly Lys Asp Pro Glu
   865                 870                 875                 880
   Lys Pro Glu Ala Ser Met Glu Val Lys Gly Gln Lys Leu Ile Ile Thr
                   885                 890                 895
   Arg Gly Leu Gln Lys Asp Tyr Arg Thr Asp Leu Val Phe Gly Thr Asp
               900                 905                 910
   Val Thr Cys Trp Phe Val His Asn Ser Gly Lys Gly Phe Ile Asp Gly
           915                 920                 925
   His Tyr Lys Asp Tyr Phe Val Pro Gln Leu Tyr Ser Phe Leu Lys Arg
   930 935 940
   Pro
   945
<210> 44
   <211> 90
   <212> PRT
   <213> Homo sapiens
<400> 44
   Met Lys Phe Leu Ala Val Leu Val Leu Leu Gly Val Ser Ile Phe Leu
   1 5 10 15
   Val Ser Ala Gln Asn Pro Thr Thr Ala Ala Pro Ala Asp Thr Tyr Pro
   20 25 30
   Ala Thr Gly Pro Ala Asp Asp Glu Ala Pro Asp Ala Glu Thr Thr Ala
   35 40 45
   Ala Ala Thr Thr Ala Thr Thr Ala Ala Pro Thr Thr Ala Thr Thr Ala
   50 55 60
   Ala Ser Thr Thr Ala Arg Lys Asp Ile Pro Val Leu Pro Lys Trp Val
   65 70 75 80
   Gly Asp Leu Pro Asn Gly Arg Val Cys Pro
   85 90
<210> 45
   <211> 90
   <212> PRT
   <213> Homo sapiens
<400> 45
   Met Ala Cys Pro Leu Asp Gln Ala Ile Gly Leu Leu Val Ala Ile Phe
   1 5 10 15
   His Lys Tyr Ser Gly Arg Glu Gly Asp Lys His Thr Leu Ser Lys Lys
   20 25 30
   Glu Leu Lys Glu Leu Ile Gln Lys Glu Leu Thr Ile Gly Ser Lys Leu
   35 40 45
   Gln Asp Ala Glu Ile Ala Arg Leu Met Glu Asp Leu Asp Arg Asn Lys
   50 55 60
   Asp Gln Glu Val Asn Phe Gln Glu Tyr Val Thr Phe Leu Gly Ala Leu
   65 70 75 80
   Ala Leu Ile Tyr Asn Glu Ala Leu Lys Gly
   85 90
<210> 46
   <211> 358
   <212> PRT
   <213> Homo sapiens
<400> 46
   Met Ser Ala Asp Ala Ala Ala Gly Ala Pro Leu Pro Arg Leu Cys Cys
   1 5 10 15
   Leu Glu Lys Gly Pro Asn Gly Tyr Gly Phe His Leu His Gly Glu Lys
   20 25 30
   Gly Lys Leu Gly Gln Tyr Ile Arg Leu Val Glu Pro Gly Ser Pro Ala
   35 40 45
   Glu Lys Ala Gly Leu Leu Ala Gly Asp Arg Leu Val Glu Val Asn Gly
   50 55 60
   Glu Asn Val Glu Lys Glu Thr His Gln Gln Val Val Ser Arg Ile Arg
   65 70 75 80
   Ala Ala Leu Asn Ala Val Arg Leu Leu Val Val Asp Pro Glu Thr Asp
   85 90 95
   Glu Gln Leu Gln Lys Leu Gly Val Gln Val Arg Glu Glu Leu Leu Arg
   100 105 110
   Ala Gln Glu Ala Pro Gly Gln Ala Glu Pro Pro Ala Ala Ala Glu Val
   115 120 125
   Gln Gly Ala Gly Asn Glu Asn Glu Pro Arg Glu Ala Asp Lys Ser His
   130 135 140
   Pro Glu Gln Arg Glu Leu Arg Pro Arg Leu Cys Thr Met Lys Lys Gly
   145 150 155 160
   Pro Ser Gly Tyr Gly Phe Asn Leu His Ser Asp Lys Ser Lys Pro Gly
   165 170 175
   Gln Phe Ile Arg Ser Val Asp Pro Asp Ser Pro Ala Glu Ala Ser Gly
   180 185 190
   Leu Arg Ala Gln Asp Arg Ile Val Glu Val Asn Gly Val Cys Met Glu
   195 200 205
   Gly Lys Gln His Gly Asp Val Val Ser Ala Ile Arg Ala Gly Gly Asp
   210 215 220
   Glu Thr Lys Leu Leu Val Val Asp Arg Glu Thr Asp Glu Phe Phe Lys
   225 230 235 240
   Lys Cys Arg Val Ile Pro Ser Gln Glu His Leu Asn Gly Pro Leu Pro
   245 250 255
   Val Pro Phe Thr Asn Gly Glu Ile Gln Lys Glu Asn Ser Arg Glu Ala
   260 265 270
   Leu Ala Glu Ala Ala Leu Glu Ser Pro Arg Pro Ala Leu Val Arg Ser
   275 280 285
   Ala Ser Ser Asp Thr Ser Glu Glu Leu Asn Ser Gln Asp Ser Pro Pro
   290 295 300
   Lys Gln Asp Ser Thr Ala Pro Ser Ser Thr Ser Ser Ser Asp Pro Ile
   305 310 315 320
   Leu Asp Phe Asn Ile Ser Leu Ala Met Ala Lys Glu Arg Ala His Gln
   325 330 335
   Lys Arg Ser Ser Lys Arg Ala Pro Gln Met Asp Trp Ser Lys Lys Asn
   340 345 350
   Glu Leu Phe Ser Asn Leu
   355
<210> 47
   <211> 75
   <212> PRT
   <213> Homo sapiens
<400> 47
   Met Ala Arg Tyr Glu Glu Val Ser Val Ser Gly Phe Glu Glu Phe His
   1 5 10 15
   Arg Ala Val Glu Gln His Asn Gly Lys Thr Ile Phe Ala Tyr Phe Thr
   20 25 30
   Gly Ser Lys Asp Ala Gly Gly Lys Ser Trp Cys Pro Asp Cys Val Gln
   35 40 45
   Ala Glu Pro Val Val Arg Glu Gly Leu Lys His Ile Ser Glu Gly Cys
   50 55 60
   Val Phe Ile Tyr Cys Gln Val Gly Glu Lys Pro
   65 70 75
<210> 48
   <211> 339
   <212> PRT
   <213> Homo sapiens
<400> 48
   Met Ala Glu Ala Ser Ser Asp Pro Gly Ala Glu Glu Arg Glu Glu Leu
   1 5 10 15
   Leu Gly Pro Thr Ala Gln Trp Ser Val Glu Asp Glu Glu Glu Ala Val
   20 25 30
   His Glu Gln Cys Gln His Glu Arg Asp Arg Gln Leu Gln Ala Gln Asp
   35 40 45
   Glu Glu Gly Gly Gly His Val Pro Glu Arg Pro Lys Gln Glu Met Leu
   50 55 60
   Leu Ser Leu Lys Pro Ser Glu Ala Pro Glu Leu Asp Glu Asp Glu Gly
   65 70 75 80
   Phe Gly Asp Trp Ser Gln Arg Pro Glu Gln Arg Gln Gln His Glu Gly
   85 90 95
   Ala Gln Gly Ala Leu Asp Ser Gly Glu Pro Pro Gln Cys Arg Ser Pro
   100 105 110
   Glu Gly Glu Gln Glu Asp Arg Pro Gly Leu His Ala Tyr Glu Lys Glu
   115 120 125
   Asp Ser Asp Glu Val His Leu Glu Glu Leu Ser Leu Ser Lys Glu Gly
   130 135 140
   Pro Gly Pro Glu Asp Thr Val Gln Asp Asn Leu Gly Ala Ala Gly Ala
   145 150 155 160
   Glu Glu Glu Gln Glu Glu His Gln Lys Cys Gln Gln Pro Arg Thr Pro
   165 170 175
   Ser Pro Leu Val Leu Glu Gly Thr Ile Glu Gln Ser Ser Pro Pro Leu
   180 185 190
   Ser Pro Thr Thr Lys Leu Ile Asp Arg Thr Glu Ser Leu Asn Arg Ser
   195 200 205
   Ile Glu Lys Ser Asn Ser Val Lys Lys Ser Gln Pro Asp Leu Pro Ile
   210 215 220
   Ser Lys Ile Asp Gln Trp Leu Glu Gln Tyr Thr Gln Ala Ile Glu Thr
   225 230 235 240
   Ala Gly Arg Thr Pro Lys Leu Ala Arg Gln Ala Ser Ile Glu Leu Pro
   245 250 255
   Ser Met Ala Val Ala Ser Thr Lys Ser Arg Trp Glu Thr Gly Glu Val
   260 265 270
   Gln Ala Gln Ser Ala Ala Lys Thr Pro Ser Cys Lys Asp Ile Val Ala
   275 280 285
   Gly Asp Met Ser Lys Lys Ser Leu Trp Glu Gln Lys Gly Gly Ser Lys
   290 295 300
   Thr Ser Ser Thr Ile Lys Ser Thr Pro Ser Gly Lys Arg Tyr Lys Phe
   305 310 315 320
   Val Ala Thr Gly His Gly Lys Tyr Glu Lys Val Leu Val Glu Gly Gly
   325 330 335
   Pro Ala Pro
<210> 49
   <211> 177
   <212> PRT
   <213> Homo sapiens
<400> 49
   Met Asp Leu Thr Thr Thr Met Ser Ser Lys Arg Thr Lys Thr Lys Thr
   1 5 10 15
   Lys Lys Arg Pro Gln Arg Ala Thr Ser Asn Val Phe Ala Met Phe Asp
   20 25 30
   Gln Ser Gln Ile Gln Glu Phe Lys Glu Ala Phe Asn Met Ile Asp Gln
   35 40 45
   Asn Arg Asp Gly Phe Ile Asp Lys Glu Asp Leu His Asp Met Leu Ala
   50 55 60
   Ser Leu Gly Lys Asn Pro Thr Asp Glu Tyr Leu Asp Ala Met Met Asn
   65 70 75 80
   Glu Ala Pro Gly Pro Ile Asn Phe Thr Met Phe Leu Thr Met Phe Gly
   85 90 95
   Glu Lys Leu Asn Gly Thr Asp Pro Glu Asp Val Ile Arg Asn Ala Phe
   100 105 110
   Ala Cys Phe Asp Glu Glu Ala Thr Gly Thr Ile Gln Glu Asp Tyr Leu
   115 120 125
   Arg Glu Leu Leu Thr Thr Met Gly Asp Arg Phe Thr Asp Glu Glu Val
   130 135 140
   Asp Glu Leu Tyr Arg Glu Ala Pro Ile Asp Lys Lys Gly Asn Phe Asn
   145 150 155 160
   Tyr Ile Glu Phe Thr Arg Ile Leu Lys His Gly Ala Lys Asp Lys Asp
   165 170 175
   Asp
<210> 50
   <211> 461
   <212> PRT
   <213> Homo sapiens
<400> 50
<210> 51
   <211> 434
   <212> PRT
   <213> Homo sapiens
<400> 51
   Met Ser Ile Leu Lys Ile His Ala Arg Glu Ile Phe Asp Ser Arg Gly
   1 5 10 15
   Asn Pro Thr Val Glu Val Asp Leu Phe Thr Ser Lys Gly Leu Phe Arg
   20 25 30
   Ala Ala Val Pro Ser Gly Ala Ser Thr Gly Ile Tyr Glu Ala Leu Glu
   35 40 45
   Leu Arg Asp Asn Asp Lys Thr Arg Tyr Met Gly Lys Gly Val Ser Lys
   50 55 60
   Ala Val Glu His Ile Asn Lys Thr Ile Ala Pro Ala Leu Val Ser Lys
   65 70 75 80
   Lys Leu Asn Val Thr Glu Gln Glu Lys Ile Asp Lys Leu Met Ile Glu
   85 90 95
   Met Asp Gly Thr Glu Asn Lys Ser Lys Phe Gly Ala Asn Ala Ile Leu
   100 105 110
   Gly Val Ser Leu Ala Val Cys Lys Ala Gly Ala Val Glu Lys Gly Val
   115 120 125
   Pro Leu Tyr Arg His Ile Ala Asp Leu Ala Gly Asn Ser Glu Val Ile
   130 135 140
   Leu Pro Val Pro Ala Phe Asn Val Ile Asn Gly Gly Ser His Ala Gly
   145 150 155 160
   Asn Lys Leu Ala Met Gln Glu Phe Met Ile Leu Pro Val Gly Ala Ala
   165 170 175
   Asn Phe Arg Glu Ala Met Arg Ile Gly Ala Glu Val Tyr His Asn Leu
   180 185 190
   Lys Asn Val Ile Lys Glu Lys Tyr Gly Lys Asp Ala Thr Asn Val Gly
   195 200 205
   Asp Glu Gly Gly Phe Ala Pro Asn Ile Leu Glu Asn Lys Glu Gly Leu
   210 215 220
   Glu Leu Leu Lys Thr Ala Ile Gly Lys Ala Gly Tyr Thr Asp Lys Val
   225 230 235 240
   Val Ile Gly Met Asp Val Ala Ala Ser Glu Phe Phe Arg Ser Gly Lys
   245 250 255
   Tyr Asp Leu Asp Phe Lys Ser Pro Asp Asp Pro Ser Arg Tyr Ile Ser
   260 265 270
   Pro Asp Gln Leu Ala Asp Leu Tyr Lys Ser Phe Ile Lys Asp Tyr Pro
   275 280 285
   Val Val Ser Ile Glu Asp Pro Phe Asp Gln Asp Asp Trp Gly Ala Trp
   290 295 300
   Gln Lys Phe Thr Ala Ser Ala Gly Ile Gln Val Val Gly Asp Asp Leu
   305 310 315 320
   Thr Val Thr Asn Pro Lys Arg Ile Ala Lys Ala Val Asn Glu Lys Ser
   325 330 335
   Cys Asn Cys Leu Leu Leu Lys Val Asn Gln Ile Gly Ser Val Thr Glu
   340 345 350
   Ser Leu Gln Ala Cys Lys Leu Ala Gln Ala Asn Gly Trp Gly Val Met
   355 360 365
   Val Ser His Arg Ser Gly Glu Thr Glu Asp Thr Phe Ile Ala Asp Leu
   370 375 380
   Val Val Gly Leu Cys Thr Gly Gln Ile Lys Thr Gly Ala Pro Cys Arg
   385 390 395 400
   Ser Glu Arg Leu Ala Lys Tyr Asn Gln Leu Leu Arg Ile Glu Glu Glu
   405 410 415
   Leu Gly Ser Lys Ala Lys Phe Ala Gly Arg Asn Phe Arg Asn Pro Leu
   420 425 430
   Ala Lys
<210> 52
   <211> 108
   <212> PRT
   <213> Homo sapiens
<400> 52
   Asp Ile Gln Met Thr Gln Ser Pro Ser Thr Leu Ser Ala Ser Val Gly
   1 5 10 15
   Asp Arg Val Thr Ile Thr Cys Arg Ala Ser Gln Ser Ile Asn Thr Trp
   20 25 30
   Leu Ala Trp Tyr Gln Gln Lys Pro Gly Lys Ala Pro Lys Leu Leu Met
   35 40 45
   Tyr Lys Ala Ser Ser Leu Glu Ser Gly Val Pro Ser Arg Phe Ile Gly
   50 55 60
   Ser Gly Ser Gly Thr Glu Phe Thr Leu Thr Ile Ser Ser Leu Gln Pro
   65 70 75 80
   Asp Asp Phe Ala Thr Tyr Tyr Cys Gln Gln Tyr Asn Ser Asp Ser Lys
   85 90 95
   Met Phe Gly Gln Gly Thr Lys Val Glu Val Lys Gly
   100 105
<210> 53
   <211> 386
   <212> PRT
   <213> Homo sapiens
<400> 53
   Met Phe Ala Glu Ile Gln Ile Gln Asp Lys Asp Arg Met Gly Thr Ala
   1               5                   10                  15
   Gly Lys Val Ile Lys Cys Lys Ala Ala Val Leu Trp Glu Gln Lys Gln
               20                  25                  30
   Pro Phe Ser Ile Glu Glu Ile Glu Val Ala Pro Pro Lys Thr Lys Glu
           35                  40                  45
   Val Arg Ile Lys Ile Leu Ala Thr Gly Ile Cys Arg Thr Asp Asp His
       50                  55                  60
   Val Ile Lys Gly Thr Met Val Ser Lys Phe Pro Val Ile Val Gly His
   65                  70                  75                  80
   Glu Ala Thr Gly Ile Val Glu Ser Ile Gly Glu Gly Val Thr Thr Val
                   85                  90                  95
   Lys Pro Gly Asp Lys Val Ile Pro Leu Phe Leu Pro Gln Cys Arg Glu
               100                 105                 110
   Cys Asn Ala Cys Arg Asn Pro Asp Gly Asn Leu Cys Ile Arg Ser Asp
           115                 120                 125
   Ile Thr Gly Arg Gly Val Leu Ala Asp Gly Thr Thr Arg Phe Thr Cys
       130                 135                 140
   Lys Gly Lys Pro Val His His Phe Met Asn Thr Ser Thr Phe Thr Glu
   145                 150                 155                 160
   Tyr Thr Val Val Asp Glu Ser Ser Val Ala Lys Ile Asp Asp Ala Ala
                   165                 170                 175
   Pro Pro Glu Lys Val Cys Leu Ile Gly Cys Gly Phe Ser Thr Gly Tyr
               180                 185                 190
   Gly Ala Ala Val Lys Thr Gly Lys Val Lys Pro Gly Ser Thr Cys Val
           195                 200                 205
   Val Phe Gly Leu Gly Gly Val Gly Leu Ser Val Ile Met Gly Cys Lys
       210                 215                 220
   Ser Ala Gly Ala Ser Arg Ile Ile Gly Ile Asp Leu Asn Lys Asp Lys
   225                 230                 235                 240
   Phe Glu Lys Ala Met Ala Val Gly Ala Thr Glu Cys Ile Ser Pro Lys
                   245                 250                 255
   Asp Ser Thr Lys Pro Ile Ser Glu Val Leu Ser Glu Met Thr Gly Asn
   260 265 270
   Asn Val Gly Tyr Thr Phe Glu Val Ile Gly His Leu Glu Thr Met Ile
   275 280 285
   Asp Ala Leu Ala Ser Cys His Met Asn Tyr Gly Thr Ser Val Val Val
   290 295 300
   Gly Val Pro Pro Ser Ala Lys Met Leu Thr Tyr Asp Pro Met Leu Leu
   305 310 315 320
   Phe Thr Gly Arg Thr Trp Lys Gly Cys Val Phe Gly Gly Leu Lys Ser
   325 330 335
   Arg Asp Asp Val Pro Lys Leu Val Thr Glu Phe Leu Ala Lys Lys Phe
   340 345 350
   Asp Leu Asp Gln Leu Ile Thr His Val Leu Pro Phe Lys Lys Ile Ser
   355 360 365
   Glu Gly Phe Glu Leu Leu Asn Ser Gly Gln Ser Ile Arg Thr Val Leu
   370 375 380
   Thr Phe
   385
<210> 54
   <211> 352
   <212> PRT
   <213> Homo sapiens
<400> 54
   Met Arg Ser Leu Gly Ala Leu Leu Leu Leu Leu Ser Ala Cys Leu Ala
   1 5 10 15
   Val Ser Ala Gly Pro Val Pro Thr Pro Pro Asp Asn Ile Gln Val Gln
   20 25 30
   Glu Asn Phe Asn Ile Ser Arg Ile Tyr Gly Lys Trp Tyr Asn Leu Ala
   35 40 45
   Ile Gly Ser Thr Cys Pro Trp Leu Lys Lys Ile Met Asp Arg Met Thr
   50 55 60
   Val Ser Thr Leu Val Leu Gly Glu Gly Ala Thr Glu Ala Glu Ile Ser
   65 70 75 80
   Met Thr Ser Thr Arg Trp Arg Lys Gly Val Cys Glu Glu Thr Ser Gly
   85 90 95
   Ala Tyr Glu Lys Thr Asp Thr Asp Gly Lys Phe Leu Tyr His Lys Ser
   100 105 110
   Lys Trp Asn Ile Thr Met Glu Ser Tyr Val Val His Thr Asn Tyr Asp
   115 120 125
   Glu Tyr Ala Ile Phe Leu Thr Lys Lys Phe Ser Arg His His Gly Pro
   130 135 140
   Thr Ile Thr Ala Lys Leu Tyr Gly Arg Ala Pro Gln Leu Arg Glu Thr
   145 150 155 160
   Leu Leu Gln Asp Phe Arg Val Val Ala Gln Gly Val Gly Ile Pro Glu
   165 170 175
   Asp Ser Ile Phe Thr Met Ala Asp Arg Gly Glu Cys Val Pro Gly Glu
   180 185 190
   Gln Glu Pro Glu Pro Ile Leu Ile Pro Arg Val Arg Arg Ala Val Leu
   195 200 205
   Pro Gln Glu Glu Glu Gly Ser Gly Gly Gly Gln Leu Val Thr Glu Val
   210 215 220
   Thr Lys Lys Glu Asp Ser Cys Gln Leu Gly Tyr Ser Ala Gly Pro Cys
   225 230 235 240
   Met Gly Met Thr Ser Arg Tyr Phe Tyr Asn Gly Thr Ser Met Ala Cys
   245 250 255
   Glu Thr Phe Gln Tyr Gly Gly Cys Met Gly Asn Gly Asn Asn Phe Val
   260 265 270
   Thr Glu Lys Glu Cys Leu Gln Thr Cys Arg Thr Val Ala Ala Cys Asn
   275 280 285
   Leu Pro Ile Val Arg Gly Pro Cys Arg Ala Phe Ile Gln Leu Trp Ala
   290 295 300
   Phe Asp Ala Val Lys Gly Lys Cys Val Leu Phe Pro Tyr Gly Gly Cys
   305 310 315 320
   Gln Gly Asn Gly Asn Lys Phe Tyr Ser Glu Lys Glu Cys Arg Glu Tyr
   325 330 335
   Cys Gly Val Pro Gly Asp Gly Asp Glu Glu Leu Leu Arg Phe Ser Asn
   340 345 350
<210> 55
   <211> 485
   <212> PRT
   <213> Homo sapiens
<400> 55
   Met Arg Lys Arg Ala Pro Gln Ser Glu Met Ala Pro Ala Gly Val Ser
   1               5                   10                  15
   Leu Arg Ala Thr Ile Leu Cys Leu Leu Ala Trp Ala Gly Leu Ala Ala
               20                  25                  30
   Gly Asp Arg Val Tyr Ile His Pro Phe His Leu Val Ile His Asn Glu
           35                  40                  45
   Ser Thr Cys Glu Gln Leu Ala Lys Ala Asn Ala Gly Lys Pro Lys Asp
       50                  55                  60
   Pro Thr Phe Ile Pro Ala Pro Ile Gln Ala Lys Thr Ser Pro Val Asp
   65                  70                  75                  80
   Glu Lys Ala Leu Gln Asp Gln Leu Val Leu Val Ala Ala Lys Leu Asp
                   85                  90                  95
   Thr Glu Asp Lys Leu Arg Ala Ala Met Val Gly Met Leu Ala Asn Phe
               100                 105                 110
   Leu Gly Phe Arg Ile Tyr Gly Met His Ser Glu Leu Trp Gly Val Val
           115                 120                 125
   His Gly Ala Thr Val Leu Ser Pro Thr Ala Val Phe Gly Thr Leu Ala
       130                 135                 140
   Ser Leu Tyr Leu Gly Ala Leu Asp His Thr Ala Asp Arg Leu Gln Ala
   145                 150                 155                 160
   Ile Leu Gly Val Pro Trp Lys Asp Lys Asn Cys Thr Ser Arg Leu Asp
                   165                 170                 175
   Ala His Lys Val Leu Ser Ala Leu Gln Ala Val Gln Gly Leu Leu Val
               180                 185                 190
   Ala Gln Gly Arg Ala Asp Ser Gln Ala Gln Leu Leu Leu Ser Thr Val
   195 200 205
   Val Gly Val Phe Thr Ala Pro Gly Leu His Leu Lys Gln Pro Phe Val
   210 215 220
   Gln Gly Leu Ala Leu Tyr Thr Pro Val Val Leu Pro Arg Ser Leu Asp
   225 230 235 240
   Phe Thr Glu Leu Asp Val Ala Ala Glu Lys Ile Asp Arg Phe Met Gln
   245 250 255
   Ala Val Thr Gly Trp Lys Thr Gly Cys Ser Leu Met Gly Ala Ser Val
   260 265 270
   Asp Ser Thr Leu Ala Phe Asn Thr Tyr Val His Phe Gln Gly Lys Met
   275 280 285
   Lys Gly Phe Ser Leu Leu Ala Glu Pro Gln Glu Phe Trp Val Asp Asn
   290 295 300
   Ser Thr Ser Val Ser Val Pro Met Leu Ser Gly Met Gly Thr Phe Gln
   305 310 315 320
   His Trp Ser Asp Ile Gln Asp Asn Phe Ser Val Thr Gln Val Pro Phe
   325 330 335
   Thr Glu Ser Ala Cys Leu Leu Leu Ile Gln Pro His Tyr Ala Ser Asp
   340 345 350
   Leu Asp Lys Val Glu Gly Leu Thr Phe Gln Gln Asn Ser Leu Asn Trp
   355 360 365
   Met Lys Lys Leu Ser Pro Arg Thr Ile His Leu Thr Met Pro Gln Leu
   370 375 380
   Val Leu Gln Gly Ser Tyr Asp Leu Gln Asp Leu Leu Ala Gln Ala Glu
   385 390 395 400
   Leu Pro Ala Ile Leu His Thr Glu Leu Asn Leu Gln Lys Leu Ser Asn
   405 410 415
   Asp Arg Ile Arg Val Gly Glu Val Leu Asn Ser Ile Phe Phe Glu Leu
   420 425 430
   Glu Ala Asp Glu Arg Glu Pro Thr Glu Ser Thr Gln Gln Leu Asn Lys
   435 440 445
   Pro Glu Val Leu Glu Val Thr Leu Asn Arg Pro Phe Leu Phe Ala Val
   450 455 460
   Tyr Asp Gln Ser Ala Thr Ala Leu His Phe Leu Gly Arg Val Ala Asn
   465 470 475 480
   Pro Leu Ser Thr Ala
   485
<210> 56
   <211> 464
   <212> PRT
   <213> Homo sapiens
<400> 56
<210> 57
   <211> 100
   <212> PRT
   <213> Homo sapiens
<400> 57
   Met Lys Leu Leu Ala Ala Thr Val Leu Leu Leu Thr Ile Cys Ser Leu
   1 5 10 15
   Glu Gly Ala Leu Val Arg Arg Gln Ala Lys Glu Pro Cys Val Glu Ser
   20 25 30
   Leu Val Ser Gln Tyr Phe Gln Thr Val Thr Asp Tyr Gly Lys Asp Leu
   35 40 45
   Met Glu Lys Val Lys Ser Pro Glu Leu Gln Ala Glu Ala Lys Ser Tyr
   50 55 60
   Phe Glu Lys Ser Lys Glu Gln Leu Thr Pro Leu Ile Lys Lys Ala Gly
   65 70 75 80
   Thr Glu Leu Val Asn Phe Leu Ser Tyr Phe Val Glu Leu Gly Thr Gln
   85 90 95
   Pro Ala Thr Gln
   100
<210> 58
   <211> 345
   <212> PRT
   <213> Homo sapiens
<400> 58
   Met Ile Ser Pro Val Leu Ile Leu Phe Ser Ser Phe Leu Cys His Val
   1 5 10 15
   Ala Ile Ala Gly Arg Thr Cys Pro Lys Pro Asp Asp Leu Pro Phe Ser
   20 25 30
   Thr Val Val Pro Leu Lys Thr Phe Tyr Glu Pro Gly Glu Glu Ile Thr
   35 40 45
   Tyr Ser Cys Lys Pro Gly Tyr Val Ser Arg Gly Gly Met Arg Lys Phe
       50                  55                  60
   Ile Cys Pro Leu Thr Gly Leu Trp Pro Ile Asn Thr Leu Lys Cys Thr
   65                  70                  75                  80
   Pro Arg Val Cys Pro Phe Ala Gly Ile Leu Glu Asn Gly Ala Val Arg
                   85                  90                  95
   Tyr Thr Thr Phe Glu Tyr Pro Asn Thr Ile Ser Phe Ser Cys Asn Thr
               100                 105                 110
   Gly Phe Tyr Leu Asn Gly Ala Asp Ser Ala Lys Cys Thr Glu Glu Gly
           115                 120                 125
   Lys Trp Ser Pro Glu Leu Pro Val Cys Ala Pro Ile Ile Cys Pro Pro
       130                 135                 140
   Pro Ser Ile Pro Thr Phe Ala Thr Leu Arg Val Tyr Lys Pro Ser Ala
   145                 150                 155                 160
   Gly Asn Asn Ser Leu Tyr Arg Asp Thr Ala Val Phe Glu Cys Leu Pro
                   165                 170                 175
   Gln His Ala Met Phe Gly Asn Asp Thr Ile Thr Cys Thr Thr His Gly
               180                 185                 190
   Asn Trp Thr Lys Leu Pro Glu Cys Arg Glu Val Lys Cys Pro Phe Pro
           195                 200                 205
   Ser Arg Pro Asp Asn Gly Phe Val Asn Tyr Pro Ala Lys Pro Thr Leu
       210                 215                 220
   Tyr Tyr Lys Asp Lys Ala Thr Phe Gly Cys His Asp Gly Tyr Ser Leu
   225                 230                 235                 240
   Asp Gly Pro Glu Glu Ile Glu Cys Thr Lys Leu Gly Asn Trp Ser Ala
                   245                 250                 255
   Met Pro Ser Cys Lys Ala Ser Cys Lys Val Pro Val Lys Lys Ala Thr
               260                 265                 270
   Val Val Tyr Gln Gly Glu Arg Val Lys Ile Gln Glu Lys Phe Lys Asn
           275                 280                 285
   Gly Met Leu His Gly Asp Lys Val Ser Phe Phe Cys Lys Asn Lys Glu
       290                 295                 300
   Lys Lys Cys Ser Tyr Thr Glu Asp Ala Gln Cys Ile Asp Gly Thr Ile
   305 310 315 320
   Glu Val Pro Lys Cys Phe Lys Glu His Ser Ser Leu Ala Phe Trp Lys
   325 330 335
   Thr Asp Ala Ser Asp Val Lys Pro Cys
   340 345
<210> 59
   <211> 694
   <212> PRT
   <213> Homo sapiens
<400> 59
   Met Glu Gly Pro His Leu Pro Asn Lys Lys Lys His Lys Lys Gln Ala
   1 5 10 15
   Val Lys Thr Glu Pro Glu Lys Lys Ser Gln Ser Thr Lys Leu Ser Val
   20 25 30
   Val His Glu Lys Lys Ser Gln Glu Gly Lys Pro Lys Glu His Thr Glu
   35 40 45
   Pro Lys Ser Leu Pro Lys Gln Ala Ser Asp Thr Gly Ser Asn Asp Ala
   50 55 60
   His Asn Lys Lys Ala Val Ser Arg Ser Ala Glu Gln Gln Pro Ser Glu
   65 70 75 80
   Lys Ser Thr Glu Pro Lys Thr Lys Pro Gln Asp Met Ile Ser Ala Gly
   85 90 95
   Gly Glu Ser Val Ala Gly Ile Thr Ala Ile Ser Gly Lys Pro Gly Asp
   100 105 110
   Lys Lys Lys Glu Lys Lys Ser Leu Thr Pro Ala Val Pro Val Glu Ser
   115 120 125
   Lys Pro Asp Lys Pro Ser Gly Lys Ser Gly Met Asp Ala Ala Leu Asp
   130 135 140
   Asp Leu Ile Asp Thr Leu Gly Gly Pro Glu Glu Thr Glu Glu Glu Asn
   145 150 155 160
   Thr Thr Tyr Thr Gly Pro Glu Val Ser Asp Pro Met Ser Ser Thr Tyr
   165 170 175
   Ile Glu Glu Leu Gly Lys Arg Glu Val Thr Ile Pro Pro Lys Tyr Arg
               180                 185                 190
   Glu Leu Leu Ala Lys Lys Glu Gly Ile Thr Gly Pro Pro Ala Asp Ser
           195                 200                 205
   Ser Lys Pro Ile Gly Pro Asp Asp Ala Ile Asp Ala Leu Ser Ser Asp
       210                 215                 220
   Phe Thr Cys Gly Ser Pro Thr Ala Ala Gly Lys Lys Thr Glu Lys Glu
   225                 230                 235                 240
   Glu Ser Thr Glu Val Leu Lys Ala Gln Ser Ala Gly Thr Val Arg Ser
                   245                 250                 255
   Ala Ala Pro Pro Gln Glu Lys Lys Arg Lys Val Glu Lys Asp Thr Met
               260                 265                 270
   Ser Asp Gln Ala Leu Glu Ala Leu Ser Ala Ser Leu Gly Thr Arg Gln
           275                 280                 285
   Ala Glu Pro Glu Leu Asp Leu Arg Ser Ile Lys Glu Val Asp Glu Ala
       290                 295                 300
   Lys Ala Lys Glu Glu Lys Leu Glu Lys Cys Gly Glu Asp Asp Glu Thr
   305                 310                 315                 320
   Ile Pro Ser Glu Tyr Arg Leu Lys Pro Ala Thr Asp Lys Asp Gly Lys
                   325                 330                 335
   Pro Leu Leu Pro Glu Pro Glu Glu Lys Pro Lys Pro Arg Ser Glu Ser
               340                 345                 350
   Glu Leu Ile Asp Glu Leu Ser Glu Asp Phe Asp Arg Ser Glu Cys Lys
           355                 360                 365
   Glu Lys Pro Ser Lys Pro Thr Glu Lys Thr Glu Glu Ser Lys Ala Ala
       370                 375                 380
   Ala Pro Ala Pro Val Ser Glu Ala Val Cys Arg Thr Ser Met Cys Ser
   385                 390                 395                 400
   Ile Gln Ser Ala Pro Pro Glu Pro Ala Thr Leu Lys Gly Thr Val Pro
                   405                 410                 415
   Asp Asp Ala Val Glu Ala Leu Ala Asp Ser Leu Gly Lys Lys Glu Ala
               420                 425                 430
   Asp Pro Glu Asp Gly Lys Pro Val Met Asp Lys Val Lys Glu Lys Ala
           435                 440                 445
   Lys Glu Glu Asp Arg Glu Lys Leu Gly Glu Lys Glu Glu Thr Ile Pro
       450                 455                 460
   Pro Asp Tyr Arg Leu Glu Glu Val Lys Asp Lys Asp Gly Lys Pro Leu
   465                 470                 475                 480
   Leu Pro Lys Glu Ser Lys Glu Gln Leu Pro Pro Met Ser Glu Asp Phe
                   485                 490                 495
   Leu Leu Asp Ala Leu Ser Glu Asp Phe Ser Gly Pro Gln Asn Ala Ser
               500                 505                 510
   Ser Leu Lys Phe Glu Asp Ala Lys Leu Ala Ala Ala Ile Ser Glu Val
           515                 520                 525
   Val Ser Gln Thr Pro Ala Ser Thr Thr Gln Ala Gly Ala Pro Pro Arg
       530                 535                 540
   Asp Thr Ser Gln Ser Asp Lys Asp Leu Asp Asp Ala Leu Asp Lys Leu
   545                 550                 555                 560
   Ser Asp Ser Leu Gly Gln Arg Gln Pro Asp Pro Asp Glu Asn Lys Pro
                   565                 570                 575
   Met Glu Asp Lys Val Lys Glu Lys Ala Lys Ala Glu His Arg Asp Lys
               580                 585                 590
   Leu Gly Glu Arg Asp Asp Thr Ile Pro Pro Glu Tyr Arg His Leu Leu
           595                 600                 605
   Asp Asp Asn Gly Gln Asp Lys Pro Val Lys Pro Pro Thr Lys Lys Ser
       610                 615                 620
   Glu Asp Ser Lys Lys Pro Ala Asp Asp Gln Asp Pro Ile Asp Ala Leu
   625                 630                 635                 640
   Ser Gly Asp Leu Asp Ser Cys Pro Ser Thr Thr Glu Thr Ser Gln Asn
                   645                 650                 655
   Thr Ala Lys Asp Lys Cys Lys Lys Ala Ala Ser Ser Ser Lys Ala Pro
               660                 665                 670
   Lys Asn Gly Gly Lys Ala Lys Asp Ser Ala Lys Thr Thr Glu Glu Thr
   675 680 685
   Ser Lys Pro Lys Asp Asp
   690
<210> 60
   <211> 227
   <212> PRT
   <213> Homo sapiens
<400> 60
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Thr Glu
               20                  25                  30
   Glu Glu Gly Thr Pro Lys Glu Ser Glu Pro Gln Ala Ala Ala Glu Pro
           35                  40                  45
   Ala Glu Ala Lys Glu Gly Lys Glu Lys Pro Asp Gln Asp Ala Glu Gly
       50                  55                  60
   Lys Ala Glu Glu Lys Glu Gly Glu Lys Asp Ala Ala Ala Ala Lys Glu
   65                  70                  75                  80
   Glu Ala Pro Lys Ala Glu Pro Glu Lys Thr Glu Gly Ala Ala Glu Ala
                   85                  90                  95
   Lys Ala Glu Pro Pro Lys Ala Pro Glu Gln Glu Gln Ala Ala Pro Gly
               100                 105                 110
   Pro Ala Ala Gly Gly Glu Ala Pro Lys Ala Ala Glu Ala Ala Ala Ala
           115                 120                 125
   Pro Ala Glu Ser Ala Ala Pro Ala Ala Gly Glu Glu Pro Ser Lys Glu
       130                 135                 140
   Glu Gly Glu Pro Lys Lys Thr Glu Ala Pro Ala Ala Pro Ala Ala Gln
   145                 150                 155                 160
   Glu Thr Lys Ser Asp Gly Ala Pro Ala Ser Asp Ser Lys Pro Gly Ser
                   165                 170                 175
   Ser Glu Ala Ala Pro Ser Ser Lys Glu Thr Pro Ala Ala Thr Glu Ala
               180                 185                 190
   Pro Ser Ser Thr Pro Lys Ala Gln Gly Pro Ala Ala Ser Ala Glu Glu
           195                 200                 205
   Pro Lys Pro Val Glu Ala Pro Ala Ala Asn Ser Asp Gln Thr Val Thr
       210                 215                 220
   Val Lys Glu
   225
<210> 61
   <211> 368
   <212> PRT
   <213> Homo sapiens
<400> 61
   Met Lys Ser Leu Val Leu Leu Leu Cys Leu Ala Gln Leu Trp Gly Cys
   1               5                   10                  15
   His Ser Ala Pro His Gly Pro Gly Leu Ile Tyr Arg Gln Pro Asn Cys
               20                  25                  30
   Asp Asp Pro Glu Thr Glu Glu Ala Ala Leu Val Ala Ile Asp Tyr Ile
           35                  40                  45
   Asn Gln Asn Leu Pro Trp Gly Tyr Lys His Thr Leu Asn Gln Ile Asp
       50                  55                  60
   Glu Val Lys Val Trp Pro Gln Gln Pro Ser Gly Glu Leu Phe Glu Ile
   65                  70                  75                  80
   Glu Ile Asp Thr Leu Glu Thr Thr Cys His Val Leu Asp Pro Thr Pro
                   85                  90                  95
   Val Ala Arg Cys Ser Val Arg Gln Leu Lys Glu His Ala Val Glu Gly
               100                 105                 110
   Asp Cys Asp Phe Gln Leu Leu Lys Leu Asp Gly Lys Phe Ser Val Val
           115                 120                 125
   Tyr Ala Lys Cys Asp Ser Ser Pro Ala Asp Ser Ala Glu Asp Val Arg
       130                 135                 140
   Lys Val Cys Gln Asp Cys Pro Leu Leu Ala Pro Leu Asn Asp Thr Arg
   145                 150                 155                 160
   Val Val His Ala Ala Lys Ala Ala Leu Ala Ala Phe Asn Ala Gln Asn
                   165                 170                 175
   Asn Gly Ser Asn Phe Gln Leu Glu Glu Ile Ser Arg Ala Gln Leu Val
               180                 185                 190
   Pro Leu Pro Pro Ser Thr Tyr Val Glu Phe Thr Val Ser Gly Thr Asp
           195                 200                 205
   Cys Val Ala Lys Glu Ala Thr Glu Ala Ala Lys Cys Asn Leu Leu Ala
       210                 215                 220
   Glu Lys Gln Tyr Gly Phe Cys Lys Ala Thr Leu Ser Glu Lys Leu Gly
   225                 230                 235                 240
   Gly Ala Glu Val Ala Val Thr Cys Met Val Phe Gln Thr Gln Pro Val
                   245                 250                 255
   Ser Ser Gln Pro Gln Pro Glu Gly Ala Asn Glu Ala Val Pro Thr Pro
               260                 265                 270
   Val Val Asp Pro Asp Ala Pro Pro Ser Pro Pro Leu Gly Ala Pro Gly
           275                 280                 285
   Leu Pro Pro Ala Gly Ser Pro Pro Asp Ser His Val Leu Leu Ala Ala
       290                 295                 300
   Pro Pro Gly His Gln Leu His Arg Ala His Tyr Asp Leu Arg His Thr
   305                 310                 315                 320
   Phe Met Gly Val Val Ser Leu Gly Ser Pro Ser Gly Glu Val Ser His
                   325                 330                 335
   Pro Arg Lys Thr Arg Thr Val Val Gln Pro Ser Val Gly Ala Ala Ala
               340                 345                 350
   Gly Pro Val Val Pro Pro Cys Pro Gly Arg Ile Arg His Phe Lys Val
           355                 360                 365
<210> 62
   <211> 417
   <212> PRT
   <213> Homo sapiens
<400> 62
   Met Leu Leu Ser Val Pro Leu Leu Leu Gly Leu Leu Gly Leu Ala Val
   1               5                   10                  15
   Ala Glu Pro Ala Val Tyr Phe Lys Glu Gln Phe Leu Asp Gly Asp Gly
               20                  25                  30
   Trp Thr Ser Arg Trp Ile Glu Ser Lys His Lys Ser Asp Phe Gly Lys
   35 40 45
   Phe Val Leu Ser Ser Gly Lys Phe Tyr Gly Asp Glu Glu Lys Asp Lys
   50 55 60
   Gly Leu Gln Thr Ser Gln Asp Ala Arg Phe Tyr Ala Leu Ser Ala Ser
   65 70 75 80
   Phe Glu Pro Phe Ser Asn Lys Gly Gln Thr Leu Val Val Gln Phe Thr
   85 90 95
   Val Lys His Glu Gln Asn Ile Asp Cys Gly Gly Gly Tyr Val Lys Leu
   100 105 110
   Phe Pro Asn Ser Leu Asp Gln Thr Asp Met His Gly Asp Ser Glu Tyr
   115 120 125
   Asn Ile Met Phe Gly Pro Asp Ile Cys Gly Pro Gly Thr Lys Lys Val
   130 135 140
   His Val Ile Phe Asn Tyr Lys Gly Lys Asn Val Leu Ile Asn Lys Asp
   145 150 155 160
   Ile Arg Cys Lys Asp Asp Glu Phe Thr His Leu Tyr Thr Leu Ile Val
   165 170 175
   Arg Pro Asp Asn Thr Tyr Glu Val Lys Ile Asp Asn Ser Gln Val Glu
   180 185 190
   Ser Gly Ser Leu Glu Asp Asp Trp Asp Phe Leu Pro Pro Lys Lys Ile
   195 200 205
   Lys Asp Pro Asp Ala Ser Lys Pro Glu Asp Trp Asp Glu Arg Ala Lys
   210 215 220
   Ile Asp Asp Pro Thr Asp Ser Lys Pro Glu Asp Trp Asp Lys Pro Glu
   225 230 235 240
   His Ile Pro Asp Pro Asp Ala Lys Lys Pro Glu Asp Trp Asp Glu Glu
   245 250 255
   Met Asp Gly Glu Trp Glu Pro Pro Val Ile Gln Asn Pro Glu Tyr Lys
   260 265 270
   Gly Glu Trp Lys Pro Arg Gln Ile Asp Asn Pro Asp Tyr Lys Gly Thr
   275 280 285
   Trp Ile His Pro Glu Ile Asp Asn Pro Glu Tyr Ser Pro Asp Pro Ser
   290 295 300
   Ile Tyr Ala Tyr Asp Asn Phe Gly Val Leu Gly Leu Asp Leu Trp Gln
   305 310 315 320
   Val Lys Ser Gly Thr Ile Phe Asp Asn Phe Leu Ile Thr Asn Asp Glu
   325 330 335
   Ala Tyr Ala Glu Glu Phe Gly Asn Glu Thr Trp Gly Val Thr Lys Ala
   340 345 350
   Ala Glu Lys Gln Met Lys Asp Lys Gln Asp Glu Glu Gln Arg Leu Lys
   355 360 365
   Glu Glu Glu Glu Asp Lys Lys Arg Lys Glu Glu Glu Glu Ala Glu Asp
   370 375 380
   Lys Glu Asp Asp Glu Asp Lys Asp Glu Asp Glu Glu Asp Glu Glu Asp
   385 390 395 400
   Lys Glu Glu Asp Glu Glu Glu Asp Val Pro Gly Gln Ala Lys Asp Glu
   405 410 415
   Leu
<210> 63
   <211> 714
   <212> PRT
   <213> Homo sapiens
<400> 63
<210> 64
   <211> 191
   <212> PRT
   <213> Homo sapiens
<400> 64
   Met Gln Thr Ile Lys Cys Val Val Val Gly Asp Gly Ala Val Gly Lys
   1 5 10 15
   Thr Cys Leu Leu Ile Ser Tyr Thr Thr Asn Lys Phe Pro Ser Glu Tyr
   20 25 30
   Val Pro Thr Val Phe Asp Asn Tyr Ala Val Thr Val Met Ile Gly Gly
   35 40 45
   Glu Pro Tyr Thr Leu Gly Leu Phe Asp Thr Ala Gly Gln Glu Asp Tyr
   50 55 60
   Asp Arg Leu Arg Pro Leu Ser Tyr Pro Gln Thr Asp Val Phe Leu Val
   65 70 75 80
   Cys Phe Ser Val Val Ser Pro Ser Ser Phe Glu Asn Val Lys Glu Lys
   85 90 95
   Trp Val Pro Glu Ile Thr His His Cys Pro Lys Thr Pro Phe Leu Leu
   100 105 110
   Val Gly Thr Gln Ile Asp Leu Arg Asp Asp Pro Ser Thr Ile Glu Lys
   115 120 125
   Leu Ala Lys Asn Lys Gln Lys Pro Ile Thr Pro Glu Thr Ala Glu Lys
   130 135 140
   Leu Ala Arg Asp Leu Lys Ala Val Lys Tyr Val Glu Cys Ser Ala Leu
   145 150 155 160
   Thr Gln Lys Gly Leu Lys Asn Val Phe Asp Glu Ala Ile Leu Ala Ala
   165 170 175
   Leu Glu Pro Pro Glu Pro Lys Lys Ser Arg Arg Cys Val Leu Leu
   180 185 190
<210> 65
   <211> 1663
   <212> PRT
   <213> Homo sapiens
<400> 65
<210> 66
   <211> 461
   <212> PRT
   <213> Homo sapiens
<400> 66
<210> 67
   <211> 868
   <212> PRT
   <213> Homo sapiens
<400> 67
   Met Ala Thr Phe Ile Ser Val Gln Leu Lys Lys Thr Ser Glu Val Asp
   1 5 10 15
   Leu Ala Lys Pro Leu Val Lys Phe Ile Gln Gln Thr Tyr Pro Ser Gly
   20 25 30
   Gly Glu Glu Gln Ala Gln Tyr Cys Arg Ala Ala Glu Glu Leu Ser Lys
   35 40 45
   Leu Arg Arg Ala Ala Val Gly Arg Pro Leu Asp Lys His Glu Gly Ala
   50 55 60
   Leu Glu Thr Leu Leu Arg Tyr Tyr Asp Gln Ile Cys Ser Ile Glu Pro
   65 70 75 80
   Lys Phe Pro Phe Ser Glu Asn Gln Ile Cys Leu Thr Phe Thr Trp Lys
   85 90 95
   Asp Ala Phe Asp Lys Gly Ser Leu Phe Gly Gly Ser Val Lys Leu Ala
   100 105 110
   Leu Ala Ser Leu Gly Tyr Glu Lys Ser Cys Val Leu Phe Asn Cys Ala
   115 120 125
   Ala Leu Ala Ser Gln Ile Ala Ala Glu Gln Asn Leu Asp Asn Asp Glu
   130 135 140
   Gly Leu Lys Ile Ala Ala Lys His Tyr Gln Phe Ala Ser Gly Ala Phe
   145 150 155 160
   Leu His Ile Lys Glu Thr Val Leu Ser Ala Leu Ser Arg Glu Pro Thr
                   165                 170                 175
   Val Asp Ile Ser Pro Asp Thr Val Gly Thr Leu Ser Leu Ile Met Leu
               180                 185                 190
   Ala Gln Ala Gln Glu Val Phe Phe Leu Lys Ala Thr Arg Asp Lys Met
           195                 200                 205
   Lys Asp Ala Ile Ile Ala Lys Leu Ala Asn Gln Ala Ala Asp Tyr Phe
       210                 215                 220
   Gly Asp Ala Phe Lys Gln Cys Gln Tyr Lys Asp Thr Leu Pro Lys Glu
   225                 230                 235                 240
   Val Phe Pro Val Leu Ala Ala Lys His Cys Ile Met Gln Ala Asn Ala
                   245                 250                 255
   Glu Tyr His Gln Ser Ile Leu Ala Lys Gln Gln Lys Lys Phe Gly Glu
               260                 265                 270
   Glu Ile Ala Arg Leu Gln His Ala Ala Glu Leu Ile Lys Thr Val Ala
           275                 280                 285
   Ser Arg Tyr Asp Glu Tyr Val Asn Val Lys Asp Phe Ser Asp Lys Ile
       290                 295                 300
   Asn Arg Ala Leu Ala Ala Ala Lys Lys Asp Asn Asp Phe Ile Tyr His
   305                 310                 315                 320
   Asp Arg Val Pro Asp Leu Lys Asp Leu Asp Pro Ile Gly Lys Ala Thr
                   325                 330                 335
   Leu Val Lys Ser Thr Pro Val Asn Val Pro Ile Ser Gln Lys Phe Thr
               340                 345                 350
   Asp Leu Phe Glu Lys Met Val Pro Val Ser Val Gln Gln Ser Leu Ala
           355                 360                 365
   Ala Tyr Asn Gln Arg Lys Ala Asp Leu Val Asn Arg Ser Ile Ala Gln
       370                 375                 380
   Met Arg Glu Ala Thr Thr Leu Ala Asn Gly Val Leu Ala Ser Leu Asn
   385                 390                 395                 400
   Leu Pro Ala Ala Ile Glu Asp Val Ser Gly Asp Thr Val Pro Gln Ser
                   405                 410                 415
   Ile Leu Thr Lys Ser Arg Ser Val Ile Glu Gln Gly Gly Ile Gln Thr
   420 425 430
   Val Asp Gln Leu Ile Lys Glu Leu Pro Glu Leu Leu Gln Arg Asn Arg
   435 440 445
   Glu Ile Leu Asp Glu Ser Leu Arg Leu Leu Asp Glu Glu Glu Ala Thr
   450 455 460
   Asp Asn Asp Leu Arg Ala Lys Phe Lys Glu Arg Trp Gln Arg Thr Pro
   465 470 475 480
   Ser Asn Glu Leu Tyr Lys Pro Leu Arg Ala Glu Gly Thr Asn Phe Arg
   485 490 495
   Thr Val Leu Asp Lys Ala Val Gln Ala Asp Gly Gln Val Lys Glu Cys
   500 505 510
   Tyr Gln Ser His Arg Asp Thr Ile Val Leu Leu Cys Lys Pro Glu Pro
   515 520 525
   Glu Leu Asn Ala Ala Ile Pro Ser Ala Asn Pro Ala Lys Thr Met Gln
   530 535 540
   Gly Ser Glu Val Val Asn Val Leu Lys Ser Leu Leu Ser Asn Leu Asp
   545 550 555 560
   Glu Val Lys Lys Glu Arg Glu Gly Leu Glu Asn Asp Leu Lys Ser Val
   565 570 575
   Asn Phe Asp Met Thr Ser Lys Phe Leu Thr Ala Leu Ala Gln Asp Gly
   580 585 590
   Val Ile Asn Glu Glu Ala Leu Ser Val Thr Glu Leu Asp Arg Val Tyr
   595 600 605
   Gly Gly Leu Thr Thr Lys Val Gln Glu Ser Leu Lys Lys Gln Glu Gly
   610 615 620
   Leu Leu Lys Asn Ile Gln Val Ser His Gln Glu Phe Ser Lys Met Lys
   625 630 635 640
   Gln Ser Asn Asn Glu Ala Asn Leu Arg Glu Glu Val Leu Lys Asn Leu
   645 650 655
   Ala Thr Ala Tyr Asp Asn Phe Val Glu Leu Val Ala Asn Leu Lys Glu
                       660                 665                 670
   Gly Thr Lys Phe Tyr Asn Glu Leu Thr Glu Ile Leu Val Arg Phe Gln
           675                 680                 685
   Asn Lys Cys Ser Asp Ile Val Phe Ala Arg Lys Thr Glu Arg Asp Glu
       690                 695                 700
   Leu Leu Lys Asp Leu Gln Gln Ser Ile Ala Arg Glu Pro Ser Ala Pro
   705                 710                 715                 720
   Ser Ile Pro Thr Pro Ala Tyr Gln Ser Ser Pro Ala Gly Gly His Ala
                   725                 730                 735
   Pro Thr Pro Pro Thr Pro Ala Pro Arg Thr Met Pro Pro Thr Lys Pro
               740                 745                 750
   Gln Pro Pro Ala Arg Pro Pro Pro Pro Val Leu Pro Ala Asn Arg Ala
           755                 760                 765
   Pro Ser Ala Thr Ala Pro Ser Pro Val Gly Ala Gly Thr Ala Ala Pro
       770                 775                 780
   Ala Pro Ser Gln Thr Pro Gly Ser Ala Pro Pro Pro Gln Ala Gln Gly
   785                 790                 795                 800
   Pro Pro Tyr Pro Thr Tyr Pro Gly Tyr Pro Gly Tyr Cys Gln Met Pro
                   805                 810                 815
   Met Pro Met Gly Tyr Asn Pro Tyr Ala Tyr Gly Gln Tyr Asn Met Pro
               820                 825                 830
   Tyr Pro Pro Val Tyr His Gln Ser Pro Gly Gln Ala Pro Tyr Pro Gly
           835                 840                 845
   Pro Gln Gln Pro Ser Tyr Pro Phe Pro Gln Pro Pro Gln Gln Ser Tyr
       850                 855                 860
   Tyr Pro Gln Gln
   865
<210> 68
   <211> 94
   <212> PRT
   <213> Homo sapiens
<400> 68
   Met Arg Thr Leu Ala Ile Leu Ala Ala Ile Leu Leu Val Ala Leu Gln
   1 5 10 15
   Ala Gln Ala Glu Pro Leu Gln Ala Arg Ala Asp Glu Val Ala Ala Ala
   20 25 30
   Pro Glu Gln Ile Ala Ala Asp Ile Pro Glu Val Val Val Ser Leu Ala
   35 40 45
   Trp Asp Glu Ser Leu Ala Pro Lys His Pro Gly Ser Arg Lys Asn Met
   50 55 60
   Ala Cys Tyr Cys Arg Ile Pro Ala Cys Ile Ala Gly Glu Arg Arg Tyr
   65 70 75 80
   Gly Thr Cys Ile Tyr Gln Gly Arg Leu Trp Ala Phe Cys Cys
   85 90
<210> 69
   <211> 275
   <212> PRT
   <213> Homo sapiens
<400> 69
   Met Glu Glu Val Arg Glu Gly His Ala Leu Gly Gly Gly Met Glu Ala
   1               5                   10                  15
   Asp Gly Pro Ala Ser Leu Gln Glu Leu Pro Pro Ser Pro Arg Ser Pro
               20                  25                  30
   Ser Pro Pro Pro Ser Pro Pro Pro Leu Pro Ser Pro Pro Ser Leu Pro
           35                  40                  45
   Ser Pro Ala Ala Pro Glu Ala Pro Glu Leu Pro Glu Pro Ala Gln Pro
       50                  55                  60
   Ser Glu Ala His Ala Arg Gln Leu Leu Leu Glu Glu Trp Gly Pro Leu
   65                  70                  75                  80
   Ser Gly Gly Leu Glu Leu Pro Gln Arg Leu Thr Trp Lys Leu Leu Leu
                   85                  90                  95
   Leu Arg Arg Pro Leu Tyr Arg Asn Leu Leu Arg Ser Pro Asn Pro Glu
               100                 105                 110
   Gly Ile Asn Ile Tyr Glu Pro Ala Pro Pro Thr Gly Pro Thr Gln Arg
           115                 120                 125
   Pro Leu Glu Thr Leu Gly Asn Phe Arg Gly Trp Tyr Ile Arg Thr Glu
   130 135 140
   Lys Leu Gln Gln Asn Gln Ser Trp Thr Val Lys Gln Gln Cys Val Asp
   145 150 155 160
   Leu Leu Ala Glu Gly Leu Trp Glu Glu Leu Leu Asp Asp Glu Gln Pro
   165 170 175
   Ala Ile Thr Val Met Asp Trp Phe Glu Asp Ser Arg Leu Asp Ala Cys
   180 185 190
   Val Tyr Glu Leu His Val Trp Leu Leu Ala Ala Asp Arg Arg Thr Val
   195 200 205
   Ile Ala Gln His His Val Ala Pro Arg Thr Ser Gly Arg Gly Pro Pro
   210 215 220
   Gly Arg Trp Val Gln Val Ser His Val Phe Arg His Tyr Gly Pro Gly
   225 230 235 240
   Val Arg Phe Ile His Phe Leu His Lys Ala Lys Asn Arg Met Glu Pro
   245 250 255
   Gly Gly Leu Arg Arg Thr Arg Val Thr Asp Ser Ser Val Ser Val Gln
   260 265 270
   Leu Arg Glu
   275
<210> 70
   <211> 188
   <212> PRT
   <213> Homo sapiens
<400> 70
   Met Ala Asn Ser Gly Cys Lys Asp Val Thr Gly Pro Asp Glu Glu Ser
   1               5                   10                  15
   Phe Leu Tyr Phe Ala Tyr Gly Ser Asn Leu Leu Thr Glu Arg Ile His
               20                  25                  30
   Leu Arg Asn Pro Ser Ala Ala Phe Phe Cys Val Ala Arg Leu Gln Asp
           35                  40                  45
   Phe Lys Leu Asp Phe Gly Asn Ser Gln Gly Lys Thr Ser Gln Thr Trp
       50                  55                  60
   His Gly Gly Ile Ala Thr Ile Phe Gln Ser Pro Gly Asp Glu Val Trp
   65                  70                  75                  80
   Gly Val Val Trp Lys Met Asn Lys Ser Asn Leu Asn Ser Leu Asp Glu
                   85                  90                  95
   Gln Glu Gly Val Lys Ser Gly Met Tyr Val Val Ile Glu Val Lys Val
               100                 105                 110
   Ala Thr Gln Glu Gly Lys Glu Ile Thr Cys Arg Ser Tyr Leu Met Thr
           115                 120                 125
   Asn Tyr Glu Ser Ala Pro Pro Ser Pro Gln Tyr Lys Lys Ile Ile Cys
       130                 135                 140
   Met Gly Ala Lys Glu Asn Gly Leu Pro Leu Glu Tyr Gln Glu Lys Leu
   145                 150                 155                 160
   Lys Ala Ile Glu Pro Asn Asp Tyr Thr Gly Lys Val Ser Glu Glu Ile
                   165                 170                 175
   Glu Asp Ile Ile Lys Lys Gly Glu Thr Gln Thr Leu
               180                 185
<210> 71
   <211> 522
   <212> PRT
   <213> Homo sapiens
<400> 71
   Met Ala Leu Leu Pro Arg Ala Leu Ser Ala Gly Ala Gly Pro Ser Trp
   1               5                   10                  15
   Arg Arg Ala Ala Arg Ala Phe Arg Gly Phe Leu Leu Leu Leu Pro Glu
               20                  25                  30
   Pro Ala Ala Leu Thr Arg Ala Leu Ser Arg Ala Met Ala Cys Arg Gln
           35                  40                  45
   Glu Pro Gln Pro Gln Gly Pro Pro Pro Ala Ala Gly Ala Val Ala Ser
       50                  55                  60
   Tyr Asp Tyr Leu Val Ile Gly Gly Gly Ser Gly Gly Leu Ala Ser Ala
   65                  70                  75                  80
   Arg Arg Ala Ala Glu Leu Gly Ala Arg Ala Ala Val Val Glu Ser His
                   85                  90                  95
   Lys Leu Gly Gly Thr Cys Val Asn Val Gly Cys Val Pro Lys Lys Val
   100 105 110
   Met Trp Asn Thr Ala Val His Ser Glu Phe Met His Asp His Ala Asp
   115 120 125
   Tyr Gly Phe Pro Ser Cys Glu Gly Lys Phe Asn Trp Arg Val Ile Lys
   130 135 140
   Glu Lys Arg Asp Ala Tyr Val Ser Arg Leu Asn Ala Ile Tyr Gln Asn
   145 150 155 160
   Asn Leu Thr Lys Ser His Ile Glu Ile Ile Arg Gly His Ala Ala Phe
   165 170 175
   Thr Ser Asp Pro Lys Pro Thr Ile Glu Val Ser Gly Lys Lys Tyr Thr
   180 185 190
   Ala Pro His Ile Leu Ile Ala Thr Gly Gly Met Pro Ser Thr Pro His
   195 200 205
   Glu Ser Gln Ile Pro Gly Ala Ser Leu Gly Ile Thr Ser Asp Gly Phe
   210 215 220
   Phe Gln Leu Glu Glu Leu Pro Gly Arg Ser Val Ile Val Gly Ala Gly
   225 230 235 240
   Tyr Ile Ala Val Glu Met Ala Gly Ile Leu Ser Ala Leu Gly Ser Lys
   245 250 255
   Thr Ser Leu Met Ile Arg His Asp Lys Val Leu Arg Ser Phe Asp Ser
   260 265 270
   Met Ile Ser Thr Asn Cys Thr Glu Glu Leu Glu Asn Ala Gly Val Glu
   275 280 285
   Val Leu Lys Phe Ser Gln Val Lys Glu Val Lys Lys Thr Leu Ser Gly
   290 295 300
   Leu Glu Val Ser Met Val Thr Ala Val Pro Gly Arg Leu Pro Val Met
   305 310 315 320
   Thr Met Ile Pro Asp Val Asp Cys Leu Leu Trp Ala Ile Gly Arg Val
   325 330 335
   Pro Asn Thr Lys Asp Leu Ser Leu Asn Lys Leu Gly Ile Gln Thr Asp
   340 345 350
   Asp Lys Gly His Ile Ile Val Asp Glu Phe Gln Asn Thr Asn Val Lys
   355 360 365
   Gly Ile Tyr Ala Val Gly Asp Val Cys Gly Lys Ala Leu Leu Thr Pro
   370 375 380
   Val Ala Ile Ala Ala Gly Arg Lys Leu Ala His Arg Leu Phe Glu Tyr
   385 390 395 400
   Lys Glu Asp Ser Lys Leu Asp Tyr Asn Asn Ile Pro Thr Val Val Phe
   405 410 415
   Ser His Pro Pro Ile Gly Thr Val Gly Leu Thr Glu Asp Glu Ala Ile
   420 425 430
   His Lys Tyr Gly Ile Glu Asn Val Lys Thr Tyr Ser Thr Ser Phe Thr
   435 440 445
   Pro Met Tyr His Ala Val Thr Lys Arg Lys Thr Lys Cys Val Met Lys
   450 455 460
   Met Val Cys Ala Asn Lys Glu Glu Lys Val Val Gly Ile His Met Gln
   465 470 475 480
   Gly Leu Gly Cys Asp Glu Met Leu Gln Gly Phe Ala Val Ala Val Lys
   485 490 495
   Met Gly Ala Thr Lys Ala Asp Phe Asp Asn Thr Val Ala Ile His Pro
   500 505 510
   Thr Ser Ser Glu Glu Leu Val Thr Leu Arg
   515 520
<210> 72
   <211> 644
   <212> PRT
   <213> Homo sapiens
<400> 72
<210> 73
   <211> 196
   <212> PRT
   <213> Homo sapiens
<400> 73
<210> 74
   <211> 630
   <212> PRT
   <213> Homo sapiens
<400> 74
   Met Ala Leu Pro Thr Ala Arg Pro Leu Leu Gly Ser Cys Gly Thr Pro
   1 5 10 15
   Ala Leu Gly Ser Leu Leu Phe Leu Leu Phe Ser Leu Gly Trp Val Gln
   20 25 30
   Pro Ser Arg Thr Leu Ala Gly Glu Thr Gly Gln Glu Ala Ala Pro Leu
   35 40 45
   Asp Gly Val Leu Ala Asn Pro Pro Asn Ile Ser Ser Leu Ser Pro Arg
   50 55 60
   Gln Leu Leu Gly Phe Pro Cys Ala Glu Val Ser Gly Leu Ser Thr Glu
   65 70 75 80
   Arg Val Arg Glu Leu Ala Val Ala Leu Ala Gln Lys Asn Val Lys Leu
   85 90 95
   Ser Thr Glu Gln Leu Arg Cys Leu Ala His Arg Leu Ser Glu Pro Pro
   100 105 110
   Glu Asp Leu Asp Ala Leu Pro Leu Asp Leu Leu Leu Phe Leu Asn Pro
   115 120 125
   Asp Ala Phe Ser Gly Pro Gln Ala Cys Thr Arg Phe Phe Ser Arg Ile
   130 135 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385 390 395 400
   Val Asn Lys Gly His Glu Met Ser Pro Gln Ala Pro Arg Arg Pro Leu
   405 410 415
   Pro Gln Val Ala Thr Leu Ile Asp Arg Phe Val Lys Gly Arg Gly Gln
   420 425 430
   Leu Asp Lys Asp Thr Leu Asp Thr Leu Thr Ala Phe Tyr Pro Gly Tyr
   435 440 445
   Leu Cys Ser Leu Ser Pro Glu Glu Leu Ser Ser Val Pro Pro Ser Ser
   450 455 460
   Ile Trp Ala Val Arg Pro Gln Asp Leu Asp Thr Cys Asp Pro Arg Gln
   465 470 475 480
   Leu Asp Val Leu Tyr Pro Lys Ala Arg Leu Ala Phe Gln Asn Met Asn
   485 490 495
   Gly Ser Glu Tyr Phe Val Lys Ile Gln Ser Phe Leu Gly Gly Ala Pro
   500 505 510
   Thr Glu Asp Leu Lys Ala Leu Ser Gln Gln Asn Val Ser Met Asp Leu
   515 520 525
   Ala Thr Phe Met Lys Leu Arg Thr Asp Ala Val Leu Pro Leu Thr Val
   530 535 540
   Ala Glu Val Gln Lys Leu Leu Gly Pro His Val Glu Gly Leu Lys Ala
   545 550 555 560
   Glu Glu Arg His Arg Pro Val Arg Asp Trp Ile Leu Arg Gln Arg Gln
   565 570 575
   Asp Asp Leu Asp Thr Leu Gly Leu Gly Leu Gln Gly Gly Ile Pro Asn
   580 585 590
   Gly Tyr Leu Val Leu Asp Leu Ser Met Gln Glu Ala Leu Ser Gly Thr
   595 600 605
   Pro Cys Leu Leu Gly Pro Gly Pro Val Leu Thr Val Leu Ala Leu Leu
   610 615 620
   Leu Ala Ser Thr Leu Ala
   625 630
<210> 75
   <211> 576
   <212> PRT
   <213> Homo sapiens
<400> 75
   Met Ala Gln Gly Val Leu Trp Ile Leu Leu Gly Leu Leu Leu Trp Ser
   1               5                   10                  15
   Asp Pro Gly Thr Ala Ser Leu Pro Leu Leu Met Asp Ser Val Ile Gln
               20                  25                  30
   Ala Leu Ala Glu Leu Glu Gln Lys Val Pro Ala Ala Lys Thr Arg His
           35                  40                  45
   Thr Ala Ser Ala Trp Leu Met Ser Ala Pro Asn Ser Gly Pro His Asn
       50                  55                  60
   Arg Leu Tyr His Phe Leu Leu Gly Ala Trp Ser Leu Asn Ala Thr Glu
   65                  70                  75                  80
   Leu Asp Pro Cys Pro Leu Ser Pro Glu Leu Leu Gly Leu Thr Lys Glu
                   85                  90                  95
   Val Ala Arg His Asp Val Arg Glu Gly Lys Glu Tyr Gly Val Val Leu
               100                 105                 110
   Ala Pro Asp Gly Ser Thr Val Ala Val Glu Pro Leu Leu Ala Gly Leu
           115                 120                 125
   Glu Ala Gly Leu Gln Gly Arg Arg Val Ile Asn Leu Pro Leu Asp Ser
       130                 135                 140
   Met Ala Ala Pro Trp Glu Thr Gly Asp Thr Phe Pro Asp Val Val Ala
   145                 150                 155                 160
   Ile Ala Pro Asp Val Arg Ala Thr Ser Ser Pro Gly Leu Arg Asp Gly
                   165                 170                 175
   Ser Pro Asp Val Thr Thr Ala Asp Ile Gly Ala Asn Thr Pro Asp Ala
               180                 185                 190
   Thr Lys Gly Cys Pro Asp Val Gln Ala Ser Leu Pro Asp Ala Lys Ala
           195                 200                 205
   Lys Ser Pro Pro Thr Met Val Asp Ser Leu Leu Ala Val Thr Leu Ala
       210                 215                 220
   Gly Asn Leu Gly Leu Thr Phe Leu Arg Gly Ser Gln Thr Gln Ser His
   225                 230                 235                 240
   Pro Asp Leu Gly Thr Glu Gly Cys Trp Asp Gln Leu Ser Ala Pro Arg
                   245                 250                 255
   Thr Phe Thr Leu Leu Asp Pro Lys Ala Ser Leu Leu Thr Met Ala Phe
               260                 265                 270
   Leu Asn Gly Ala Leu Asp Gly Val Ile Leu Gly Asp Tyr Leu Ser Arg
           275                 280                 285
   Thr Pro Glu Pro Arg Pro Ser Leu Ser His Leu Leu Ser Gln Tyr Tyr
       290                 295                 300
   Gly Ala Gly Val Ala Arg Asp Pro Gly Phe Arg Ser Asn Phe Arg Arg
   305                 310                 315                 320
   Gln Asn Gly Ala Ala Leu Thr Ser Ala Ser Ile Leu Ala Gln Gln Val
                   325                 330                 335
   Trp Gly Thr Leu Val Leu Leu Gln Arg Leu Glu Pro Val His Leu Gln
               340                 345                 350
   Leu Gln Cys Met Ser Gln Glu Gln Leu Ala Gln Val Ala Ala Asn Ala
           355                 360                 365
   Thr Lys Glu Phe Thr Glu Ala Phe Leu Gly Cys Pro Ala Ile His Pro
       370                 375                 380
   Arg Cys Arg Trp Gly Ala Ala Pro Tyr Arg Gly Arg Pro Lys Leu Leu
   385                 390                 395                 400
   Gln Leu Pro Leu Gly Phe Leu Tyr Val His His Thr Tyr Val Pro Ala
                   405                 410                 415
   Pro Pro Cys Thr Asp Phe Thr Arg Cys Ala Ala Asn Met Arg Ser Met
               420                 425                 430
   Gln Arg Tyr His Gln Asp Thr Gln Gly Trp Gly Asp Ile Gly Tyr Ser
           435                 440                 445
   Phe Val Val Gly Ser Asp Gly Tyr Val Tyr Glu Gly Arg Gly Trp His
       450                 455                 460
   Trp Val Gly Ala His Thr Leu Gly His Asn Ser Arg Gly Phe Gly Val
   465                 470                 475                 480
   Ala Ile Val Gly Asn Tyr Thr Ala Ala Leu Pro Thr Glu Ala Ala Leu
   485 490 495
   Arg Thr Val Arg Asp Thr Leu Pro Ser Cys Ala Val Arg Ala Gly Leu
   500 505 510
   Leu Arg Pro Asp Tyr Ala Leu Leu Gly His Arg Gln Leu Val Arg Thr
   515 520 525
   Asp Cys Pro Gly Asp Ala Leu Phe Asp Leu Leu Arg Thr Trp Pro His
   530 535 540
   Phe Thr Ala Thr Val Lys Pro Arg Pro Ala Arg Ser Val Ser Lys Arg
   545 550 555 560
   Ser Arg Arg Glu Pro Pro Pro Arg Thr Leu Pro Ala Thr Asp Leu Gln
   565 570 575
<210> 76
   <211> 538
   <212> PRT
   <213> Homo sapiens
<400> 76
   Met Ala Ala Glu Gln Asp Pro Glu Ala Arg Ala Ala Ala Arg Pro Leu
   1 5 10 15
   Leu Thr Asp Leu Tyr Gln Ala Thr Met Ala Leu Gly Tyr Trp Arg Ala
   20 25 30
   Gly Arg Ala Arg Asp Ala Ala Glu Phe Glu Leu Phe Phe Arg Arg Cys
   35 40 45
   Pro Phe Gly Gly Ala Phe Ala Leu Ala Ala Gly Leu Arg Asp Cys Val
   50 55 60
   Arg Phe Leu Arg Ala Phe Arg Leu Arg Asp Ala Asp Val Gln Phe Leu
   65 70 75 80
   Ala Ser Val Leu Pro Pro Asp Thr Asp Pro Ala Phe Phe Glu His Leu
   85 90 95
   Arg Ala Leu Asp Cys Ser Glu Val Thr Val Arg Ala Leu Pro Glu Gly
   100 105 110
   Ser Leu Ala Phe Pro Gly Val Pro Leu Leu Gln Val Ser Gly Pro Leu
   115 120 125
   Leu Val Val Gln Leu Leu Glu Thr Pro Leu Leu Cys Leu Val Ser Tyr
       130                 135                 140
   Ala Ser Leu Val Ala Thr Asn Ala Ala Arg Leu Arg Leu Ile Ala Gly
   145                 150                 155                 160
   Pro Glu Lys Arg Leu Leu Glu Met Gly Leu Arg Arg Ala Gln Gly Pro
                   165                 170                 175
   Asp Gly Gly Leu Thr Ala Ser Thr Tyr Ser Tyr Leu Gly Gly Phe Asp
               180                 185                 190
   Ser Ser Ser Asn Val Leu Ala Gly Gln Leu Arg Gly Val Pro Val Ala
           195                 200                 205
   Gly Thr Leu Ala His Ser Phe Val Thr Ser Phe Ser Gly Ser Glu Val
       210                 215                 220
   Pro Pro Asp Pro Met Leu Ala Pro Ala Ala Gly Glu Gly Pro Gly Val
   225                 230                 235                 240
   Asp Leu Ala Ala Lys Ala Gln Val Trp Leu Glu Gln Val Cys Ala His
                   245                 250                 255
   Leu Gly Leu Gly Val Gln Glu Pro His Pro Gly Glu Arg Ala Ala Phe
               260                 265                 270
   Val Ala Tyr Ala Leu Ala Phe Pro Arg Ala Phe Gln Gly Leu Leu Asp
           275                 280                 285
   Thr Tyr Ser Val Trp Arg Ser Gly Leu Pro Asn Phe Leu Ala Val Ala
       290                 295                 300
   Leu Ala Leu Gly Glu Leu Gly Tyr Arg Ala Val Gly Val Arg Leu Asp
   305                 310                 315                 320
   Ser Gly Asp Leu Leu Gln Gln Ala Gln Glu Ile Arg Lys Val Phe Arg
                   325                 330                 335
   Ala Ala Ala Ala Gln Phe Gln Val Pro Trp Leu Glu Ser Val Leu Ile
               340                 345                 350
   Val Val Ser Asn Asn Ile Asp Glu Glu Ala Leu Ala Arg Leu Ala Gln
           355                 360                 365
   Glu Gly Ser Glu Val Asn Val Ile Gly Ile Gly Thr Ser Val Val Thr
       370                 375                 380
   Cys Pro Gln Gln Pro Ser Leu Gly Gly Val Tyr Lys Leu Val Ala Val
   385                 390                 395                 400
   Gly Gly Gln Pro Arg Met Lys Leu Thr Glu Asp Pro Glu Lys Gln Thr
                   405                 410                 415
   Leu Pro Gly Ser Lys Ala Ala Phe Arg Leu Leu Gly Ser Asp Gly Ser
               420                 425                 430
   Pro Leu Met Asp Met Leu Gln Leu Ala Glu Glu Pro Val Pro Gln Ala
           435                 440                 445
   Gly Gln Glu Leu Arg Val Trp Pro Pro Gly Ala Gln Glu Pro Cys Thr
       450                 455                 460
   Val Arg Pro Ala Gln Val Glu Pro Leu Leu Arg Leu Cys Leu Gln Gln
   465                 470                 475                 480
   Gly Gln Leu Cys Glu Pro Leu Pro Ser Leu Ala Glu Ser Arg Ala Leu
                   485                 490                 495
   Ala Gln Leu Ser Leu Ser Arg Leu Ser Pro Glu His Arg Arg Leu Arg
               500                 505                 510
   Ser Pro Ala Gln Tyr Gln Val Val Leu Ser Glu Arg Leu Gln Ala Leu
           515                 520                 525
   Val Asn Ser Leu Cys Ala Gly Gln Ser Pro
       530                 535
<210> 77
   <211> 911
   <212> PRT
   <213> Homo sapiens
<400> 77
   Met Asp Gly Ala Met Gly Pro Arg Gly Leu Leu Leu Cys Met Tyr Leu
   1               5                   10                  15
   Val Ser Leu Leu Ile Leu Gln Ala Met Pro Ala Leu Gly Ser Ala Thr
               20                  25                  30
   Gly Arg Ser Lys Ser Ser Glu Lys Arg Gln Ala Val Asp Thr Ala Val
           35                  40                  45
   Asp Gly Val Phe Ile Arg Ser Leu Lys Val Asn Cys Lys Val Thr Ser
       50                  55                  60
   Arg Phe Ala His Tyr Val Val Thr Ser Gln Val Val Asn Thr Ala Asn
   65                  70                  75                  80
   Glu Ala Arg Glu Val Ala Phe Asp Leu Glu Ile Pro Lys Thr Ala Phe
                   85                  90                  95
   Ile Ser Asp Phe Ala Val Thr Ala Asp Gly Asn Ala Phe Ile Gly Asp
               100                 105                 110
   Ile Lys Asp Lys Val Thr Ala Trp Lys Gln Tyr Arg Lys Ala Ala Ile
           115                 120                 125
   Ser Gly Glu Asn Ala Gly Leu Val Arg Ala Ser Gly Arg Thr Met Glu
       130                 135                 140
   Gln Phe Thr Ile His Leu Thr Val Asn Pro Gln Ser Lys Val Thr Phe
   145                 150                 155                 160
   Gln Leu Thr Tyr Glu Glu Val Leu Lys Arg Asn His Met Gln Tyr Glu
                   165                 170                 175
   Ile Val Ile Lys Val Lys Pro Lys Gln Leu Val His His Phe Glu Ile
               180                 185                 190
   Asp Val Asp Ile Phe Glu Pro Gln Gly Ile Ser Lys Leu Asp Ala Gln
           195                 200                 205
   Ala Ser Phe Leu Pro Lys Glu Leu Ala Ala Gln Thr Ile Lys Lys Ser
       210                 215                 220
   Phe Ser Gly Lys Lys Gly His Val Leu Phe Arg Pro Thr Val Ser Gln
   225                 230                 235                 240
   Gln Gln Ser Cys Pro Thr Cys Ser Thr Ser Leu Leu Asn Gly His Phe
                   245                 250                 255
   Lys Val Thr Tyr Asp Val Ser Arg Asp Lys Ile Cys Asp Leu Leu Val
               260                 265                 270
   Ala Asn Asn His Phe Ala His Phe Phe Ala Pro Gln Asn Leu Thr Asn
           275                 280                 285
   Met Asn Lys Asn Val Val Phe Val Ile Asp Ile Ser Gly Ser Met Arg
       290                 295                 300
   Gly Gln Lys Val Lys Gln Thr Lys Glu Ala Leu Leu Lys Ile Leu Gly
   305 310 315 320
   Asp Met Gln Pro Gly Asp Tyr Phe Asp Leu Val Leu Phe Gly Thr Arg
   325 330 335
   Val Gln Ser Trp Lys Gly Ser Leu Val Gln Ala Ser Glu Ala Asn Leu
   340 345 350
   Gln Ala Ala Gln Asp Phe Val Arg Gly Phe Ser Leu Asp Glu Ala Thr
   355 360 365
   Asn Leu Asn Gly Gly Leu Leu Arg Gly Ile Glu Ile Leu Asn Gln Val
   370 375 380
   Gln Glu Ser Leu Pro Glu Leu Ser Asn His Ala Ser Ile Leu Ile Met
   385 390 395 400
   Leu Thr Asp Gly Asp Pro Thr Glu Gly Val Thr Asp Arg Ser Gln Ile
   405 410 415
   Leu Lys Asn Val Arg Asn Ala Ile Arg Gly Arg Phe Pro Leu Tyr Asn
   420 425 430
   Leu Gly Phe Gly His Asn Val Asp Phe Asn Phe Leu Glu Val Met Ser
   435 440 445
   Met Glu Asn Asn Gly Arg Ala Gln Arg Ile Tyr Glu Asp His Asp Ala
   450 455 460
   Thr Gln Gln Leu Gln Gly Phe Tyr Ser Gln Val Ala Lys Pro Leu Leu
   465 470 475 480
   Val Asp Val Asp Leu Gln Tyr Pro Gln Asp Ala Val Leu Ala Leu Thr
   485 490 495
   Gln Asn His His Lys Gln Tyr Tyr Glu Gly Ser Glu Ile Val Val Ala
   500 505 510
   Gly Arg Ile Ala Asp Asn Lys Gln Ser Ser Phe Lys Ala Asp Val Gln
   515 520 525
   Ala His Gly Glu Gly Gln Glu Phe Ser Ile Thr Cys Leu Val Asp Glu
   530 535 540
   Glu Glu Met Lys Lys Leu Leu Arg Glu Arg Gly His Met Leu Glu Asn
   545 550 555 560
   His Val Glu Arg Leu Trp Ala Tyr Leu Thr Ile Gln Glu Leu Leu Ala
                   565                 570                 575
   Lys Arg Met Lys Val Asp Arg Glu Glu Arg Ala Asn Leu Ser Ser Gln
               580                 585                 590
   Ala Leu Gln Met Ser Leu Asp Tyr Gly Phe Val Thr Pro Leu Thr Ser
           595                 600                 605
   Met Ser Ile Arg Gly Met Ala Asp Gln Asp Gly Leu Lys Pro Thr Ile
       610                 615                 620
   Asp Lys Pro Ser Glu Asp Ser Pro Pro Leu Glu Met Leu Gly Pro Arg
   625                 630                 635                 640
   Arg Thr Phe Val Leu Ser Ala Leu Gln Pro Ser Pro Thr His Ser Ser
                   645                 650                 655
   Ser Asn Thr Gln Arg Leu Pro Asp Arg Val Thr Gly Val Asp Thr Asp
               660                 665                 670
   Pro His Phe Ile Ile His Val Pro Gln Lys Glu Asp Thr Leu Cys Phe
           675                 680                 685
   Asn Ile Asn Glu Glu Pro Gly Val Ile Leu Ser Leu Val Gln Asp Pro
       690                 695                 700
   Asn Thr Gly Phe Ser Val Asn Gly Gln Leu Ile Gly Asn Lys Ala Arg
   705                 710                 715                 720
   Ser Pro Gly Gln His Asp Gly Thr Tyr Phe Gly Arg Leu Gly Ile Ala
                   725                 730                 735
   Asn Pro Ala Thr Asp Phe Gln Leu Glu Val Thr Pro Gln Asn Ile Thr
               740                 745                 750
   Leu Asn Pro Gly Phe Gly Gly Pro Val Phe Ser Trp Arg Asp Gln Ala
           755                 760                 765
   Val Leu Arg Gln Asp Gly Val Val Val Thr Ile Asn Lys Lys Arg Asn
       770                 775                 780
   Leu Val Val Ser Val Asp Asp Gly Gly Thr Phe Glu Val Val Leu His
   785                 790                 795                 800
   Arg Val Trp Lys Gly Ser Ser Val His Gln Asp Phe Leu Gly Phe Tyr
                   805                 810                 815
   Val Leu Asp Ser His Arg Met Ser Ala Arg Thr His Gly Leu Leu Gly
   820 825 830
   Gln Phe Phe His Pro Ile Gly Phe Glu Val Ser Asp Ile His Pro Gly
   835 840 845
   Ser Asp Pro Thr Lys Pro Asp Ala Thr Met Val Val Arg Asn Arg Arg
   850 855 860
   Leu Thr Val Thr Arg Gly Leu Gln Lys Asp Tyr Ser Lys Asp Pro Trp
   865 870 875 880
   His Gly Ala Glu Val Ser Cys Trp Phe Ile His Asn Asn Gly Ala Gly
   885 890 895
   Leu Ile Asp Gly Ala Tyr Thr Asp Tyr Ile Val Pro Asp Ile Phe
   900 905 910
<210> 78
   <211> 306
   <212> PRT
   <213> Homo sapiens
<400> 78
<210> 79
   <211> 222
   <212> PRT
   <213> Homo sapiens
<400> 79
<210> 80
   <211> 169
   <212> PRT
   <213> Homo sapiens
<400> 80
   Met Ser Ser Tyr Gln Gln Lys Gln Thr Phe Thr Pro Pro Pro Gln Leu
   1 5 10 15
   Gln Gln Gln Gln Val Lys Gln Pro Ser Gln Pro Pro Pro Gln Glu Ile
   20 25 30
   Phe Val Pro Thr Thr Lys Glu Pro Cys His Ser Lys Val Pro Gln Pro
   35 40 45
   Gly Asn Thr Lys Ile Pro Glu Pro Gly Cys Thr Lys Val Pro Glu Pro
   50 55 60
   Gly Cys Thr Lys Val Pro Glu Pro Gly Cys Thr Lys Val Pro Glu Pro
   65 70 75 80
   Gly Cys Thr Lys Val Pro Glu Pro Gly Cys Thr Lys Val Pro Glu Pro
   85 90 95
   Gly Cys Thr Lys Val Pro Glu Pro Gly Tyr Thr Lys Val Pro Glu Pro
   100 105 110
   Gly Ser Ile Lys Val Pro Asp Gln Gly Phe Ile Lys Phe Pro Glu Pro
   115 120 125
   Gly Ala Ile Lys Val Pro Glu Gln Gly Tyr Thr Lys Val Pro Val Pro
   130 135 140
   Gly Tyr Thr Lys Leu Pro Glu Pro Cys Pro Ser Thr Val Thr Pro Gly
   145 150 155 160
   Pro Ala Gln Gln Lys Thr Lys Gln Lys
   165
<210> 81
   <211> 550
   <212> PRT
   <213> Homo sapiens
<400> 81
   Met Trp Lys Ala Ser Ala Gly His Ala Val Ser Ile Ala Gln Asp Asp
   1 5 10 15
   Ala Gly Ala Asp Asp Trp Glu Thr Asp Pro Asp Phe Val Asn Asp Val
   20 25 30
   Ser Glu Lys Glu Gln Arg Trp Gly Ala Lys Thr Val Gln Gly Ser Gly
   35 40 45
   His Gln Glu His Ile Asn Ile His Lys Leu Arg Glu Asn Val Phe Gln
   50 55 60
   Glu His Gln Thr Leu Lys Glu Lys Glu Leu Glu Thr Gly Pro Lys Ala
   65 70 75 80
   Ser His Gly Tyr Gly Gly Lys Phe Gly Val Glu Gln Asp Arg Met Asp
                   85                  90                  95
   Lys Ser Ala Val Gly His Glu Tyr Gln Ser Lys Leu Ser Lys His Cys
               100                 105                 110
   Ser Gln Val Asp Ser Val Arg Gly Phe Gly Gly Lys Phe Gly Val Gln
           115                 120                 125
   Met Asp Arg Val Asp Gln Ser Ala Val Gly Phe Glu Tyr Gln Gly Lys
       130                 135                 140
   Thr Glu Lys His Ala Ser Gln Lys Asp Tyr Ser Ser Gly Phe Gly Gly
   145                 150                 155                 160
   Lys Tyr Gly Val Gln Ala Asp Arg Val Asp Lys Ser Ala Val Gly Phe
                   165                 170                 175
   Asp Tyr Gln Gly Lys Thr Glu Lys His Glu Ser Gln Arg Asp Tyr Ser
               180                 185                 190
   Lys Gly Phe Gly Gly Lys Tyr Gly Ile Asp Lys Asp Lys Val Asp Lys
           195                 200                 205
   Ser Ala Val Gly Phe Glu Tyr Gln Gly Lys Thr Glu Lys His Glu Ser
       210                 215                 220
   Gln Lys Asp Tyr Val Lys Gly Phe Gly Gly Lys Phe Gly Val Gln Thr
   225                 230                 235                 240
   Asp Arg Gln Asp Lys Cys Ala Leu Gly Trp Asp His Gln Glu Lys Leu
                   245                 250                 255
   Gln Leu His Glu Ser Gln Lys Asp Tyr Lys Thr Gly Phe Gly Gly Lys
               260                 265                 270
   Phe Gly Val Gln Ser Glu Arg Gln Asp Ser Ala Ala Val Gly Phe Asp
           275                 280                 285
   Tyr Lys Glu Lys Leu Ala Lys His Glu Ser Gln Gln Asp Tyr Ser Lys
       290                 295                 300
   Gly Phe Gly Gly Lys Tyr Gly Val Gln Lys Asp Arg Met Asp Lys Asn
   305                 310                 315                 320
   Ala Ser Thr Phe Glu Asp Val Thr Gln Val Ser Ser Ala Tyr Gln Lys
                   325                 330                 335
   Thr Val Pro Val Glu Ala Val Thr Ser Lys Thr Ser Asn Ile Arg Ala
               340                 345                 350
   Asn Phe Glu Asn Leu Ala Lys Glu Lys Glu Gln Glu Asp Arg Arg Lys
           355                 360                 365
   Ala Glu Ala Glu Arg Ala Gln Arg Met Ala Lys Glu Arg Gln Glu Gln
       370                 375                 380
   Glu Glu Ala Arg Arg Lys Leu Glu Glu Gln Ala Arg Ala Lys Thr Gln
   385                 390                 395                 400
   Thr Pro Pro Val Ser Pro Ala Pro Gln Pro Thr Glu Glu Arg Leu Pro
                   405                 410                 415
   Ser Ser Pro Val Tyr Glu Asp Ala Ala Ser Phe Lys Ala Glu Leu Ser
               420                 425                 430
   Tyr Arg Gly Pro Val Ser Gly Thr Glu Pro Glu Pro Val Tyr Ser Met
           435                 440                 445
   Glu Ala Ala Asp Tyr Arg Glu Ala Ser Ser Gln Gln Gly Leu Ala Tyr
       450                 455                 460
   Ala Thr Glu Ala Val Tyr Glu Ser Ala Glu Ala Pro Gly His Tyr Pro
   465                 470                 475                 480
   Ala Glu Asp Ser Thr Tyr Asp Glu Tyr Glu Asn Asp Leu Gly Ile Thr
                   485                 490                 495
   Ala Val Ala Leu Tyr Asp Tyr Gln Ala Ala Gly Asp Asp Glu Ile Ser
               500                 505                 510
   Phe Asp Pro Asp Asp Ile Ile Thr Asn Ile Glu Met Ile Asp Asp Gly
           515                 520                 525
   Trp Trp Arg Gly Val Cys Lys Gly Arg Tyr Gly Leu Phe Pro Ala Asn
       530                 535                 540
   Tyr Val Glu Leu Arg Gln
   545                 550
<210> 82
   <211> 445
   <212> PRT
   <213> Homo sapiens
<400> 82
   Met Arg Glu Ile Val His Leu Gln Ala Gly Gln Cys Gly Asn Gln Ile
   1 5 10 15
   Gly Ala Lys Phe Trp Glu Val Ile Ser Asp Glu His Gly Ile Asp Pro
   20 25 30
   Thr Gly Thr Tyr His Gly Asp Ser Asp Leu Gln Leu Glu Arg Ile Asn
   35 40 45
   Val Tyr Tyr Asn Glu Ala Thr Gly Gly Lys Tyr Val Pro Arg Ala Val
   50 55 60
   Leu Val Asp Leu Glu Pro Gly Thr Met Asp Ser Val Arg Ser Gly Pro
   65 70 75 80
   Phe Gly Gln Ile Phe Arg Pro Asp Asn Phe Val Phe Gly Gln Ser Gly
   85 90 95
   Ala Gly Asn Asn Trp Ala Lys Gly His Tyr Thr Glu Gly Ala Glu Leu
   100 105 110
   Val Asp Ser Val Leu Asp Val Val Arg Lys Glu Ala Glu Ser Cys Asp
   115 120 125
   Cys Leu Gln Gly Phe Gln Leu Thr His Ser Leu Gly Gly Gly Thr Gly
   130 135 140
   Ser Gly Met Gly Thr Leu Leu Ile Ser Lys Ile Arg Glu Glu Tyr Pro
   145 150 155 160
   Asp Arg Ile Met Asn Thr Phe Ser Val Val Pro Ser Pro Lys Val Ser
   165 170 175
   Asp Thr Val Val Glu Pro Tyr Asn Ala Thr Leu Ser Val His Gln Leu
   180 185 190
   Val Glu Asn Thr Asp Glu Thr Tyr Cys Ile Asp Asn Glu Ala Leu Tyr
   195 200 205
   Asp Ile Cys Phe Arg Thr Leu Lys Leu Thr Thr Pro Thr Tyr Gly Asp
   210 215 220
   Leu Asn His Leu Val Ser Ala Thr Met Ser Gly Val Thr Thr Cys Leu
   225 230 235 240
   Arg Phe Pro Gly Gln Leu Asn Ala Asp Leu Arg Lys Leu Ala Val Asn
   245 250 255
   Met Val Pro Phe Pro Arg Leu His Phe Phe Met Pro Gly Phe Ala Pro
   260 265 270
   Leu Thr Ser Arg Gly Ser Gln Gln Tyr Arg Ala Leu Thr Val Pro Glu
   275 280 285
   Leu Thr Gln Gln Met Phe Asp Ala Lys Asn Met Met Ala Ala Cys Asp
   290 295 300
   Pro Arg His Gly Arg Tyr Leu Thr Val Ala Ala Val Phe Arg Gly Arg
   305 310 315 320
   Met Ser Met Lys Glu Val Asp Glu Gln Met Leu Asn Val Gln Asn Lys
   325 330 335
   Asn Ser Ser Tyr Phe Val Glu Trp Ile Pro Asn Asn Val Lys Thr Ala
   340 345 350
   Val Cys Asp Ile Pro Pro Arg Gly Leu Lys Met Ser Ala Thr Phe Ile
   355 360 365
   Gly Asn Ser Thr Ala Ile Gln Glu Leu Phe Lys Arg Ile Ser Glu Gln
   370 375 380
   Phe Thr Ala Met Phe Arg Arg Lys Ala Phe Leu His Trp Tyr Thr Gly
   385 390 395 400
   Glu Gly Met Asp Glu Met Glu Phe Thr Glu Ala Glu Ser Asn Met Asn
   405 410 415
   Asp Leu Val Ser Glu Tyr Gln Gln Tyr Gln Asp Ala Thr Ala Glu Glu
   420 425 430
   Glu Gly Glu Phe Glu Glu Glu Ala Glu Glu Glu Val Ala
   435 440 445
<210> 83
   <211> 227
   <212> PRT
   <213> Homo sapiens
<400> 83
   Met Ala Gly Ile Thr Thr Ile Glu Ala Val Lys Arg Lys Ile Gln Val
   1               5                   10                  15
   Leu Gln Gln Gln Ala Asp Asp Ala Glu Glu Arg Ala Glu Arg Leu Gln
               20                  25                  30
   Arg Glu Val Glu Gly Glu Arg Arg Ala Arg Glu Gln Ala Glu Ala Glu
           35                  40                  45
   Val Ala Ser Leu Asn Arg Arg Ile Gln Leu Val Glu Glu Glu Leu Asp
       50                  55                  60
   Arg Ala Gln Glu Arg Leu Ala Thr Ala Leu Gln Lys Leu Glu Glu Ala
   65                  70                  75                  80
   Glu Lys Ala Ala Asp Glu Ser Glu Arg Gly Met Lys Val Ile Glu Asn
                   85                  90                  95
   Arg Ala Leu Lys Asp Glu Glu Lys Met Glu Leu Gln Glu Ile Gln Leu
               100                 105                 110
   Lys Glu Ala Lys His Ile Ala Glu Glu Ala Asp Arg Lys Tyr Glu Glu
           115                 120                 125
   Val Ala Arg Lys Leu Val Ile Ile Glu Gly Asp Leu Glu Arg Thr Glu
       130                 135                 140
   Glu Arg Ala Glu Leu Ala Glu Ser Arg Cys Arg Glu Met Asp Glu Gln
   145                 150                 155                 160
   Ile Arg Leu Met Asp Gln Asn Leu Lys Cys Leu Ser Ala Ala Glu Glu
                   165                 170                 175
   Lys Ala Glu Thr Arg Ala Glu Phe Ala Glu Arg Ser Val Ala Lys Leu
               180                 185                 190
   Glu Lys Thr Ile Asp Asp Leu Glu Asp Lys Leu Lys Cys Thr Lys Glu
           195                 200                 205
   Glu His Leu Cys Thr Gln Arg Met Leu Asp Gln Thr Leu Leu Asp Leu
       210                 215                 220
   Asn Glu Met
   225
<210> 84
   <211> 698
   <212> PRT
   <213> Homo sapiens
<400> 84
   Met Arg Leu Ala Val Gly Ala Leu Leu Val Cys Ala Val Leu Gly Leu
   1               5                   10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65                  70                  75                  80
   Leu Asp Ala Gly Leu Val Tyr Asp Ala Tyr Leu Ala Pro Asn Asn Leu
                   85                  90                  95
   Lys Pro Val Val Ala Glu Phe Tyr Gly Ser Lys Glu Asp Pro Gln Thr
               100                 105                 110
   Phe Tyr Tyr Ala Val Ala Val Val Lys Lys Asp Ser Gly Phe Gln Met
           115                 120                 125
   Asn Gln Leu Arg Gly Lys Lys Ser Cys His Thr Gly Leu Gly Arg Ser
       130                 135                 140
   Ala Gly Trp Asn Ile Pro Ile Gly Leu Leu Tyr Cys Asp Leu Pro Glu
   145                 150                 155                 160
   Pro Arg Lys Pro Leu Glu Lys Ala Val Ala Asn Phe Phe Ser Gly Ser
                   165                 170                 175
   Cys Ala Pro Cys Ala Asp Gly Thr Asp Phe Pro Gln Leu Cys Gln Leu
               180                 185                 190
   Cys Pro Gly Cys Gly Cys Ser Thr Leu Asn Gln Tyr Phe Gly Tyr Ser
           195                 200                 205
   Gly Ala Phe Lys Cys Leu Lys Asp Gly Ala Gly Asp Val Ala Phe Val
       210                 215                 220
   Lys His Ser Thr Ile Phe Glu Asn Leu Ala Asn Lys Ala Asp Arg Asp
   225                 230                 235                 240
   Gln Tyr Glu Leu Leu Cys Leu Asp Asn Thr Arg Lys Pro Val Asp Glu
                   245                 250                 255
   Tyr Lys Asp Cys His Leu Ala Gln Val Pro Ser His Thr Val Val Ala
               260                 265                 270
   Arg Ser Met Gly Gly Lys Glu Asp Leu Ile Trp Glu Leu Leu Asn Gln
           275                 280                 285
   Ala Gln Glu His Phe Gly Lys Asp Lys Ser Lys Glu Phe Gln Leu Phe
       290                 295                 300
   Ser Ser Pro His Gly Lys Asp Leu Leu Phe Lys Asp Ser Ala His Gly
   305                 310                 315                 320
   Phe Leu Lys Val Pro Pro Arg Met Asp Ala Lys Met Tyr Leu Gly Tyr
                   325                 330                 335
   Glu Tyr Val Thr Ala Ile Arg Asn Leu Arg Glu Gly Thr Cys Pro Glu
               340                 345                 350
   Ala Pro Thr Asp Glu Cys Lys Pro Val Lys Trp Cys Ala Leu Ser His
           355                 360                 365
   His Glu Arg Leu Lys Cys Asp Glu Trp Ser Val Asn Ser Val Gly Lys
       370                 375                 380
   Ile Glu Cys Val Ser Ala Glu Thr Thr Glu Asp Cys Ile Ala Lys Ile
   385                 390                 395                 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Ile
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
   515 520 525
   Asn Lys Glu Gly Tyr Tyr Gly Tyr Thr Gly Ala Phe Arg Cys Leu Val
   530 535 540
   Glu Lys Gly Asp Val Ala Phe Val Lys His Gln Thr Val Pro Gln Asn
   545 550 555 560
   Thr Gly Gly Lys Asn Pro Asp Pro Trp Ala Lys Asn Leu Asn Glu Lys
   565 570 575
   Asp Tyr Glu Leu Leu Cys Leu Asp Gly Thr Arg Lys Pro Val Glu Glu
   580 585 590
   Tyr Ala Asn Cys His Leu Ala Arg Ala Pro Asn His Ala Val Val Thr
   595 600 605
   Arg Lys Asp Lys Glu Ala Cys Val His Lys Ile Leu Arg Gln Gln Gln
   610 615 620
   His Leu Phe Gly Ser Asn Val Thr Asp Cys Ser Gly Asn Phe Cys Leu
   625 630 635 640
   Phe Arg Ser Glu Thr Lys Asp Leu Leu Phe Arg Asp Asp Thr Val Cys
   645 650 655
   Leu Ala Lys Leu His Asp Arg Asn Thr Tyr Glu Lys Tyr Leu Gly Glu
   660 665 670
   Glu Tyr Val Lys Ala Val Gly Asn Leu Arg Lys Cys Ser Thr Ser Ser
   675 680 685
   Leu Leu Glu Ala Cys Thr Phe Arg Arg Pro
   690 695
<210> 85
   <211> 210
   <212> PRT
   <213> Homo sapiens
<400> 85
   Met Pro Pro Tyr Thr Val Val Tyr Phe Pro Val Arg Gly Arg Cys Ala
   1 5 10 15
   Ala Leu Arg Met Leu Leu Ala Asp Gln Gly Gln Ser Trp Lys Glu Glu
   20 25 30
   Val Val Thr Val Glu Thr Trp Gln Glu Gly Ser Leu Lys Ala Ser Cys
   35 40 45
   Leu Tyr Gly Gln Leu Pro Lys Phe Gln Asp Gly Asp Leu Thr Leu Tyr
   50 55 60
   Gln Ser Asn Thr Ile Leu Arg His Leu Gly Arg Thr Leu Gly Leu Tyr
   65 70 75 80
   Gly Lys Asp Gln Gln Glu Ala Ala Leu Val Asp Met Val Asn Asp Gly
   85 90 95
   Val Glu Asp Leu Arg Cys Lys Tyr Ile Ser Leu Ile Tyr Thr Asn Tyr
   100 105 110
   Glu Ala Gly Lys Asp Asp Tyr Val Lys Ala Leu Pro Gly Gln Leu Lys
   115 120 125
   Pro Phe Glu Thr Leu Leu Ser Gln Asn Gln Gly Gly Lys Thr Phe Ile
   130 135 140
   Val Gly Asp Gln Ile Ser Phe Ala Asp Tyr Asn Leu Leu Asp Leu Leu
   145 150 155 160
   Leu Ile His Glu Val Leu Ala Pro Gly Cys Leu Asp Ala Phe Pro Leu
   165 170 175
   Leu Ser Ala Tyr Val Gly Arg Leu Ser Ala Arg Pro Lys Leu Lys Ala
   180 185 190
   Phe Leu Ala Ser Pro Glu Tyr Val Asn Leu Pro Ile Asn Gly Asn Gly
   195 200 205
   Lys Gln
   210
<210> 86
   <211> 478
   <212> PRT
   <213> Homo sapiens
<400> 86
   Met Ala Pro Leu Arg Pro Leu Leu Ile Leu Ala Leu Leu Ala Trp Val
   1 5 10 15
   Ala Leu Ala Asp Gln Glu Ser Cys Lys Gly Arg Cys Thr Glu Gly Phe
   20 25 30
   Asn Val Asp Lys Lys Cys Gln Cys Asp Glu Leu Cys Ser Tyr Tyr Gln
           35                  40                  45
   Ser Cys Cys Thr Asp Tyr Thr Ala Glu Cys Lys Pro Gln Val Thr Arg
       50                  55                  60
   Gly Asp Val Phe Thr Met Pro Glu Asp Glu Tyr Thr Val Tyr Asp Asp
   65                  70                  75                  80
   Gly Glu Glu Lys Asn Asn Ala Thr Val His Glu Gln Val Gly Gly Pro
                   85                  90                  95
   Ser Leu Thr Ser Asp Leu Gln Ala Gln Ser Lys Gly Asn Pro Glu Gln
               100                 105                 110
   Thr Pro Val Leu Lys Pro Glu Glu Glu Ala Pro Ala Pro Glu Val Gly
           115                 120                 125
   Ala Ser Lys Pro Glu Gly Ile Asp Ser Arg Pro Glu Thr Leu His Pro
       130                 135                 140
   Gly Arg Pro Gln Pro Pro Ala Glu Glu Glu Leu Cys Ser Gly Lys Pro
   145                 150                 155                 160
   Phe Asp Ala Phe Thr Asp Leu Lys Asn Gly Ser Leu Phe Ala Phe Arg
                   165                 170                 175
   Gly Gln Tyr Cys Tyr Glu Leu Asp Glu Lys Ala Val Arg Pro Gly Tyr
               180                 185                 190
   Pro Lys Leu Ile Arg Asp Val Trp Gly Ile Glu Gly Pro Ile Asp Ala
           195                 200                 205
   Ala Phe Thr Arg Ile Asn Cys Gln Gly Lys Thr Tyr Leu Phe Lys Gly
       210                 215                 220
   Ser Gln Tyr Trp Arg Phe Glu Asp Gly Val Leu Asp Pro Asp Tyr Pro
   225                 230                 235                 240
   Arg Asn Ile Ser Asp Gly Phe Asp Gly Ile Pro Asp Asn Val Asp Ala
                   245                 250                 255
   Ala Leu Ala Leu Pro Ala His Ser Tyr Ser Gly Arg Glu Arg Val Tyr
               260                 265                 270
   Phe Phe Lys Gly Lys Gln Tyr Trp Glu Tyr Gln Phe Gln His Gln Pro
           275                 280                 285
   Ser Gln Glu Glu Cys Glu Gly Ser Ser Leu Ser Ala Val Phe Glu His
   290 295 300
   Phe Ala Met Met Gln Arg Asp Ser Trp Glu Asp Ile Phe Glu Leu Leu
   305 310 315 320
   Phe Trp Gly Arg Thr Ser Ala Gly Thr Arg Gln Pro Gln Phe Ile Ser
   325 330 335
   Arg Asp Trp His Gly Val Pro Gly Gln Val Asp Ala Ala Met Ala Gly
   340 345 350
   Arg Ile Tyr Ile Ser Gly Met Ala Pro Arg Pro Ser Leu Ala Lys Lys
   355 360 365
   Gln Arg Phe Arg His Arg Asn Arg Lys Gly Tyr Arg Ser Gln Arg Gly
   370 375 380
   His Ser Arg Gly Arg Asn Gln Asn Ser Arg Arg Pro Ser Arg Ala Thr
   385 390 395 400
   Trp Leu Ser Leu Phe Ser Ser Glu Glu Ser Asn Leu Gly Ala Asn Asn
   405 410 415
   Tyr Asp Asp Tyr Arg Met Asp Trp Leu Val Pro Ala Thr Cys Glu Pro
   420 425 430
   Ile Gln Ser Val Phe Phe Phe Ser Gly Asp Lys Tyr Tyr Arg Val Asn
   435 440 445
   Leu Arg Thr Arg Arg Val Asp Thr Val Asp Pro Pro Tyr Pro Arg Ser
   450 455 460
   Ile Ala Gln Tyr Trp Leu Gly Cys Pro Ala Pro Gly His Leu
   465 470 475
<210> 87
   <211> 114
   <212> PRT
   <213> Homo sapiens
<400> 87
   Met Thr Cys Lys Met Ser Gln Leu Glu Arg Asn Ile Glu Thr Ile Ile
   1 5 10 15
   Asn Thr Phe His Gln Tyr Ser Val Lys Leu Gly His Pro Asp Thr Leu
   20 25 30
   Asn Gln Gly Glu Phe Lys Glu Leu Val Arg Lys Asp Leu Gln Asn Phe
   35 40 45
   Leu Lys Lys Glu Asn Lys Asn Glu Lys Val Ile Glu His Ile Met Glu
   50 55 60
   Asp Leu Asp Thr Asn Ala Asp Lys Gln Leu Ser Phe Glu Glu Phe Ile
   65 70 75 80
   Met Leu Met Ala Arg Leu Thr Trp Ala Ser His Glu Lys Met His Glu
   85 90 95
   Gly Asp Glu Gly Pro Gly His His His Lys Pro Gly Leu Gly Glu Gly
   100 105 110
   Thr Pro
<210> 88
   <211> 15
   <212> PRT
   <213> Homo sapiens
<400> 88
   Ser Cys Cys Glu Glu Gln Asn Lys Val Asn Cys Leu Gln Thr Arg
   1 5 10 15
<210> 89
   <211> 14
   <212> PRT
   <213> Homo sapiens
<400> 89
   Ala Glu Ser Pro Glu Val Cys Phe Asn Glu Glu Ser Pro Lys
   1 5 10
<210> 90
   <211> 14
   <212> PRT
   <213> Homo sapiens
<400> 90
   Glu Gly Lys Phe Thr Asp Ser Glu Asn Val Cys Gln Glu Arg
   1 5 10
<210> 91
   <211> 13
   <212> PRT
   <213> Homo sapiens
<400> 91
   Glu Ser Leu Leu Asn His Phe Leu Tyr Glu Val Ala Arg
   1 5 10
<210> 92
   <211> 11
   <212> PRT
   <213> Homo sapiens
<400> 92
   Phe Thr Asp Ser Glu Asn Val Cys Gln Glu Arg
   1 5 10
<210> 93
   <211> 12
   <212> PRT
   <213> Homo sapiens
<400> 93
   Arg His Pro Asp Leu Ser Ile Pro Glu Leu Leu Arg
   1 5 10
<210> 94
   <211> 17
   <212> PRT
   <213> Homo sapiens
<400> 94
   Ser Asp Val Gly Phe Leu Pro Pro Phe Pro Thr Leu Asp Pro Glu Glu
   1 5 10 15
   Lys
<210> 95
   <211> 28
   <212> PRT
   <213> Homo sapiens
<400> 95
   Cys Ser Pro His Leu Val Leu Ser Ala Leu Thr Ser Asp Asn His Gly
   1 5 10 15
   Ala Thr Tyr Ala Phe Ser Gly Thr His Tyr Trp Arg
   20 25
<210> 96
   <211> 9
   <212> PRT
   <213> Homo sapiens
<400> 96
   Asp Tyr Phe Met Pro Cys Pro Gly Arg
   1 5
<210> 97
   <211> 24
   <212> PRT
   <213> Homo sapiens
<400> 97
   Glu Val Gly Thr Pro His Gly Ile Ile Leu Asp Ser Val Asp Ala Ala
   1 5 10 15
   Phe Ile Cys Pro Gly Ser Ser Arg
   20
<210> 98
   <211> 14
   <212> PRT
   <213> Homo sapiens
<400> 98
   Glu Trp Phe Trp Asp Leu Ala Thr Gly Thr Met Lys Glu Arg
   1 5 10
<210> 99
   <211> 12
   <212> PRT
   <213> Homo sapiens
<400> 99
   Gly Asp Lys Val Trp Val Tyr Pro Pro Glu Lys Lys
   1 5 10
<210> 100
   <211> 15
   <212> PRT
   <213> Homo sapiens
<400> 100
   Gly Glu Cys Gln Ala Glu Gly Val Leu Phe Phe Gln Gly Asp Arg
   1 5 10 15
<210> 101
   <211> 11
   <212> PRT
   <213> Homo sapiens
<400> 101
   Gly Gly Tyr Thr Leu Val Ser Gly Tyr Pro Lys
   1 5 10
<210> 102
   <211> 21
   <212> PRT
   <213> Homo sapiens
<400> 102
   Leu Leu Gln Asp Glu Phe Pro Gly Ile Pro Ser Pro Leu Asp Ala Ala
   1 5 10 15
   Val Glu Cys His Arg
   20
<210> 103
   <211> 11
   <212> PRT
   <213> Homo sapiens
<400> 103
   Asn Phe Pro Ser Pro Val Asp Ala Ala Phe Arg
   1 5 10
<210> 104
   <211> 10
   <212> PRT
   <213> Homo sapiens
<400> 104
   Gln Gly His Asn Ser Val Phe Leu Ile Lys
   1 5 10
<210> 105
   <211> 8
   <212> PRT
   <213> Homo sapiens
<400> 105
   Arg Leu Trp Trp Leu Asp Leu Lys
   1 5
<210> 106
   <211> 16
   <212> PRT
   <213> Homo sapiens
<400> 106
   Ser Gly Ala Gln Ala Thr Trp Thr Glu Leu Pro Trp Pro His Glu Lys
   1 5 10 15
<210> 107
   <211> 30
   <212> PRT
   <213> Homo sapiens
<400> 107
   Ser Leu Gly Pro Asn Ser Cys Ser Ala Asn Gly Pro Gly Leu Tyr Leu
   1               5                   10                  15
   Ile His Gly Pro Asn Leu Tyr Cys Tyr Ser Asp Val Glu Lys
               20                  25                  30
<210> 108
   <211> 35
   <212> PRT
   <213> Homo sapiens
<400> 108
   Ser Leu Gly Pro Asn Ser Cys Ser Ala Asn Gly Pro Gly Leu Tyr Leu
   1               5                   10                  15
   Ile His Gly Pro Asn Leu Tyr Cys Tyr Ser Asp Val Glu Lys Leu Asn
               20                  25                  30
   Ala Ala Lys
           35
<210> 109
   <211> 9
   <212> PRT
   <213> Homo sapiens
<400> 109
   Val Trp Val Tyr Pro Pro Glu Lys Lys
   1               5
<210> 110
   <211> 13
   <212> PRT
   <213> Homo sapiens
<400> 110
   Trp Lys Asn Phe Pro Ser Pro Val Asp Ala Ala Phe Arg
   1               5                   10
<210> 111
   <211> 15
   <212> PRT
   <213> Homo sapiens
<400> 111
   Trp Leu Gly Arg Tyr Tyr Cys Phe Gln Gly Asn Gln Phe Leu Arg
   1 5 10 15
<210> 112
   <211> 11
   <212> PRT
   <213> Homo sapiens
<400> 112
   Tyr Tyr Cys Phe Gln Gly Asn Gln Phe Leu Arg
   1 5 10
<210> 113
   <211> 20
   <212> PRT
   <213> Homo sapiens
<400> 113
   Ala Asp Glu Ile Ala Lys Ala Gln Val Ala Arg Pro Gly Gly Asp Thr
   1 5 10 15
   Ile Phe Gly Lys
   20
<210> 114
   <211> 20
   <212> PRT
   <213> Homo sapiens
<400> 114
   Cys Leu Ala Phe His Asp Ile Ser Pro Gln Ala Pro Thr His Phe Leu
   1 5 10 15
   Val Ile Pro Lys
   20
<210> 115
   <211> 26
   <212> PRT
   <213> Homo sapiens
<400> 115
   Cys Cys Val Glu Cys Pro Pro Cys Pro Ala Pro Pro Val Ala Gly Pro
   1 5 10 15
   Ser Val Phe Leu Phe Pro Pro Lys Pro Lys
   20 25
<210> 116
   <211> 14
   <212> PRT
   <213> Homo sapiens
<400> 116
   Cys Lys Val Ser Asn Lys Gly Leu Pro Ala Pro Ile Glu Lys
   1               5                   10
<210> 117
   <211> 7
   <212> PRT
   <213> Homo sapiens
<400> 117
   Asp Thr Leu Met Ile Ser Arg
   1               5
<210> 118
   <211> 12
   <212> PRT
   <213> Homo sapiens
<400> 118
   Gly Pro Ser Val Phe Pro Leu Ala Pro Cys Ser Arg
   1               5                   10
<210> 119
   <211> 27
   <212> PRT
   <213> Homo sapiens
<400> 119
   Lys Cys Cys Val Glu Cys Pro Pro Cys Pro Ala Pro Pro Val Ala Gly
   1               5                   10                  15
   Pro Ser Val Phe Leu Phe Pro Pro Lys Pro Lys
               20                  25
<210> 120
   <211> 10
   <212> PRT
   <213> Homo sapiens
<400> 120
   Asn Gln Val Ser Leu Thr Cys Leu Val Lys
   1               5                   10
<210> 121
   <211> 14
   <212> PRT
   <213> Homo sapiens
<400> 121
   Ser Thr Ser Glu Ser Thr Ala Ala Leu Gly Cys Leu Val Lys
   1 5 10
<210> 122
   <211> 17
   <212> PRT
   <213> Homo sapiens
<400> 122
   Thr Lys Gly Gln Pro Arg Glu Pro Gln Val Tyr Thr Leu Pro Pro Ser
   1 5 10 15
   Arg
<210> 123
   <211> 17
   <212> PRT
   <213> Homo sapiens
<400> 123
   Thr Thr Pro Pro Met Leu Asp Ser Asp Gly Ser Phe Phe Leu Tyr Ser
   1 5 10 15
   Lys
<210> 124
   <211> 12
   <212> PRT
   <213> Homo sapiens
<400> 124
   Val Ser Asn Lys Gly Leu Pro Ala Pro Ile Glu Lys
   1 5 10
<210> 125
   <211> 16
   <212> PRT
   <213> Homo sapiens
<400> 125
   Val Val Ser Val Leu Thr Val Val His Gln Asp Trp Leu Asn Gly Lys
   1 5 10 15
<210> 126
   <211> 19
   <212> PRT
   <213> Homo sapiens
<400> 126
   Val Val Ser Val Leu Thr Val Val His Gln Asp Trp Leu Asn Gly Lys
   1 5 10 15
   Glu Tyr Lys
<210> 127
   <211> 11
   <212> PRT
   <213> Homo sapiens
<400> 127
   Ala Glu Asp Thr Ala Val Tyr Tyr Cys Ala Lys
   1 5 10
<210> 128
   <211> 19
   <212> PRT
   <213> Homo sapiens
<400> 128
   Glu Val Gln Leu Leu Glu Ser Gly Gly Gly Leu Val Gln Pro Gly Gly
   1 5 10 15
   Ser Leu Arg
<210> 129
   <211> 19
   <212> PRT
   <213> Homo sapiens
<400> 129
   Ala Ala Pro Ser Val Thr Leu Phe Pro Pro Ser Ser Glu Glu Leu Gln
   1 5 10 15
   Ala Asn Lys
<210> 130
   <211> 17
   <212> PRT
   <213> Homo sapiens
<400> 130
   Ala Asp Ser Ser Pro Val Lys Ala Gly Val Glu Thr Thr Thr Pro Ser
   1 5 10 15
   Lys
<210> 131
   <211> 22
   <212> PRT
   <213> Homo sapiens
<400> 131
   Ala Asp Ser Ser Pro Val Lys Ala Gly Val Glu Thr Thr Thr Pro Ser
   1 5 10 15
   Lys Gln Ser Asn Asn Lys
   20
<210> 132
   <211> 10
   <212> PRT
   <213> Homo sapiens
<400> 132
   Ala Gly Val Glu Thr Thr Thr Pro Ser Lys
   1 5 10
<210> 133
   <211> 30
   <212> PRT
   <213> Homo sapiens
<400> 133
   Ala Gly Val Glu Thr Thr Thr Pro Ser Lys Gln Ser Asn Asn Lys Tyr
   1 5 10 15
   Ala Ala Ser Ser Tyr Leu Ser Leu Thr Pro Glu Gln Trp Lys
   20 25 30
<210> 134
   <211> 20
   <212> PRT
   <213> Homo sapiens
<400> 134
   Gln Ser Asn Asn Lys Tyr Ala Ala Ser Ser Tyr Leu Ser Leu Thr Pro
   1 5 10 15
   Glu Gln Trp Lys
   20
<210> 135
   <211> 18
   <212> PRT
   <213> Homo sapiens
<400> 135
   Ser His Lys Ser Tyr Ser Cys Gln Val Thr His Glu Gly Ser Thr Val
   1 5 10 15
   Glu Lys
<210> 136
   <211> 15
   <212> PRT
   <213> Homo sapiens
<400> 136
   Ser Tyr Ser Cys Gln Val Thr His Glu Gly Ser Thr Val Glu Lys
   1 5 10 15
<210> 137
   <211> 23
   <212> PRT
   <213> Homo sapiens
<400> 137
   Ser Tyr Ser Cys Gln Val Thr His Glu Gly Ser Thr Val Glu Lys Thr
   1 5 10 15
   Val Ala Pro Thr Glu Cys Ser
   20
<210> 138
   <211> 8
   <212> PRT
   <213> Homo sapiens
<400> 138
   Thr Val Ala Pro Thr Glu Cys Ser
   1 5
<210> 139
   <211> 15
   <212> PRT
   <213> Homo sapiens
<400> 139
   Tyr Ala Ala Ser Ser Tyr Leu Ser Leu Thr Pro Glu Gln Trp Lys
   1 5 10 15
<210> 140
   <211> 11
   <212> PRT
   <213> Homo sapiens
<400> 140
   Ala Ile Ser Gly Glu Asn Ala Gly Leu Val Arg
   1 5 10
<210> 141
   <211> 10
   <212> PRT
   <213> Homo sapiens
<400> 141
   Glu Val Ala Phe Asp Leu Glu Ile Pro Lys
   1 5 10
<210> 142
   <211> 18
   <212> PRT
   <213> Homo sapiens
<400> 142
   Phe Ala His Tyr Val Val Thr Ser Gln Val Val Asn Thr Ala Asn Glu
   1 5 10 15
   Ala Arg
<210> 143
   <211> 13
   <212> PRT
   <213> Homo sapiens
<400> 143
   Gln Tyr Tyr Glu Gly Ser Glu Ile Val Val Ala Gly Arg
   1 5 10
<210> 144
   <211> 15
   <212> PRT
   <213> Homo sapiens
<400> 144
   Ala Glu Asp His Phe Ser Val Ile Asp Phe Asn Gln Asn Ile Arg
   1 5 10 15
<210> 145
   <211> 13
   <212> PRT
   <213> Homo sapiens
<400> 145
   Ala His Val Ser Phe Lys Pro Thr Val Ala Gln Gln Arg
   1 5 10
<210> 146
   <211> 20
   <212> PRT
   <213> Homo sapiens
<400> 146
   Phe Leu His Val Pro Asp Thr Phe Glu Gly His Phe Asp Gly Val Pro
   1 5 10 15
   Val Ile Ser Lys
   20
<210> 147
   <211> 11
   <212> PRT
   <213> Homo sapiens
<400> 147
   Phe Tyr Asn Gln Val Ser Thr Pro Leu Leu Arg
   1 5 10
<210> 148
   <211> 15
   <212> PRT
   <213> Homo sapiens
<400> 148
   Ile Gln Pro Ser Gly Gly Thr Asn Ile Asn Glu Ala Leu Leu Arg
   1 5 10 15
<210> 149
   <211> 10
   <212> PRT
   <213> Homo sapiens
<400> 149
   Thr Glu Val Asn Val Leu Pro Gly Ala Lys
   1 5 10
<210> 150
   <211> 11
   <212> PRT
   <213> Homo sapiens
<400> 150
   Val Gln Phe Glu Leu His Tyr Gln Glu Val Lys
   1 5 10
<210> 151
   <211> 19
   <212> PRT
   <213> Homo sapiens
<400> 151
   Val Val Asn Asn Ser Pro Gln Pro Gln Asn Val Val Phe Asp Val Gln
   1               5                   10                  15
   Ile Pro Lys
<210> 152
   <211> 24
   <212> PRT
   <213> Homo sapiens
<400> 152
   His Arg Gln Gly Pro Val Asn Leu Leu Ser Asp Pro Glu Gln Gly Val
   1               5                   10                  15
   Glu Val Thr Gly Gln Tyr Glu Arg
               20
<210> 153
   <211> 9
   <212> PRT
   <213> Homo sapiens
<400> 153
   Leu Gly Val Tyr Glu Leu Leu Leu Lys
   1               5
<210> 154
   <211> 10
   <212> PRT
   <213> Homo sapiens
<400> 154
   Ala Glu Ala Glu Ser Leu Tyr Gln Ser Lys
   1               5                   10
<210> 155
   <211> 9
   <212> PRT
   <213> Homo sapiens
<400> 155
   Ala Gln Tyr Glu Asp Ile Ala Gln Lys
   1               5
<210> 156
   <211> 18
   <212> PRT
   <213> Homo sapiens
<400> 156
   Glu Gln Ile Lys Ser Leu Asn Asn Gln Phe Ala Ser Phe Ile Asp Lys
   1               5                   10                  15
   Val Arg
<210> 157
   <211> 12
   <212> PRT
   <213> Homo sapiens
<400> 157
   Phe Leu Glu Gln Gln Asn Gln Val Leu Gln Thr Lys
   1               5                   10
<210> 158
   <211> 24
   <212> PRT
   <213> Homo sapiens
<400> 158
   Phe Leu Glu Gln Gln Asn Gln Val Leu Gln Thr Lys Trp Glu Leu Leu
   1               5                   10                  15
   Gln Gln Val Asp Thr Ser Thr Arg
               20
<210> 159
   <211> 20
   <212> PRT
   <213> Homo sapiens
<400> 159
   Phe Ser Ser Cys Gly Gly Gly Gly Gly Ser Phe Gly Ala Gly Gly Gly
   1               5                   10                  15
   Phe Gly Ser Arg
               20
<210> 160
   <211> 31
   <212> PRT
   <213> Homo sapiens
<400> 160
   Gly Gly Gly Gly Gly Gly Tyr Gly Ser Gly Gly Ser Ser Tyr Gly Ser
   1               5                   10                  15
   Gly Gly Gly Ser Tyr Gly Ser Gly Gly Gly Gly Gly Gly Gly Arg
               20                  25                  30
<210> 161
   <211> 14
   <212> PRT
   <213> Homo sapiens
<400> 161
   Gly Ser Gly Gly Gly Ser Ser Gly Gly Ser Ile Gly Gly Arg
   1 5 10
<210> 162
   <211> 8
   <212> PRT
   <213> Homo sapiens
<400> 162
   Ile Glu Ile Ser Glu Leu Asn Arg
   1 5
<210> 163
   <211> 11
   <212> PRT
   <213> Homo sapiens
<400> 163
   Leu Ala Leu Asp Leu Glu Ile Ala Thr Tyr Arg
   1 5 10
<210> 164
   <211> 16
   <212> PRT
   <213> Homo sapiens
<400> 164
   Leu Asp Ser Glu Leu Lys Asn Met Gln Asp Met Val Glu Asp Tyr Arg
   1 5 10 15
<210> 165
   <211> 12
   <212> PRT
   <213> Homo sapiens
<400> 165
   Leu Leu Arg Asp Tyr Gln Glu Leu Met Asn Thr Lys
   1 5 10
<210> 166
   <211> 12
   <212> PRT
   <213> Homo sapiens
<400> 166
   Leu Asn Asp Leu Glu Asp Ala Leu Gln Gln Ala Lys
   1 5 10
<210> 167
   <211> 10
   <212> PRT
   <213> Homo sapiens
<400> 167
   Leu Arg Ser Glu Ile Asp Asn Val Lys Lys
   1 5 10
<210> 168
   <211> 14
   <212> PRT
   <213> Homo sapiens
<400> 168
   Asn Lys Leu Asn Asp Leu Glu Asp Ala Leu Gln Gln Ala Lys
   1 5 10
<210> 169
   <211> 19
   <212> PRT
   <213> Homo sapiens
<400> 169
   Asn Lys Leu Asn Asp Leu Glu Asp Ala Leu Gln Gln Ala Lys Glu Asp
   1 5 10 15
   Leu Ala Arg
<210> 170
   <211> 10
   <212> PRT
   <213> Homo sapiens
<400> 170
   Asn Lys Tyr Glu Asp Glu Ile Asn Lys Arg
   1 5 10
<210> 171
   <211> 15
   <212> PRT
   <213> Homo sapiens
<400> 171
   Asn Ser Lys Ile Glu Ile Ser Glu Leu Asn Arg Val Ile Gln Arg
   1 5 10 15
<210> 172
   <211> 15
   <212> PRT
   <213> Homo sapiens
<400> 172
   Gln Ile Ser Asn Leu Gln Gln Ser Ile Ser Asp Ala Glu Gln Arg
   1 5 10 15
<210> 173
   <211> 23
   <212> PRT
   <213> Homo sapiens
<400> 173
   Ser Glu Ile Asp Asn Val Lys Lys Gln Ile Ser Asn Leu Gln Gln Ser
   1 5 10 15
   Ile Ser Asp Ala Glu Gln Arg
   20
<210> 174
   <211> 17
   <212> PRT
   <213> Homo sapiens
<400> 174
   Ser Gly Gly Gly Phe Ser Ser Gly Ser Ala Gly Ile Ile Asn Tyr Gln
   1 5 10 15
   Arg
<210> 175
   <211> 18
   <212> PRT
   <213> Homo sapiens
<400> 175
   Ser Gly Gly Gly Phe Ser Ser Gly Ser Ala Gly Ile Ile Asn Tyr Gln
   1 5 10 15
   Arg Arg
<210> 176
   <211> 27
   <212> PRT
   <213> Homo sapiens
<400> 176
   Ser Gly Gly Gly Gly Gly Arg Phe Ser Ser Cys Gly Gly Gly Gly Gly
   1 5 10 15
   Ser Phe Gly Ala Gly Gly Gly Phe Gly Ser Arg
   20 25
<210> 177
   <211> 21
   <212> PRT
   <213> Homo sapiens
<400> 177
   Ser Gly Tyr Arg Ser Gly Gly Gly Phe Ser Ser Gly Ser Ala Gly Ile
   1 5 10 15
   Ile Asn Tyr Gln Arg
   20
<210> 178
   <211> 8
   <212> PRT
   <213> Homo sapiens
<400> 178
   Ser Ile Ser Ile Ser Val Ala Arg
   1 5
<210> 179
   <211> 12
   <212> PRT
   <213> Homo sapiens
<400> 179
   Ser Lys Ala Glu Ala Glu Ser Leu Tyr Gln Ser Lys
   1 5 10
<210> 180
   <211> 22
   <212> PRT
   <213> Homo sapiens
<400> 180
   Ser Lys Ala Glu Ala Glu Ser Leu Tyr Gln Ser Lys Tyr Glu Glu Leu
   1 5 10 15
   Gln Ile Thr Ala Gly Arg
   20
<210> 181
   <211> 12
   <212> PRT
   <213> Homo sapiens
<400> 181
   Ser Leu Asp Leu Asp Ser Ile Ile Ala Glu Val Lys
   1 5 10
<210> 182
   <211> 12
   <212> PRT
   <213> Homo sapiens
<400> 182
   Ser Leu Asn Asn Gln Phe Ala Ser Phe Ile Asp Lys
   1 5 10
<210> 183
   <211> 14
   <212> PRT
   <213> Homo sapiens
<400> 183
   Ser Leu Asn Asn Gln Phe Ala Ser Phe Ile Asp Lys Val Arg
   1 5 10
<210> 184
   <211> 8
   <212> PRT
   <213> Homo sapiens
<400> 184
   Leu Val Asn Leu Gly Gly Ser Lys
   1 5
<210> 185
   <211> 17
   <212> PRT
   <213> Homo sapiens
<400> 185
   Ser Leu Val Asn Leu Gly Gly Ser Lys Ser Ile Ser Ile Ser Val Ala
   1 5 10 15
   Arg
<210> 186
   <211> 16
   <212> PRT
   <213> Homo sapiens
<400> 186
   Thr His Asn Leu Glu Pro Tyr Phe Glu Ser Phe Ile Asn Asn Leu Arg
   1 5 10 15
<210> 187
   <211> 17
   <212> PRT
   <213> Homo sapiens
<400> 187
   Thr His Asn Leu Glu Pro Tyr Phe Glu Ser Phe Ile Asn Asn Leu Arg
   1 5 10 15
   Arg
<210> 188
   <211> 9
   <212> PRT
   <213> Homo sapiens
<400> 188
   Thr Leu Leu Glu Gly Glu Glu Ser Arg
   1 5
<210> 189
   <211> 11
   <212> PRT
   <213> Homo sapiens
<400> 189
   Thr Asn Ala Glu Asn Glu Phe Val Thr Ile Lys
   1 5 10
<210> 190
   <211> 12
   <212> PRT
   <213> Homo sapiens
<400> 190
   Thr Asn Ala Glu Asn Glu Phe Val Thr Ile Lys Lys
   1 5 10
<210> 191
   <211> 12
   <212> PRT
   <213> Homo sapiens
<400> 191
   Trp Glu Leu Leu Gln Gln Val Asp Thr Ser Thr Arg
   1 5 10
<210> 192
   <211> 10
   <212> PRT
   <213> Homo sapiens
<400> 192
   Tyr Glu Glu Leu Gln Ile Thr Ala Gly Arg
   1 5 10
<210> 193
   <211> 16
   <212> PRT
   <213> Homo sapiens
<400> 193
   Tyr Glu Glu Leu Gln Ile Thr Ala Gly Arg His Gly Asp Ser Val Arg
   1 5 10 15
<210> 194
   <211> 20
   <212> PRT
   <213> Homo sapiens
<400> 194
   Ala Glu Ala Glu Ser Leu Tyr Gln Ser Lys Tyr Glu Glu Leu Gln Ile
   1 5 10 15
   Thr Ala Gly Arg
   20
<210> 195
   <211> 13
   <212> PRT
   <213> Homo sapiens
<400> 195
   Ala Gly Lys Gly Met His Gly Gly Val Pro Gly Gly Lys
   1 5 10
<210> 196
   <211> 10
   <212> PRT
   <213> Homo sapiens
<400> 196
   Gly Met His Gly Gly Val Pro Gly Gly Lys
   1 5 10
<210> 197
   <211> 7
   <212> PRT
   <213> Homo sapiens
<400> 197
   Gln Glu Leu Asn Pro Leu Lys
   1               5
<210> 198
   <211> 10
   <212> PRT
   <213> Homo sapiens
<400> 198
   Gln Phe Ile Glu Ser Glu Cys Ile Pro Arg
   1               5                   10
<210> 199
   <211> 13
   <212> PRT
   <213> Homo sapiens
<400> 199
   Ala Gly Lys Gly Met His Gly Gly Val Pro Gly Gly Lys
   1               5                   10
<210> 200
   <211> 25
   <212> PRT
   <213> Homo sapiens
<400> 200
   Ala Gly Lys Gly Met His Gly Gly Val Pro Gly Gly Lys Gln Phe Ile
   1               5                   10                  15
   Glu Asn Gly Ser Glu Phe Ala Gln Lys
               20                  25
<210> 201
   <211> 8
   <212> PRT
   <213> Homo sapiens
<400> 201
   Phe Ser Leu Leu Lys Pro Trp Ala
   1               5
<210> 202
   <211> 10
   <212> PRT
   <213> Homo sapiens
<400> 202
   Gly Met His Gly Gly Val Pro Gly Gly Lys
   1               5                   10
<210> 203
   <211> 22
   <212> PRT
   <213> Homo sapiens
<400> 203
   Gly Met His Gly Gly Val Pro Gly Gly Lys Gln Phe Ile Glu Asn Gly
   1               5                   10                  15
   Ser Glu Phe Ala Gln Lys
               20
<210> 204
   <211> 25
   <212> PRT
   <213> Homo sapiens
<400> 204
   Gly Met His Gly Gly Val Pro Gly Gly Lys Gln Phe Ile Glu Asn Gly
   1               5                   10                  15
   Ser Glu Phe Ala Gln Lys Leu Leu Lys
               20                  25
<210> 205
   <211> 9
   <212> PRT
   <213> Homo sapiens
<400> 205
   Lys Phe Ser Leu Leu Lys Pro Trp Ala
   1               5
<210> 206
   <211> 7
   <212> PRT
   <213> Homo sapiens
<400> 206
   Gln Glu Leu Asn Pro Leu Lys
   1               5
<210> 207
   <211> 12
   <212> PRT
   <213> Homo sapiens
<400> 207
   Gln Glu Leu Asn Pro Leu Lys Ser Ile Val Glu Lys
   1               5                   10
<210> 208
   <211> 12
   <212> PRT
   <213> Homo sapiens
<400> 208
   Gln Phe Ile Glu Asn Gly Ser Glu Phe Ala Gln Lys
   1 5 10
<210> 209
   <211> 15
   <212> PRT
   <213> Homo sapiens
<400> 209
   Gln Phe Ile Glu Asn Gly Ser Glu Phe Ala Gln Lys Leu Leu Lys
   1 5 10 15
<210> 210
   <211> 16
   <212> PRT
   <213> Homo sapiens
<400> 210
   Gln Phe Ile Glu Asn Gly Ser Glu Phe Ala Gln Lys Leu Leu Lys Lys
   1 5 10 15
<210> 211
   <211> 11
   <212> PRT
   <213> Homo sapiens
<400> 211
   Ser Ile Leu Leu Thr Glu Gln Ala Leu Ala Lys
   1 5 10
<210> 212
   <211> 14
   <212> PRT
   <213> Homo sapiens
<400> 212
   Ser Ile Leu Leu Thr Glu Gln Ala Leu Ala Lys Ala Gly Lys
   1 5 10
<210> 213
   <211> 16
   <212> PRT
   <213> Homo sapiens
<400> 213
   Ser Ile Val Glu Lys Ser Ile Leu Leu Thr Glu Gln Ala Leu Ala Lys
   1 5 10 15
<210> 214
   <211> 19
   <212> PRT
   <213> Homo sapiens
<400> 214
   Ser Ile Val Glu Lys Ser Ile Leu Leu Thr Glu Gln Ala Leu Ala Lys
   1 5 10 15
   Ala Gly Lys
<210> 215
   <211> 29
   <212> PRT
   <213> Homo sapiens
<400> 215
   Asp Ile Glu Asn Gln Tyr Glu Thr Gln Ile Thr Gln Ile Glu His Glu
   1 5 10 15
   Val Ser Ser Ser Gly Gln Glu Val Gln Ser Ser Ala Lys
   20 25
<210> 216
   <211> 23
   <212> PRT
   <213> Homo sapiens
<400> 216
   Glu Ile Glu Thr Tyr His Asn Leu Leu Glu Gly Gly Gln Glu Asp Phe
   1 5 10 15
   Glu Ser Ser Gly Ala Gly Lys
   20
<210> 217
   <211> 25
   <212> PRT
   <213> Homo sapiens
<400> 217
   Phe Ser Ser Ser Gly Gly Gly Gly Gly Gly Gly Arg Phe Ser Ser Ser
   1 5 10 15
   Ser Gly Tyr Gly Gly Gly Ser Ser Arg
   20 25
<210> 218
   <211> 13
   <212> PRT
   <213> Homo sapiens
<400> 218
   Phe Ser Ser Ser Ser Gly Tyr Gly Gly Gly Ser Ser Arg
   1 5 10
<210> 219
   <211> 32
   <212> PRT
   <213> Homo sapiens
<400> 219
   Gly Gly Gly Gly Ser Phe Gly Tyr Ser Tyr Gly Gly Gly Ser Gly Gly
   1 5 10 15
   Gly Phe Ser Ala Ser Ser Leu Gly Gly Gly Phe Gly Gly Gly Ser Arg
   20 25 30
<210> 220
   <211> 23
   <212> PRT
   <213> Homo sapiens
<400> 220
   Gly Gly Ser Gly Gly Ser Tyr Gly Gly Gly Gly Ser Gly Gly Gly Tyr
   1 5 10 15
   Gly Gly Gly Ser Gly Ser Arg
   20
<210> 221
   <211> 16
   <212> PRT
   <213> Homo sapiens
<400> 221
   His Gly Val Gln Glu Leu Glu Ile Glu Leu Gln Ser Gln Leu Ser Lys
   1 5 10 15
<210> 222
   <211> 17
   <212> PRT
   <213> Homo sapiens
<400> 222
   His Gly Val Gln Glu Leu Glu Ile Glu Leu Gln Ser Gln Leu Ser Lys
   1 5 10 15
   Lys
<210> 223
   <211> 7
   <212> PRT
   <213> Homo sapiens
<400> 223
   Leu Ala Ser Tyr Leu Asp Lys
   1 5
<210> 224
   <211> 21
   <212> PRT
   <213> Homo sapiens
<400> 224
   Leu Ala Ser Tyr Leu Asp Lys Val Gln Ala Leu Glu Glu Ala Asn Asn
   1 5 10 15
   Asp Leu Glu Asn Lys
   20
<210> 225
   <211> 26
   <212> PRT
   <213> Homo sapiens
<400> 225
   Leu Glu Lys Glu Ile Glu Thr Tyr His Asn Leu Leu Glu Gly Gly Gln
   1 5 10 15
   Glu Asp Phe Glu Ser Ser Gly Ala Gly Lys
   20 25
<210> 226
   <211> 32
   <212> PRT
   <213> Homo sapiens
<400> 226
   Asn Arg Lys Asp Ile Glu Asn Gln Tyr Glu Thr Gln Ile Thr Gln Ile
   1 5 10 15
   Glu His Glu Val Ser Ser Ser Gly Gln Glu Val Gln Ser Ser Ala Lys
   20 25 30
<210> 227
   <211> 9
   <212> PRT
   <213> Homo sapiens
<400> 227
   Gln Glu Tyr Glu Gln Leu Ile Ala Lys
   1 5
<210> 228
   <211> 11
   <212> PRT
   <213> Homo sapiens
<400> 228
   Gln Gly Val Asp Ala Asp Ile Asn Gly Leu Arg
   1 5 10
<210> 229
   <211> 10
   <212> PRT
   <213> Homo sapiens
<400> 229
   Gln Val Leu Asp Asn Leu Thr Met Glu Lys
   1 5 10
<210> 230
   <211> 12
   <212> PRT
   <213> Homo sapiens
<400> 230
   Ser Cys Arg Gln Phe Ser Ser Ser Tyr Leu Ser Arg
   1 5 10
<210> 231
   <211> 16
   <212> PRT
   <213> Homo sapiens
<400> 231
   Ser Gly Gly Gly Gly Gly Gly Gly Leu Gly Ser Gly Gly Ser Ile Arg
   1 5 10 15
<210> 232
   <211> 9
   <212> PRT
   <213> Homo sapiens
<400> 232
   Ser Thr Met Gln Glu Leu Asn Ser Arg
   1 5
<210> 233
   <211> 9
   <212> PRT
   <213> Homo sapiens
<400> 233
   Thr Leu Leu Asp Ile Asp Asn Thr Arg
   1 5
<210> 234
   <211> 15
   <212> PRT
   <213> Homo sapiens
<400> 234
   Thr Leu Asn Asp Met Arg Gln Glu Tyr Glu Gln Leu Ile Ala Lys
   1 5 10 15
<210> 235
   <211> 14
   <212> PRT
   <213> Homo sapiens
<400> 235
   Val Gln Ala Leu Glu Glu Ala Asn Asn Asp Leu Glu Asn Lys
   1 5 10
<210> 236
   <211> 10
   <212> PRT
   <213> Homo sapiens
<400> 236
   Asp Leu Leu Leu Pro Gln Pro Asp Leu Arg
   1 5 10
<210> 237
   <211> 7
   <212> PRT
   <213> Homo sapiens
<400> 237
   Gly Pro Leu Gln Leu Glu Arg
   1 5
<210> 238
   <211> 10
   <212> PRT
   <213> Homo sapiens
<400> 238
   Val Ala Ala Gly Ala Phe Gln Gly Leu Arg
   1 5 10
<210> 239
   <211> 13
   <212> PRT
   <213> Homo sapiens
<400> 239
   Ala Thr Leu Lys Asp Gln Leu Ile Tyr Asn Leu Leu Lys
   1 5 10
<210> 240
   <211> 21
   <212> PRT
   <213> Homo sapiens
<400> 240
   Ala Thr Leu Lys Asp Gln Leu Ile Tyr Asn Leu Leu Lys Glu Glu Gln
   1 5 10 15
   Thr Pro Gln Asn Lys
   20
<210> 241
   <211> 15
   <212> PRT
   <213> Homo sapiens
<400> 241
   Asp Leu Ala Asp Glu Leu Ala Leu Val Asp Val Ile Glu Asp Lys
   1 5 10 15
<210> 242
   <211> 9
   <212> PRT
   <213> Homo sapiens
<400> 242
   Asp Gln Leu Ile Tyr Asn Leu Leu Lys
   1 5
<210> 243
   <211> 17
   <212> PRT
   <213> Homo sapiens
<400> 243
   Asp Gln Leu Ile Tyr Asn Leu Leu Lys Glu Glu Gln Thr Pro Gln Asn
   1 5 10 15
   Lys
<210> 244
   <211> 9
   <212> PRT
   <213> Homo sapiens
<400> 244
   Leu Val Ile Ile Thr Ala Gly Ala Arg
   1 5
<210> 245
   <211> 14
   <212> PRT
   <213> Homo sapiens
<400> 245
   Asn Arg Val Ile Gly Ser Gly Cys Asn Leu Asp Ser Ala Arg
   1 5 10
<210> 246
   <211> 14
   <212> PRT
   <213> Homo sapiens
<400> 246
   Asn Val Asn Ile Phe Lys Phe Ile Ile Pro Asn Val Val Lys
   1 5 10
<210> 247
   <211> 11
   <212> PRT
   <213> Homo sapiens
<400> 247
   Gln Val Val Glu Ser Ala Tyr Glu Val Ile Lys
   1 5 10
<210> 248
   <211> 10
   <212> PRT
   <213> Homo sapiens
<400> 248
   Ser Ala Asp Thr Leu Trp Gly Ile Gln Lys
   1 5 10
<210> 249
   <211> 16
   <212> PRT
   <213> Homo sapiens
<400> 249
   Thr Leu His Pro Asp Leu Gly Thr Asp Lys Asp Lys Glu Gln Trp Lys
   1 5 10 15
<210> 250
   <211> 12
   <212> PRT
   <213> Homo sapiens
<400> 250
   Val Ile Gly Ser Gly Cys Asn Leu Asp Ser Ala Arg
   1 5 10
<210> 251
   <211> 10
   <212> PRT
   <213> Homo sapiens
<400> 251
   Val Thr Leu Thr Ser Glu Glu Glu Ala Arg
   1 5 10
<210> 252
   <211> 14
   <212> PRT
   <213> Homo sapiens
<400> 252
   Ile Val Ala Asp Lys Asp Tyr Ser Val Thr Ala Asn Ser Lys
   1 5 10
<210> 253
   <211> 16
   <212> PRT
   <213> Homo sapiens
<400> 253
   Leu Ile Ala Pro Val Ala Glu Glu Glu Ala Thr Val Pro Asn Asn Lys
   1 5 10 15
<210> 254
   <211> 10
   <212> PRT
   <213> Homo sapiens
<400> 254
   Ser Ala Asp Thr Leu Trp Asp Ile Gln Lys
   1 5 10
<210> 255
   <211> 15
   <212> PRT
   <213> Homo sapiens
<400> 255
   Ser Leu Ala Asp Glu Leu Ala Leu Val Asp Val Leu Glu Asp Lys
   1               5                   10                  15
<210> 256
   <211> 12
   <212> PRT
   <213> Homo sapiens
<400> 256
   Val Ile Gly Ser Gly Cys Asn Leu Asp Ser Ala Arg
   1               5                   10
<210> 257
   <211> 12
   <212> PRT
   <213> Homo sapiens
<400> 257
   Ala Glu Ala Ser Ser Asp Pro Gly Ala Glu Glu Arg
   1               5                   10
<210> 258
   <211> 20
   <212> PRT
   <213> Homo sapiens
<400> 258
   Asp Arg Gln Leu Gln Ala Gln Asp Glu Glu Gly Gly Gly His Val Pro
   1               5                   10                  15
   Glu Arg Pro Lys
               20
<210> 259
   <211> 27
   <212> PRT
   <213> Homo sapiens
<400> 259
   Glu Gly Pro Gly Pro Glu Asp Thr Val Gln Asp Asn Leu Gly Ala Ala
   1               5                   10                  15
   Gly Ala Glu Glu Glu Gln Glu Glu His Gln Lys
               20                  25
<210> 260
   <211> 13
   <212> PRT
   <213> Homo sapiens
<400> 260
   Trp Glu Thr Gly Glu Val Gln Ala Gln Ser Ala Ala Lys
   1               5                   10
<210> 261
   <211> 17
   <212> PRT
   <213> Homo sapiens
<400> 261
   Ser Gln Glu Thr Glu Cys Thr Tyr Phe Ser Thr Pro Leu Leu Leu Gly
   1               5                   10                  15
   Lys
<210> 262
   <211> 19
   <212> PRT
   <213> Homo sapiens
<400> 262
   Val Ala Val Leu Gly Ala Ser Gly Gly Ile Gly Gln Pro Leu Ser Leu
   1               5                   10                  15
   Leu Leu Lys
<210> 263
   <211> 12
   <212> PRT
   <213> Homo sapiens
<400> 263
   Ala Leu Gly Gly Leu Ala Cys Asp Leu Pro Gly Arg
   1               5                   10
<210> 264
   <211> 24
   <212> PRT
   <213> Homo sapiens
<400> 264
   Ala Leu Gly Gly Leu Ala Cys Asp Leu Pro Gly Arg Phe Val Ala Glu
   1               5                   10                  15
   Ser Ala Glu Val Leu Leu Pro Arg
               20
<210> 265
   <211> 10
   <212> PRT
   <213> Homo sapiens
<400> 265
   Glu Ile Asp Glu Ser Leu Ile Phe Tyr Lys
   1 5 10
<210> 266
   <211> 12
   <212> PRT
   <213> Homo sapiens
<400> 266
   Phe Val Ala Glu Ser Ala Glu Val Leu Leu Pro Arg
   1 5 10
<210> 267
   <211> 12
   <212> PRT
   <213> Homo sapiens
<400> 267
   Gly Leu Leu Pro Val Leu Gly Gln Pro Ile Ile Arg
   1 5 10
<210> 268
   <211> 10
   <212> PRT
   <213> Homo sapiens
<400> 268
   Gly Ser Leu Leu Ser Glu Ala Asp Val Arg
   1 5 10
<210> 269
   <211> 14
   <212> PRT
   <213> Homo sapiens
<400> 269
   Ile Gln Ser Phe Leu Gly Gly Ala Pro Thr Glu Asp Leu Lys
   1 5 10
<210> 270
   <211> 11
   <212> PRT
   <213> Homo sapiens
<400> 270
   Ile Thr Lys Ala Asn Val Asp Leu Leu Pro Arg
   1               5                   10
<210> 271
   <211> 10
   <212> PRT
   <213> Homo sapiens
<400> 271
   Leu Leu Gly Pro His Val Glu Gly Leu Lys
   1               5                   10
<210> 272
   <211> 14
   <212> PRT
   <213> Homo sapiens
<400> 272
   Leu Leu Gly Pro His Val Glu Gly Leu Lys Ala Glu Glu Arg
   1               5                   10
<210> 273
   <211> 12
   <212> PRT
   <213> Homo sapiens
<400> 273
   Leu Leu Pro Ala Ala Leu Ala Cys Trp Gly Val Arg
   1               5                   10
<210> 274
   <211> 16
   <212> PRT
   <213> Homo sapiens
<400> 274
   Leu Arg Thr Asp Ala Val Leu Pro Leu Thr Val Ala Glu Val Gln Lys
   1               5                   10                  15
<210> 275
   <211> 17
   <212> PRT
   <213> Homo sapiens
<400> 275
   Leu Val Ser Cys Pro Gly Pro Leu Asp Gln Asp Gln Gln Glu Ala Ala
   1               5                   10                  15
   Arg
<210> 276
   <211> 8
   <212> PRT
   <213> Homo sapiens
<400> 276
   Gln Leu Asp Val Leu Tyr Pro Lys
   1 5
<210> 277
   <211> 17
   <212> PRT
   <213> Homo sapiens
<400> 277
   Gln Leu Leu Gly Phe Pro Cys Ala Glu Val Ser Gly Leu Ser Thr Glu
   1 5 10 15
   Arg
<210> 278
   <211> 14
   <212> PRT
   <213> Homo sapiens
<400> 278
   Thr Asp Ala Val Leu Pro Leu Thr Val Ala Glu Val Gln Lys
   1 5 10
<210> 279
   <211> 15
   <212> PRT
   <213> Homo sapiens
<400> 279
   Val Asn Ala Ile Pro Phe Thr Tyr Glu Gln Leu Asp Val Leu Lys
   1 5 10 15
<210> 280
   <211> 18
   <212> PRT
   <213> Homo sapiens
<400> 280
   His Leu Ala Cys Leu Pro Arg Glu Pro Gly Leu Cys Thr Trp Gln Ser
   1 5 10 15
   Leu Arg
<210> 281
   <211> 25
   <212> PRT
   <213> Homo sapiens
<400> 281
   Leu Gln Asp Gly Leu Leu His Ile Thr Thr Cys Ser Phe Val Ala Pro
   1               5                   10                  15
   Trp Asn Ser Leu Ser Leu Ala Gln Arg
               20                  25
<210> 282
   <211> 19
   <212> PRT
   <213> Homo sapiens
<400> 282
   Leu Gln Ser Gly Thr His Cys Leu Trp Thr Asp Gln Leu Leu Gln Gly
   1               5                   10                  15
   Ser Glu Lys
<210> 283
   <211> 20
   <212> PRT
   <213> Homo sapiens
<400> 283
   Thr Tyr Thr Val Gly Cys Glu Glu Cys Thr Val Phe Pro Cys Leu Ser
   1               5                   10                  15
   Ile Pro Cys Lys
               20
<210> 284
   <211> 9
   <212> PRT
   <213> Homo sapiens
<400> 284
   Trp Val Gly Asp Leu Pro Asn Gly Arg
   1               5
<210> 285
   <211> 12
   <212> PRT
   <213> Homo sapiens
<400> 285
   Trp Val Gly Asp Leu Pro Asn Gly Arg Val Cys Pro
   1 5 10
<210> 286
   <211> 11
   <212> PRT
   <213> Homo sapiens
<400> 286
   Phe Thr Asp Glu Glu Val Asp Glu Leu Tyr Arg
   1 5 10
<210> 287
   <211> 10
   <212> PRT
   <213> Homo sapiens
<400> 287
   Gly Asn Phe Asn Tyr Ile Glu Phe Thr Arg
   1 5 10
<210> 288
   <211> 11
   <212> PRT
   <213> Homo sapiens
<400> 288
   Leu Asn Gly Thr Asp Pro Glu Asp Val Ile Arg
   1 5 10
<210> 289
   <211> 17
   <212> PRT
   <213> Homo sapiens
<400> 289
   Glu Ala Leu Ala Glu Ala Ala Leu Glu Ser Pro Arg Pro Ala Leu Val
   1 5 10 15
   Arg
<210> 290
   <211> 31
   <212> PRT
   <213> Homo sapiens
<400> 290
   Glu Glu Leu Leu Arg Ala Gln Glu Ala Pro Gly Gln Ala Glu Pro Pro
   1               5                   10                  15
   Ala Ala Ala Glu Val Gln Gly Ala Gly Asn Glu Asn Glu Pro Arg
               20                  25                  30
<210> 291
   <211> 14
   <212> PRT
   <213> Homo sapiens
<400> 291
   Leu Leu Val Val Asp Pro Glu Thr Asp Glu Gln Leu Gln Lys
   1               5                   10
<210> 292
   <211> 10
   <212> PRT
   <213> Homo sapiens
<400> 292
   Leu Val Glu Pro Gly Ser Pro Ala Glu Lys
   1               5                   10
<210> 293
   <211> 14
   <212> PRT
   <213> Homo sapiens
<400> 293
   Ser Val Asp Pro Asp Ser Pro Ala Glu Ala Ser Gly Leu Arg
   1               5                   10
<210> 294
   <211> 23
   <212> PRT
   <213> Homo sapiens
<400> 294
   Gly Ser Gln Thr Gln Ser His Pro Asp Leu Gly Thr Glu Gly Cys Trp
   1               5                   10                  15
   Asp Gln Leu Ser Ala Pro Arg
               20
<210> 295
   <211> 13
   <212> PRT
   <213> Homo sapiens
<400> 295
   Thr Asp Cys Pro Gly Asp Ala Leu Phe Asp Leu Leu Arg
   1 5 10
<210> 296
   <211> 9
   <212> PRT
   <213> Homo sapiens
<400> 296
   Ala Ala Glu Gln Asp Pro Glu Ala Arg
   1 5
<210> 297
   <211> 14
   <212> PRT
   <213> Homo sapiens
<400> 297
   Leu Gln Ala Leu Val Asn Ser Leu Cys Ala Gly Gln Ser Pro
   1 5 10
<210> 298
   <211> 14
   <212> PRT
   <213> Homo sapiens
<400> 298
   Leu Arg Ser Pro Ala Gln Tyr Gln Val Val Leu Ser Glu Arg
   1 5 10
<210> 299
   <211> 12
   <212> PRT
   <213> Homo sapiens
<400> 299
   Asp Ser Pro Ser Val Trp Ala Ala Val Pro Gly Lys
   1 5 10
<210> 300
   <211> 11
   <212> PRT
   <213> Homo sapiens
<400> 300
   Glu Gly Val His Gly Gly Leu Ile Asn Lys Lys
   1 5 10
<210> 301
   <211> 14
   <212> PRT
   <213> Homo sapiens
<400> 301
   Ser Ser Phe Tyr Val Asn Gly Leu Thr Leu Gly Gly Gln Lys
   1               5                   10
<210> 302
   <211> 19
   <212> PRT
   <213> Homo sapiens
<400> 302
   Ser Ser Phe Tyr Val Asn Gly Leu Thr Leu Gly Gly Gln Lys Cys Ser
   1               5                   10                  15
   Val Ile Arg
<210> 303
   <211> 14
   <212> PRT
   <213> Homo sapiens
<400> 303
   Ser Thr Gly Gly Ala Pro Thr Phe Asn Val Thr Val Thr Lys
   1               5                   10
<210> 304
   <211> 17
   <212> PRT
   <213> Homo sapiens
<400> 304
   Ser Thr Gly Gly Ala Pro Thr Phe Asn Val Thr Val Thr Lys Thr Asp
   1               5                   10                  15
   Lys
<210> 305
   <211> 16
   <212> PRT
   <213> Homo sapiens
<400> 305
   Thr Phe Val Asn Ile Thr Pro Ala Glu Val Gly Val Leu Val Gly Lys
   1               5                   10                  15
<210> 306
   <211> 18
   <212> PRT
   <213> Homo sapiens
<400> 306
   Thr Phe Val Asn Ile Thr Pro Ala Glu Val Gly Val Leu Val Gly Lys
   1 5 10 15
   Asp Arg
<210> 307
   <211> 16
   <212> PRT
   <213> Homo sapiens
<400> 307
   Thr Lys Ser Thr Gly Gly Ala Pro Thr Phe Asn Val Thr Val Thr Lys
   1 5 10 15
<210> 308
   <211> 19
   <212> PRT
   <213> Homo sapiens
<400> 308
   Thr Lys Ser Thr Gly Gly Ala Pro Thr Phe Asn Val Thr Val Thr Lys
   1 5 10 15
   Thr Asp Lys
<210> 309
   <211> 10
   <212> PRT
   <213> Homo sapiens
<400> 309
   Ala Thr Phe Ile Ser Val Gln Leu Lys Lys
   1 5 10
<210> 310
   <211> 11
   <212> PRT
   <213> Homo sapiens
<400> 310
   Phe Tyr Asn Glu Leu Thr Glu Ile Leu Val Arg
   1 5 10
<210> 311
   <211> 14
   <212> PRT
   <213> Homo sapiens
<400> 311
   Leu Ala Asn Gln Ala Ala Asp Tyr Phe Gly Asp Ala Phe Lys
   1 5 10
<210> 312
   <211> 13
   <212> PRT
   <213> Homo sapiens
<400> 312
   Leu Leu Asp Glu Glu Glu Ala Thr Asp Asn Asp Leu Arg
   1 5 10
<210> 313
   <211> 16
   <212> PRT
   <213> Homo sapiens
<400> 313
   Ser Val Ile Glu Gln Gly Gly Ile Gln Thr Val Asp Gln Leu Ile Lys
   1 5 10 15
<210> 314
   <211> 26
   <212> PRT
   <213> Homo sapiens
<400> 314
   Cys Ile Trp Gly Pro Ser Tyr Trp Cys Gln Asn Thr Glu Thr Ala Ala
   1 5 10 15
   Gln Cys Asn Ala Val Glu His Cys Lys Arg
   20 25
<210> 315
   <211> 15
   <212> PRT
   <213> Homo sapiens
<400> 315
   Glu Ile Val Asp Ser Tyr Leu Pro Val Ile Leu Asp Ile Ile Lys
   1 5 10 15
<210> 316
   <211> 10
   <212> PRT
   <213> Homo sapiens
<400> 316
   Gly Ser Ala Val Trp Cys Gln Asn Val Lys
   1 5 10
<210> 317
   <211> 13
   <212> PRT
   <213> Homo sapiens
<400> 317
   His Cys Leu Gln Thr Val Trp Asn Lys Pro Thr Val Lys
   1 5 10
<210> 318
   <211> 9
   <212> PRT
   <213> Homo sapiens
<400> 318
   His Leu Ala Glu Leu Asn His Gln Lys
   1 5
<210> 319
   <211> 12
   <212> PRT
   <213> Homo sapiens
<400> 319
   Ala Phe Ile Gln Leu Trp Ala Phe Asp Ala Val Lys
   1 5 10
<210> 320
   <211> 14
   <212> PRT
   <213> Homo sapiens
<400> 320
   Ala Phe Ile Gln Leu Trp Ala Phe Asp Ala Val Lys Gly Lys
   1 5 10
<210> 321
   <211> 15
   <212> PRT
   <213> Homo sapiens
<400> 321
   Cys Val Leu Phe Pro Tyr Gly Gly Cys Gln Gly Asn Gly Asn Lys
   1 5 10 15
<210> 322
   <211> 15
   <212> PRT
   <213> Homo sapiens
<400> 322
   Thr Val Ala Ala Cys Asn Leu Pro Ile Val Arg Gly Pro Cys Arg
   1 5 10 15
<210> 323
   <211> 8
   <212> PRT
   <213> Homo sapiens
<400> 323
   Glu Thr Leu Leu Gln Asp Phe Arg
   1 5
<210> 324
   <211> 15
   <212> PRT
   <213> Homo sapiens
<400> 324
   Glu Tyr Cys Gly Val Pro Gly Asp Gly Asp Glu Glu Leu Leu Arg
   1 5 10 15
<210> 325
   <211> 11
   <212> PRT
   <213> Homo sapiens
<400> 325
   Thr Val Ala Ala Cys Asn Leu Pro Ile Val Arg
   1 5 10
<210> 326
   <211> 20
   <212> PRT
   <213> Homo sapiens
<400> 326
   Asp Lys Leu Ala Ala Cys Leu Glu Gly Asn Cys Ala Glu Gly Leu Gly
   1 5 10 15
   Thr Asn Tyr Arg
   20
<210> 327
   <211> 10
   <212> PRT
   <213> Homo sapiens
<400> 327
   Glu Leu Leu Glu Ser Tyr Ile Asp Gly Arg
   1 5 10
<210> 328
   <211> 16
   <212> PRT
   <213> Homo sapiens
<400> 328
   Lys Ser Pro Gln Glu Leu Leu Cys Gly Ala Ser Leu Ile Ser Asp Arg
   1 5 10 15
<210> 329
   <211> 19
   <212> PRT
   <213> Homo sapiens
<400> 329
   Leu Ala Val Thr Thr His Gly Leu Pro Cys Leu Ala Trp Ala Ser Ala
   1 5 10 15
   Gln Ala Lys
<210> 330
   <211> 9
   <212> PRT
   <213> Homo sapiens
<400> 330
   Ser Gly Ile Glu Cys Gln Leu Trp Arg
   1 5
<210> 331
   <211> 13
   <212> PRT
   <213> Homo sapiens
<400> 331
   Thr Ala Thr Ser Glu Tyr Gln Thr Phe Phe Asn Pro Arg
   1 5 10
<210> 332
   <211> 26
   <212> PRT
   <213> Homo sapiens
<400> 332
   Ser Glu Gly Ser Ser Val Asn Leu Ser Pro Pro Leu Glu Gln Cys Val
   1 5 10 15
   Pro Asp Arg Gly Gln Gln Tyr Gln Gly Arg
   20 25
<210> 333
   <211> 15
   <212> PRT
   <213> Homo sapiens
<400> 333
   Ala Asn Asn Thr Phe Tyr Gly Leu Ser Ala Gly Val Phe Thr Lys
   1 5 10 15
<210> 334
   <211> 14
   <212> PRT
   <213> Homo sapiens
<400> 334
   Glu Leu Gly Glu Tyr Gly Phe His Glu Tyr Thr Glu Val Lys
   1 5 10
<210> 335
   <211> 10
   <212> PRT
   <213> Homo sapiens
<400> 335
   Phe Lys Ser Leu Asp Asp Val Ile Lys Arg
   1 5 10
<210> 336
   <211> 17
   <212> PRT
   <213> Homo sapiens
<400> 336
   Gly Tyr Phe Val Gln Pro Thr Val Phe Ser Asn Val Thr Asp Glu Met
   1 5 10 15
   Arg
<210> 337
   <211> 14
   <212> PRT
   <213> Homo sapiens
<400> 337
   Ile Phe Ile Asn Asn Glu Trp His Asp Ser Val Ser Gly Lys
   1 5 10
<210> 338
   <211> 13
   <212> PRT
   <213> Homo sapiens
<400> 338
   Ile Phe Val Glu Glu Ser Ile Tyr Asp Glu Phe Val Arg
   1 5 10
<210> 339
   <211> 14
   <212> PRT
   <213> Homo sapiens
<400> 339
   Ile Phe Val Glu Glu Ser Ile Tyr Asp Glu Phe Val Arg Arg
   1 5 10
<210> 340
   <211> 31
   <212> PRT
   <213> Homo sapiens
<400> 340
   Ile Gly Pro Ala Leu Ser Cys Gly Asn Thr Val Val Val Lys Pro Ala
   1 5 10 15
   Glu Gln Thr Pro Leu Thr Ala Leu His Val Ala Ser Leu Ile Lys
   20 25 30
<210> 341
   <211> 10
   <212> PRT
   <213> Homo sapiens
<400> 341
   Ile Leu Asp Leu Ile Glu Ser Gly Lys Lys
   1 5 10
<210> 342
   <211> 26
   <212> PRT
   <213> Homo sapiens
<400> 342
   Lys Phe Pro Val Phe Asn Pro Ala Thr Glu Glu Glu Leu Cys Gln Val
   1 5 10 15
   Glu Glu Gly Asp Lys Glu Asp Val Asp Lys
   20 25
<210> 343
   <211> 25
   <212> PRT
   <213> Homo sapiens
<400> 343
   Lys Tyr Ile Leu Gly Asn Pro Leu Thr Pro Gly Val Thr Gln Gly Pro
   1 5 10 15
   Gln Ile Asp Lys Glu Gln Tyr Asp Lys
   20 25
<210> 344
   <211> 7
   <212> PRT
   <213> Homo sapiens
<400> 344
   Leu Ala Asp Leu Ile Glu Arg
   1 5
<210> 345
   <211> 11
   <212> PRT
   <213> Homo sapiens
<400> 345
   Leu Glu Cys Gly Gly Gly Pro Trp Gly Asn Lys
   1 5 10
<210> 346
   <211> 11
   <212> PRT
   <213> Homo sapiens
<400> 346
   Leu Leu Tyr Lys Leu Ala Asp Leu Ile Glu Arg
   1 5 10
<210> 347
   <211> 15
   <212> PRT
   <213> Homo sapiens
<400> 347
   Leu Tyr Ser Asn Ala Tyr Leu Asn Asp Leu Ala Gly Cys Ile Lys
   1 5 10 15
<210> 348
   <211> 16
   <212> PRT
   <213> Homo sapiens
<400> 348
   Arg Ala Asn Asn Thr Phe Tyr Gly Leu Ser Ala Gly Val Phe Thr Lys
   1 5 10 15
<210> 349
   <211> 23
   <212> PRT
   <213> Homo sapiens
<400> 349
   Ser Leu Asp Asp Val Ile Lys Arg Ala Asn Asn Thr Phe Tyr Gly Leu
   1 5 10 15
   Ser Ala Gly Val Phe Thr Lys
   20
<210> 350
   <211> 16
   <212> PRT
   <213> Homo sapiens
<400> 350
   Ser Ser Ser Gly Thr Pro Asp Leu Pro Val Leu Leu Thr Asp Leu Lys
   1 5 10 15
<210> 351
   <211> 13
   <212> PRT
   <213> Homo sapiens
<400> 351
   Thr Ile Pro Ile Asp Gly Asn Phe Phe Thr Tyr Thr Arg
   1 5 10
<210> 352
   <211> 12
   <212> PRT
   <213> Homo sapiens
<400> 352
   Tyr Cys Ala Gly Trp Ala Asp Lys Ile Gln Gly Arg
   1 5 10
<210> 353
   <211> 24
   <212> PRT
   <213> Homo sapiens
<400> 353
   Tyr Ile Leu Gly Asn Pro Leu Thr Pro Gly Val Thr Gln Gly Pro Gln
   1 5 10 15
   Ile Asp Lys Glu Gln Tyr Asp Lys
   20
<210> 354
   <211> 11
   <212> PRT
   <213> Homo sapiens
<400> 354
   Val Ala Phe Thr Gly Ser Thr Glu Val Gly Lys
   1 5 10
<210> 355
   <211> 9
   <212> PRT
   <213> Homo sapiens
<400> 355
   Asp Leu Cys Gly Ile Val Ala Ser Lys
   1 5
<210> 356
   <211> 14
   <212> PRT
   <213> Homo sapiens
<400> 356
   Glu Leu Cys Gln Gly Leu Gly Gln Pro Gly Ser Val Leu Arg
   1 5 10
<210> 357
   <211> 15
   <212> PRT
   <213> Homo sapiens
<400> 357
   Glu Leu Ser Leu Ala Gly Asn Glu Leu Gly Asp Glu Gly Ala Arg
   1 5 10 15
<210> 358
   <211> 10
   <212> PRT
   <213> Homo sapiens
<400> 358
   Leu Asp Asp Cys Gly Leu Thr Glu Ala Arg
   1 5 10
<210> 359
   <211> 22
   <212> PRT
   <213> Homo sapiens
<400> 359
   Ser Asn Glu Leu Gly Asp Val Gly Val His Cys Val Leu Gln Gly Leu
   1 5 10 15
   Gln Thr Pro Ser Cys Lys
   20
<210> 360
   <211> 12
   <212> PRT
   <213> Homo sapiens
<400> 360
   Thr Leu Trp Ile Trp Glu Cys Gly Ile Thr Ala Lys
   1 5 10
<210> 361
   <211> 11
   <212> PRT
   <213> Homo sapiens
<400> 361
   Val Asn Pro Ala Leu Ala Glu Leu Asn Leu Arg
   1 5 10
<210> 362
   <211> 15
   <212> PRT
   <213> Homo sapiens
<400> 362
   Trp Ala Glu Leu Leu Pro Leu Leu Gln Gln Cys Gln Val Val Arg
   1 5 10 15
<210> 363
   <211> 12
   <212> PRT
   <213> Homo sapiens
<400> 363
   Leu Glu Ser Cys Gly Val Thr Ser Asp Asn Cys Arg
   1 5 10
<210> 364
   <211> 10
   <212> PRT
   <213> Homo sapiens
<400> 364
   Glu Leu Asp Leu Asn Ser Val Leu Leu Lys
   1 5 10
<210> 365
   <211> 13
   <212> PRT
   <213> Homo sapiens
<400> 365
   Gln Glu Val Trp Leu Ala Asn Gly Ala Ala Glu Ser Arg
   1 5 10
<210> 366
   <211> 21
   <212> PRT
   <213> Homo sapiens
<400> 366
   Leu Gly Ile Lys Pro Ser Ile Asn Tyr Tyr Gln Val Ala Asp Phe Lys
   1               5                   10                  15
   Asp Ala Leu Ala Arg
               20
<210> 367
   <211> 16
   <212> PRT
   <213> Homo sapiens
<400> 367
   His Gly Thr Cys Ala Ala Gln Val Asp Ala Leu Asn Ser Gln Lys Lys
   1               5                   10                  15
<210> 368
   <211> 15
   <212> PRT
   <213> Homo sapiens
<400> 368
   His Gly Thr Cys Ala Ala Gln Val Asp Ala Leu Asn Ser Gln Lys
   1               5                   10                  15
<210> 369
   <211> 8
   <212> PRT
   <213> Homo sapiens
<400> 369
   Leu Ala Leu Leu Glu Glu Ala Arg
   1               5
<210> 370
   <211> 11
   <212> PRT
   <213> Homo sapiens
<400> 370
   Ala Leu Asn Ser Ile Ile Asp Val Tyr His Lys
   1               5                   10
<210> 371
   <211> 16
   <212> PRT
   <213> Homo sapiens
<400> 371
   Ala Leu Asn Ser Ile Ile Asp Val Tyr His Lys Tyr Ser Leu Ile Lys
   1 5 10 15
<210> 372
   <211> 16
   <212> PRT
   <213> Homo sapiens
<400> 372
   Asp Asp Leu Lys Lys Leu Leu Glu Thr Glu Cys Pro Gln Tyr Ile Arg
   1 5 10 15
<210> 373
   <211> 21
   <212> PRT
   <213> Homo sapiens
<400> 373
   Glu Leu Asp Ile Asn Thr Asp Gly Ala Val Asn Phe Gln Glu Phe Leu
   1 5 10 15
   Ile Leu Val Ile Lys
   20
<210> 374
   <211> 7
   <212> PRT
   <213> Homo sapiens
<400> 374
   Gly Ala Asp Val Trp Phe Lys
   1 5
<210> 375
   <211> 8
   <212> PRT
   <213> Homo sapiens
<400> 375
   Gly Asn Phe His Ala Val Tyr Arg
   1 5
<210> 376
   <211> 12
   <212> PRT
   <213> Homo sapiens
<400> 376
   Gly Asn Phe His Ala Val Tyr Arg Asp Asp Leu Lys
   1 5 10
<210> 377
   <211> 13
   <212> PRT
   <213> Homo sapiens
<400> 377
   Gly Asn Phe His Ala Val Tyr Arg Asp Asp Leu Lys Lys
   1 5 10
<210> 378
   <211> 12
   <212> PRT
   <213> Homo sapiens
<400> 378
   Lys Leu Leu Glu Thr Glu Cys Pro Gln Tyr Ile Arg
   1 5 10
<210> 379
   <211> 11
   <212> PRT
   <213> Homo sapiens
<400> 379
   Leu Leu Glu Thr Glu Cys Pro Gln Tyr Ile Arg
   1 5 10
<210> 380
   <211> 8
   <212> PRT
   <213> Homo sapiens
<400> 380
   Met Gly Val Ala Ala His Lys Lys
   1 5
<210> 381
   <211> 7
   <212> PRT
   <213> Homo sapiens
<400> 381
   Met Leu Thr Glu Leu Glu Lys
   1 5
<210> 382
   <211> 7
   <212> PRT
   <213> Homo sapiens
<400> 382
   Asp Leu Gln Asn Phe Leu Lys
   1 5
<210> 383
   <211> 8
   <212> PRT
   <213> Homo sapiens
<400> 383
   Asp Leu Gln Asn Phe Leu Lys Lys
   1 5
<210> 384
   <211> 11
   <212> PRT
   <213> Homo sapiens
<400> 384
   Asp Leu Gln Asn Phe Leu Lys Lys Glu Asn Lys
   1 5 10
<210> 385
   <211> 12
   <212> PRT
   <213> Homo sapiens
<400> 385
   Glu Leu Val Arg Lys Asp Leu Gln Asn Phe Leu Lys
   1 5 10
<210> 386
   <211> 8
   <212> PRT
   <213> Homo sapiens
<400> 386
   Lys Asp Leu Gln Asn Phe Leu Lys
   1 5
<210> 387
   <211> 9
   <212> PRT
   <213> Homo sapiens
<400> 387
   Lys Asp Leu Gln Asn Phe Leu Lys Lys
   1 5
<210> 388
   <211> 13
   <212> PRT
   <213> Homo sapiens
<400> 388
   Leu Gly His Pro Asp Thr Leu Asn Gln Gly Glu Phe Lys
   1 5 10
<210> 389
   <211> 17
   <212> PRT
   <213> Homo sapiens
<400> 389
   Leu Gly His Pro Asp Thr Leu Asn Gln Gly Glu Phe Lys Glu Leu Val
   1 5 10 15
   Arg
<210> 390
   <211> 8
   <212> PRT
   <213> Homo sapiens
<400> 390
   Leu Thr Trp Ala Ser His Glu Lys
   1 5
<210> 391
   <211> 21
   <212> PRT
   <213> Homo sapiens
<400> 391
   Met His Glu Gly Asp Glu Gly Pro Gly His His His Lys Pro Gly Leu
   1 5 10 15
   Gly Glu Gly Thr Pro
   20
<210> 392
   <211> 15
   <212> PRT
   <213> Homo sapiens
<400> 392
   Asn Ile Glu Thr Ile Ile Asn Thr Phe His Gln Tyr Ser Val Lys
   1 5 10 15
<210> 393
   <211> 28
   <212> PRT
   <213> Homo sapiens
<400> 393
   Asn Ile Glu Thr Ile Ile Asn Thr Phe His Gln Tyr Ser Val Lys Leu
   1 5 10 15
   Gly His Pro Asp Thr Leu Asn Gln Gly Glu Phe Lys
   20 25
<210> 394
   <211> 9
   <212> PRT
   <213> Homo sapiens
<400> 394
   Thr Cys Lys Met Ser Gln Leu Glu Arg
   1 5
<210> 395
   <211> 15
   <212> PRT
   <213> Homo sapiens
<400> 395
   Val Ile Glu His Ile Met Glu Asp Leu Asp Thr Asn Ala Asp Lys
   1 5 10 15
<210> 396
   <211> 17
   <212> PRT
   <213> Homo sapiens
<400> 396
   Ala Cys Pro Leu Asp Gln Ala Ile Gly Leu Leu Val Ala Ile Phe His
   1 5 10 15
   Lys
<210> 397
   <211> 12
   <212> PRT
   <213> Homo sapiens
<400> 397
   Glu Leu Ile Gln Lys Glu Leu Thr Ile Gly Ser Lys
   1 5 10
<210> 398
   <211> 20
   <212> PRT
   <213> Homo sapiens
<400> 398
   Glu Leu Ile Gln Lys Glu Leu Thr Ile Gly Ser Lys Leu Gln Asp Ala
   1               5                   10                  15
   Glu Ile Ala Arg
               20
<210> 399
   <211> 15
   <212> PRT
   <213> Homo sapiens
<400> 399
   Glu Leu Lys Glu Leu Ile Gln Lys Glu Leu Thr Ile Gly Ser Lys
   1               5                   10                  15
<210> 400
   <211> 15
   <212> PRT
   <213> Homo sapiens
<400> 400
   Glu Leu Thr Ile Gly Ser Lys Leu Gln Asp Ala Glu Ile Ala Arg
   1               5                   10                  15
<210> 401
   <211> 9
   <212> PRT
   <213> Homo sapiens
<400> 401
   Lys Glu Leu Lys Glu Leu Ile Gln Lys
   1               5
<210> 402
   <211> 8
   <212> PRT
   <213> Homo sapiens
<400> 402
   Leu Gln Asp Ala Glu Ile Ala Arg
   1               5
<210> 403
   <211> 28
   <212> PRT
   <213> Homo sapiens
<400> 403
   Asn Lys Asp Gln Glu Val Asn Phe Gln Glu Tyr Val Thr Phe Leu Gly
   1 5 10 15
   Ala Leu Ala Leu Ile Tyr Asn Glu Ala Leu Lys Gly
   20 25
<210> 404
   <211> 13
   <212> PRT
   <213> Homo sapiens
<400> 404
   Tyr Ser Gly Arg Glu Gly Asp Lys His Thr Leu Ser Lys
   1 5 10
<210> 405
   <211> 16
   <212> PRT
   <213> Homo sapiens
<400> 405
   Phe Pro Glu Pro Gly Ala Ile Lys Val Pro Glu Gln Gly Tyr Thr Lys
   1 5 10 15
<210> 406
   <211> 17
   <212> PRT
   <213> Homo sapiens
<400> 406
   Leu Pro Glu Pro Cys Pro Ser Thr Val Thr Pro Gly Pro Ala Gln Gln
   1 5 10 15
   Lys
<210> 407
   <211> 16
   <212> PRT
   <213> Homo sapiens
<400> 407
   Gln Pro Ser Gln Pro Pro Pro Gln Glu Ile Phe Val Pro Thr Thr Lys
   1 5 10 15
<210> 408
   <211> 22
   <212> PRT
   <213> Homo sapiens
<400> 408
   Gln Pro Ser Gln Pro Pro Pro Gln Glu Ile Phe Val Pro Thr Thr Lys
   1               5                   10                  15
   Glu Pro Cys His Ser Lys
               20
<210> 409
   <211> 15
   <212> PRT
   <213> Homo sapiens
<400> 409
   Gln Thr Phe Thr Pro Pro Pro Gln Leu Gln Gln Gln Gln Val Lys
   1               5                   10                  15
<210> 410
   <211> 16
   <212> PRT
   <213> Homo sapiens
<400> 410
   Val Pro Asp Gln Gly Phe Ile Lys Phe Pro Glu Pro Gly Ala Ile Lys
   1               5                   10                  15
<210> 411
   <211> 16
   <212> PRT
   <213> Homo sapiens
<400> 411
   Val Pro Glu Pro Gly Cys Thr Lys Val Pro Glu Pro Gly Cys Thr Lys
   1               5                   10                  15
<210> 412
   <211> 16
   <212> PRT
   <213> Homo sapiens
<400> 412
   Val Pro Gln Pro Gly Asn Thr Lys Ile Pro Glu Pro Gly Cys Thr Lys
   1               5                   10                  15
<210> 413
   <211> 12
   <212> PRT
   <213> Homo sapiens
<400> 413
   Val Ala Gly Gln Asp Gly Ser Val Val Gln Phe Lys
   1               5                   10
<210> 414
   <211> 10
   <212> PRT
   <213> Homo sapiens
<400> 414
   Glu Glu Asp Ile Asp Glu Asn Leu Leu Phe
   1 5 10
<210> 415
   <211> 29
   <212> PRT
   <213> Homo sapiens
<400> 415
   Glu His Ala Asn Ser Lys Gln Glu Glu Asp Asn Thr Gln Ser Asp Asp
   1 5 10 15
   Ile Leu Glu Glu Ser Asp Gln Pro Thr Gln Val Ser Lys
   20 25
<210> 416
   <211> 15
   <212> PRT
   <213> Homo sapiens
<400> 416
   Thr Gly Leu Glu Ala Ile Ser Asn His Lys Glu Thr Glu Glu Lys
   1 5 10 15
<210> 417
   <211> 13
   <212> PRT
   <213> Homo sapiens
<400> 417
   Gly His Gln Leu Gln Leu Asp Tyr Phe Gly Ala Cys Lys
   1 5 10
<210> 418
   <211> 19
   <212> PRT
   <213> Homo sapiens
<400> 418
   His Ser Ala Ser Asp Asp Tyr Phe Ile Pro Ser Gln Ala Phe Leu Glu
   1 5 10 15
   Ala Glu Arg
<210> 419
   <211> 21
   <212> PRT
   <213> Homo sapiens
<400> 419
   Asn His Gly Val Asp Asp Asp Gly Asp Asp Asp Gly Asp Asp Gly Gly
   1 5 10 15
   Thr Asp Gly Pro Arg
   20
<210> 420
   <211> 16
   <212> PRT
   <213> Homo sapiens
<400> 420
   Ser Ile Pro Thr Cys Thr Asp Phe Glu Val Ile Gln Phe Pro Leu Arg
   1 5 10 15
<210> 421
   <211> 8
   <212> PRT
   <213> Homo sapiens
<400> 421
   Ala Asn Phe Glu Asn Leu Ala Lys
   1 5
<210> 422
   <211> 13
   <212> PRT
   <213> Homo sapiens
<400> 422
   Tyr Gly Leu Phe Pro Ala Asn Tyr Val Glu Leu Arg Gln
   1 5 10
<210> 423
   <211> 10
   <212> PRT
   <213> Homo sapiens
<400> 423
   Tyr Gly Val Gln Ala Asp Arg Val Asp Lys
   1 5 10
<210> 424
   <211> 14
   <212> PRT
   <213> Homo sapiens
<400> 424
   Ala Asp Asp Leu Gly Lys Gly Gly Asn Glu Glu Ser Thr Lys
   1               5                   10
<210> 425
   <211> 21
   <212> PRT
   <213> Homo sapiens
<400> 425
   Ala Asp Asp Leu Gly Lys Gly Gly Asn Glu Glu Ser Thr Lys Thr Gly
   1               5                   10                  15
   Asn Ala Gly Ser Arg
               20
<210> 426
   <211> 23
   <212> PRT
   <213> Homo sapiens
<400> 426
   Ala Thr Lys Ala Val Cys Val Leu Lys Gly Asp Gly Pro Val Gln Gly
   1               5                   10                  15
   Ile Ile Asn Phe Glu Gln Lys
               20
<210> 427
   <211> 20
   <212> PRT
   <213> Homo sapiens
<400> 427
   Ala Val Cys Val Leu Lys Gly Asp Gly Pro Val Gln Gly Ile Ile Asn
   1               5                   10                  15
   Phe Glu Gln Lys
               20
<210> 428
   <211> 13
   <212> PRT
   <213> Homo sapiens
<400> 428
   Glu Ser Asn Gly Pro Val Lys Val Trp Gly Ser Ile Lys
   1               5                   10
<210> 429
   <211> 14
   <212> PRT
   <213> Homo sapiens
<400> 429
   Gly Asp Gly Pro Val Gln Gly Ile Ile Asn Phe Glu Gln Lys
   1 5 10
<210> 430
   <211> 36
   <212> PRT
   <213> Homo sapiens
<400> 430
   His Val Gly Asp Leu Gly Asn Val Thr Ala Asp Lys Asp Gly Val Ala
   1 5 10 15
   Asp Val Ser Ile Glu Asp Ser Val Ile Ser Leu Ser Gly Asp His Cys
   20 25 30
   Ile Ile Gly Arg
   35
<210> 431
   <211> 14
   <212> PRT
   <213> Homo sapiens
<400> 431
   Ala Ile Trp Asn Val Ile Asn Trp Glu Asn Val Thr Glu Arg
   1 5 10
<210> 432
   <211> 14
   <212> PRT
   <213> Homo sapiens
<400> 432
   Gly Asp Val Thr Ala Gln Ile Ala Leu Gln Pro Ala Leu Lys
   1 5 10
<210> 433
   <211> 19
   <212> PRT
   <213> Homo sapiens
<400> 433
   Asp Ala Gly Gly Lys Ser Trp Cys Pro Asp Cys Val Gln Ala Glu Pro
   1 5 10 15
   Val Val Arg
<210> 434
   <211> 14
   <212> PRT
   <213> Homo sapiens
<400> 434
   Ser Trp Cys Pro Asp Cys Val Gln Ala Glu Pro Val Val Arg
   1               5                   10
<210> 435
   <211> 10
   <212> PRT
   <213> Homo sapiens
<400> 435
   Thr Ile Phe Ala Tyr Phe Thr Gly Ser Lys
   1               5                   10
<210> 436
   <211> 13
   <212> PRT
   <213> Homo sapiens
<400> 436
   Ala Ala Asp Asp Thr Trp Glu Pro Phe Ala Ser Gly Lys
   1               5                   10
<210> 437
   <211> 13
   <212> PRT
   <213> Homo sapiens
<400> 437
   Gly Ser Pro Ala Ile Asn Val Ala Val His Val Phe Arg
   1               5                   10
<210> 438
   <211> 14
   <212> PRT
   <213> Homo sapiens
<400> 438
   Lys Ala Ala Asp Asp Thr Trp Glu Pro Phe Ala Ser Gly Lys
   1               5                   10
<210> 439
   <211> 24
   <212> PRT
   <213> Homo sapiens
<400> 439
   Arg Tyr Thr Ile Ala Ala Leu Leu Ser Pro Tyr Ser Tyr Ser Thr Thr
   1               5                   10                  15
   Ala Val Val Thr Asn Pro Lys Glu
               20
<210> 440
   <211> 22
   <212> PRT
   <213> Homo sapiens
<400> 440
   Thr Ser Glu Ser Gly Glu Leu His Gly Leu Thr Thr Glu Glu Glu Phe
   1               5                   10                  15
   Val Glu Gly Ile Tyr Lys
               20
<210> 441
   <211> 19
   <212> PRT
   <213> Homo sapiens
<400> 441
   Val Leu Asp Ala Val Arg Gly Ser Pro Ala Ile Asn Val Ala Val His
   1               5                   10                  15
   Val Phe Arg
<210> 442
   <211> 23
   <212> PRT
   <213> Homo sapiens
<400> 442
   Tyr Thr Ile Ala Ala Leu Leu Ser Pro Tyr Ser Tyr Ser Thr Thr Ala
   1               5                   10                  15
   Val Val Thr Asn Pro Lys Glu
               20
<210> 443
   <211> 11
   <212> PRT
   <213> Homo sapiens
<400> 443
   Ala Gly Ile Thr Thr Ile Glu Ala Val Lys Arg
   1               5                   10
<210> 444
   <211> 15
   <212> PRT
   <213> Homo sapiens
<400> 444
   His Ile Ala Glu Glu Ala Asp Arg Lys Tyr Glu Glu Val Ala Arg
   1               5                   10                  15
<210> 445
   <211> 10
   <212> PRT
   <213> Homo sapiens
<400> 445
   Ile Gln Leu Val Glu Glu Glu Leu Asp Arg
   1               5                   10
<210> 446
   <211> 14
   <212> PRT
   <213> Homo sapiens
<400> 446
   Ile Gln Leu Val Glu Glu Glu Leu Asp Arg Ala Gln Glu Arg
   1               5                   10
<210> 447
   <211> 14
   <212> PRT
   <213> Homo sapiens
<400> 447
   Ile Gln Val Leu Gln Gln Gln Ala Asp Asp Ala Glu Glu Arg
   1               5                   10
<210> 448
   <211> 17
   <212> PRT
   <213> Homo sapiens
<400> 448
   Ile Gln Val Leu Gln Gln Gln Ala Asp Asp Ala Glu Glu Arg Ala Glu
   1               5                   10                  15
   Arg
<210> 449
   <211> 15
   <212> PRT
   <213> Homo sapiens
<400> 449
   Lys Ile Gln Val Leu Gln Gln Gln Ala Asp Asp Ala Glu Glu Arg
   1 5 10 15
<210> 450
   <211> 15
   <212> PRT
   <213> Homo sapiens
<400> 450
   Arg Ile Gln Leu Val Glu Glu Glu Leu Asp Arg Ala Gln Glu Arg
   1 5 10 15
<210> 451
   <211> 10
   <212> PRT
   <213> Homo sapiens
<400> 451
   Thr Ile Asp Asp Leu Glu Asp Lys Leu Lys
   1 5 10
<210> 452
   <211> 16
   <212> PRT
   <213> Homo sapiens
<400> 452
   Thr Ile Thr Leu Glu Val Glu Pro Ser Asp Thr Ile Glu Asn Val Lys
   1 5 10 15
<210> 453
   <211> 16
   <212> PRT
   <213> Homo sapiens
<400> 453
   Asp Glu Arg Lys Asn Pro Asp Ser Gln Tyr Gly Glu Leu Ile Glu Lys
   1 5 10 15
<210> 454
   <211> 17
   <212> PRT
   <213> Homo sapiens
<400> 454
   Ile Val Glu Lys Tyr Gly Tyr Thr His Leu Ser Ala Gly Glu Leu Leu
   1 5 10 15
   Arg
<210> 455
   <211> 13
   <212> PRT
   <213> Homo sapiens
<400> 455
   Lys Asn Pro Asp Ser Gln Tyr Gly Glu Leu Ile Glu Lys
   1 5 10
<210> 456
   <211> 10
   <212> PRT
   <213> Homo sapiens
<400> 456
   Asn Lys Phe Leu Ile Asp Gly Phe Pro Arg
   1 5 10
<210> 457
   <211> 10
   <212> PRT
   <213> Homo sapiens
<400> 457
   Asn Gln Asp Asn Leu Gln Gly Trp Asn Lys
   1 5 10
<210> 458
   <211> 11
   <212> PRT
   <213> Homo sapiens
<400> 458
   Ser Asp Asp Asn Arg Glu Ser Leu Glu Lys Arg
   1 5 10
<210> 459
   <211> 13
   <212> PRT
   <213> Homo sapiens
<400> 459
   Tyr Gly Tyr Thr His Leu Ser Ala Gly Glu Leu Leu Arg
   1 5 10
<210> 460
   <211> 7
   <212> PRT
   <213> Homo sapiens
<400> 460
   Ala Cys Gln Gln Phe Leu Lys
   1 5
<210> 461
   <211> 9
   <212> PRT
   <213> Homo sapiens
<400> 461
   Lys Gln Cys Pro Cys Asp His Phe Lys
   1 5
<210> 462
   <211> 17
   <212> PRT
   <213> Homo sapiens
<400> 462
   Trp Leu Gln Glu Gly Gly Gln Glu Cys Glu Cys Lys Asp Trp Phe Leu
   1 5 10 15
   Arg
<210> 463
   <211> 22
   <212> PRT
   <213> Homo sapiens
<400> 463
   Glu Val Val Ser Leu Thr Glu Ala Cys Cys Ala Glu Gly Ala Asp Pro
   1 5 10 15
   Asp Cys Tyr Asp Thr Arg
   20
<210> 464
   <211> 8
   <212> PRT
   <213> Homo sapiens
<400> 464
   Phe Glu Asp Cys Cys Gln Glu Lys
   1 5
<210> 465
   <211> 22
   <212> PRT
   <213> Homo sapiens
<400> 465
   His Gln Pro Gln Glu Phe Pro Thr Tyr Val Glu Pro Thr Asn Asp Glu
   1 5 10 15
   Ile Cys Glu Ala Phe Arg
   20
<210> 466
   <211> 26
   <212> PRT
   <213> Homo sapiens
<400> 466
   His Gln Pro Gln Glu Phe Pro Thr Tyr Val Glu Pro Thr Asn Asp Glu
   1 5 10 15
   Ile Cys Glu Ala Phe Arg Lys Asp Pro Lys
   20 25
<210> 467
   <211> 15
   <212> PRT
   <213> Homo sapiens
<400> 467
   Lys Phe Pro Ser Gly Thr Phe Glu Gln Val Ser Gln Leu Val Lys
   1 5 10 15
<210> 468
   <211> 11
   <212> PRT
   <213> Homo sapiens
<400> 468
   Leu Lys Ala Lys Leu Pro Asp Ala Thr Pro Lys
   1 5 10
<210> 469
   <211> 11
   <212> PRT
   <213> Homo sapiens
<400> 469
   Asn Ser Lys Phe Glu Asp Cys Cys Gln Glu Lys
   1 5 10
<210> 470
   <211> 11
   <212> PRT
   <213> Homo sapiens
<400> 470
   Arg Thr His Leu Pro Glu Val Phe Leu Ser Lys
   1 5 10
<210> 471
   <211> 20
   <212> PRT
   <213> Homo sapiens
<400> 471
   Ser Cys Glu Ser Asn Ser Pro Phe Pro Val His Pro Gly Thr Ala Glu
   1 5 10 15
   Cys Cys Thr Lys
   20
<210> 472
   <211> 18
   <212> PRT
   <213> Homo sapiens
<400> 472
   Ser Leu Gly Glu Cys Cys Asp Val Glu Asp Ser Thr Thr Cys Phe Asn
   1 5 10 15
   Ala Lys
<210> 473
   <211> 24
   <212> PRT
   <213> Homo sapiens
<400> 473
   Ser Leu Gly Glu Cys Cys Asp Val Glu Asp Ser Thr Thr Cys Phe Asn
   1 5 10 15
   Ala Lys Gly Pro Leu Leu Lys Lys
   20
<210> 474
   <211> 10
   <212> PRT
   <213> Homo sapiens
<400> 474
   Thr His Leu Pro Glu Val Phe Leu Ser Lys
   1 5 10
<210> 475
   <211> 14
   <212> PRT
   <213> Homo sapiens
<400> 475
   Val Cys Ser Gln Tyr Ala Ala Tyr Gly Glu Lys Lys Ser Arg
   1               5                   10
<210> 476
   <211> 11
   <212> PRT
   <213> Homo sapiens
<400> 476
   Val Met Asp Lys Tyr Thr Phe Glu Leu Ser Arg
   1               5                   10
<210> 477
   <211> 27
   <212> PRT
   <213> Homo sapiens
<400> 477
   Cys Gln Cys Asp Glu Leu Cys Ser Tyr Tyr Gln Ser Cys Cys Thr Asp
   1               5                   10                  15
   Tyr Thr Ala Glu Cys Lys Pro Gln Val Thr Arg
               20                  25
<210> 478
   <211> 15
   <212> PRT
   <213> Homo sapiens
<400> 478
   Asp Val Trp Gly Ile Glu Gly Pro Ile Asp Ala Ala Phe Thr Arg
   1               5                   10                  15
<210> 479
   <211> 10
   <212> PRT
   <213> Homo sapiens
<400> 479
   Gly Gln Tyr Cys Tyr Glu Leu Asp Glu Lys
   1               5                   10
<210> 480
   <211> 15
   <212> PRT
   <213> Homo sapiens
<400> 480
   Ser Ile Ala Gln Tyr Trp Leu Gly Cys Pro Ala Pro Gly His Leu
   1               5                   10                  15
<210> 481
   <211> 9
   <212> PRT
   <213> Homo sapiens
<400> 481
   Ala Leu Phe Val Ser Glu Glu Glu Lys
   1 5
<210> 482
   <211> 10
   <212> PRT
   <213> Homo sapiens
<400> 482
   Ala Leu Phe Val Ser Glu Glu Glu Lys Lys
   1 5 10
<210> 483
   <211> 8
   <212> PRT
   <213> Homo sapiens
<400> 483
   Cys Leu Val Asn Leu Ile Glu Lys
   1 5
<210> 484
   <211> 10
   <212> PRT
   <213> Homo sapiens
<400> 484
   Asp Ala Gln Tyr Ala Pro Gly Tyr Asp Lys
   1 5 10
<210> 485
   <211> 12
   <212> PRT
   <213> Homo sapiens
<400> 485
   Asp Ala Gln Tyr Ala Pro Gly Tyr Asp Lys Val Lys
   1 5 10
<210> 486
   <211> 21
   <212> PRT
   <213> Homo sapiens
<400> 486
   Asp Ala Gln Tyr Ala Pro Gly Tyr Asp Lys Val Lys Asp Ile Ser Glu
   1               5                   10                  15
   Val Val Thr Pro Arg
               20
<210> 487
   <211> 9
   <212> PRT
   <213> Homo sapiens
<400> 487
   Asp Ile Ser Glu Val Val Thr Pro Arg
   1               5
<210> 488
   <211> 16
   <212> PRT
   <213> Homo sapiens
<400> 488
   Glu Ala Gly Ile Pro Glu Phe Tyr Asp Tyr Asp Val Ala Leu Ile Lys
   1               5                   10                  15
<210> 489
   <211> 10
   <212> PRT
   <213> Homo sapiens
<400> 489
   Glu Glu Leu Leu Pro Ala Gln Asp Ile Lys
   1               5                   10
<210> 490
   <211> 11
   <212> PRT
   <213> Homo sapiens
<400> 490
   Glu Lys Leu Gln Asp Glu Asp Leu Gly Phe Leu
   1               5                   10
<210> 491
   <211> 17
   <212> PRT
   <213> Homo sapiens
<400> 491
   Phe Leu Cys Thr Gly Gly Val Ser Pro Tyr Ala Asp Pro Asn Thr Cys
   1               5                   10                  15
   Arg
<210> 492
   <211> 28
   <212> PRT
   <213> Homo sapiens
<400> 492
   Phe Leu Cys Thr Gly Gly Val Ser Pro Tyr Ala Asp Pro Asn Thr Cys
   1               5                   10                  15
   Arg Gly Asp Ser Gly Gly Pro Leu Ile Val His Lys
               20                  25
<210> 493
   <211> 29
   <212> PRT
   <213> Homo sapiens
<400> 493
   Phe Leu Cys Thr Gly Gly Val Ser Pro Tyr Ala Asp Pro Asn Thr Cys
   1               5                   10                  15
   Arg Gly Asp Ser Gly Gly Pro Leu Ile Val His Lys Arg
               20                  25
<210> 494
   <211> 9
   <212> PRT
   <213> Homo sapiens
<400> 494
   Lys Asp Asn Glu Gln His Val Phe Lys
   1               5
<210> 495
   <211> 11
   <212> PRT
   <213> Homo sapiens
<400> 495
   Leu Glu Asp Ser Val Thr Tyr His Cys Ser Arg
   1               5                   10
<210> 496
   <211> 24
   <212> PRT
   <213> Homo sapiens
<400> 496
   Leu Leu Gln Glu Gly Gln Ala Leu Glu Tyr Val Cys Pro Ser Gly Phe
   1 5 10 15
   Tyr Pro Tyr Pro Val Gln Thr Arg
   20
<210> 497
   <211> 11
   <212> PRT
   <213> Homo sapiens
<400> 497
   Leu Pro Pro Thr Thr Thr Cys Gln Gln Gln Lys
   1 5 10
<210> 498
   <211> 21
   <212> PRT
   <213> Homo sapiens
<400> 498
   Leu Pro Pro Thr Thr Thr Cys Gln Gln Gln Lys Glu Glu Leu Leu Pro
   1 5 10 15
   Ala Gln Asp Ile Lys
   20
<210> 499
   <211> 9
   <212> PRT
   <213> Homo sapiens
<400> 499
   Leu Gln Asp Glu Asp Leu Gly Phe Leu
   1 5
<210> 500
   <211> 9
   <212> PRT
   <213> Homo sapiens
<400> 500
   Ser Thr Gly Ser Trp Ser Thr Leu Lys
   1 5
<210> 501
   <211> 17
   <212> PRT
   <213> Homo sapiens
<400> 501
   Ser Thr Gly Ser Trp Ser Thr Leu Lys Thr Gln Asp Gln Lys Thr Val
   1               5                   10                  15
   Arg
<210> 502
   <211> 9
   <212> PRT
   <213> Homo sapiens
<400> 502
   Val Ala Ser Tyr Gly Val Lys Pro Arg
   1               5
<210> 503
   <211> 14
   <212> PRT
   <213> Homo sapiens
<400> 503
   Val Ser Glu Ala Asp Ser Ser Asn Ala Asp Trp Val Thr Lys
   1               5                   10
<210> 504
   <211> 25
   <212> PRT
   <213> Homo sapiens
<400> 504
   Trp Ser Gly Gln Thr Ala Ile Cys Asp Asn Gly Ala Gly Tyr Cys Ser
   1               5                   10                  15
   Asn Pro Gly Ile Pro Ile Gly Thr Arg
               20                  25
<210> 505
   <211> 26
   <212> PRT
   <213> Homo sapiens
<400> 505
   Trp Ser Gly Gln Thr Ala Ile Cys Asp Asn Gly Ala Gly Tyr Cys Ser
   1               5                   10                  15
   Asn Pro Gly Ile Pro Ile Gly Thr Arg Lys
               20                  25
<210> 506
   <211> 11
   <212> PRT
   <213> Homo sapiens
<400> 506
   Tyr Gly Leu Val Thr Tyr Ala Thr Tyr Pro Lys
   1 5 10
<210> 507
   <211> 15
   <212> PRT
   <213> Homo sapiens
<400> 507
   Tyr Gly Leu Val Thr Tyr Ala Thr Tyr Pro Lys Ile Trp Val Lys
   1 5 10 15
<210> 508
   <211> 18
   <212> PRT
   <213> Homo sapiens
<400> 508
   Tyr Gly Gln Thr Ile Arg Pro Ile Cys Leu Pro Cys Thr Glu Gly Thr
   1 5 10 15
   Thr Arg
<210> 509
   <211> 15
   <212> PRT
   <213> Homo sapiens
<400> 509
   Glu Tyr Ala Gln Asn Ile Trp Asn Val Glu Pro Ser Asp Leu Lys
   1 5 10 15
<210> 510
   <211> 10
   <212> PRT
   <213> Homo sapiens
<400> 510
   Phe Ser Gln Phe Leu Glu Thr Glu Tyr Lys
   1 5 10
<210> 511
   <211> 12
   <212> PRT
   <213> Homo sapiens
<400> 511
   Ile Phe Val Asp Ile Glu Lys Leu Pro Trp Ser Lys
   1 5 10
<210> 512
   <211> 14
   <212> PRT
   <213> Homo sapiens
<400> 512
   Ile Gly Glu Asp Tyr Val Lys Asp Leu Ser Gln Leu Thr Lys
   1 5 10
<210> 513
   <211> 17
   <212> PRT
   <213> Homo sapiens
<400> 513
   Leu Lys Gln Glu Tyr Phe Val Val Ala Ala Thr Leu Gln Asp Ile Ile
   1 5 10 15
   Arg
<210> 514
   <211> 14
   <212> PRT
   <213> Homo sapiens
<400> 514
   Leu Val Ile Asp Gln Ile Asp Asn Gly Phe Phe Ser Pro Lys
   1 5 10
<210> 515
   <211> 14
   <212> PRT
   <213> Homo sapiens
<400> 515
   Val Pro Thr Ala Asn Val Ser Val Val Asp Leu Thr Cys Arg
   1 5 10
<210> 516
   <211> 24
   <212> PRT
   <213> Homo sapiens
<400> 516
   Val Ile His Asp Asn Phe Gly Ile Val Glu Gly Leu Met Thr Thr Val
   1 5 10 15
   His Ala Ile Thr Ala Thr Gln Lys
   20
<210> 517
   <211> 8
   <212> PRT
   <213> Homo sapiens
<400> 517
   Val Gly Val Asn Gly Phe Gly Arg
   1 5
<210> 518
   <211> 18
   <212> PRT
   <213> Homo sapiens
<400> 518
   Leu Val Ile Asn Gly Asn Pro Ile Thr Ile Phe Gln Glu Arg Asp Pro
   1 5 10 15
   Ser Lys
<210> 519
   <211> 14
   <212> PRT
   <213> Homo sapiens
<400> 519
   Leu Val Ile Asn Gly Asn Pro Ile Thr Ile Phe Gln Glu Arg
   1 5 10
<210> 520
   <211> 14
   <212> PRT
   <213> Homo sapiens
<400> 520
   Leu Ile Ser Trp Tyr Asp Asn Glu Phe Gly Tyr Ser Asn Arg
   1 5 10
<210> 521
   <211> 17
   <212> PRT
   <213> Homo sapiens
<400> 521
   Ile Ile Ser Asn Ala Ser Cys Thr Thr Asn Cys Leu Ala Pro Leu Ala
   1 5 10 15
   Lys
<210> 522
   <211> 12
   <212> PRT
   <213> Homo sapiens
<400> 522
   Gly Lys Val Lys Val Gly Val Asn Gly Phe Gly Arg
   1 5 10
<210> 523
   <211> 15
   <212> PRT
   <213> Homo sapiens
<400> 523
   Gly Ala Leu Gln Asn Ile Ile Pro Ala Ser Thr Gly Ala Ala Lys
   1 5 10 15
<210> 524
   <211> 18
   <212> PRT
   <213> Homo sapiens
<400> 524
   Asp Gly Arg Gly Ala Leu Gln Asn Ile Ile Pro Ala Ser Thr Gly Ala
   1 5 10 15
   Ala Lys
<210> 525
   <211> 23
   <212> PRT
   <213> Homo sapiens
<400> 525
   Ala Glu Asn Gly Lys Leu Val Ile Asn Gly Asn Pro Ile Thr Ile Phe
   1 5 10 15
   Gln Glu Arg Asp Pro Ser Lys
   20
<210> 526
   <211> 19
   <212> PRT
   <213> Homo sapiens
<400> 526
   Ala Glu Asn Gly Lys Leu Val Ile Asn Gly Asn Pro Ile Thr Ile Phe
   1 5 10 15
   Gln Glu Arg
<210> 527
   <211> 9
   <212> PRT
   <213> Homo sapiens
<400> 527
   Ala Ile Glu Asn Glu Leu Leu Ala Arg
   1 5
<210> 528
   <211> 11
   <212> PRT
   <213> Homo sapiens
<400> 528
   Ala Thr Asn Trp Gly Ser Leu Leu Gln Asp Lys
   1 5 10
<210> 529
   <211> 18
   <212> PRT
   <213> Homo sapiens
<400> 529
   Tyr Ile Ser Leu Ile Tyr Thr Asn Tyr Glu Ala Gly Lys Asp Asp Tyr
   1 5 10 15
   Val Lys
<210> 530
   <211> 13
   <212> PRT
   <213> Homo sapiens
<400> 530
   Tyr Ile Ser Leu Ile Tyr Thr Asn Tyr Glu Ala Gly Lys
   1 5 10
<210> 531
   <211> 13
   <212> PRT
   <213> Homo sapiens
<400> 531
   Pro Pro Tyr Thr Val Val Tyr Phe Pro Val Arg Gly Arg
   1 5 10
<210> 532
   <211> 11
   <212> PRT
   <213> Homo sapiens
<400> 532
   Pro Pro Tyr Thr Val Val Tyr Phe Pro Val Arg
   1 5 10
<210> 533
   <211> 11
   <212> PRT
   <213> Homo sapiens
<400> 533
   Met Leu Leu Ala Asp Gln Gly Gln Ser Trp Lys
   1 5 10
<210> 534
   <211> 21
   <212> PRT
   <213> Homo sapiens
<400> 534
   Leu Lys Ala Phe Leu Ala Ser Pro Glu Tyr Val Asn Leu Pro Ile Asn
   1 5 10 15
   Gly Asn Gly Lys Gln
   20
<210> 535
   <211> 20
   <212> PRT
   <213> Homo sapiens
<400> 535
   Leu Lys Ala Phe Leu Ala Ser Pro Glu Tyr Val Asn Leu Pro Ile Asn
   1 5 10 15
   Gly Asn Gly Lys
   20
<210> 536
   <211> 20
   <212> PRT
   <213> Homo sapiens
<400> 536
   Phe Gln Asp Gly Asp Leu Thr Leu Tyr Gln Ser Asn Thr Ile Leu Arg
   1               5                   10                  15
   His Leu Gly Arg
               20
<210> 537
   <211> 16
   <212> PRT
   <213> Homo sapiens
<400> 537
   Phe Gln Asp Gly Asp Leu Thr Leu Tyr Gln Ser Asn Thr Ile Leu Arg
   1               5                   10                  15
<210> 538
   <211> 15
   <212> PRT
   <213> Homo sapiens
<400> 538
   Glu Glu Val Val Thr Val Glu Thr Trp Gln Glu Gly Ser Leu Lys
   1               5                   10                  15
<210> 539
   <211> 20
   <212> PRT
   <213> Homo sapiens
<400> 539
   Cys Lys Tyr Ile Ser Leu Ile Tyr Thr Asn Tyr Glu Ala Gly Lys Asp
   1               5                   10                  15
   Asp Tyr Val Lys
               20
<210> 540
   <211> 10
   <212> PRT
   <213> Homo sapiens
<400> 540
   Ala Ser Cys Leu Tyr Gly Gln Leu Pro Lys
   1               5                   10
<210> 541
   <211> 20
   <212> PRT
   <213> Homo sapiens
<400> 541
   Ala Leu Pro Gly Gln Leu Lys Pro Phe Glu Thr Leu Leu Ser Gln Asn
   1               5                   10                  15
   Gln Gly Gly Lys
               20
<210> 542
   <211> 19
   <212> PRT
   <213> Homo sapiens
<400> 542
   Ala Phe Leu Ala Ser Pro Glu Tyr Val Asn Leu Pro Ile Asn Gly Asn
   1               5                   10                  15
   Gly Lys Gln
<210> 543
   <211> 18
   <212> PRT
   <213> Homo sapiens
<400> 543
   Ala Phe Leu Ala Ser Pro Glu Tyr Val Asn Leu Pro Ile Asn Gly Asn
   1               5                   10                  15
   Gly Lys
<210> 544
   <211> 14
   <212> PRT
   <213> Homo sapiens
<400> 544
   Ser Asn Glu Glu Gly Ser Glu Glu Lys Gly Pro Glu Val Arg
   1               5                   10
<210> 545
   <211> 15
   <212> PRT
   <213> Homo sapiens
<400> 545
   Asn Leu Leu Ser Val Ala Tyr Lys Asn Val Val Gly Gly Gln Arg
   1               5                   10                  15
<210> 546
   <211> 8
   <212> PRT
   <213> Homo sapiens
<400> 546
   Asn Leu Leu Ser Val Ala Tyr Lys
   1 5
<210> 547
   <211> 7
   <212> PRT
   <213> Homo sapiens
<400> 547
   Leu Ala Glu Gln Ala Glu Arg
   1 5
<210> 548
   <211> 9
   <212> PRT
   <213> Homo sapiens
<400> 548
   Gly Glu Glu Leu Ser Cys Glu Glu Arg
   1 5
<210> 549
   <211> 14
   <212> PRT
   <213> Homo sapiens
<400> 549
   Gly Ala Val Glu Lys Gly Glu Glu Leu Ser Cys Glu Glu Arg
   1 5 10
<210> 550
   <211> 10
   <212> PRT
   <213> Homo sapiens
<400> 550
   Asp Ser Thr Leu Ile Met Gln Leu Leu Arg
   1 5 10
<210> 551
   <211> 24
   <212> PRT
   <213> Homo sapiens
<400> 551
   Asp Asn Leu Thr Leu Trp Thr Ala Asp Asn Ala Gly Glu Glu Gly Gly
   1 5 10 15
   Glu Ala Pro Gln Glu Pro Gln Ser
   20
<210> 552
   <211> 13
   <212> PRT
   <213> Homo sapiens
<400> 552
   Tyr Leu Ala Glu Val Ala Thr Gly Asp Asp Lys Lys Arg
   1 5 10
<210> 553
   <211> 16
   <212> PRT
   <213> Homo sapiens
<400> 553
   Ala Ala Gly Val Asn Val Glu Pro Phe Trp Pro Gly Leu Phe Ala Lys
   1 5 10 15
<210> 554
   <211> 17
   <212> PRT
   <213> Homo sapiens
<400> 554
   Val Cys Leu Ile Gly Cys Gly Phe Ser Thr Gly Tyr Gly Ser Ala Val
   1 5 10 15
   Lys
<210> 555
   <211> 9
   <212> PRT
   <213> Homo sapiens
<400> 555
   Asn Pro Glu Ser Asn Tyr Cys Leu Lys
   1 5
<210> 556
   <211> 14
   <212> PRT
   <213> Homo sapiens
<400> 556
   Lys Pro Phe Ser Ile Glu Glu Val Glu Val Ala Pro Pro Lys
   1 5 10
<210> 557
   <211> 9
   <212> PRT
   <213> Homo sapiens
<400> 557
   Ile Asn Glu Gly Phe Asp Leu Leu Arg
   1               5
<210> 558
   <211> 12
   <212> PRT
   <213> Homo sapiens
<400> 558
   Ile Cys Lys Asn Pro Glu Ser Asn Tyr Cys Leu Lys
   1               5                   10
<210> 559
   <211> 14
   <212> PRT
   <213> Homo sapiens
<400> 559
   Glu Leu Gly Ala Thr Glu Cys Ile Asn Pro Gln Asp Tyr Lys
   1               5                   10
<210> 560
   <211> 8
   <212> PRT
   <213> Homo sapiens
<400> 560
   Ala Ala Val Leu Trp Glu Leu Lys
   1               5
<210> 561
   <211> 15
   <212> PRT
   <213> Homo sapiens
<400> 561
   Glu Cys Asn Ala Cys Arg Asn Pro Asp Gly Asn Leu Cys Ile Arg
   1               5                   10                  15
<210> 562
   <211> 28
   <212> PRT
   <213> Homo sapiens
<400> 562
   Phe Pro Val Ile Val Gly His Glu Ala Thr Gly Ile Val Glu Ser Ile
   1 5 10 15
   Gly Glu Gly Val Thr Thr Val Lys Pro Gly Asp Lys
   20 25
<210> 563
   <211> 10
   <212> PRT
   <213> Homo sapiens
<400> 563
   Ile Lys Ile Leu Ala Thr Gly Ile Cys Arg
   1 5 10
<210> 564
   <211> 8
   <212> PRT
   <213> Homo sapiens
<400> 564
   Ile Leu Ala Thr Gly Ile Cys Arg
   1 5
<210> 565
   <211> 15
   <212> PRT
   <213> Homo sapiens
<400> 565
   Ile Ser Glu Gly Phe Glu Leu Leu Asn Ser Gly Gln Ser Ile Arg
   1 5 10 15
<210> 566
   <211> 15
   <212> PRT
   <213> Homo sapiens
<400> 566
   Lys Phe Asp Leu Asp Gln Leu Ile Thr His Val Leu Pro Phe Lys
   1 5 10 15
<210> 567
   <211> 9
   <212> PRT
   <213> Homo sapiens
<400> 567
   Asn Pro Asp Gly Asn Leu Cys Ile Arg
   1 5
<210> 568
   <211> 17
   <212> PRT
   <213> Homo sapiens
<400> 568
   Val Cys Leu Ile Gly Cys Gly Phe Ser Thr Gly Tyr Gly Ala Ala Val
   1               5                   10                  15
   Lys
<210> 569
   <211> 10
   <212> PRT
   <213> Homo sapiens
<400> 569
   Val Ile Pro Leu Phe Leu Pro Gln Cys Arg
   1               5                   10
<210> 570
   <211> 26
   <212> PRT
   <213> Homo sapiens
<400> 570
   Ile Asp Asp Ala Ala Pro Pro Glu Lys Val Cys Leu Ile Gly Cys Gly
   1               5                   10                  15
   Phe Ser Thr Gly Tyr Gly Ala Ala Val Lys
               20                  25
<210> 571
   <211> 17
   <212> PRT
   <213> Homo sapiens
<400> 571
   Tyr Lys Pro Val Cys Asn Gln Val Glu Cys His Pro Tyr Phe Asn Gln
   1               5                   10                  15
   Arg
<210> 572
   <211> 19
   <212> PRT
   <213> Homo sapiens
<400> 572
   His Ile Asp Ser Ala His Leu Tyr Asn Asn Glu Glu Gln Val Gly Leu
   1 5 10 15
   Ala Ile Arg
<210> 573
   <211> 8
   <212> PRT
   <213> Homo sapiens
<400> 573
   Leu Ala Ile Glu Ala Gly Phe Arg
   1 5
<210> 574
   <211> 22
   <212> PRT
   <213> Homo sapiens
<400> 574
   Leu Asn Asp Gly His Phe Met Pro Val Leu Gly Phe Gly Thr Tyr Ala
   1 5 10 15
   Pro Ala Glu Val Pro Lys
   20
<210> 575
   <211> 9
   <212> PRT
   <213> Homo sapiens
<400> 575
   Arg Glu Asp Ile Phe Tyr Thr Ser Lys
   1 5
<210> 576
   <211> 16
   <212> PRT
   <213> Homo sapiens
<400> 576
   Ser Lys Asp Ile Val Leu Val Ala Tyr Ser Ala Leu Gly Ser His Arg
   1 5 10 15
<210> 577
   <211> 8
   <212> PRT
   <213> Homo sapiens
<400> 577
   Thr Pro Ala Leu Ile Ala Leu Arg
   1 5
<210> 578
   <211> 11
   <212> PRT
   <213> Homo sapiens
<400> 578
   Ile Thr Leu Leu Ser Ala Leu Val Glu Thr Arg
   1 5 10
<210> 579
   <211> 10
   <212> PRT
   <213> Homo sapiens
<400> 579
   Asn Leu Ala Val Ser Gln Val Val His Lys
   1 5 10
<210> 580
   <211> 18
   <212> PRT
   <213> Homo sapiens
<400> 580
   Leu Tyr Gly Ser Glu Ala Phe Ala Thr Asp Phe Gln Asp Ser Ala Ala
   1 5 10 15
   Ala Lys
<210> 581
   <211> 9
   <212> PRT
   <213> Homo sapiens
<400> 581
   Glu Ile Gly Glu Leu Tyr Leu Pro Lys
   1 5
<210> 582
   <211> 12
   <212> PRT
   <213> Homo sapiens
<400> 582
   Asp Glu Glu Leu Ser Cys Thr Val Val Glu Leu Lys
   1 5 10
<210> 583
   <211> 10
   <212> PRT
   <213> Homo sapiens
<400> 583
   Ala Asp Leu Ser Gly Ile Thr Gly Ala Arg
   1 5 10
<210> 584
   <211> 11
   <212> PRT
   <213> Homo sapiens
<400> 584
   Ile Thr Leu Leu Ser Ala Leu Val Glu Thr Arg
   1 5 10
<210> 585
   <211> 12
   <212> PRT
   <213> Homo sapiens
<400> 585
   Ala Val His Lys Ala Val Leu Thr Ile Asp Glu Lys
   1 5 10
<210> 586
   <211> 16
   <212> PRT
   <213> Homo sapiens
<400> 586
   Asp Thr Glu Glu Glu Asp Phe His Val Asp Gln Val Thr Thr Val Lys
   1 5 10 15
<210> 587
   <211> 15
   <212> PRT
   <213> Homo sapiens
<400> 587
   Phe Asn Lys Pro Phe Val Phe Leu Met Ile Glu Gln Asn Thr Lys
   1 5 10 15
<210> 588
   <211> 8
   <212> PRT
   <213> Homo sapiens
<400> 588
   Phe Leu Glu Asn Glu Asp Arg Arg
   1 5
<210> 589
   <211> 14
   <212> PRT
   <213> Homo sapiens
<400> 589
   Ile Thr Pro Asn Leu Ala Glu Phe Ala Phe Ser Leu Tyr Arg
   1 5 10
<210> 590
   <211> 14
   <212> PRT
   <213> Homo sapiens
<400> 590
   Lys Gln Ile Asn Asp Tyr Val Glu Lys Gly Thr Gln Gly Lys
   1 5 10
<210> 591
   <211> 10
   <212> PRT
   <213> Homo sapiens
<400> 591
   Leu Ser Ile Thr Gly Thr Tyr Asp Leu Lys
   1 5 10
<210> 592
   <211> 15
   <212> PRT
   <213> Homo sapiens
<400> 592
   Leu Gln His Leu Glu Asn Glu Leu Thr His Asp Ile Ile Thr Lys
   1 5 10 15
<210> 593
   <211> 9
   <212> PRT
   <213> Homo sapiens
<400> 593
   Leu Ser Ser Trp Val Leu Leu Met Lys
   1 5
<210> 594
   <211> 19
   <212> PRT
   <213> Homo sapiens
<400> 594
   Leu Tyr His Ser Glu Ala Phe Thr Val Asn Phe Gly Asp Thr Glu Glu
   1 5 10 15
   Ala Lys Lys
<210> 595
   <211> 8
   <212> PRT
   <213> Homo sapiens
<400> 595
   Gln Ile Asn Asp Tyr Val Glu Lys
   1 5
<210> 596
   <211> 13
   <212> PRT
   <213> Homo sapiens
<400> 596
   Gln Ile Asn Asp Tyr Val Glu Lys Gly Thr Gln Gly Lys
   1 5 10
<210> 597
   <211> 8
   <212> PRT
   <213> Homo sapiens
<400> 597
   Ser Ala Ser Leu His Leu Pro Lys
   1 5
<210> 598
   <211> 24
   <212> PRT
   <213> Homo sapiens
<400> 598
   Thr Leu Asn Gln Pro Asp Ser Gln Leu Gln Leu Thr Thr Gly Asn Gly
   1 5 10 15
   Leu Phe Leu Ser Glu Gly Leu Lys
   20
<210> 599
   <211> 10
   <212> PRT
   <213> Homo sapiens
<400> 599
   Ser Val Leu Gly Gln Leu Gly Ile Thr Lys
   1 5 10
<210> 600
   <211> 18
   <212> PRT
   <213> Homo sapiens
<400> 600
   Val Phe Ser Asn Gly Ala Asp Leu Ser Gly Val Thr Glu Glu Ala Pro
   1 5 10 15
   Leu Lys
<210> 601
   <211> 18
   <212> PRT
   <213> Homo sapiens
<400> 601
   Val Phe Ser Asn Gly Ala Asp Leu Ser Gly Val Thr Glu Glu Ala Pro
   1 5 10 15
   Leu Lys
<210> 602
   <211> 17
   <212> PRT
   <213> Homo sapiens
<400> 602
   Val Thr Leu Thr Cys Val Ala Pro Leu Ser Gly Val Asp Phe Gln Leu
   1 5 10 15
   Arg
<210> 603
   <211> 20
   <212> PRT
   <213> Homo sapiens
<400> 603
   Ser Gly Leu Ser Thr Gly Trp Thr Gln Leu Ser Lys Leu Leu Glu Leu
   1 5 10 15
   Thr Gly Pro Lys
   20
<210> 604
   <211> 12
   <212> PRT
   <213> Homo sapiens
<400> 604
   Ser Gly Leu Ser Thr Gly Trp Thr Gln Leu Ser Lys
   1 5 10
<210> 605
   <211> 10
   <212> PRT
   <213> Homo sapiens
<400> 605
   Arg Gly Glu Lys Glu Leu Leu Val Pro Arg
   1 5 10
<210> 606
   <211> 14
   <212> PRT
   <213> Homo sapiens
<400> 606
   Cys Glu Gly Pro Ile Pro Asp Val Thr Phe Glu Leu Leu Arg
   1 5 10
<210> 607
   <211> 11
   <212> PRT
   <213> Homo sapiens
<400> 607
   Ala Thr Trp Ser Gly Ala Val Leu Ala Gly Arg
   1 5 10
<210> 608
   <211> 22
   <212> PRT
   <213> Homo sapiens
<400> 608
   Thr Pro Gly Ala Ala Ala Asn Leu Glu Leu Ile Phe Val Gly Pro Gln
   1 5 10 15
   His Ala Gly Asn Tyr Arg
   20
<210> 609
   <211> 9
   <212> PRT
   <213> Homo sapiens
<400> 609
   Cys Leu Ala Pro Leu Glu Gly Ala Arg
   1 5
<210> 610
   <211> 21
   <212> PRT
   <213> Homo sapiens
<400> 610
   Thr Val Val Gln Pro Ser Val Gly Ala Ala Ala Gly Pro Val Val Pro
   1 5 10 15
   Pro Cys Pro Gly Arg
   20
<210> 611
   <211> 14
   <212> PRT
   <213> Homo sapiens
<400> 611
   Glu His Ala Val Glu Gly Asp Cys Asp Phe Gln Leu Leu Lys
   1 5 10
<210> 612
   <211> 7
   <212> PRT
   <213> Homo sapiens
<400> 612
   Phe Ser Val Val Tyr Ala Lys
   1 5
<210> 613
   <211> 10
   <212> PRT
   <213> Homo sapiens
<400> 613
   His Thr Leu Asn Gln Ile Asp Glu Val Lys
   1 5 10
<210> 614
   <211> 11
   <212> PRT
   <213> Homo sapiens
<400> 614
   Leu Asp Gly Lys Phe Ser Val Val Tyr Ala Lys
   1 5 10
<210> 615
   <211> 27
   <212> PRT
   <213> Homo sapiens
<400> 615
   Tyr Asn Ile Leu Pro Glu Lys Glu Glu Phe Pro Phe Ala Leu Gly Val
   1               5                   10                  15
   Gln Thr Leu Pro Gln Thr Cys Asp Glu Pro Lys
               20                  25
<210> 616
   <211> 14
   <212> PRT
   <213> Homo sapiens
<400> 616
   Val Thr Gly Glu Gly Cys Val Tyr Leu Gln Thr Ser Leu Lys
   1               5                   10
<210> 617
   <211> 12
   <212> PRT
   <213> Homo sapiens
<400> 617
   Val Thr Ala Ala Pro Gln Ser Val Cys Ala Leu Arg
   1               5                   10
<210> 618
   <211> 18
   <212> PRT
   <213> Homo sapiens
<400> 618
   Val Ser Val Gln Leu Glu Ala Ser Pro Ala Phe Leu Ala Val Pro Val
   1               5                   10                  15
   Glu Lys
<210> 619
   <211> 11
   <212> PRT
   <213> Homo sapiens
<400> 619
   Val Gly Phe Tyr Glu Ser Asp Val Met Gly Arg
   1               5                   10
<210> 620
   <211> 16
   <212> PRT
   <213> Homo sapiens
<400> 620
   Val Asp Ser His Phe Arg Gln Gly Ile Pro Phe Phe Gly Gln Val Arg
   1 5 10 15
<210> 621
   <211> 19
   <212> PRT
   <213> Homo sapiens
<400> 621
   Val Asp Leu Ser Phe Ser Pro Ser Gln Ser Leu Pro Ala Ser His Ala
   1 5 10 15
   His Leu Arg
<210> 622
   <211> 14
   <212> PRT
   <213> Homo sapiens
<400> 622
   Thr Glu Val Ser Ser Asn His Val Leu Ile Tyr Leu Asp Lys
   1 5 10
<210> 623
   <211> 22
   <212> PRT
   <213> Homo sapiens
<400> 623
   Ser Leu Phe Thr Asp Leu Glu Ala Glu Asn Asp Val Leu His Cys Val
   1 5 10 15
   Ala Phe Ala Val Pro Lys
   20
<210> 624
   <211> 13
   <212> PRT
   <213> Homo sapiens
<400> 624
   Ser Lys Ala Ile Gly Tyr Leu Asn Thr Gly Tyr Gln Arg
   1 5 10
<210> 625
   <211> 23
   <212> PRT
   <213> Homo sapiens
<400> 625
   Gln Gln Asn Ala Gln Gly Gly Phe Ser Ser Thr Gln Asp Thr Val Val
   1               5                   10                  15
   Ala Leu His Ala Leu Ser Lys
               20
<210> 626
   <211> 17
   <212> PRT
   <213> Homo sapiens
<400> 626
   Gln Leu Asn Tyr Lys His Tyr Asp Gly Ser Tyr Ser Thr Phe Gly Glu
   1               5                   10                  15
   Arg
<210> 627
   <211> 10
   <212> PRT
   <213> Homo sapiens
<400> 627
   Gln Gly Ile Pro Phe Phe Gly Gln Val Arg
   1               5                   10
<210> 628
   <211> 16
   <212> PRT
   <213> Homo sapiens
<400> 628
   Gln Phe Ser Phe Pro Leu Ser Ser Glu Pro Phe Gln Gly Ser Tyr Lys
   1               5                   10                  15
<210> 629
   <211> 13
   <212> PRT
   <213> Homo sapiens
<400> 629
   Asn Gln Gly Asn Thr Trp Leu Thr Ala Phe Val Leu Lys
   1               5                   10
<210> 630
   <211> 12
   <212> PRT
   <213> Homo sapiens
<400> 630
   Asn Glu Asp Ser Leu Val Phe Val Gln Thr Asp Lys
   1 5 10
<210> 631
   <211> 18
   <212> PRT
   <213> Homo sapiens
<400> 631
   Leu Leu Ile Tyr Ala Val Leu Pro Thr Gly Asp Val Ile Gly Asp Ser
   1 5 10 15
   Ala Lys
<210> 632
   <211> 19
   <212> PRT
   <213> Homo sapiens
<400> 632
   Leu His Thr Glu Ala Gln Ile Gln Glu Glu Gly Thr Val Val Glu Leu
   1 5 10 15
   Thr Gly Arg
<210> 633
   <211> 19
   <212> PRT
   <213> Homo sapiens
<400> 633
   Lys Tyr Ser Asp Ala Ser Asp Cys His Gly Glu Asp Ser Gln Ala Phe
   1 5 10 15
   Cys Glu Lys
<210> 634
   <211> 14
   <212> PRT
   <213> Homo sapiens
<400> 634
   Ile Ala Gln Trp Gln Ser Phe Gln Leu Glu Gly Gly Leu Lys
   1 5 10
<210> 635
   <211> 16
   <212> PRT
   <213> Homo sapiens
<400> 635
   Phe Ser Gly Gln Leu Asn Ser His Gly Cys Phe Tyr Gln Gln Val Lys
   1 5 10 15
<210> 636
   <211> 9
   <212> PRT
   <213> Homo sapiens
<400> 636
   Phe Glu Val Gln Val Thr Val Pro Lys
   1 5
<210> 637
   <211> 16
   <212> PRT
   <213> Homo sapiens
<400> 637
   Asp Thr Val Ile Lys Pro Leu Leu Val Glu Pro Glu Gly Leu Glu Lys
   1 5 10 15
<210> 638
   <211> 15
   <212> PRT
   <213> Homo sapiens
<400> 638
   Ala Leu Leu Ala Tyr Ala Phe Ala Leu Ala Gly Asn Gln Asp Lys
   1 5 10 15
<210> 639
   <211> 11
   <212> PRT
   <213> Homo sapiens
<400> 639
   Ala Ile Gly Tyr Leu Asn Thr Gly Tyr Gln Arg
   1 5 10
<210> 640
   <211> 15
   <212> PRT
   <213> Homo sapiens
<400> 640
   Ala Ala Gln Val Thr Ile Gln Ser Ser Gly Thr Phe Ser Ser Lys
   1 5 10 15
<210> 641
   <211> 12
   <212> PRT
   <213> Homo sapiens
<400> 641
   Val Gly Trp Glu Gln Leu Leu Thr Thr Ile Ala Arg
   1 5 10
<210> 642
   <211> 12
   <212> PRT
   <213> Homo sapiens
<400> 642
   Thr Ile Asn Glu Val Glu Asn Gln Ile Leu Thr Arg
   1 5 10
<210> 643
   <211> 13
   <212> PRT
   <213> Homo sapiens
<400> 643
   Cys Gln Leu Glu Ile Asn Phe Asn Thr Leu Gln Thr Lys
   1 5 10
<210> 644
   <211> 16
   <212> PRT
   <213> Homo sapiens
<400> 644
   Glu Thr Thr Asp Thr Asp Thr Ala Asp Gln Val Ile Ala Ser Phe Lys
   1 5 10 15
<210> 645
   <211> 11
   <212> PRT
   <213> Homo sapiens
<400> 645
   Asp Tyr Glu Thr Ala Thr Leu Ser Asp Ile Lys
   1 5 10
<210> 646
   <211> 13
   <212> PRT
   <213> Homo sapiens
<400> 646
   His Arg Asp Tyr Glu Thr Ala Thr Leu Ser Asp Ile Lys
   1 5 10
<210> 647
   <211> 26
   <212> PRT
   <213> Homo sapiens
<400> 647
   Ile Ala Glu Ser Asn His Ile Lys Leu Ser Gly Ser Asn Pro Tyr Thr
   1 5 10 15
   Thr Val Thr Pro Gln Ile Ile Asn Ser Lys
   20 25
<210> 648
   <211> 15
   <212> PRT
   <213> Homo sapiens
<400> 648
   Ile Cys Asp Gln Trp Asp Ala Leu Gly Ser Leu Thr His Ser Arg
   1 5 10 15
<210> 649
   <211> 17
   <212> PRT
   <213> Homo sapiens
<400> 649
   Lys Asp Asp Pro Val Thr Asn Leu Asn Asn Ala Phe Glu Val Ala Glu
   1 5 10 15
   Lys
<210> 650
   <211> 10
   <212> PRT
   <213> Homo sapiens
<400> 650
   Leu Ala Ser Asp Leu Leu Glu Trp Ile Arg
   1 5 10
<210> 651
   <211> 18
   <212> PRT
   <213> Homo sapiens
<400> 651
   Leu Ser Gly Ser Asn Pro Tyr Thr Thr Val Thr Pro Gln Ile Ile Asn
   1 5 10 15
   Ser Lys
<210> 652
   <211> 21
   <212> PRT
   <213> Homo sapiens
<400> 652
   Leu Ser Gly Ser Asn Pro Tyr Thr Thr Val Thr Pro Gln Ile Ile Asn
   1               5                   10                  15
   Ser Lys Trp Glu Lys
               20
<210> 653
   <211> 16
   <212> PRT
   <213> Homo sapiens
<400> 653
   Gln Phe Ala Ser Gln Ala Asn Val Val Gly Pro Trp Ile Gln Thr Lys
   1               5                   10                  15
<210> 654
   <211> 10
   <212> PRT
   <213> Homo sapiens
<400> 654
   Ser Thr Leu Pro Asp Ala Asp Arg Glu Arg
   1               5                   10
<210> 655
   <211> 15
   <212> PRT
   <213> Homo sapiens
<400> 655
   Val Leu Ala Gly Asp Lys Asn Phe Ile Thr Ala Glu Glu Leu Arg
   1               5                   10                  15
<210> 656
   <211> 13
   <212> PRT
   <213> Homo sapiens
<400> 656
   Gly Asn Pro Thr Val Glu Val Asp Leu Phe Thr Ser Lys
   1               5                   10
<210> 657
   <211> 8
   <212> PRT
   <213> Homo sapiens
<400> 657
   Ser Cys Asn Cys Leu Leu Leu Lys
   1 5
<210> 658
   <211> 15
   <212> PRT
   <213> Homo sapiens
<400> 658
   Val Asn Gln Ile Gly Ser Val Thr Glu Ser Leu Gln Ala Cys Lys
   1 5 10 15
<210> 659
   <211> 18
   <212> PRT
   <213> Homo sapiens
<400> 659
   Ala Ala Val Pro Ser Gly Ala Ser Thr Gly Ile Tyr Glu Ala Leu Glu
   1 5 10 15
   Leu Arg
<210> 660
   <211> 12
   <212> PRT
   <213> Homo sapiens
<400> 660
   Ala Gly Ala Val Glu Lys Gly Val Pro Leu Tyr Arg
   1 5 10
<210> 661
   <211> 7
   <212> PRT
   <213> Homo sapiens
<400> 661
   Ala Val Glu His Ile Asn Lys
   1 5
<210> 662
   <211> 16
   <212> PRT
   <213> Homo sapiens
<400> 662
   Ala Val Glu His Ile Asn Lys Thr Ile Ala Pro Ala Leu Val Ser Lys
   1 5 10 15
<210> 663
   <211> 19
   <212> PRT
   <213> Homo sapiens
<400> 663
   Asp Ala Thr Asn Val Gly Asp Glu Gly Gly Phe Ala Pro Asn Ile Leu
   1               5                   10                  15
   Glu Asn Lys
<210> 664
   <211> 26
   <212> PRT
   <213> Homo sapiens
<400> 664
   Asp Ala Thr Asn Val Gly Asp Glu Gly Gly Phe Ala Pro Asn Ile Leu
   1               5                   10                  15
   Glu Asn Lys Glu Gly Leu Glu Leu Leu Lys
               20                  25
<210> 665
   <211> 19
   <212> PRT
   <213> Homo sapiens
<400> 665
   Glu Ile Phe Asp Ser Arg Gly Asn Pro Thr Val Glu Val Asp Leu Phe
   1               5                   10                  15
   Thr Ser Lys
<210> 666
   <211> 23
   <212> PRT
   <213> Homo sapiens
<400> 666
   Glu Ile Phe Asp Ser Arg Gly Asn Pro Thr Val Glu Val Asp Leu Phe
   1               5                   10                  15
   Thr Ser Lys Gly Leu Phe Arg
               20
<210> 667
   <211> 24
   <212> PRT
   <213> Homo sapiens
<400> 667
   Glu Lys Tyr Gly Lys Asp Ala Thr Asn Val Gly Asp Glu Gly Gly Phe
   1               5                   10                  15
   Ala Pro Asn Ile Leu Glu Asn Lys
               20
<210> 668
   <211> 21
   <212> PRT
   <213> Homo sapiens
<400> 668
   Phe Thr Ala Ser Ala Gly Ile Gln Val Val Gly Asp Asp Leu Thr Val
   1               5                   10                  15
   Thr Asn Pro Lys Arg
               20
<210> 669
   <211> 30
   <212> PRT
   <213> Homo sapiens
<400> 669
   His Ile Ala Asp Leu Ala Gly Asn Ser Glu Val Ile Leu Pro Val Pro
   1               5                   10                  15
   Ala Phe Asn Val Ile Asn Gly Gly Ser His Ala Gly Asn Lys
               20                  25                  30
<210> 670
   <211> 8
   <212> PRT
   <213> Homo sapiens
<400> 670
   Ile Glu Glu Glu Leu Gly Ser Lys
   1               5
<210> 671
   <211> 10
   <212> PRT
   <213> Homo sapiens
<400> 671
   Ile Glu Glu Glu Leu Gly Ser Lys Ala Lys
   1               5                   10
<210> 672
   <211> 14
   <212> PRT
   <213> Homo sapiens
<400> 672
   Ile Glu Glu Glu Leu Gly Ser Lys Ala Lys Phe Ala Gly Arg
   1 5 10
<210> 673
   <211> 10
   <212> PRT
   <213> Homo sapiens
<400> 673
   Ile Gly Ala Glu Val Tyr His Asn Leu Lys
   1 5 10
<210> 674
   <211> 14
   <212> PRT
   <213> Homo sapiens
<400> 674
   Ile Gly Ala Glu Val Tyr His Asn Leu Lys Asn Val Ile Lys
   1 5 10
<210> 675
   <211> 9
   <212> PRT
   <213> Homo sapiens
<400> 675
   Lys Leu Asn Val Thr Glu Gln Glu Lys
   1 5
<210> 676
   <211> 12
   <212> PRT
   <213> Homo sapiens
<400> 676
   Lys Leu Asn Val Thr Glu Gln Glu Lys Ile Asp Lys
   1 5 10
<210> 677
   <211> 14
   <212> PRT
   <213> Homo sapiens
<400> 677
   Leu Ala Gln Ala Asn Gly Trp Gly Val Met Val Ser His Arg
   1 5 10
<210> 678
   <211> 8
   <212> PRT
   <213> Homo sapiens
<400> 678
   Asn Phe Arg Asn Pro Leu Ala Lys
   1 5
<210> 679
   <211> 22
   <212> PRT
   <213> Homo sapiens
<400> 679
   Ser Gly Glu Thr Glu Asp Thr Phe Ile Ala Asp Leu Val Val Gly Leu
   1 5 10 15
   Cys Thr Gly Gln Ile Lys
   20
<210> 680
   <211> 16
   <212> PRT
   <213> Homo sapiens
<400> 680
   Ser Gly Lys Tyr Asp Leu Asp Phe Lys Ser Pro Asp Asp Pro Ser Arg
   1 5 10 15
<210> 681
   <211> 8
   <212> PRT
   <213> Homo sapiens
<400> 681
   Ser Ile Leu Lys Ile His Ala Arg
   1 5
<210> 682
   <211> 19
   <212> PRT
   <213> Homo sapiens
<400> 682
   Ser Pro Asp Asp Pro Ser Arg Tyr Ile Ser Pro Asp Gln Leu Ala Asp
   1 5 10 15
   Leu Tyr Lys
<210> 683
   <211> 9
   <212> PRT
   <213> Homo sapiens
<400> 683
   Thr Ile Ala Pro Ala Leu Val Ser Lys
   1               5
<210> 684
   <211> 22
   <212> PRT
   <213> Homo sapiens
<400> 684
   Tyr Gly Lys Asp Ala Thr Asn Val Gly Asp Glu Gly Gly Phe Ala Pro
   1               5                   10                  15
   Asn Ile Leu Glu Asn Lys
               20
<210> 685
   <211> 29
   <212> PRT
   <213> Homo sapiens
<400> 685
   Tyr Gly Lys Asp Ala Thr Asn Val Gly Asp Glu Gly Gly Phe Ala Pro
   1               5                   10                  15
   Asn Ile Leu Glu Asn Lys Glu Gly Leu Glu Leu Leu Lys
               20                  25
<210> 686
   <211> 12
   <212> PRT
   <213> Homo sapiens
<400> 686
   Tyr Ile Ser Pro Asp Gln Leu Ala Asp Leu Tyr Lys
   1               5                   10
<210> 687
   <211> 6
   <212> PRT
   <213> Homo sapiens
<400> 687
   Tyr Asn Gln Leu Leu Arg
   1               5
<210> 688
   <211> 14
   <212> PRT
   <213> Homo sapiens
<400> 688
   Tyr Asn Gln Leu Leu Arg Ile Glu Glu Glu Leu Gly Ser Lys
   1 5 10
<210> 689
   <211> 16
   <212> PRT
   <213> Homo sapiens
<400> 689
   Tyr Asn Gln Leu Leu Arg Ile Glu Glu Glu Leu Gly Ser Lys Ala Lys
   1 5 10 15
<210> 690
   <211> 13
   <212> PRT
   <213> Homo sapiens
<400> 690
   Tyr Asp Leu Asp Phe Lys Ser Pro Asp Asp Pro Ser Arg
   1 5 10
<210> 691
   <211> 17
   <212> PRT
   <213> Homo sapiens
<400> 691
   Val Leu Ser Ala Leu Gln Ala Val Gln Gly Leu Leu Val Ala Gln Gly
   1 5 10 15
   Arg
<210> 692
   <211> 13
   <212> PRT
   <213> Homo sapiens
<400> 692
   Ser Leu Asp Phe Thr Glu Leu Asp Val Ala Ala Glu Lys
   1 5 10
<210> 693
   <211> 17
   <212> PRT
   <213> Homo sapiens
<400> 693
   Val Leu Ser Ala Leu Gln Ala Val Gln Gly Leu Leu Val Ala Gln Gly
   1 5 10 15
   Arg
<210> 694
   <211> 19
   <212> PRT
   <213> Homo sapiens
<400> 694
   Ala Asn Ala Gly Lys Pro Lys Asp Pro Thr Phe Ile Pro Ala Pro Ile
   1 5 10 15
   Gln Ala Lys
<210> 695
   <211> 10
   <212> PRT
   <213> Homo sapiens
<400> 695
   Ala Leu Leu Leu Leu Cys Gly Glu Asp Asp
   1 5 10
<210> 696
   <211> 17
   <212> PRT
   <213> Homo sapiens
<400> 696
   Ala Gln Val Leu Arg Gly Thr Val Thr Asp Phe Pro Gly Phe Asp Glu
   1 5 10 15
   Arg
<210> 697
   <211> 13
   <212> PRT
   <213> Homo sapiens
<400> 697
   Asp Leu Leu Asp Asp Leu Lys Ser Glu Leu Thr Gly Lys
   1 5 10
<210> 698
   <211> 16
   <212> PRT
   <213> Homo sapiens
<400> 698
   Asp Leu Leu Asp Asp Leu Lys Ser Glu Leu Thr Gly Lys Phe Glu Lys
   1 5 10 15
<210> 699
   <211> 16
   <212> PRT
   <213> Homo sapiens
<400> 699
   Gly Leu Gly Thr Asp Glu Glu Ser Ile Leu Thr Leu Leu Thr Ser Arg
   1 5 10 15
<210> 700
   <211> 20
   <212> PRT
   <213> Homo sapiens
<400> 700
   Gly Thr Val Thr Asp Phe Pro Gly Phe Asp Glu Arg Ala Asp Ala Glu
   1 5 10 15
   Thr Leu Arg Lys
   20
<210> 701
   <211> 11
   <212> PRT
   <213> Homo sapiens
<400> 701
   Asn Phe Ala Thr Ser Leu Tyr Ser Met Ile Lys
   1 5 10
<210> 702
   <211> 9
   <212> PRT
   <213> Homo sapiens
<400> 702
   Ser Glu Ile Asp Leu Phe Asn Ile Arg
   1 5
<210> 703
   <211> 9
   <212> PRT
   <213> Homo sapiens
<400> 703
   Val Leu Thr Glu Ile Ile Ala Ser Arg
   1 5
<210> 704
   <211> 15
   <212> PRT
   <213> Homo sapiens
<400> 704
   Trp Gly Thr Asp Glu Glu Lys Phe Ile Thr Ile Phe Gly Thr Arg
   1 5 10 15
<210> 705
   <211> 12
   <212> PRT
   <213> Homo sapiens
<400> 705
   Gly Thr Val Thr Asp Phe Pro Gly Phe Asp Glu Arg
   1 5 10
<210> 706
   <211> 10
   <212> PRT
   <213> Homo sapiens
<400> 706
   Val Phe Ala Glu Asn Lys Glu Ile Gln Lys
   1 5 10
<210> 707
   <211> 18
   <212> PRT
   <213> Homo sapiens
<400> 707
   Leu Tyr Ala Tyr Glu Pro Ala Asp Thr Ala Leu Leu Leu Asp Asn Met
   1 5 10 15
   Lys Lys
<210> 708
   <211> 7
   <212> PRT
   <213> Homo sapiens
<400> 708
   Leu Pro Gln Thr Leu Ser Arg
   1 5
<210> 709
   <211> 28
   <212> PRT
   <213> Homo sapiens
<400> 709
   Leu Ala Glu Gln Phe Val Leu Leu Asn Leu Val Tyr Glu Thr Thr Asp
   1 5 10 15
   Lys His Leu Ser Pro Asp Gly Gln Tyr Val Pro Arg
   20 25
<210> 710
   <211> 17
   <212> PRT
   <213> Homo sapiens
<400> 710
   Leu Ala Glu Gln Phe Val Leu Leu Asn Leu Val Tyr Glu Thr Thr Asp
   1 5 10 15
   Lys
<210> 711
   <211> 11
   <212> PRT
   <213> Homo sapiens
<400> 711
   Ile Met Phe Val Asp Pro Ser Leu Thr Val Arg
   1 5 10
<210> 712
   <211> 12
   <212> PRT
   <213> Homo sapiens
<400> 712
   Asp Ser Arg Pro Lys Leu Pro Gln Thr Leu Ser Arg
   1 5 10
<210> 713
   <211> 10
   <212> PRT
   <213> Homo sapiens
<400> 713
   Ala Asn Arg Pro Phe Leu Val Phe Ile Arg
   1 5 10
<210> 714
   <211> 17
   <212> PRT
   <213> Homo sapiens
<400> 714
   Ala Thr Glu Asp Glu Gly Ser Glu Gln Lys Ile Pro Glu Ala Thr Asn
   1 5 10 15
   Arg
<210> 715
   <211> 18
   <212> PRT
   <213> Homo sapiens
<400> 715
   Ala Thr Glu Asp Glu Gly Ser Glu Gln Lys Ile Pro Glu Ala Thr Asn
   1 5 10 15
   Arg Arg
<210> 716
   <211> 12
   <212> PRT
   <213> Homo sapiens
<400> 716
   Glu Val Pro Leu Asn Thr Ile Ile Phe Met Gly Arg
   1 5 10
<210> 717
   <211> 10
   <212> PRT
   <213> Homo sapiens
<400> 717
   Phe Arg Ile Glu Asp Gly Phe Ser Leu Lys
   1 5 10
<210> 718
   <211> 25
   <212> PRT
   <213> Homo sapiens
<400> 718
   Ile Thr Asp Val Ile Pro Ser Glu Ala Ile Asn Glu Leu Thr Val Leu
   1 5 10 15
   Val Leu Val Asn Thr Ile Tyr Phe Lys
   20 25
<210> 719
   <211> 18
   <212> PRT
   <213> Homo sapiens
<400> 719
   Lys Ala Thr Glu Asp Glu Gly Ser Glu Gln Lys Ile Pro Glu Ala Thr
   1 5 10 15
   Asn Arg
<210> 720
   <211> 10
   <212> PRT
   <213> Homo sapiens
<400> 720
   Ser Lys Phe Ser Pro Glu Asn Thr Arg Lys
   1 5 10
<210> 721
   <211> 22
   <212> PRT
   <213> Homo sapiens
<400> 721
   Ser Lys Leu Pro Gly Ile Val Ala Glu Gly Arg Asp Asp Leu Tyr Val
   1 5 10 15
   Ser Asp Ala Phe His Lys
   20
<210> 722
   <211> 11
   <212> PRT
   <213> Homo sapiens
<400> 722
   Thr Ser Asp Gln Ile His Phe Phe Phe Ala Lys
   1 5 10
<210> 723
   <211> 8
   <212> PRT
   <213> Homo sapiens
<400> 723
   Ala Glu Leu Gln Glu Gly Ala Arg
   1 5
<210> 724
   <211> 18
   <212> PRT
   <213> Homo sapiens
<400> 724
   Ala His Val Asp Ala Leu Arg Thr His Leu Ala Pro Tyr Ser Asp Glu
   1 5 10 15
   Leu Arg
<210> 725
   <211> 11
   <212> PRT
   <213> Homo sapiens
<400> 725
   Ala Thr Glu His Leu Ser Thr Leu Ser Glu Lys
   1 5 10
<210> 726
   <211> 11
   <212> PRT
   <213> Homo sapiens
<400> 726
   Asp Leu Ala Thr Val Tyr Val Asp Val Leu Lys
   1 5 10
<210> 727
   <211> 17
   <212> PRT
   <213> Homo sapiens
<400> 727
   Asp Ser Gly Arg Asp Tyr Val Ser Gln Phe Glu Gly Ser Ala Leu Gly
   1 5 10 15
   Lys
<210> 728
   <211> 13
   <212> PRT
   <213> Homo sapiens
<400> 728
   Asp Tyr Val Ser Gln Phe Glu Gly Ser Ala Leu Gly Lys
   1 5 10
<210> 729
   <211> 16
   <212> PRT
   <213> Homo sapiens
<400> 729
   Glu Gln Leu Gly Pro Val Thr Gln Glu Phe Trp Asp Asn Leu Glu Lys
   1 5 10 15
<210> 730
   <211> 18
   <212> PRT
   <213> Homo sapiens
<400> 730
   Leu Ala Glu Tyr His Ala Lys Ala Thr Glu His Leu Ser Thr Leu Ser
   1               5                   10                  15
   Glu Lys
<210> 731
   <211> 11
   <212> PRT
   <213> Homo sapiens
<400> 731
   Leu Glu Ala Leu Lys Glu Asn Gly Gly Ala Arg
   1               5                   10
<210> 732
   <211> 7
   <212> PRT
   <213> Homo sapiens
<400> 732
   Leu His Glu Leu Gln Glu Lys
   1               5
<210> 733
   <211> 14
   <212> PRT
   <213> Homo sapiens
<400> 733
   Leu Leu Asp Asn Trp Asp Ser Val Thr Ser Thr Phe Ser Lys
   1               5                   10
<210> 734
   <211> 18
   <212> PRT
   <213> Homo sapiens
<400> 734
   Leu Arg Glu Gln Leu Gly Pro Val Thr Gln Glu Phe Trp Asp Asn Leu
   1               5                   10                  15
   Glu Lys
<210> 735
   <211> 11
   <212> PRT
   <213> Homo sapiens
<400> 735
   Gln Gly Leu Leu Pro Val Leu Glu Ser Phe Lys
   1 5 10
<210> 736
   <211> 15
   <212> PRT
   <213> Homo sapiens
<400> 736
   Gln Lys Val Glu Pro Leu Arg Ala Glu Leu Gln Glu Gly Ala Arg
   1 5 10 15
<210> 737
   <211> 11
   <212> PRT
   <213> Homo sapiens
<400> 737
   Thr His Leu Ala Pro Tyr Ser Asp Glu Leu Arg
   1 5 10
<210> 738
   <211> 13
   <212> PRT
   <213> Homo sapiens
<400> 738
   Thr His Leu Ala Pro Tyr Ser Asp Glu Leu Arg Gln Arg
   1 5 10
<210> 739
   <211> 13
   <212> PRT
   <213> Homo sapiens
<400> 739
   Val Glu Pro Leu Arg Ala Glu Leu Gln Glu Gly Ala Arg
   1 5 10
<210> 740
   <211> 13
   <212> PRT
   <213> Homo sapiens
<400> 740
   Val Lys Asp Leu Ala Thr Val Tyr Val Asp Val Leu Lys
   1 5 10
<210> 741
   <211> 11
   <212> PRT
   <213> Homo sapiens
<400> 741
   Val Gln Pro Tyr Leu Asp Asp Phe Gln Lys Lys
   1 5 10
<210> 742
   <211> 12
   <212> PRT
   <213> Homo sapiens
<400> 742
   Val Ser Phe Leu Ser Ala Leu Glu Glu Tyr Thr Lys
   1 5 10
<210> 743
   <211> 13
   <212> PRT
   <213> Homo sapiens
<400> 743
   Val Ser Phe Leu Ser Ala Leu Glu Glu Tyr Thr Lys Lys
   1 5 10
<210> 744
   <211> 11
   <212> PRT
   <213> Homo sapiens
<400> 744
   Val Lys Ser Pro Glu Leu Gln Ala Glu Ala Lys
   1 5 10
<210> 745
   <211> 9
   <212> PRT
   <213> Homo sapiens
<400> 745
   Ser Pro Glu Leu Gln Ala Glu Ala Lys
   1 5
<210> 746
   <211> 11
   <212> PRT
   <213> Homo sapiens
<400> 746
   Ser Lys Glu Gln Leu Thr Pro Leu Ile Lys Lys
   1 5 10
<210> 747
   <211> 10
   <212> PRT
   <213> Homo sapiens
<400> 747
   Ser Lys Glu Gln Leu Thr Pro Leu Ile Lys
   1 5 10
<210> 748
   <211> 22
   <212> PRT
   <213> Homo sapiens
<400> 748
   Ala Gly Thr Glu Leu Val Asn Phe Leu Ser Tyr Phe Val Glu Leu Gly
   1 5 10 15
   Thr Gln Pro Ala Thr Gln
   20
<210> 749
   <211> 9
   <212> PRT
   <213> Homo sapiens
<400> 749
   Ile Ser Ala Ser Ala Glu Glu Leu Arg
   1 5
<210> 750
   <211> 13
   <212> PRT
   <213> Homo sapiens
<400> 750
   Leu Gly Glu Val Asn Thr Tyr Ala Gly Asp Leu Gln Lys
   1 5 10
<210> 751
   <211> 14
   <212> PRT
   <213> Homo sapiens
<400> 751
   Ser Glu Leu Thr Gln Gln Leu Asn Ala Leu Phe Gln Asp Lys
   1 5 10
<210> 752
   <211> 17
   <212> PRT
   <213> Homo sapiens
<400> 752
   Ser Leu Ala Glu Leu Gly Gly His Leu Asp Gln Gln Val Glu Glu Phe
   1 5 10 15
   Arg
<210> 753
   <211> 21
   <212> PRT
   <213> Homo sapiens
<400> 753
   Ala Thr Phe Gly Cys His Asp Gly Tyr Ser Leu Asp Gly Pro Glu Glu
   1 5 10 15
   Ile Glu Cys Thr Lys
   20
<210> 754
   <211> 9
   <212> PRT
   <213> Homo sapiens
<400> 754
   Ala Thr Val Val Tyr Gln Gly Glu Arg
   1 5
<210> 755
   <211> 12
   <212> PRT
   <213> Homo sapiens
<400> 755
   Cys Phe Lys Glu His Ser Ser Leu Ala Phe Trp Lys
   1 5 10
<210> 756
   <211> 15
   <212> PRT
   <213> Homo sapiens
<400> 756
   Phe Ile Cys Pro Leu Thr Gly Leu Trp Pro Ile Asn Thr Leu Lys
   1 5 10 15
<210> 757
   <211> 19
   <212> PRT
   <213> Homo sapiens
<400> 757
   Lys Cys Ser Tyr Thr Glu Asp Ala Gln Cys Ile Asp Gly Thr Ile Glu
   1               5                   10                  15
   Val Pro Lys
<210> 758
   <211> 17
   <212> PRT
   <213> Homo sapiens
<400> 758
   Thr Cys Pro Lys Pro Asp Asp Leu Pro Phe Ser Thr Val Val Pro Leu
   1               5                   10                  15
   Lys
<210> 759
   <211> 9
   <212> PRT
   <213> Homo sapiens
<400> 759
   Thr Asp Ala Ser Asp Val Lys Pro Cys
   1               5
<210> 760
   <211> 14
   <212> PRT
   <213> Homo sapiens
<400> 760
   Val Cys Pro Phe Ala Gly Ile Leu Glu Asn Gly Ala Val Arg
   1               5                   10
<210> 761
   <211> 21
   <212> PRT
   <213> Homo sapiens
<400> 761
   Ser Asp Gly Ala Pro Ala Ser Asp Ser Lys Pro Gly Ser Ser Glu Ala
   1               5                   10                  15
   Ala Pro Ser Ser Lys
               20
<210> 762
   <211> 14
   <212> PRT
   <213> Homo sapiens
<400> 762
   Glu Thr Pro Ala Ala Thr Glu Ala Pro Ser Ser Thr Pro Lys
   1 5 10
<210> 763
   <211> 24
   <212> PRT
   <213> Homo sapiens
<400> 763
   Ala Glu Pro Pro Lys Ala Pro Glu Gln Glu Gln Ala Ala Pro Gly Pro
   1 5 10 15
   Ala Ala Gly Gly Glu Ala Pro Lys
   20
<210> 764
   <211> 23
   <212> PRT
   <213> Homo sapiens
<400> 764
   Ala Phe Ser Ala Val Asp Thr Asp Gly Asn Gly Thr Ile Asn Ala Gln
   1 5 10 15
   Glu Leu Gly Ala Ala Leu Lys
   20
<210> 765
   <211> 13
   <212> PRT
   <213> Homo sapiens
<400> 765
   Ala Gly Glu Leu Thr Pro Glu Glu Glu Ala Gln Tyr Lys
   1 5 10
<210> 766
   <211> 11
   <212> PRT
   <213> Homo sapiens
<400> 766
   Ala Gly Leu Glu Asp Leu Gln Val Ala Phe Arg
   1 5 10
<210> 767
   <211> 17
   <212> PRT
   <213> Homo sapiens
<400> 767
   Glu Ala Asp Val Asp Gln Asp Gly Arg Val Asn Tyr Glu Glu Phe Ala
   1               5                   10                  15
   Arg
<210> 768
   <211> 10
   <212> PRT
   <213> Homo sapiens
<400> 768
   Tyr Leu Gly Gln Asp Tyr Glu Gln Leu Arg
   1               5                   10
<210> 769
   <211> 11
   <212> PRT
   <213> Homo sapiens
<400> 769
   Trp Asn Thr Thr Leu Tyr Glu Gly Thr Trp Arg
   1               5                   10
<210> 770
   <211> 18
   <212> PRT
   <213> Homo sapiens
<400> 770
   Arg Pro Thr Glu Leu Leu Ser Asn Pro Gln Phe Ile Val Asp Gly Ala
   1               5                   10                  15
   Thr Arg
<210> 771
   <211> 13
   <212> PRT
   <213> Homo sapiens
<400> 771
   Asn Tyr Pro Ala Thr Phe Trp Val Asn Pro Gln Phe Lys
   1               5                   10
<210> 772
   <211> 24
   <212> PRT
   <213> Homo sapiens
<400> 772
   Leu Pro Pro Gly Glu Tyr Val Val Val Pro Ser Thr Phe Glu Pro Asn
   1 5 10 15
   Lys Glu Gly Asp Phe Val Leu Arg
   20
<210> 773
   <211> 12
   <212> PRT
   <213> Homo sapiens
<400> 773
   Leu Glu Ile Cys Asn Leu Thr Pro Asp Ala Leu Lys
   1 5 10
<210> 774
   <211> 9
   <212> PRT
   <213> Homo sapiens
<400> 774
   Asp Phe Phe Leu Ala Asn Ala Ser Arg
   1 5
<210> 775
   <211> 11
   <212> PRT
   <213> Homo sapiens
<400> 775
   Ala Pro Ser Asp Leu Tyr Gln Ile Ile Leu Lys
   1 5 10
<210> 776
   <211> 13
   <212> PRT
   <213> Homo sapiens
<400> 776
   Ala Ala Ala Pro Ala Pro Val Ser Glu Ala Val Cys Arg
   1 5 10
<210> 777
   <211> 13
   <212> PRT
   <213> Homo sapiens
<400> 777
   Cys Gly Glu Asp Asp Glu Thr Ile Pro Ser Glu Tyr Arg
   1 5 10
<210> 778
   <211> 16
   <212> PRT
   <213> Homo sapiens
<400> 778
   Glu Glu Ser Thr Glu Val Leu Lys Ala Gln Ser Ala Gly Thr Val Arg
   1 5 10 15
<210> 779
   <211> 9
   <212> PRT
   <213> Homo sapiens
<400> 779
   Gln Ala Glu Pro Glu Leu Asp Leu Arg
   1 5
<210> 780
   <211> 15
   <212> PRT
   <213> Homo sapiens
<400> 780
   Ser Glu Ser Glu Leu Ile Asp Glu Leu Ser Glu Asp Phe Asp Arg
   1 5 10 15
<210> 781
   <211> 19
   <212> PRT
   <213> Homo sapiens
<400> 781
   Ser Glu Ser Glu Leu Ile Asp Glu Leu Ser Glu Asp Phe Asp Arg Ser
   1 5 10 15
   Glu Cys Lys
<210> 782
   <211> 15
   <212> PRT
   <213> Homo sapiens
<400> 782
   Ile Lys Asp Pro Asp Ala Ser Lys Pro Glu Asp Trp Asp Glu Arg
   1 5 10 15
<210> 783
   <211> 22
   <212> PRT
   <213> Homo sapiens
<400> 783
   Ile Asp Asn Ser Gln Val Glu Ser Gly Ser Leu Glu Asp Asp Trp Asp
   1 5 10 15
   Phe Leu Pro Pro Lys Lys
   20
<210> 784
   <211> 18
   <212> PRT
   <213> Homo sapiens
<400> 784
   Phe Tyr Gly Asp Glu Glu Lys Asp Lys Gly Leu Gln Thr Ser Gln Asp
   1 5 10 15
   Ala Arg
<210> 785
   <211> 12
   <212> PRT
   <213> Homo sapiens
<400> 785
   Glu Gln Phe Leu Asp Gly Asp Gly Trp Thr Ser Arg
   1 5 10
<210> 786
   <211> 14
   <212> PRT
   <213> Homo sapiens
<400> 786
   Phe Tyr Ala Leu Ser Ala Ser Phe Glu Pro Phe Ser Asn Lys
   1 5 10
<210> 787
   <211> 10
   <212> PRT
   <213> Homo sapiens
<400> 787
   Glu Gln Trp Pro Gln Cys Pro Thr Ile Lys
   1 5 10
<210> 788
   <211> 17
   <212> PRT
   <213> Homo sapiens
<400> 788
   Gly Gln Asp His Cys Gly Ile Glu Ser Glu Val Val Ala Gly Ile Pro
   1 5 10 15
   Arg
<210> 789
   <211> 11
   <212> PRT
   <213> Homo sapiens
<400> 789
   Ile Cys Glu Pro Gly Tyr Ser Pro Thr Tyr Lys
   1 5 10
<210> 790
   <211> 14
   <212> PRT
   <213> Homo sapiens
<400> 790
   Ile Cys Glu Pro Gly Tyr Ser Pro Thr Tyr Lys Gln Asp Lys
   1 5 10
<210> 791
   <211> 19
   <212> PRT
   <213> Homo sapiens
<400> 791
   Ile Leu Arg Gly Gln Asp His Cys Gly Ile Glu Ser Glu Val Val Ala
   1 5 10 15
   Gly Ile Pro
<210> 792
   <211> 14
   <212> PRT
   <213> Homo sapiens
<400> 792
   Leu Cys Gly Thr Phe Leu Gly Gly Pro Lys Pro Pro Gln Arg
   1 5 10
<210> 793
   <211> 8
   <212> PRT
   <213> Homo sapiens
<400> 793
   Thr Asp Gln Tyr Trp Glu Lys Ile
   1               5
<210> 794
   <211> 10
   <212> PRT
   <213> Homo sapiens
<400> 794
   Tyr Val Glu Cys Ser Ala Leu Thr Gln Lys
   1               5                   10
<210> 795
   <211> 17
   <212> PRT
   <213> Homo sapiens
<400> 795
   Asn Val Phe Asp Glu Ala Ile Leu Ala Ala Leu Glu Pro Pro Glu Pro
   1               5                   10                  15
   Lys
<210> 796
   <211> 20
   <212> PRT
   <213> Homo sapiens
<400> 796
   Gly Leu Lys Asn Val Phe Asp Glu Ala Ile Leu Ala Ala Leu Glu Pro
   1               5                   10                  15
   Pro Glu Pro Lys
               20
<210> 797
   <211> 21
   <212> PRT
   <213> Homo sapiens
<400> 797
   Ala Asp Phe Asp Asn Thr Val Ala Ile His Pro Thr Ser Ser Glu Glu
   1               5                   10                  15
   Leu Val Thr Leu Arg
               20
<210> 798
   <211> 12
   <212> PRT
   <213> Homo sapiens
<400> 798
   Ala Leu Leu Thr Pro Val Ala Ile Ala Ala Gly Arg
   1               5                   10
<210> 799
   <211> 18
   <212> PRT
   <213> Homo sapiens
<400> 799
   Gly His Ala Ala Phe Thr Ser Asp Pro Lys Pro Thr Ile Glu Val Ser
   1               5                   10                  15
   Gly Lys
<210> 800
   <211> 11
   <212> PRT
   <213> Homo sapiens
<400> 800
   Gly Ile Tyr Ala Val Gly Asp Val Cys Gly Lys
   1               5                   10
<210> 801
   <211> 13
   <212> PRT
   <213> Homo sapiens
<400> 801
   Leu Gly Gly Thr Cys Val Asn Val Gly Cys Val Pro Lys
   1               5                   10
<210> 802
   <211> 22
   <212> PRT
   <213> Homo sapiens
<400> 802
   Leu Gly Ile Gln Thr Asp Asp Lys Gly His Ile Ile Val Asp Glu Phe
   1               5                   10                  15
   Gln Asn Thr Asn Val Lys
               20
<210> 803
   <211> 11
   <212> PRT
   <213> Homo sapiens
<400> 803
   Leu Asn Ala Ile Tyr Gln Asn Asn Leu Thr Lys
   1               5                   10
<210> 804
   <211> 10
   <212> PRT
   <213> Homo sapiens
<400> 804
   Val Ser Glu Glu Ile Glu Asp Ile Ile Lys
   1               5                   10
<210> 805
   <211> 11
   <212> PRT
   <213> Homo sapiens
<400> 805
   Asn Pro Ser Ala Ala Phe Phe Cys Val Ala Arg
   1               5                   10
<210> 806
   <211> 14
   <212> PRT
   <213> Homo sapiens
<400> 806
   Leu Gln Asp Phe Lys Leu Asp Phe Gly Asn Ser Gln Gly Lys
   1               5                   10
<210> 807
   <211> 14
   <212> PRT
   <213> Homo sapiens
<400> 807
   Glu Leu Ser Glu Ala Leu Gly Gln Ile Phe Asp Ser Gln Arg
   1 5 10
<210> 808
   <211> 11
   <212> PRT
   <213> Homo sapiens
<400> 808
   Leu Ala Asp Gly Gly Ala Thr Asn Gln Gly Arg
   1 5 10
<210> 809
   <211> 13
   <212> PRT
   <213> Homo sapiens
<400> 809
   Ser Asp Leu Ala Val Pro Ser Glu Leu Ala Leu Leu Lys
   1 5 10
<210> 810
   <211> 16
   <212> PRT
   <213> Homo sapiens
<400> 810
   Ser Thr Ser Ser Phe Pro Cys Pro Ala Gly His Phe Asn Gly Phe Arg
   1 5 10 15
<210> 811
   <211> 17
   <212> PRT
   <213> Homo sapiens
<400> 811
   Thr Leu Gln Ala Leu Glu Phe His Thr Val Pro Phe Gln Leu Leu Ala
   1 5 10 15
   Arg
<210> 812
   <211> 13
   <212> PRT
   <213> Homo sapiens
<400> 812
   Tyr Ser Ser Asp Tyr Phe Gln Ala Pro Ser Asp Tyr Arg
   1 5 10
<210> 813
   <211> 19
   <212> PRT
   <213> Homo sapiens
<400> 813
   Cys Leu Thr Thr Asp Glu Tyr Asp Gly His Ser Thr Tyr Pro Ser His
   1               5                   10                  15
   Gln Tyr Gln
<210> 814
   <211> 10
   <212> PRT
   <213> Homo sapiens
<400> 814
   Leu Gln Ala Val Thr Asp Asp His Ile Arg
   1               5                   10
<210> 815
   <211> 12
   <212> PRT
   <213> Homo sapiens
<400> 815
   Val Thr Asp Ser Ser Val Ser Val Gln Leu Arg Glu
   1               5                   10
<210> 816
   <211> 14
   <212> PRT
   <213> Homo sapiens
<400> 816
   Thr Arg Val Thr Asp Ser Ser Val Ser Val Gln Leu Arg Glu
   1               5                   10
<210> 817
   <211> 30
   <212> PRT
   <213> Homo sapiens
<400> 817
   Ser Pro Asn Pro Glu Gly Ile Asn Ile Tyr Glu Pro Ala Pro Pro Thr
   1               5                   10                  15
   Gly Pro Thr Gln Arg Pro Leu Glu Thr Leu Gly Asn Phe Arg
               20                  25                  30
<210> 818
   <211> 19
   <212> PRT
   <213> Homo sapiens
<400> 818
   Gln Leu Leu Leu Glu Glu Trp Gly Pro Leu Ser Gly Gly Leu Glu Leu
   1 5 10 15
   Pro Gln Arg
<210> 819
   <211> 15
   <212> PRT
   <213> Homo sapiens
<400> 819
   Tyr Gly Thr Cys Ile Tyr Gln Gly Arg Leu Trp Ala Phe Cys Cys
   1 5 10 15
<210> 820
   <211> 9
   <212> PRT
   <213> Homo sapiens
<400> 820
   Tyr Gly Thr Cys Ile Tyr Gln Gly Arg
   1 5
<210> 821
   <211> 10
   <212> PRT
   <213> Homo sapiens
<400> 821
   Arg Tyr Gly Thr Cys Ile Tyr Gln Gly Arg
   1 5 10
<210> 822
   <211> 6
   <212> PRT
   <213> Homo sapiens
<400> 822
   Leu Trp Ala Phe Cys Cys
   1 5
<210> 823
   <211> 10
   <212> PRT
   <213> Homo sapiens
<400> 823
   Ile Pro Ala Cys Ile Ala Gly Glu Arg Arg
   1 5 10
<210> 824
   <211> 9
   <212> PRT
   <213> Homo sapiens
<400> 824
   Ile Pro Ala Cys Ile Ala Gly Glu Arg
   1 5
<210> 825
   <211> 11
   <212> PRT
   <213> Homo sapiens
<400> 825
   Ala Leu His Phe Ala Ile Ser Glu Tyr Asn Lys
   1 5 10
<210> 826
   <211> 14
   <212> PRT
   <213> Homo sapiens
<400> 826
   Ala Leu His Phe Ala Ile Ser Glu Tyr Asn Lys Ala Thr Lys
   1 5 10
<210> 827
   <211> 19
   <212> PRT
   <213> Homo sapiens
<400> 827
   Ala Arg Gln Gln Thr Val Gly Gly Val Asn Tyr Phe Phe Asp Val Glu
   1 5 10 15
   Val Gly Arg
<210> 828
   <211> 8
   <212> PRT
   <213> Homo sapiens
<400> 828
   Ala Thr Lys Asp Asp Tyr Tyr Arg
   1 5
<210> 829
   <211> 22
   <212> PRT
   <213> Homo sapiens
<400> 829
   Glu Glu Asp Arg Ile Ile Pro Gly Gly Ile Tyr Asn Ala Asp Leu Asn
   1 5 10 15
   Asp Glu Trp Val Gln Arg
   20
<210> 830
   <211> 17
   <212> PRT
   <213> Homo sapiens
<400> 830
   Lys Gln Leu Cys Ser Phe Glu Ile Tyr Glu Val Pro Trp Glu Asn Arg
   1 5 10 15
   Arg
<210> 831
   <211> 15
   <212> PRT
   <213> Homo sapiens
<400> 831
   Gln Leu Cys Ser Phe Glu Ile Tyr Glu Val Pro Trp Glu Asn Arg
   1 5 10 15
<210> 832
   <211> 16
   <212> PRT
   <213> Homo sapiens
<400> 832
   Gln Leu Cys Ser Phe Glu Ile Tyr Glu Val Pro Trp Glu Asn Arg Arg
   1 5 10 15
<210> 833
   <211> 17
   <212> PRT
   <213> Homo sapiens
<400> 833
   Gln Gln Thr Val Gly Gly Val Asn Tyr Phe Phe Asp Val Glu Val Gly
   1 5 10 15
   Arg
<210> 834
   <211> 10
   <212> PRT
   <213> Homo sapiens
<400> 834
   Ser Leu Val Lys Ser Arg Cys Gln Glu Ser
   1 5 10
<210> 835
   <211> 18
   <212> PRT
   <213> Homo sapiens
<400> 835
   Ser Gln Pro Asn Leu Asp Thr Cys Ala Phe His Glu Gln Pro Glu Leu
   1 5 10 15
   Gln Lys
<210> 836
   <211> 19
   <212> PRT
   <213> Homo sapiens
<400> 836
   Ser Gln Pro Asn Leu Asp Thr Cys Ala Phe His Glu Gln Pro Glu Leu
   1 5 10 15
   Gln Lys Lys
<210> 837
   <211> 18
   <212> PRT
   <213> Homo sapiens
<400> 837
   Ile Ile Pro Gly Gly Ile Tyr Asn Ala Asp Leu Asn Asp Glu Trp Val
   1 5 10 15
   Gln Arg
<210> 838
   <211> 16
   <212> PRT
   <213> Homo sapiens
<400> 838
   Val Ser Gln Thr Thr Trp Asp Ser Gly Phe Cys Ala Val Asn Pro Lys
   1 5 10 15
<210> 839
   <211> 11
   <212> PRT
   <213> Homo sapiens
<400> 839
   Lys Leu Gln Ala Thr Val Gln Glu Leu Gln Lys
   1 5 10
<210> 840
   <211> 10
   <212> PRT
   <213> Homo sapiens
<400> 840
   Asp Ala Gly Pro Leu Leu Ile Ser Leu Lys
   1 5 10
<210> 841
   <211> 9
   <212> PRT
   <213> Homo sapiens
<400> 841
   Ala Asp Gln Cys Tyr Glu Asp Val Arg
   1 5
<210> 842
   <211> 16
   <212> PRT
   <213> Homo sapiens
<400> 842
   Ala Ala Pro Glu Ala Ser Gly Thr Pro Ser Ser Asp Ala Val Ser Arg
   1 5 10 15
<210> 843
   <211> 13
   <212> PRT
   <213> Homo sapiens
<400> 843
   Ala Ala Val Tyr His His Phe Ile Ser Asp Gly Val Arg
   1 5 10
<210> 844
   <211> 14
   <212> PRT
   <213> Homo sapiens
<400> 844
   Ala Ala Val Tyr His His Phe Ile Ser Asp Gly Val Arg Lys
   1 5 10
<210> 845
   <211> 11
   <212> PRT
   <213> Homo sapiens
<400> 845
   Ala Cys Glu Pro Gly Val Asp Tyr Val Tyr Lys
   1 5 10
<210> 846
   <211> 14
   <212> PRT
   <213> Homo sapiens
<400> 846
   Ala Gly Asp Phe Leu Glu Ala Asn Tyr Met Asn Leu Gln Arg
   1 5 10
<210> 847
   <211> 10
   <212> PRT
   <213> Homo sapiens
<400> 847
   Cys Ala Glu Glu Asn Cys Phe Ile Gln Lys
   1 5 10
<210> 848
   <211> 10
   <212> PRT
   <213> Homo sapiens
<400> 848
   Asp Phe Asp Phe Val Pro Pro Val Val Arg
   1 5 10
<210> 849
   <211> 20
   <212> PRT
   <213> Homo sapiens
<400> 849
   Asp Thr Trp Val Glu His Trp Pro Glu Glu Asp Glu Cys Gln Asp Glu
   1 5 10 15
   Glu Asn Gln Lys
   20
<210> 850
   <211> 8
   <212> PRT
   <213> Homo sapiens
<400> 850
   Glu Ala Leu Lys Leu Glu Glu Lys
   1               5
<210> 851
   <211> 25
   <212> PRT
   <213> Homo sapiens
<400> 851
   Glu Gly Val Gln Lys Glu Asp Ile Pro Pro Ala Asp Leu Ser Asp Gln
   1               5                   10                  15
   Val Pro Asp Thr Glu Ser Glu Thr Arg
               20                  25
<210> 852
   <211> 16
   <212> PRT
   <213> Homo sapiens
<400> 852
   Glu Tyr Val Leu Pro Ser Phe Glu Val Ile Val Glu Pro Thr Glu Lys
   1               5                   10                  15
<210> 853
   <211> 9
   <212> PRT
   <213> Homo sapiens
<400> 853
   Gly Tyr Thr Gln Gln Leu Ala Phe Arg
   1               5
<210> 854
   <211> 20
   <212> PRT
   <213> Homo sapiens
<400> 854
   Gly Tyr Thr Gln Gln Leu Ala Phe Arg Gln Pro Ser Ser Ala Phe Ala
   1               5                   10                  15
   Ala Phe Val Lys
               20
<210> 855
   <211> 13
   <212> PRT
   <213> Homo sapiens
<400> 855
   Ile Phe Thr Val Asn His Lys Leu Leu Pro Val Gly Arg
   1 5 10
<210> 856
   <211> 20
   <212> PRT
   <213> Homo sapiens
<400> 856
   Ile Leu Leu Gln Gly Thr Pro Val Ala Gln Met Thr Glu Asp Ala Val
   1 5 10 15
   Asp Ala Glu Arg
   20
<210> 857
   <211> 10
   <212> PRT
   <213> Homo sapiens
<400> 857
   Ile His Trp Glu Ser Ala Ser Leu Leu Arg
   1 5 10
<210> 858
   <211> 14
   <212> PRT
   <213> Homo sapiens
<400> 858
   Ile Pro Ile Glu Asp Gly Ser Gly Glu Val Val Leu Ser Arg
   1 5 10
<210> 859
   <211> 11
   <212> PRT
   <213> Homo sapiens
<400> 859
   Ile Arg Tyr Tyr Thr Tyr Leu Ile Met Asn Lys
   1 5 10
<210> 860
   <211> 12
   <212> PRT
   <213> Homo sapiens
<400> 860
   Lys Gln Glu Leu Ser Glu Ala Glu Gln Ala Thr Arg
   1 5 10
<210> 861
   <211> 14
   <212> PRT
   <213> Homo sapiens
<400> 861
   Lys Val Phe Leu Asp Cys Cys Asn Tyr Ile Thr Glu Leu Arg
   1 5 10
<210> 862
   <211> 15
   <212> PRT
   <213> Homo sapiens
<400> 862
   Lys Val Phe Leu Asp Cys Cys Asn Tyr Ile Thr Glu Leu Arg Arg
   1 5 10 15
<210> 863
   <211> 11
   <212> PRT
   <213> Homo sapiens
<400> 863
   Lys Val Leu Leu Asp Gly Val Gln Asn Pro Arg
   1 5 10
<210> 864
   <211> 14
   <212> PRT
   <213> Homo sapiens
<400> 864
   Leu Asp Lys Ala Cys Glu Pro Gly Val Asp Tyr Val Tyr Lys
   1 5 10
<210> 865
   <211> 7
   <212> PRT
   <213> Homo sapiens
<400> 865
   Leu Pro Tyr Ser Val Val Arg
   1 5
<210> 866
   <211> 17
   <212> PRT
   <213> Homo sapiens
<400> 866
   Leu Ser Ile Asn Thr His Pro Ser Gln Lys Pro Leu Ser Ile Thr Val
   1 5 10 15
   Arg
<210> 867
   <211> 14
   <212> PRT
   <213> Homo sapiens
<400> 867
   Leu Val Ala Tyr Tyr Thr Leu Ile Gly Ala Ser Gly Gln Arg
   1 5 10
<210> 868
   <211> 9
   <212> PRT
   <213> Homo sapiens
<400> 868
   Asn Thr Leu Ile Ile Tyr Leu Asp Lys
   1 5
<210> 869
   <211> 11
   <212> PRT
   <213> Homo sapiens
<400> 869
   Gln Glu Leu Ser Glu Ala Glu Gln Ala Thr Arg
   1 5 10
<210> 870
   <211> 15
   <212> PRT
   <213> Homo sapiens
<400> 870
   Arg Ile Pro Ile Glu Asp Gly Ser Gly Glu Val Val Leu Ser Arg
   1 5 10 15
<210> 871
   <211> 10
   <212> PRT
   <213> Homo sapiens
<400> 871
   Ser Asp Asp Lys Val Thr Leu Glu Glu Arg
   1 5 10
<210> 872
   <211> 13
   <212> PRT
   <213> Homo sapiens
<400> 872
   Ser Asp Asp Lys Val Thr Leu Glu Glu Arg Leu Asp Lys
   1 5 10
<210> 873
   <211> 17
   <212> PRT
   <213> Homo sapiens
<400> 873
   Ser Glu Glu Thr Lys Glu Asn Glu Gly Phe Thr Val Thr Ala Glu Gly
   1 5 10 15
   Lys
<210> 874
   <211> 16
   <212> PRT
   <213> Homo sapiens
<400> 874
   Ser Gly Ile Pro Ile Val Thr Ser Pro Tyr Gln Ile His Phe Thr Lys
   1 5 10 15
<210> 875
   <211> 12
   <212> PRT
   <213> Homo sapiens
<400> 875
   Ser Gly Ser Asp Glu Val Gln Val Gly Gln Gln Arg
   1 5 10
<210> 876
   <211> 16
   <212> PRT
   <213> Homo sapiens
<400> 876
   Ser Asn Leu Asp Glu Asp Ile Ile Ala Glu Glu Asn Ile Val Ser Arg
   1 5 10 15
<210> 877
   <211> 13
   <212> PRT
   <213> Homo sapiens
<400> 877
   Ser Ser Leu Ser Val Pro Tyr Val Ile Val Pro Leu Lys
   1 5 10
<210> 878
   <211> 22
   <212> PRT
   <213> Homo sapiens
<400> 878
   Ser Ser Leu Ser Val Pro Tyr Val Ile Val Pro Leu Lys Thr Gly Leu
   1 5 10 15
   Gln Glu Val Glu Val Lys
   20
<210> 879
   <211> 8
   <212> PRT
   <213> Homo sapiens
<400> 879
   Ser Val Gln Leu Thr Glu Lys Arg
   1 5
<210> 880
   <211> 16
   <212> PRT
   <213> Homo sapiens
<400> 880
   Thr Glu Leu Arg Pro Gly Glu Thr Leu Asn Val Asn Phe Leu Leu Arg
   1 5 10 15
<210> 881
   <211> 9
   <212> PRT
   <213> Homo sapiens
<400> 881
   Thr Gly Leu Gln Glu Val Glu Val Lys
   1 5
<210> 882
   <211> 14
   <212> PRT
   <213> Homo sapiens
<400> 882
   Thr Lys Lys Gln Glu Leu Ser Glu Ala Glu Gln Ala Thr Arg
   1 5 10
<210> 883
   <211> 23
   <212> PRT
   <213> Homo sapiens
<400> 883
   Thr Met Gln Ala Leu Pro Tyr Ser Thr Val Gly Asn Ser Asn Asn Tyr
   1 5 10 15
   Leu His Leu Ser Val Leu Arg
   20
<210> 884
   <211> 12
   <212> PRT
   <213> Homo sapiens
<400> 884
   Thr Arg Phe Ile Ser Leu Gly Glu Ala Cys Lys Lys
   1 5 10
<210> 885
   <211> 16
   <212> PRT
   <213> Homo sapiens
<400> 885
   Val Glu Leu Leu His Asn Pro Ala Phe Cys Ser Leu Ala Thr Thr Lys
   1 5 10 15
<210> 886
   <211> 13
   <212> PRT
   <213> Homo sapiens
<400> 886
   Val Phe Leu Asp Cys Cys Asn Tyr Ile Thr Glu Leu Arg
   1 5 10
<210> 887
   <211> 14
   <212> PRT
   <213> Homo sapiens
<400> 887
   Val Phe Leu Asp Cys Cys Asn Tyr Ile Thr Glu Leu Arg Arg
   1 5 10
<210> 888
   <211> 16
   <212> PRT
   <213> Homo sapiens
<400> 888
   Val His Gln Tyr Phe Asn Val Glu Leu Ile Gln Pro Gly Ala Val Lys
   1 5 10 15
<210> 889
   <211> 10
   <212> PRT
   <213> Homo sapiens
<400> 889
   Val Leu Leu Asp Gly Val Gln Asn Pro Arg
   1 5 10
<210> 890
   <211> 22
   <212> PRT
   <213> Homo sapiens
<400> 890
   Val Pro Val Ala Val Gln Gly Glu Asp Thr Val Gln Ser Leu Thr Gln
   1 5 10 15
   Gly Asp Gly Val Ala Lys
   20
<210> 891
   <211> 12
   <212> PRT
   <213> Homo sapiens
<400> 891
   Val Ser His Ser Glu Asp Asp Cys Leu Ala Phe Lys
   1 5 10
<210> 892
   <211> 11
   <212> PRT
   <213> Homo sapiens
<400> 892
   Val Thr Ile Lys Pro Ala Pro Glu Thr Glu Lys
   1 5 10
<210> 893
   <211> 16
   <212> PRT
   <213> Homo sapiens
<400> 893
   Val Val Leu Val Ala Val Asp Lys Gly Val Phe Val Leu Asn Lys Lys
   1 5 10 15
<210> 894
   <211> 13
   <212> PRT
   <213> Homo sapiens
<400> 894
   Val Tyr Ala Tyr Tyr Asn Leu Glu Glu Ser Cys Thr Arg
   1 5 10
<210> 895
   <211> 9
   <212> PRT
   <213> Homo sapiens
<400> 895
   Tyr Tyr Thr Tyr Leu Ile Met Asn Lys
   1 5
<210> 896
   <211> 17
   <212> PRT
   <213> Homo sapiens
<400> 896
   Glu Ile Val His Leu Gln Ala Gly Gln Cys Gly Asn Gln Ile Gly Ala
   1 5 10 15
   Lys
<210> 897
   <211> 18
   <212> PRT
   <213> Homo sapiens
<400> 897
   Gly His Tyr Thr Glu Gly Ala Glu Leu Val Asp Ser Val Leu Asp Val
   1 5 10 15
   Val Arg
<210> 898
   <211> 19
   <212> PRT
   <213> Homo sapiens
<400> 898
   Gly His Tyr Thr Glu Gly Ala Glu Leu Val Asp Ser Val Leu Asp Val
   1 5 10 15
   Val Arg Lys
<210> 899
   <211> 16
   <212> PRT
   <213> Homo sapiens
<400> 899
   Ile Asn Val Tyr Tyr Asn Glu Ala Thr Gly Gly Lys Tyr Val Pro Arg
   1 5 10 15
<210> 900
   <211> 14
   <212> PRT
   <213> Homo sapiens
<400> 900
   Asn Ser Ser Tyr Phe Val Glu Trp Ile Pro Asn Asn Val Lys
   1 5 10
<210> 901
   <211> 26
   <212> PRT
   <213> Homo sapiens
<400> 901
   Ser Gly Pro Phe Gly Gln Ile Phe Arg Pro Asp Asn Phe Val Phe Gly
   1 5 10 15
   Gln Ser Gly Ala Gly Asn Asn Trp Ala Lys
   20 25
<210> 902
   <211> 9
   <212> PRT
   <213> Homo sapiens
<400> 902
   Tyr Leu Thr Val Ala Ala Val Phe Arg
   1 5
<210> 903
   <211> 16
   <212> PRT
   <213> Homo sapiens
<400> 903
   Phe Ser Gly Ser Asn Ser Gly Asn Thr Ala Thr Leu Thr Ile Ser Arg
   1 5 10 15
<210> 904
   <211> 8
   <212> PRT
   <213> Homo sapiens
<400> 904
   Leu Thr Val Leu Ser Gln Pro Lys
   1 5
<210> 905
   <211> 18
   <212> PRT
   <213> Homo sapiens
<400> 905
   Tyr Val Leu Thr Gln Pro Pro Ser Val Ser Val Ala Pro Gly Glu Thr
   1 5 10 15
   Ala Arg
<210> 906
   <211> 12
   <212> PRT
   <213> Homo sapiens
<400> 906
   Thr Phe Gly Gln Gly Thr Lys Val Glu Ile Lys Arg
   1 5 10
<210> 907
   <211> 9
   <212> PRT
   <213> Homo sapiens
<400> 907
   Leu Leu Ile Tyr Gly Ala Ser Ser Arg
   1 5
<210> 908
   <211> 16
   <212> PRT
   <213> Homo sapiens
<400> 908
   Phe Ser Gly Ser Gly Ser Gly Thr Asp Phe Thr Leu Thr Ile Ser Arg
   1               5                   10                  15
<210> 909
   <211> 23
   <212> PRT
   <213> Homo sapiens
<400> 909
   Ala Thr Gly Ile Pro Asp Arg Phe Ser Gly Ser Gly Ser Gly Thr Asp
   1               5                   10                  15
   Phe Thr Leu Thr Ile Ser Arg
               20
<210> 910
   <211> 22
   <212> PRT
   <213> Homo sapiens
<400> 910
   Ala Ser Gln Ser Val Ser Ser Ser Tyr Leu Ala Trp Tyr Gln Gln Lys
   1               5                   10                  15
   Pro Gly Gln Ala Pro Arg
               20
<210> 911
   <211> 11
   <212> PRT
   <213> Homo sapiens
<400> 911
   Ala Ser Ser Leu Glu Ser Gly Val Pro Ser Arg
   1               5                   10
<210> 912
   <211> 18
   <212> PRT
   <213> Homo sapiens
<400> 912
   Asp Ile Gln Met Thr Gln Ser Pro Ser Thr Leu Ser Ala Ser Val Gly
   1               5                   10                  15
   Asp Arg
<210> 913
   <211> 24
   <212> PRT
   <213> Homo sapiens
<400> 913
   Tyr Leu Thr Trp Ala Ser Arg Gln Glu Pro Ser Gln Gly Thr Thr Thr
   1               5                   10                  15
   Phe Ala Val Thr Ser Ile Leu Arg
               20
<210> 914
   <211> 7
   <212> PRT
   <213> Homo sapiens
<400> 914
   Tyr Leu Thr Trp Ala Ser Arg
   1               5
<210> 915
   <211> 19
   <212> PRT
   <213> Homo sapiens
<400> 915
   Trp Leu Gln Gly Ser Gln Glu Leu Pro Arg Glu Lys Tyr Leu Thr Trp
   1               5                   10                  15
   Ala Ser Arg
<210> 916
   <211> 12
   <212> PRT
   <213> Homo sapiens
<400> 916
   Trp Leu Gln Gly Ser Gln Glu Leu Pro Arg Glu Lys
   1               5                   10
<210> 917
   <211> 8
   <212> PRT
   <213> Homo sapiens
<400> 917
   Val Ala Ala Glu Asp Trp Lys Lys
   1               5
<210> 918
   <211> 7
   <212> PRT
   <213> Homo sapiens
<400> 918
   Val Ala Ala Glu Asp Trp Lys
   1 5
<210> 919
   <211> 9
   <212> PRT
   <213> Homo sapiens
<400> 919
   Thr Pro Leu Thr Ala Thr Leu Ser Lys
   1 5
<210> 920
   <211> 21
   <212> PRT
   <213> Homo sapiens
<400> 920
   Thr Phe Thr Cys Thr Ala Ala Tyr Pro Glu Ser Lys Thr Pro Leu Thr
   1 5 10 15
   Ala Thr Leu Ser Lys
   20
<210> 921
   <211> 12
   <212> PRT
   <213> Homo sapiens
<400> 921
   Thr Phe Thr Cys Thr Ala Ala Tyr Pro Glu Ser Lys
   1 5 10
<210> 922
   <211> 7
   <212> PRT
   <213> Homo sapiens
<400> 922
   Ser Val Thr Cys His Val Lys
   1 5
<210> 923
   <211> 32
   <212> PRT
   <213> Homo sapiens
<400> 923
   Ser Gly Asn Thr Phe Arg Pro Glu Val His Leu Leu Pro Pro Pro Ser
   1               5                   10                  15
   Glu Glu Leu Ala Leu Asn Glu Leu Val Thr Leu Thr Cys Leu Ala Arg
               20                  25                  30
<210> 924
   <211> 32
   <212> PRT
   <213> Homo sapiens
<400> 924
   Ser Ala Val Gln Gly Pro Pro Glu Arg Asp Leu Cys Gly Cys Tyr Ser
   1               5                   10                  15
   Val Ser Ser Val Leu Pro Gly Cys Ala Glu Pro Trp Asn His Gly Lys
               20                  25                  30
<210> 925
   <211> 9
   <212> PRT
   <213> Homo sapiens
<400> 925
   Ser Ala Val Gln Gly Pro Pro Glu Arg
   1               5
<210> 926
   <211> 17
   <212> PRT
   <213> Homo sapiens
<400> 926
   Gln Glu Pro Ser Gln Gly Thr Thr Thr Phe Ala Val Thr Ser Ile Leu
   1               5                   10                  15
   Arg
<210> 927
   <211> 30
   <212> PRT
   <213> Homo sapiens
<400> 927
   Asn Phe Pro Pro Ser Gln Asp Ala Ser Gly Asp Leu Tyr Thr Thr Ser
   1               5                   10                  15
   Ser Gln Leu Thr Leu Pro Ala Thr Gln Cys Leu Ala Gly Lys
               20                  25                  30
<210> 928
   <211> 21
   <212> PRT
   <213> Homo sapiens
<400> 928
   Lys Gly Asp Thr Phe Ser Cys Met Val Gly His Glu Ala Leu Pro Leu
   1 5 10 15
   Ala Phe Thr Gln Lys
   20
<210> 929
   <211> 20
   <212> PRT
   <213> Homo sapiens
<400> 929
   Gly Phe Ser Pro Lys Asp Val Leu Val Arg Trp Leu Gln Gly Ser Gln
   1 5 10 15
   Glu Leu Pro Arg
   20
<210> 930
   <211> 10
   <212> PRT
   <213> Homo sapiens
<400> 930
   Gly Phe Ser Pro Lys Asp Val Leu Val Arg
   1 5 10
<210> 931
   <211> 20
   <212> PRT
   <213> Homo sapiens
<400> 931
   Gly Asp Thr Phe Ser Cys Met Val Gly His Glu Ala Leu Pro Leu Ala
   1 5 10 15
   Phe Thr Gln Lys
   20
<210> 932
   <211> 26
   <212> PRT
   <213> Homo sapiens
<400> 932
   Glu Lys Tyr Leu Thr Trp Ala Ser Arg Gln Glu Pro Ser Gln Gly Thr
   1 5 10 15
   Thr Thr Phe Ala Val Thr Ser Ile Leu Arg
   20 25
<210> 933
   <211> 9
   <212> PRT
   <213> Homo sapiens
<400> 933
   Glu Lys Tyr Leu Thr Trp Ala Ser Arg
   1 5
<210> 934
   <211> 17
   <212> PRT
   <213> Homo sapiens
<400> 934
   Asp Val Leu Val Arg Trp Leu Gln Gly Ser Gln Glu Leu Pro Arg Glu
   1 5 10 15
   Lys
<210> 935
   <211> 15
   <212> PRT
   <213> Homo sapiens
<400> 935
   Asp Val Leu Val Arg Trp Leu Gln Gly Ser Gln Glu Leu Pro Arg
   1 5 10 15
<210> 936
   <211> 23
   <212> PRT
   <213> Homo sapiens
<400> 936
   Asp Leu Cys Gly Cys Tyr Ser Val Ser Ser Val Leu Pro Gly Cys Ala
   1 5 10 15
   Glu Pro Trp Asn His Gly Lys
   20
<210> 937
   <211> 15
   <212> PRT
   <213> Homo sapiens
<400> 937
   Asp Ala Ser Gly Val Thr Phe Thr Trp Thr Pro Ser Ser Gly Lys
   1 5 10 15
<210> 938
   <211> 12
   <212> PRT
   <213> Homo sapiens
<400> 938
   Ala Glu Thr Gly Asp Lys Val Tyr Val His Leu Lys
   1 5 10
<210> 939
   <211> 12
   <212> PRT
   <213> Homo sapiens
<400> 939
   Ala Leu Tyr Leu Gln Tyr Thr Asp Glu Thr Phe Arg
   1 5 10
<210> 940
   <211> 14
   <212> PRT
   <213> Homo sapiens
<400> 940
   Asp Ile Ala Ser Gly Leu Ile Gly Pro Leu Ile Ile Cys Lys
   1 5 10
<210> 941
   <211> 14
   <212> PRT
   <213> Homo sapiens
<400> 941
   Asp Leu Tyr Ser Gly Leu Ile Gly Pro Leu Ile Val Cys Arg
   1 5 10
<210> 942
   <211> 14
   <212> PRT
   <213> Homo sapiens
<400> 942
   Glu Val Gly Pro Thr Asn Ala Asp Pro Val Cys Leu Ala Lys
   1               5                   10
<210> 943
   <211> 10
   <212> PRT
   <213> Homo sapiens
<400> 943
   Glu Tyr Thr Asp Ala Ser Phe Thr Asn Arg
   1               5                   10
<210> 944
   <211> 19
   <212> PRT
   <213> Homo sapiens
<400> 944
   Phe Asn Lys Asn Asn Glu Gly Thr Tyr Tyr Ser Pro Asn Tyr Asn Pro
   1               5                   10                  15
   Gln Ser Arg
<210> 945
   <211> 13
   <212> PRT
   <213> Homo sapiens
<400> 945
   Gly Ala Tyr Pro Leu Ser Ile Glu Pro Ile Gly Val Arg
   1               5                   10
<210> 946
   <211> 20
   <212> PRT
   <213> Homo sapiens
<400> 946
   His Tyr Tyr Ile Gly Ile Ile Glu Thr Thr Trp Asp Tyr Ala Ser Asp
   1               5                   10                  15
   His Gly Glu Lys
               20
<210> 947
   <211> 23
   <212> PRT
   <213> Homo sapiens
<400> 947
   Lys Ala Glu Glu Glu His Leu Gly Ile Leu Gly Pro Gln Leu His Ala
   1               5                   10                  15
   Asp Val Gly Asp Lys Val Lys
               20
<210> 948
   <211> 13
   <212> PRT
   <213> Homo sapiens
<400> 948
   Lys Ala Leu Tyr Leu Gln Tyr Thr Asp Glu Thr Phe Arg
   1               5                   10
<210> 949
   <211> 20
   <212> PRT
   <213> Homo sapiens
<400> 949
   Lys Leu Ile Ser Val Asp Thr Glu His Ser Asn Ile Tyr Leu Gln Asn
   1               5                   10                  15
   Gly Pro Asp Arg
               20
<210> 950
   <211> 19
   <212> PRT
   <213> Homo sapiens
<400> 950
   Leu Ile Ser Val Asp Thr Glu His Ser Asn Ile Tyr Leu Gln Asn Gly
   1               5                   10                  15
   Pro Asp Arg
<210> 951
   <211> 16
   <212> PRT
   <213> Homo sapiens
<400> 951
   Asn Asn Glu Gly Thr Tyr Tyr Ser Pro Asn Tyr Asn Pro Gln Ser Arg
   1               5                   10                  15
<210> 952
   <211> 12
   <212> PRT
   <213> Homo sapiens
<400> 952
   Gln Ser Glu Asp Ser Thr Phe Tyr Leu Gly Glu Arg
   1 5 10
<210> 953
   <211> 23
   <212> PRT
   <213> Homo sapiens
<400> 953
   Ser Gly Ala Gly Thr Glu Asp Ser Ala Cys Ile Pro Trp Ala Tyr Tyr
   1 5 10 15
   Ser Thr Val Asp Gln Val Lys
   20
<210> 954
   <211> 23
   <212> PRT
   <213> Homo sapiens
<400> 954
   Ser Val Pro Pro Ser Ala Ser His Val Ala Pro Thr Glu Thr Phe Thr
   1 5 10 15
   Tyr Glu Trp Thr Val Pro Lys
   20
<210> 955
   <211> 21
   <212> PRT
   <213> Homo sapiens
<400> 955
   Thr Tyr Cys Ser Glu Pro Glu Lys Val Asp Lys Asp Asn Glu Asp Phe
   1 5 10 15
   Gln Glu Ser Asn Arg
   20
<210> 956
   <211> 13
   <212> PRT
   <213> Homo sapiens
<400> 956
   Val Asn Lys Asp Asp Glu Glu Phe Ile Glu Ser Asn Lys
   1 5 10
<210> 957
   <211> 7
   <212> PRT
   <213> Homo sapiens
<400> 957
   Tyr Thr Val Asn Gln Cys Arg
   1 5
<210> 958
   <211> 15
   <212> PRT
   <213> Homo sapiens
<400> 958
   Ala Asp Arg Asp Gln Tyr Glu Leu Leu Cys Leu Asp Asn Thr Arg
   1 5 10 15
<210> 959
   <211> 9
   <212> PRT
   <213> Homo sapiens
<400> 959
   Ala Pro Asn His Ala Val Val Thr Arg
   1 5
<210> 960
   <211> 8
   <212> PRT
   <213> Homo sapiens
<400> 960
   Ala Ser Tyr Leu Asp Cys Ile Arg
   1 5
<210> 961
   <211> 11
   <212> PRT
   <213> Homo sapiens
<400> 961
   Cys Asp Glu Trp Ser Val Asn Ser Val Gly Lys
   1 5 10
<210> 962
   <211> 13
   <212> PRT
   <213> Homo sapiens
<400> 962
   Cys Leu Lys Asp Gly Ala Gly Asp Val Ala Phe Val Lys
   1 5 10
<210> 963
   <211> 12
   <212> PRT
   <213> Homo sapiens
<400> 963
   Cys Leu Val Glu Lys Gly Asp Val Ala Phe Val Lys
   1 5 10
<210> 964
   <211> 13
   <212> PRT
   <213> Homo sapiens
<400> 964
   Cys Ser Thr Ser Ser Leu Leu Glu Ala Cys Thr Phe Arg
   1 5 10
<210> 965
   <211> 15
   <212> PRT
   <213> Homo sapiens
<400> 965
   Cys Ser Thr Ser Ser Leu Leu Glu Ala Cys Thr Phe Arg Arg Pro
   1 5 10 15
<210> 966
   <211> 15
   <212> PRT
   <213> Homo sapiens
<400> 966
   Asp Cys His Leu Ala Gln Val Pro Ser His Thr Val Val Ala Arg
   1 5 10 15
<210> 967
   <211> 8
   <212> PRT
   <213> Homo sapiens
<400> 967
   Asp Asp Thr Val Cys Leu Ala Lys
   1 5
<210> 968
   <211> 10
   <212> PRT
   <213> Homo sapiens
<400> 968
   Asp Gly Ala Gly Asp Val Ala Phe Val Lys
   1 5 10
<210> 969
   <211> 8
   <212> PRT
   <213> Homo sapiens
<400> 969
   Asp Lys Glu Ala Cys Val His Lys
   1 5
<210> 970
   <211> 11
   <212> PRT
   <213> Homo sapiens
<400> 970
   Asp Lys Glu Ala Cys Val His Lys Ile Leu Arg
   1 5 10
<210> 971
   <211> 13
   <212> PRT
   <213> Homo sapiens
<400> 971
   Asp Leu Leu Phe Lys Asp Ser Ala His Gly Phe Leu Lys
   1 5 10
<210> 972
   <211> 17
   <212> PRT
   <213> Homo sapiens
<400> 972
   Asp Leu Leu Phe Lys Asp Ser Ala His Gly Phe Leu Lys Val Pro Pro
   1 5 10 15
   Arg
<210> 973
   <211> 13
   <212> PRT
   <213> Homo sapiens
<400> 973
   Asp Leu Leu Phe Arg Asp Asp Thr Val Cys Leu Ala Lys
   1 5 10
<210> 974
   <211> 12
   <212> PRT
   <213> Homo sapiens
<400> 974
   Asp Ser Ala His Gly Phe Leu Lys Val Pro Pro Arg
   1 5 10
<210> 975
   <211> 10
   <212> PRT
   <213> Homo sapiens
<400> 975
   Asp Ser Gly Phe Gln Met Asn Gln Leu Arg
   1 5 10
<210> 976
   <211> 11
   <212> PRT
   <213> Homo sapiens
<400> 976
   Asp Tyr Glu Leu Leu Cys Leu Asp Gly Thr Arg
   1 5 10
<210> 977
   <211> 12
   <212> PRT
   <213> Homo sapiens
<400> 977
   Asp Tyr Glu Leu Leu Cys Leu Asp Gly Thr Arg Lys
   1 5 10
<210> 978
   <211> 24
   <212> PRT
   <213> Homo sapiens
<400> 978
   Asp Tyr Glu Leu Leu Cys Leu Asp Gly Thr Arg Lys Pro Val Glu Glu
   1 5 10 15
   Tyr Ala Asn Cys His Leu Ala Arg
   20
<210> 979
   <211> 14
   <212> PRT
   <213> Homo sapiens
<400> 979
   Glu Asp Pro Gln Thr Phe Tyr Tyr Ala Val Ala Val Val Lys
   1 5 10
<210> 980
   <211> 15
   <212> PRT
   <213> Homo sapiens
<400> 980
   Glu Asp Pro Gln Thr Phe Tyr Tyr Ala Val Ala Val Val Lys Lys
   1 5 10 15
<210> 981
   <211> 11
   <212> PRT
   <213> Homo sapiens
<400> 981
   Glu Phe Gln Leu Phe Ser Ser Pro His Gly Lys
   1 5 10
<210> 982
   <211> 16
   <212> PRT
   <213> Homo sapiens
<400> 982
   Glu Phe Gln Leu Phe Ser Ser Pro His Gly Lys Asp Leu Leu Phe Lys
   1 5 10 15
<210> 983
   <211> 11
   <212> PRT
   <213> Homo sapiens
<400> 983
   Glu Gly Tyr Tyr Gly Tyr Thr Gly Ala Phe Arg
   1 5 10
<210> 984
   <211> 16
   <212> PRT
   <213> Homo sapiens
<400> 984
   Glu Gly Thr Cys Pro Glu Ala Pro Thr Asp Glu Cys Lys Pro Val Lys
   1               5                   10                  15
<210> 985
   <211> 14
   <212> PRT
   <213> Homo sapiens
<400> 985
   Phe Asp Glu Phe Phe Ser Glu Gly Cys Ala Pro Gly Ser Lys
   1               5                   10
<210> 986
   <211> 15
   <212> PRT
   <213> Homo sapiens
<400> 986
   Phe Asp Glu Phe Phe Ser Glu Gly Cys Ala Pro Gly Ser Lys Lys
   1               5                   10                  15
<210> 987
   <211> 18
   <212> PRT
   <213> Homo sapiens
<400> 987
   Gly Asp Val Ala Phe Val Lys His Gln Thr Val Pro Gln Asn Thr Gly
   1               5                   10                  15
   Gly Lys
<210> 988
   <211> 21
   <212> PRT
   <213> Homo sapiens
<400> 988
   Phe Asp Glu Phe Phe Ser Glu Gly Cys Ala Pro Gly Ser Lys Lys Asp
   1               5                   10                  15
   Ser Ser Leu Cys Lys
               20
<210> 989
   <211> 11
   <212> PRT
   <213> Homo sapiens
<400> 989
   His Gln Thr Val Pro Gln Asn Thr Gly Gly Lys
   1 5 10
<210> 990
   <211> 18
   <212> PRT
   <213> Homo sapiens
<400> 990
   His Gln Thr Val Pro Gln Asn Thr Gly Gly Lys Asn Pro Asp Pro Trp
   1 5 10 15
   Ala Lys
<210> 991
   <211> 23
   <212> PRT
   <213> Homo sapiens
<400> 991
   His Gln Thr Val Pro Gln Asn Thr Gly Gly Lys Asn Pro Asp Pro Trp
   1 5 10 15
   Ala Lys Asn Leu Asn Glu Lys
   20
<210> 992
   <211> 11
   <212> PRT
   <213> Homo sapiens
<400> 992
   His Ser Thr Ile Phe Glu Asn Leu Ala Asn Lys
   1 5 10
<210> 993
   <211> 14
   <212> PRT
   <213> Homo sapiens
<400> 993
   His Ser Thr Ile Phe Glu Asn Leu Ala Asn Lys Ala Asp Arg
   1 5 10
<210> 994
   <211> 15
   <212> PRT
   <213> Homo sapiens
<400> 994
   Ile Glu Cys Val Ser Ala Glu Thr Thr Glu Asp Cys Ile Ala Lys
   1 5 10 15
<210> 995
   <211> 19
   <212> PRT
   <213> Homo sapiens
<400> 995
   Ile Asn His Cys Arg Phe Asp Glu Phe Phe Ser Glu Gly Cys Ala Pro
   1 5 10 15
   Gly Ser Lys
<210> 996
   <211> 20
   <212> PRT
   <213> Homo sapiens
<400> 996
   Ile Asn His Cys Arg Phe Asp Glu Phe Phe Ser Glu Gly Cys Ala Pro
   1 5 10 15
   Gly Ser Lys Lys
   20
<210> 997
   <211> 14
   <212> PRT
   <213> Homo sapiens
<400> 997
   Lys Cys Ser Thr Ser Ser Leu Leu Glu Ala Cys Thr Phe Arg
   1 5 10
<210> 998
   <211> 11
   <212> PRT
   <213> Homo sapiens
<400> 998
   Lys Asp Ser Gly Phe Gln Met Asn Gln Leu Arg
   1 5 10
<210> 999
   <211> 13
   <212> PRT
   <213> Homo sapiens
<400> 999
   Lys Pro Val Glu Glu Tyr Ala Asn Cys His Leu Ala Arg
   1 5 10
<210> 1000
   <211> 12
   <212> PRT
   <213> Homo sapiens
<400> 1000
   Lys Ser Ala Ser Asp Leu Thr Trp Asp Asn Leu Lys
   1 5 10
<210> 1001
   <211> 15
   <212> PRT
   <213> Homo sapiens
<400> 1001
   Leu Cys Met Gly Ser Gly Leu Asn Leu Cys Glu Pro Asn Asn Lys
   1 5 10 15
<210> 1002
   <211> 26
   <212> PRT
   <213> Homo sapiens
<400> 1002
   Leu Cys Met Gly Ser Gly Leu Asn Leu Cys Glu Pro Asn Asn Lys Glu
   1 5 10 15
   Gly Tyr Tyr Gly Tyr Thr Gly Ala Phe Arg
   20 25
<210> 1003
   <211> 17
   <212> PRT
   <213> Homo sapiens
<400> 1003
   Leu His Asp Arg Asn Thr Tyr Glu Lys Tyr Leu Gly Glu Glu Tyr Val
   1 5 10 15
   Lys
<210> 1004
   <211> 13
   <212> PRT
   <213> Homo sapiens
<400> 1004
   Leu Lys Cys Asp Glu Trp Ser Val Asn Ser Val Gly Lys
   1 5 10
<210> 1005
   <211> 28
   <212> PRT
   <213> Homo sapiens
<400> 1005
   Leu Lys Cys Asp Glu Trp Ser Val Asn Ser Val Gly Lys Ile Glu Cys
   1 5 10 15
   Val Ser Ala Glu Thr Thr Glu Asp Cys Ile Ala Lys
   20 25
<210> 1006
   <211> 12
   <212> PRT
   <213> Homo sapiens
<400> 1006
   Met Tyr Leu Gly Tyr Glu Tyr Val Thr Ala Ile Arg
   1 5 10
<210> 1007
   <211> 15
   <212> PRT
   <213> Homo sapiens
<400> 1007
   Met Tyr Leu Gly Tyr Glu Tyr Val Thr Ala Ile Arg Asn Leu Arg
   1 5 10 15
<210> 1008
   <211> 16
   <212> PRT
   <213> Homo sapiens
<400> 1008
   Asn Leu Asn Glu Lys Asp Tyr Glu Leu Leu Cys Leu Asp Gly Thr Arg
   1 5 10 15
<210> 1009
   <211> 17
   <212> PRT
   <213> Homo sapiens
<400> 1009
   Asn Leu Asn Glu Lys Asp Tyr Glu Leu Leu Cys Leu Asp Gly Thr Arg
   1               5                   10                  15
   Lys
<210> 1010
   <211> 19
   <212> PRT
   <213> Homo sapiens
<400> 1010
   Asn Leu Arg Glu Gly Thr Cys Pro Glu Ala Pro Thr Asp Glu Cys Lys
   1               5                   10                  15
   Pro Val Lys
<210> 1011
   <211> 7
   <212> PRT
   <213> Homo sapiens
<400> 1011
   Asn Pro Asp Pro Trp Ala Lys
   1               5
<210> 1012
   <211> 19
   <212> PRT
   <213> Homo sapiens
<400> 1012
   Ser Ala Gly Trp Asn Ile Pro Ile Gly Leu Leu Tyr Cys Asp Leu Pro
   1               5                   10                  15
   Glu Pro Arg
<210> 1013
   <211> 24
   <212> PRT
   <213> Homo sapiens
<400> 1013
   Ser Ala Gly Trp Asn Ile Pro Ile Gly Leu Leu Tyr Cys Asp Leu Pro
   1               5                   10                  15
   Glu Pro Arg Lys Pro Leu Glu Lys
               20
<210> 1014
   <211> 11
   <212> PRT
   <213> Homo sapiens
<400> 1014
   Ser Ala Ser Asp Leu Thr Trp Asp Asn Leu Lys
   1               5                   10
<210> 1015
   <211> 13
   <212> PRT
   <213> Homo sapiens
<400> 1015
   Ser Ala Ser Asp Leu Thr Trp Asp Asn Leu Lys Gly Lys
   1               5                   10
<210> 1016
   <211> 14
   <212> PRT
   <213> Homo sapiens
<400> 1016
   Ser Ala Ser Asp Leu Thr Trp Asp Asn Leu Lys Gly Lys Lys
   1               5                   10
<210> 1017
   <211> 17
   <212> PRT
   <213> Homo sapiens
<400> 1017
   Ser Glu Thr Lys Asp Leu Leu Phe Arg Asp Asp Thr Val Cys Leu Ala
   1               5                   10                  15
   Lys
<210> 1018
   <211> 13
   <212> PRT
   <213> Homo sapiens
<400> 1018
   Ser Lys Glu Phe Gln Leu Phe Ser Ser Pro His Gly Lys
   1               5                   10
<210> 1019
   <211> 18
   <212> PRT
   <213> Homo sapiens
<400> 1019
   Ser Lys Glu Phe Gln Leu Phe Ser Ser Pro His Gly Lys Asp Leu Leu
   1 5 10 15
   Phe Lys
<210> 1020
   <211> 22
   <212> PRT
   <213> Homo sapiens
<400> 1020
   Ser Met Gly Gly Lys Glu Asp Leu Ile Trp Glu Leu Leu Asn Gln Ala
   1 5 10 15
   Gln Glu His Phe Gly Lys
   20
<210> 1021
   <211> 14
   <212> PRT
   <213> Homo sapiens
<400> 1021
   Ser Val Ile Pro Ser Asp Gly Pro Ser Val Ala Cys Val Lys
   1 5 10
<210> 1022
   <211> 11
   <212> PRT
   <213> Homo sapiens
<400> 1022
   Trp Cys Ala Val Ser Glu His Glu Ala Thr Lys
   1 5 10
<210> 1023
   <211> 14
   <212> PRT
   <213> Homo sapiens
<400> 1023
   Thr Ala Gly Trp Asn Ile Pro Met Gly Leu Leu Tyr Asn Lys
   1 5 10
<210> 1024
   <211> 8
   <212> PRT
   <213> Homo sapiens
<400> 1024
   Tyr Leu Gly Glu Glu Tyr Val Lys
   1 5
<210> 1025
   <211> 15
   <212> PRT
   <213> Homo sapiens
<400> 1025
   Tyr Leu Gly Glu Glu Tyr Val Lys Ala Val Gly Asn Leu Arg Lys
   1 5 10 15
<210> 1026
   <211> 339
   <212> PRT
   <213> Homo sapiens
<400> 1026
   Met Trp Gln Leu Trp Ala Ser Leu Cys Cys Leu Leu Val Leu Ala Asn
   1 5 10 15
   Ala Arg Ser Arg Pro Ser Phe His Pro Leu Ser Asp Glu Leu Val Asn
   20 25 30
   Tyr Val Asn Lys Arg Asn Thr Thr Trp Gln Ala Gly His Asn Phe Tyr
   35 40 45
   Asn Val Asp Met Ser Tyr Leu Lys Arg Leu Cys Gly Thr Phe Leu Gly
   50 55 60
   Gly Pro Lys Pro Pro Gln Arg Val Met Phe Thr Glu Asp Leu Lys Leu
   65 70 75 80
   Pro Ala Ser Phe Asp Ala Arg Glu Gln Trp Pro Gln Cys Pro Thr Ile
   85 90 95
   Lys Glu Ile Arg Asp Gln Gly Ser Cys Gly Ser Cys Trp Ala Phe Gly
               100                 105                 110
   Ala Val Glu Ala Ile Ser Asp Arg Ile Cys Ile His Thr Asn Ala His
           115                 120                 125
   Val Ser Val Glu Val Ser Ala Glu Asp Leu Leu Thr Cys Cys Gly Ser
       130                 135                 140
   Met Cys Gly Asp Gly Cys Asn Gly Gly Tyr Pro Ala Glu Ala Trp Asn
   145                 150                 155                 160
   Phe Trp Thr Arg Lys Gly Leu Val Ser Gly Gly Leu Tyr Glu Ser His
                   165                 170                 175
   Val Gly Cys Arg Pro Tyr Ser Ile Pro Pro Cys Glu His His Val Asn
               180                 185                 190
   Gly Ser Arg Pro Pro Cys Thr Gly Glu Gly Asp Thr Pro Lys Cys Ser
           195                 200                 205
   Lys Ile Cys Glu Pro Gly Tyr Ser Pro Thr Tyr Lys Gln Asp Lys His
       210                 215                 220
   Tyr Gly Tyr Asn Ser Tyr Ser Val Ser Asn Ser Glu Lys Asp Ile Met
   225                 230                 235                 240
   Ala Glu Ile Tyr Lys Asn Gly Pro Val Glu Gly Ala Phe Ser Val Tyr
                   245                 250                 255
   Ser Asp Phe Leu Leu Tyr Lys Ser Gly Val Tyr Gln His Val Thr Gly
               260                 265                 270
   Glu Met Met Gly Gly His Ala Ile Arg Ile Leu Gly Trp Gly Val Glu
           275                 280                 285
   Asn Gly Thr Pro Tyr Trp Leu Val Ala Asn Ser Trp Asn Thr Asp Trp
       290                 295                 300
   Gly Asp Asn Gly Phe Phe Lys Ile Leu Arg Gly Gln Asp His Cys Gly
   305                 310                 315                 320
   Ile Glu Ser Glu Val Val Ala Gly Ile Pro Arg Thr Asp Gln Tyr Trp
                   325                 330                 335
   Glu Lys Ile
<210> 1027
   <211> 1065
   <212> PRT
   <213> Homo sapiens
<400> 1027
   Met Lys Ile Leu Ile Leu Gly Ile Phe Leu Phe Leu Cys Ser Thr Pro
   1               5                   10                  15
   Ala Trp Ala Lys Glu Lys His Tyr Tyr Ile Gly Ile Ile Glu Thr Thr
               20                  25                  30
   Trp Asp Tyr Ala Ser Asp His Gly Glu Lys Lys Leu Ile Ser Val Asp
           35                  40                  45
   Thr Glu His Ser Asn Ile Tyr Leu Gln Asn Gly Pro Asp Arg Ile Gly
       50                  55                  60
   Arg Leu Tyr Lys Lys Ala Leu Tyr Leu Gln Tyr Thr Asp Glu Thr Phe
   65                  70                  75                  80
   Arg Thr Thr Ile Glu Lys Pro Val Trp Leu Gly Phe Leu Gly Pro Ile
                   85                  90                  95
   Ile Lys Ala Glu Thr Gly Asp Lys Val Tyr Val His Leu Lys Asn Leu
               100                 105                 110
   Ala Ser Arg Pro Tyr Thr Phe His Ser His Gly Ile Thr Tyr Tyr Lys
           115                 120                 125
   Glu His Glu Gly Ala Ile Tyr Pro Asp Asn Thr Thr Asp Phe Gln Arg
       130                 135                 140
   Ala Asp Asp Lys Val Tyr Pro Gly Glu Gln Tyr Thr Tyr Met Leu Leu
   145                 150                 155                 160
   Ala Thr Glu Glu Gln Ser Pro Gly Glu Gly Asp Gly Asn Cys Val Thr
                   165                 170                 175
   Arg Ile Tyr His Ser His Ile Asp Ala Pro Lys Asp Ile Ala Ser Gly
               180                 185                 190
   Leu Ile Gly Pro Leu Ile Ile Cys Lys Lys Asp Ser Leu Asp Lys Glu
           195                 200                 205
   Lys Glu Lys His Ile Asp Arg Glu Phe Val Val Met Phe Ser Val Val
   210 215 220
   Asp Glu Asn Phe Ser Trp Tyr Leu Glu Asp Asn Ile Lys Thr Tyr Cys
   225 230 235 240
   Ser Glu Pro Glu Lys Val Asp Lys Asp Asn Glu Asp Phe Gln Glu Ser
   245 250 255
   Asn Arg Met Tyr Ser Val Asn Gly Tyr Thr Phe Gly Ser Leu Pro Gly
   260 265 270
   Leu Ser Met Cys Ala Glu Asp Arg Val Lys Trp Tyr Leu Phe Gly Met
   275 280 285
   Gly Asn Glu Val Asp Val His Ala Ala Phe Phe His Gly Gln Ala Leu
   290 295 300
   Thr Asn Lys Asn Tyr Arg Ile Asp Thr Ile Asn Leu Phe Pro Ala Thr
   305 310 315 320
   Leu Phe Asp Ala Tyr Met Val Ala Gln Asn Pro Gly Glu Trp Met Leu
   325 330 335
   Ser Cys Gln Asn Leu Asn His Leu Lys Ala Gly Leu Gln Ala Phe Phe
   340 345 350
   Gln Val Gln Glu Cys Asn Lys Ser Ser Ser Lys Asp Asn Ile Arg Gly
   355 360 365
   Lys His Val Arg His Tyr Tyr Ile Ala Ala Glu Glu Ile Ile Trp Asn
   370 375 380
   Tyr Ala Pro Ser Gly Ile Asp Ile Phe Thr Lys Glu Asn Leu Thr Ala
   385 390 395 400
   Pro Gly Ser Asp Ser Ala Val Phe Phe Glu Gln Gly Thr Thr Arg Ile
   405 410 415
   Gly Gly Ser Tyr Lys Lys Leu Val Tyr Arg Glu Tyr Thr Asp Ala Ser
   420 425 430
   Phe Thr Asn Arg Lys Glu Arg Gly Pro Glu Glu Glu His Leu Gly Ile
   435 440 445
   Leu Gly Pro Val Ile Trp Ala Glu Val Gly Asp Thr Ile Arg Val Thr
   450 455 460
   Phe His Asn Lys Gly Ala Tyr Pro Leu Ser Ile Glu Pro Ile Gly Val
   465 470 475 480
   Arg Phe Asn Lys Asn Asn Glu Gly Thr Tyr Tyr Ser Pro Asn Tyr Asn
   485 490 495
   Pro Gln Ser Arg Ser Val Pro Pro Ser Ala Ser His Val Ala Pro Thr
   500 505 510
   Glu Thr Phe Thr Tyr Glu Trp Thr Val Pro Lys Glu Val Gly Pro Thr
   515 520 525
   Asn Ala Asp Pro Val Cys Leu Ala Lys Met Tyr Tyr Ser Ala Val Asp
   530 535 540
   Pro Thr Lys Asp Ile Phe Thr Gly Leu Ile Gly Pro Met Lys Ile Cys
   545 550 555 560
   Lys Lys Gly Ser Leu His Ala Asn Gly Arg Gln Lys Asp Val Asp Lys
   565 570 575
   Glu Phe Tyr Leu Phe Pro Thr Val Phe Asp Glu Asn Glu Ser Leu Leu
   580 585 590
   Leu Glu Asp Asn Ile Arg Met Phe Thr Thr Ala Pro Asp Gln Val Asp
   595 600 605
   Lys Glu Asp Glu Asp Phe Gln Glu Ser Asn Lys Met His Ser Met Asn
   610 615 620
   Gly Phe Met Tyr Gly Asn Gln Pro Gly Leu Thr Met Cys Lys Gly Asp
   625 630 635 640
   Ser Val Val Trp Tyr Leu Phe Ser Ala Gly Asn Glu Ala Asp Val His
   645 650 655
   Gly Ile Tyr Phe Ser Gly Asn Thr Tyr Leu Trp Arg Gly Glu Arg Arg
   660 665 670
   Asp Thr Ala Asn Leu Phe Pro Gln Thr Ser Leu Thr Leu His Met Trp
   675 680 685
   Pro Asp Thr Glu Gly Thr Phe Asn Val Glu Cys Leu Thr Thr Asp His
   690 695 700
   Tyr Thr Gly Gly Met Lys Gln Lys Tyr Thr Val Asn Gln Cys Arg Arg
   705 710 715 720
   Gln Ser Glu Asp Ser Thr Phe Tyr Leu Gly Glu Arg Thr Tyr Tyr Ile
                   725                 730                 735
   Ala Ala Val Glu Val Glu Trp Asp Tyr Ser Pro Gln Arg Glu Trp Glu
               740                 745                 750
   Lys Glu Leu His His Leu Gln Glu Gln Asn Val Ser Asn Ala Phe Leu
           755                 760                 765
   Asp Lys Gly Glu Phe Tyr Ile Gly Ser Lys Tyr Lys Lys Val Val Tyr
       770                 775                 780
   Arg Gln Tyr Thr Asp Ser Thr Phe Arg Val Pro Val Glu Arg Lys Ala
   785                 790                 795                 800
   Glu Glu Glu His Leu Gly Ile Leu Gly Pro Gln Leu His Ala Asp Val
                   805                 810                 815
   Gly Asp Lys Val Lys Ile Ile Phe Lys Asn Met Ala Thr Arg Pro Tyr
               820                 825                 830
   Ser Ile His Ala His Gly Val Gln Thr Glu Ser Ser Thr Val Thr Pro
           835                 840                 845
   Thr Leu Pro Gly Glu Thr Leu Thr Tyr Val Trp Lys Ile Pro Glu Arg
       850                 855                 860
   Ser Gly Ala Gly Thr Glu Asp Ser Ala Cys Ile Pro Trp Ala Tyr Tyr
   865                 870                 875                 880
   Ser Thr Val Asp Gln Val Lys Asp Leu Tyr Ser Gly Leu Ile Gly Pro
                   885                 890                 895
   Leu Ile Val Cys Arg Arg Pro Tyr Leu Lys Val Phe Asn Pro Arg Arg
               900                 905                 910
   Lys Leu Glu Phe Ala Leu Leu Phe Leu Val Phe Asp Glu Asn Glu Ser
           915                 920                 925
   Trp Tyr Leu Asp Asp Asn Ile Lys Thr Tyr Ser Asp His Pro Glu Lys
       930                 935                 940
   Val Asn Lys Asp Asp Glu Glu Phe Ile Glu Ser Asn Lys Met His Ala
   945                 950                 955                 960
   Ile Asn Gly Arg Met Phe Gly Asn Leu Gln Gly Leu Thr Met His Val
                   965                 970                 975
   Gly Asp Glu Val Asn Trp Tyr Leu Met Gly Met Gly Asn Glu Ile Asp
   980 985 990
   Leu His Thr Val His Phe His Gly His Ser Phe Gln Tyr Lys His Arg
   995 1000 1005
   Gly Val Tyr Ser Ser Asp Val Phe Asp Ile Phe Pro Gly Thr Tyr
   1010 1015 1020
   Gln Thr Leu Glu Met Phe Pro Arg Thr Pro Gly Ile Trp Leu Leu
   1025 1030 1035
   His Cys His Val Thr Asp His Ile His Ala Gly Met Glu Thr Thr
   1040 1045 1050
   Tyr Thr Val Leu Gln Asn Glu Asp Thr Lys Ser Gly
   1055 1060 1065
<210> 1028
   <211> 248
   <212> PRT
   <213> Homo sapiens
<400> 1028
<210> 1029
   <211> 1474
   <212> PRT
   <213> Homo sapiens
<400> 1029
   Met Gly Lys Asn Lys Leu Leu His Pro Ser Leu Val Leu Leu Leu Leu
   1 5 10 15
   Val Leu Leu Pro Thr Asp Ala Ser Val Ser Gly Lys Pro Gln Tyr Met
   20 25 30
   Val Leu Val Pro Ser Leu Leu His Thr Glu Thr Thr Glu Lys Gly Cys
   35 40 45
   Val Leu Leu Ser Tyr Leu Asn Glu Thr Val Thr Val Ser Ala Ser Leu
   50 55 60
   Glu Ser Val Arg Gly Asn Arg Ser Leu Phe Thr Asp Leu Glu Ala Glu
   65 70 75 80
   Asn Asp Val Leu His Cys Val Ala Phe Ala Val Pro Lys Ser Ser Ser
   85 90 95
   Asn Glu Glu Val Met Phe Leu Thr Val Gln Val Lys Gly Pro Thr Gln
               100                 105                 110
   Glu Phe Lys Lys Arg Thr Thr Val Met Val Lys Asn Glu Asp Ser Leu
           115                 120                 125
   Val Phe Val Gln Thr Asp Lys Ser Ile Tyr Lys Pro Gly Gln Thr Val
       130                 135                 140
   Lys Phe Arg Val Val Ser Met Asp Glu Asn Phe His Pro Leu Asn Glu
   145                 150                 155                 160
   Leu Ile Pro Leu Val Tyr Ile Gln Asp Pro Lys Gly Asn Arg Ile Ala
                   165                 170                 175
   Gln Trp Gln Ser Phe Gln Leu Glu Gly Gly Leu Lys Gln Phe Ser Phe
               180                 185                 190
   Pro Leu Ser Ser Glu Pro Phe Gln Gly Ser Tyr Lys Val Val Val Gln
           195                 200                 205
   Lys Lys Ser Gly Gly Arg Thr Glu His Pro Phe Thr Val Glu Glu Phe
       210                 215                 220
   Val Leu Pro Lys Phe Glu Val Gln Val Thr Val Pro Lys Ile Ile Thr
   225                 230                 235                 240
   Ile Leu Glu Glu Glu Met Asn Val Ser Val Cys Gly Leu Tyr Thr Tyr
                   245                 250                 255
   Gly Lys Pro Val Pro Gly His Val Thr Val Ser Ile Cys Arg Lys Tyr
               260                 265                 270
   Ser Asp Ala Ser Asp Cys His Gly Glu Asp Ser Gln Ala Phe Cys Glu
           275                 280                 285
   Lys Phe Ser Gly Gln Leu Asn Ser His Gly Cys Phe Tyr Gln Gln Val
       290                 295                 300
   Lys Thr Lys Val Phe Gln Leu Lys Arg Lys Glu Tyr Glu Met Lys Leu
   305                 310                 315                 320
   His Thr Glu Ala Gln Ile Gln Glu Glu Gly Thr Val Val Glu Leu Thr
                   325                 330                 335
   Gly Arg Gln Ser Ser Glu Ile Thr Arg Thr Ile Thr Lys Leu Ser Phe
   340 345 350
   Val Lys Val Asp Ser His Phe Arg Gln Gly Ile Pro Phe Phe Gly Gln
   355 360 365
   Val Arg Leu Val Asp Gly Lys Gly Val Pro Ile Pro Asn Lys Val Ile
   370 375 380
   Phe Ile Arg Gly Asn Glu Ala Asn Tyr Tyr Ser Asn Ala Thr Thr Asp
   385 390 395 400
   Glu His Gly Leu Val Gln Phe Ser Ile Asn Thr Thr Asn Val Met Gly
   405 410 415
   Thr Ser Leu Thr Val Arg Val Asn Tyr Lys Asp Arg Ser Pro Cys Tyr
   420 425 430
   Gly Tyr Gln Trp Val Ser Glu Glu His Glu Glu Ala His His Thr Ala
   435 440 445
   Tyr Leu Val Phe Ser Pro Ser Lys Ser Phe Val His Leu Glu Pro Met
   450 455 460
   Ser His Glu Leu Pro Cys Gly His Thr Gln Thr Val Gln Ala His Tyr
   465 470 475 480
   Ile Leu Asn Gly Gly Thr Leu Leu Gly Leu Lys Lys Leu Ser Phe Tyr
   485 490 495
   Tyr Leu Ile Met Ala Lys Gly Gly Ile Val Arg Thr Gly Thr His Gly
   500 505 510
   Leu Leu Val Lys Gln Glu Asp Met Lys Gly His Phe Ser Ile Ser Ile
   515 520 525
   Pro Val Lys Ser Asp Ile Ala Pro Val Ala Arg Leu Leu Ile Tyr Ala
   530 535 540
   Val Leu Pro Thr Gly Asp Val Ile Gly Asp Ser Ala Lys Tyr Asp Val
   545 550 555 560
   Glu Asn Cys Leu Ala Asn Lys Val Asp Leu Ser Phe Ser Pro Ser Gln
   565 570 575
   Ser Leu Pro Ala Ser His Ala His Leu Arg Val Thr Ala Ala Pro Gln
   580 585 590
   Ser Val Cys Ala Leu Arg Ala Val Asp Gln Ser Val Leu Leu Met Lys
   595 600 605
   Pro Asp Ala Glu Leu Ser Ala Ser Ser Val Tyr Asn Leu Leu Pro Glu
   610 615 620
   Lys Asp Leu Thr Gly Phe Pro Gly Pro Leu Asn Asp Gln Asp Asn Glu
   625 630 635 640
   Asp Cys Ile Asn Arg His Asn Val Tyr Ile Asn Gly Ile Thr Tyr Thr
   645 650 655
   Pro Val Ser Ser Thr Asn Glu Lys Asp Met Tyr Ser Phe Leu Glu Asp
   660 665 670
   Met Gly Leu Lys Ala Phe Thr Asn Ser Lys Ile Arg Lys Pro Lys Met
   675 680 685
   Cys Pro Gln Leu Gln Gln Tyr Glu Met His Gly Pro Glu Gly Leu Arg
   690 695 700
   Val Gly Phe Tyr Glu Ser Asp Val Met Gly Arg Gly His Ala Arg Leu
   705 710 715 720
   Val His Val Glu Glu Pro His Thr Glu Thr Val Arg Lys Tyr Phe Pro
   725 730 735
   Glu Thr Trp Ile Trp Asp Leu Val Val Val Asn Ser Ala Gly Val Ala
   740 745 750
   Glu Val Gly Val Thr Val Pro Asp Thr Ile Thr Glu Trp Lys Ala Gly
   755 760 765
   Ala Phe Cys Leu Ser Glu Asp Ala Gly Leu Gly Ile Ser Ser Thr Ala
   770 775 780
   Ser Leu Arg Ala Phe Gln Pro Phe Phe Val Glu Leu Thr Met Pro Tyr
   785 790 795 800
   Ser Val Ile Arg Gly Glu Ala Phe Thr Leu Lys Ala Thr Val Leu Asn
   805 810 815
   Tyr Leu Pro Lys Cys Ile Arg Val Ser Val Gln Leu Glu Ala Ser Pro
   820 825 830
   Ala Phe Leu Ala Val Pro Val Glu Lys Glu Gln Ala Pro His Cys Ile
   835 840 845
   Cys Ala Asn Gly Arg Gln Thr Val Ser Trp Ala Val Thr Pro Lys Ser
       850                 855                 860
   Leu Gly Asn Val Asn Phe Thr Val Ser Ala Glu Ala Leu Glu Ser Gln
   865                 870                 875                 880
   Glu Leu Cys Gly Thr Glu Val Pro Ser Val Pro Glu His Gly Arg Lys
                   885                 890                 895
   Asp Thr Val Ile Lys Pro Leu Leu Val Glu Pro Glu Gly Leu Glu Lys
               900                 905                 910
   Glu Thr Thr Phe Asn Ser Leu Leu Cys Pro Ser Gly Gly Glu Val Ser
           915                 920                 925
   Glu Glu Leu Ser Leu Lys Leu Pro Pro Asn Val Val Glu Glu Ser Ala
       930                 935                 940
   Arg Ala Ser Val Ser Val Leu Gly Asp Ile Leu Gly Ser Ala Met Gln
   945                 950                 955                 960
   Asn Thr Gln Asn Leu Leu Gln Met Pro Tyr Gly Cys Gly Glu Gln Asn
                   965                 970                 975
   Met Val Leu Phe Ala Pro Asn Ile Tyr Val Leu Asp Tyr Leu Asn Glu
               980                 985                 990
   Thr Gln Gln Leu Thr Pro Glu Ile  Lys Ser Lys Ala Ile  Gly Tyr Leu
           995                 1000                 1005
   Asn Thr  Gly Tyr Gln Arg Gln  Leu Asn Tyr Lys His  Tyr Asp Gly
       1010                 1015                 1020
   Ser Tyr  Ser Thr Phe Gly Glu  Arg Tyr Gly Arg Asn  Gln Gly Asn
       1025                 1030                 1035
   Thr Trp  Leu Thr Ala Phe Val  Leu Lys Thr Phe Ala  Gln Ala Arg
       1040                 1045                 1050
   Ala Tyr  Ile Phe Ile Asp Glu  Ala His Ile Thr Gln  Ala Leu Ile
       1055                 1060                 1065
   Trp Leu  Ser Gln Arg Gln Lys  Asp Asn Gly Cys Phe  Arg Ser Ser
       1070                 1075                 1080
   Gly Ser  Leu Leu Asn Asn Ala  Ile Lys Gly Gly Val  Glu Asp Glu
       1085                 1090                 1095
   Val Thr  Leu Ser Ala Tyr Ile  Thr Ile Ala Leu Leu  Glu Ile Pro
       1100                 1105                 1110
   Leu Thr  Val Thr His Pro Val  Val Arg Asn Ala Leu  Phe Cys Leu
       1115                 1120                 1125
   Glu Ser  Ala Trp Lys Thr Ala  Gln Glu Gly Asp His  Gly Ser His
       1130                 1135                 1140
   Val Tyr  Thr Lys Ala Leu Leu  Ala Tyr Ala Phe Ala  Leu Ala Gly
       1145                 1150                 1155
   Asn Gln  Asp Lys Arg Lys Glu  Val Leu Lys Ser Leu  Asn Glu Glu
       1160                 1165                 1170
   Ala Val  Lys Lys Asp Asn Ser  Val His Trp Glu Arg  Pro Gln Lys
       1175                 1180                 1185
   Pro Lys  Ala Pro Val Gly His  Phe Tyr Glu Pro Gln  Ala Pro Ser
       1190                 1195                 1200
   Ala Glu  Val Glu Met Thr Ser  Tyr Val Leu Leu Ala  Tyr Leu Thr
       1205                 1210                 1215
   Ala Gln  Pro Ala Pro Thr Ser  Glu Asp Leu Thr Ser  Ala Thr Asn
       1220                 1225                 1230
   Ile Val  Lys Trp Ile Thr Lys  Gln Gln Asn Ala Gln  Gly Gly Phe
       1235                 1240                 1245
   Ser Ser  Thr Gln Asp Thr Val  Val Ala Leu His Ala  Leu Ser Lys
       1250                 1255                 1260
   Tyr Gly  Ala Ala Thr Phe Thr  Arg Thr Gly Lys Ala  Ala Gln Val
       1265                 1270                 1275
   Thr Ile  Gln Ser Ser Gly Thr  Phe Ser Ser Lys Phe  Gln Val Asp
       1280                 1285                 1290
   Asn Asn  Asn Arg Leu Leu Leu  Gln Gln Val Ser Leu  Pro Glu Leu
       1295                 1300                 1305
   Pro Gly  Glu Tyr Ser Met Lys  Val Thr Gly Glu Gly  Cys Val Tyr
       1310                 1315                 1320
   Leu Gln  Thr Ser Leu Lys Tyr  Asn Ile Leu Pro Glu  Lys Glu Glu
   1325 1330 1335
   Phe Pro Phe Ala Leu Gly Val Gln Thr Leu Pro Gln Thr Cys Asp
   1340 1345 1350
   Glu Pro Lys Ala His Thr Ser Phe Gln Ile Ser Leu Ser Val Ser
   1355 1360 1365
   Tyr Thr Gly Ser Arg Ser Ala Ser Asn Met Ala Ile Val Asp Val
   1370 1375 1380
   Lys Met Val Ser Gly Phe Ile Pro Leu Lys Pro Thr Val Lys Met
   1385 1390 1395
   Leu Glu Arg Ser Asn His Val Ser Arg Thr Glu Val Ser Ser Asn
   1400 1405 1410
   His Val Leu Ile Tyr Leu Asp Lys Val Ser Asn Gln Thr Leu Ser
   1415 1420 1425
   Leu Phe Phe Thr Val Leu Gln Asp Val Pro Val Arg Asp Leu Lys
   1430 1435 1440
   Pro Ala Ile Val Lys Val Tyr Asp Tyr Tyr Glu Thr Asp Glu Phe
   1445 1450 1455
   Ala Ile Ala Glu Tyr Asn Ala Pro Cys Ser Lys Asp Leu Gly Asn
   1460 1465 1470
   Ala
<210> 1030
   <211> 147
   <212> PRT
   <213> Homo sapiens
<400> 1030
<210> 1031
   <211> 527
   <212> PRT
   <213> Homo sapiens
<400> 1031
<210> 1032
   <211> 426
   <212> PRT
   <213> Homo sapiens
<400> 1032
   Met Phe Trp Glu Val Ile Ser Asp Glu His Gly Ile Asp Pro Thr Gly
   1 5 10 15
   Thr Tyr His Gly Asp Ser Asp Leu Gln Leu Asp Arg Ile Ser Val Tyr
   20 25 30
   Tyr Asn Glu Ala Thr Gly Gly Lys Tyr Val Pro Arg Ala Ile Leu Val
   35 40 45
   Asp Leu Glu Pro Gly Thr Met Asp Ser Val Arg Ser Gly Pro Phe Gly
   50 55 60
   Gln Ile Phe Arg Pro Asp Asn Phe Val Phe Gly Gln Ser Gly Ala Gly
   65                  70                  75                  80
   Asn Asn Trp Ala Lys Gly His Tyr Thr Glu Gly Ala Glu Leu Val Asp
                   85                  90                  95
   Ser Val Leu Asp Val Val Arg Lys Glu Ala Glu Ser Cys Asp Cys Leu
               100                 105                 110
   Gln Gly Phe Gln Leu Thr His Ser Leu Gly Gly Gly Thr Gly Ser Gly
           115                 120                 125
   Met Gly Thr Leu Leu Ile Ser Lys Ile Arg Glu Glu Tyr Pro Asp Arg
       130                 135                 140
   Ile Met Asn Thr Phe Ser Val Val Pro Ser Pro Lys Val Ser Asp Thr
   145                 150                 155                 160
   Val Val Glu Pro Tyr Asn Ala Thr Leu Ser Val His Gln Leu Val Glu
                   165                 170                 175
   Asn Thr Asp Glu Thr Tyr Cys Ile Asp Asn Glu Ala Leu Tyr Asp Ile
               180                 185                 190
   Cys Phe Arg Thr Leu Lys Leu Thr Thr Pro Thr Tyr Gly Asp Leu Asn
           195                 200                 205
   His Leu Val Ser Ala Thr Met Ser Gly Val Thr Thr Cys Leu Arg Phe
       210                 215                 220
   Pro Gly Gln Leu Asn Ala Asp Leu Arg Lys Leu Ala Val Asn Met Val
   225                 230                 235                 240
   Pro Phe Pro Arg Leu His Phe Phe Met Pro Gly Phe Ala Pro Leu Thr
                   245                 250                 255
   Ser Arg Gly Ser Gln Gln Tyr Arg Ala Leu Thr Val Pro Glu Leu Thr
               260                 265                 270
   Gln Gln Val Phe Asp Ala Lys Asn Met Met Ala Ala Cys Asp Pro Arg
           275                 280                 285
   His Gly Arg Tyr Leu Thr Val Ala Ala Val Phe Arg Gly Arg Met Ser
       290                 295                 300
   Met Lys Glu Val Asp Glu Gln Met Leu Asn Val Gln Asn Lys Asn Ser
   305                 310                 315                 320
   Ser Tyr Phe Val Glu Trp Ile Pro Asn Asn Val Lys Thr Ala Val Cys
                   325                 330                 335
   Asp Ile Pro Pro Arg Gly Leu Lys Met Ala Val Thr Phe Ile Gly Asn
               340                 345                 350
   Ser Thr Ala Ile Gln Glu Leu Phe Lys Arg Ile Ser Glu Gln Phe Thr
           355                 360                 365
   Ala Met Phe Arg Arg Lys Ala Phe Leu His Trp Tyr Thr Gly Glu Gly
       370                 375                 380
   Met Asp Glu Met Glu Phe Thr Glu Ala Glu Ser Asn Met Asn Asp Leu
   385                 390                 395                 400
   Val Ser Glu Tyr Gln Gln Tyr Gln Asp Ala Thr Ala Glu Glu Glu Glu
                   405                 410                 415
   Asp Phe Gly Glu Glu Ala Glu Glu Glu Ala
               420                 425
<210> 1033
   <211> 117
   <212> PRT
   <213> Homo sapiens
<400> 1033
   Met Ala Trp Thr Val Leu Leu Leu Gly Leu Leu Ser His Cys Thr Gly
   1               5                   10                  15
   Ser Val Thr Ser Tyr Val Leu Thr Gln Pro Pro Ser Val Ser Val Ala
               20                  25                  30
   Pro Gly Gln Thr Ala Arg Ile Thr Cys Gly Gly Asn Asn Ile Gly Ser
           35                  40                  45
   Lys Ser Val His Trp Tyr Gln Gln Lys Pro Gly Gln Ala Pro Val Leu
       50                  55                  60
   Val Val Tyr Asp Asp Ser Asp Arg Pro Ser Gly Ile Pro Glu Arg Phe
   65                  70                  75                  80
   Ser Gly Ser Asn Ser Gly Asn Thr Ala Thr Leu Thr Ile Ser Arg Val
                   85                  90                  95
   Glu Ala Gly Asp Glu Ala Asp Tyr Tyr Cys Gln Val Trp Asp Ser Ser
               100                 105                 110
   Ser Asp His Pro Thr
   115
<210> 1034
   <211> 109
   <212> PRT
   <213> Homo sapiens
<400> 1034
<210> 1035
   <211> 129
   <212> PRT
   <213> Homo sapiens
<400> 1035
   Met Glu Thr Pro Ala Gln Leu Leu Phe Leu Leu Leu Leu Trp Leu Pro
   1 5 10 15
   Asp Thr Thr Gly Glu Ile Val Leu Thr Gln Ser Pro Gly Thr Leu Ser
   20 25 30
   Leu Ser Pro Gly Glu Arg Ala Thr Leu Ser Cys Arg Ala Ser Gln Ser
   35 40 45
   Val Ser Ser Ser Tyr Leu Ala Trp Tyr Gln Gln Lys Pro Gly Gln Ala
   50 55 60
   Pro Arg Leu Leu Ile Tyr Gly Ala Ser Ser Arg Ala Thr Gly Ile Pro
   65 70 75 80
   Asp Arg Phe Ser Gly Ser Gly Ser Gly Thr Asp Phe Thr Leu Thr Ile
   85 90 95
   Ser Arg Leu Glu Pro Glu Asp Phe Ala Val Tyr Tyr Cys Gln Gln Tyr
   100 105 110
   Gly Thr Ser Pro Arg Thr Phe Gly Gln Gly Thr Lys Val Glu Ile Lys
   115 120 125
   Arg
<210> 1036
   <211> 22
   <212> PRT
   <213> Homo sapiens
<400> 1036
   Ala Ser Gln Ser Val Ser Asn Ser Tyr Leu Ala Trp Tyr Gln Gln Lys
   1 5 10 15
   Pro Gly Gln Ala Pro Arg
   20
<210> 1037
   <211> 23
   <212> PRT
   <213> Homo sapiens
<400> 1037
   Ala Thr Gly Ile Pro Asp Arg Phe Ser Gly Ser Gly Ser Gly Thr Asp
   1 5 10 15
   Phe Thr Leu Thr Ile Ser Arg
   20
<210> 1038
   <211> 18
   <212> PRT
   <213> Homo sapiens
<400> 1038
   Glu Ile Val Leu Thr Gln Ser Pro Gly Thr Leu Ser Leu Ser Pro Gly
   1 5 10 15
   Glu Arg
<210> 1039
   <211> 24
   <212> PRT
   <213> Homo sapiens
<400> 1039
   Glu Ile Val Leu Thr Gln Ser Pro Gly Thr Leu Ser Leu Ser Pro Gly
   1               5                   10                  15
   Glu Arg Ala Thr Leu Ser Cys Arg
               20
<210> 1040
   <211> 16
   <212> PRT
   <213> Homo sapiens
<400> 1040
   Phe Ser Gly Ser Gly Ser Gly Thr Asp Phe Thr Leu Thr Ile Ser Arg
   1               5                   10                  15
<210> 1041
   <211> 9
   <212> PRT
   <213> Homo sapiens
<400> 1041
   Leu Leu Ile Tyr Gly Ala Ser Ser Arg
   1               5
<210> 1042
   <211> 16
   <212> PRT
   <213> Homo sapiens
<400> 1042
   Phe Ser Gly Ser Asn Ser Gly Asn Thr Ala Thr Leu Thr Ile Ser Arg
   1               5                   10                  15
<210> 1043
   <211> 30
   <212> PRT
   <213> Homo sapiens
<400> 1043
   Ser Val His Trp Tyr Gln Gln Lys Pro Gly Gln Ala Pro Val Leu Val
   1               5                   10                  15
   Val Tyr Asp Asp Ser Asp Arg Pro Ser Gly Ile Pro Glu Arg
               20                  25                  30
<210> 1044
   <211> 15
   <212> PRT
   <213> Homo sapiens
<400> 1044
   Ala Leu Thr Val Pro Glu Leu Thr Gln Gln Val Phe Asp Ala Lys
   1 5 10 15
<210> 1045
   <211> 19
   <212> PRT
   <213> Homo sapiens
<400> 1045
   Gly His Tyr Thr Glu Gly Ala Glu Leu Val Asp Ser Val Leu Asp Val
   1 5 10 15
   Val Arg Lys
<210> 1046
   <211> 14
   <212> PRT
   <213> Homo sapiens
<400> 1046
   Asn Ser Ser Tyr Phe Val Glu Trp Ile Pro Asn Asn Val Lys
   1 5 10
<210> 1047
   <211> 26
   <212> PRT
   <213> Homo sapiens
<400> 1047
   Ser Gly Pro Phe Gly Gln Ile Phe Arg Pro Asp Asn Phe Val Phe Gly
   1 5 10 15
   Gln Ser Gly Ala Gly Asn Asn Trp Ala Lys
   20 25
<210> 1048
   <211> 9
   <212> PRT
   <213> Homo sapiens
<400> 1048
   Tyr Leu Thr Val Ala Ala Val Phe Arg
   1 5
<210> 1049
   <211> 12
   <212> PRT
   <213> Homo sapiens
<400> 1049
   Asp Ile Ala Pro Thr Leu Thr Leu Tyr Val Gly Lys
   1 5 10
<210> 1050
   <211> 13
   <212> PRT
   <213> Homo sapiens
<400> 1050
   Asp Ile Ala Pro Thr Leu Thr Leu Tyr Val Gly Lys Lys
   1 5 10
<210> 1051
   <211> 6
   <212> PRT
   <213> Homo sapiens
<400> 1051
   Phe Thr Asp His Leu Lys
   1 5
<210> 1052
   <211> 10
   <212> PRT
   <213> Homo sapiens
<400> 1052
   His Tyr Glu Gly Ser Thr Val Pro Glu Lys
   1 5 10
<210> 1053
   <211> 15
   <212> PRT
   <213> Homo sapiens
<400> 1053
   Leu Arg Thr Glu Gly Asp Gly Val Tyr Thr Leu Asn Asn Glu Lys
   1 5 10 15
<210> 1054
   <211> 20
   <212> PRT
   <213> Homo sapiens
<400> 1054
   Leu Arg Thr Glu Gly Asp Gly Val Tyr Thr Leu Asn Asn Glu Lys Gln
   1 5 10 15
   Trp Ile Asn Lys
   20
<210> 1055
   <211> 7
   <212> PRT
   <213> Homo sapiens
<400> 1055
   Gln Leu Val Glu Ile Glu Lys
   1 5
<210> 1056
   <211> 13
   <212> PRT
   <213> Homo sapiens
<400> 1056
   Thr Glu Gly Asp Gly Val Tyr Thr Leu Asn Asn Glu Lys
   1 5 10
<210> 1057
   <211> 18
   <212> PRT
   <213> Homo sapiens
<400> 1057
   Thr Glu Gly Asp Gly Val Tyr Thr Leu Asn Asn Glu Lys Gln Trp Ile
   1 5 10 15
   Asn Lys
<210> 1058
   <211> 9
   <212> PRT
   <213> Homo sapiens
<400> 1058
   Val Gly Tyr Val Ser Gly Trp Gly Arg
   1 5
<210> 1059
   <211> 16
   <212> PRT
   <213> Homo sapiens
<400> 1059
   Val Val Leu His Pro Asn Tyr Ser Gln Val Asp Ile Gly Leu Ile Lys
   1               5                   10                  15
<210> 1060
   <211> 8
   <212> PRT
   <213> Homo sapiens
<400> 1060
   Tyr Gln Cys Lys Asn Tyr Tyr Lys
   1               5
<210> 1061
   <211> 10
   <212> PRT
   <213> Homo sapiens
<400> 1061
   Tyr Gln Cys Lys Asn Tyr Tyr Lys Leu Arg
   1               5                   10
<210> 1062
   <211> 14
   <212> PRT
   <213> Homo sapiens
<400> 1062
   Tyr Val Met Leu Pro Val Ala Asp Gln Asp Gln Cys Ile Arg
   1               5                   10
<210> 1063
   <211> 222
   <212> PRT
   <213> Homo sapiens
<400> 1063
   Met Ser Ala Leu Gly Ala Val Ile Ala Leu Leu Leu Trp Gly Gln Leu
   1               5                   10                  15
   Phe Ala Val Asp Ser Gly Asn Asp Val Thr Asp Ile Ala Asp Asp Gly
               20                  25                  30
   Cys Pro Lys Pro Pro Glu Ile Ala His Gly Tyr Val Glu His Ser Val
           35                  40                  45
   Arg Tyr Gln Cys Lys Asn Tyr Tyr Lys Leu Arg Thr Glu Gly Asp Gly
       50                  55                  60
   Val Tyr Thr Leu Asn Asn Glu Lys Gln Trp Ile Asn Lys Ala Val Gly
   65                  70                  75                  80
   Asp Lys Leu Pro Glu Cys Glu Ala Gly Ala Thr Leu Ile Asn Glu Gln
   85 90 95
   Trp Leu Leu Thr Thr Ala Lys Asn Leu Phe Leu Asn His Ser Glu Asn
   100 105 110
   Ala Thr Ala Lys Asp Ile Ala Pro Thr Leu Thr Leu Tyr Val Gly Lys
   115 120 125
   Lys Gln Leu Val Glu Ile Glu Lys Val Val Leu His Pro Asn Tyr Ser
   130 135 140
   Gln Val Asp Ile Gly Leu Ile Lys Leu Lys Gln Lys Val Ser Val Asn
   145 150 155 160
   Glu Arg Val Met Pro Ile Cys Leu Pro Ser Lys Asp Tyr Ala Glu Val
   165 170 175
   Gly Arg Val Gly Tyr Val Ser Gly Trp Gly Arg Asn Ala Asn Phe Lys
   180 185 190
   Phe Thr Asp His Leu Lys Tyr Val Met Leu Pro Val Ala Asp Gln Asp
   195 200 205
   Gln Cys Ile Arg His Tyr Glu Gly Ser Thr Val Pro Glu Lys
   210 215 220
<210> 1064
   <211> 31
   <212> PRT
   <213> Homo sapiens
<400> 1064
   Ala Val Gly Asp Lys Leu Pro Glu Cys Glu Ala Asp Asp Gly Cys Pro
   1 5 10 15
   Lys Pro Pro Glu Ile Ala His Gly Tyr Val Glu His Ser Val Arg
   20 25 30
<210> 1065
   <211> 17
   <212> PRT
   <213> Homo sapiens
<400> 1065
   Ala Val Gly Asp Lys Leu Pro Glu Cys Glu Ala Val Cys Gly Lys Pro
   1 5 10 15
   Lys
<210> 1066
   <211> 12
   <212> PRT
   <213> Homo sapiens
<400> 1066
   Asp Ile Ala Pro Thr Leu Thr Leu Tyr Val Gly Lys
   1               5                   10
<210> 1067
   <211> 13
   <212> PRT
   <213> Homo sapiens
<400> 1067
   Asp Ile Ala Pro Thr Leu Thr Leu Tyr Val Gly Lys Lys
   1               5                   10
<210> 1068
   <211> 6
   <212> PRT
   <213> Homo sapiens
<400> 1068
   Phe Thr Asp His Leu Lys
   1               5
<210> 1069
   <211> 8
   <212> PRT
   <213> Homo sapiens
<400> 1069
   Gly Ser Phe Pro Trp Gln Ala Lys
   1               5
<210> 1070
   <211> 10
   <212> PRT
   <213> Homo sapiens
<400> 1070
   His Tyr Glu Gly Ser Thr Val Pro Glu Lys
   1               5                   10
<210> 1071
   <211> 11
   <212> PRT
   <213> Homo sapiens
<400> 1071
   His Tyr Glu Gly Ser Thr Val Pro Glu Lys Lys
   1               5                   10
<210> 1072
   <211> 9
   <212> PRT
   <213> Homo sapiens
<400> 1072
   Ile Leu Gly Gly His Leu Asp Ala Lys
   1               5
<210> 1073
   <211> 17
   <212> PRT
   <213> Homo sapiens
<400> 1073
   Ile Leu Gly Gly His Leu Asp Ala Lys Gly Ser Phe Pro Trp Gln Ala
   1               5                   10                  15
   Lys
<210> 1074
   <211> 14
   <212> PRT
   <213> Homo sapiens
<400> 1074
   Leu Arg Thr Glu Gly Asp Gly Val Tyr Thr Leu Asn Asp Lys
   1               5                   10
<210> 1075
   <211> 15
   <212> PRT
   <213> Homo sapiens
<400> 1075
   Leu Arg Thr Glu Gly Asp Gly Val Tyr Thr Leu Asn Asp Lys Lys
   1               5                   10                  15
<210> 1076
   <211> 15
   <212> PRT
   <213> Homo sapiens
<400> 1076
   Leu Arg Thr Glu Gly Asp Gly Val Tyr Thr Leu Asn Asn Glu Lys
   1               5                   10                  15
<210> 1077
   <211> 20
   <212> PRT
   <213> Homo sapiens
<400> 1077
   Leu Arg Thr Glu Gly Asp Gly Val Tyr Thr Leu Asn Asn Glu Lys Gln
   1 5 10 15
   Trp Ile Asn Lys
   20
<210> 1078
   <211> 8
   <212> PRT
   <213> Homo sapiens
<400> 1078
   Asn Pro Ala Asn Pro Val Gln Arg
   1 5
<210> 1079
   <211> 7
   <212> PRT
   <213> Homo sapiens
<400> 1079
   Gln Leu Val Glu Ile Glu Lys
   1 5
<210> 1080
   <211> 12
   <212> PRT
   <213> Homo sapiens
<400> 1080
   Ser Cys Ala Val Ala Glu Tyr Gly Val Tyr Val Lys
   1 5 10
<210> 1081
   <211> 20
   <212> PRT
   <213> Homo sapiens
<400> 1081
   Ser Pro Val Gly Val Gln Pro Ile Leu Asn Glu His Thr Phe Cys Ala
   1 5 10 15
   Gly Met Ser Lys
   20
<210> 1082
   <211> 12
   <212> PRT
   <213> Homo sapiens
<400> 1082
   Thr Glu Gly Asp Gly Val Tyr Thr Leu Asn Asp Lys
   1 5 10
<210> 1083
   <211> 18
   <212> PRT
   <213> Homo sapiens
<400> 1083
   Thr Glu Gly Asp Gly Val Tyr Thr Leu Asn Asp Lys Lys Gln Trp Ile
   1 5 10 15
   Asn Lys
<210> 1084
   <211> 13
   <212> PRT
   <213> Homo sapiens
<400> 1084
   Thr Glu Gly Asp Gly Val Tyr Thr Leu Asn Asn Glu Lys
   1 5 10
<210> 1085
   <211> 18
   <212> PRT
   <213> Homo sapiens
<400> 1085
   Thr Glu Gly Asp Gly Val Tyr Thr Leu Asn Asn Glu Lys Gln Trp Ile
   1 5 10 15
   Asn Lys
<210> 1086
   <211> 9
   <212> PRT
   <213> Homo sapiens
<400> 1086
   Val Gly Tyr Val Ser Gly Trp Gly Arg
   1 5
<210> 1087
   <211> 17
   <212> PRT
   <213> Homo sapiens
<400> 1087
   Glu Ile Val His Leu Gln Ala Gly Gln Cys Gly Asn Gln Ile Gly Ala
   1 5 10 15
   Lys
<210> 1088
   <211> 18
   <212> PRT
   <213> Homo sapiens
<400> 1088
   Gly His Tyr Thr Glu Gly Ala Glu Leu Val Asp Ser Val Leu Asp Val
   1 5 10 15
   Val Arg
<210> 1089
   <211> 19
   <212> PRT
   <213> Homo sapiens
<400> 1089
   Gly His Tyr Thr Glu Gly Ala Glu Leu Val Asp Ser Val Leu Asp Val
   1 5 10 15
   Val Arg Lys
<210> 1090
   <211> 16
   <212> PRT
   <213> Homo sapiens
<400> 1090
   Ile Asn Val Tyr Tyr Asn Glu Ala Thr Gly Gly Lys Tyr Val Pro Arg
   1 5 10 15
<210> 1091
   <211> 14
   <212> PRT
   <213> Homo sapiens
<400> 1091
   Asn Ser Ser Tyr Phe Val Glu Trp Ile Pro Asn Asn Val Lys
   1 5 10
<210> 1092
   <211> 26
   <212> PRT
   <213> Homo sapiens
<400> 1092
   Ser Gly Pro Phe Gly Gln Ile Phe Arg Pro Asp Asn Phe Val Phe Gly
   1               5                   10                  15
   Gln Ser Gly Ala Gly Asn Asn Trp Ala Lys
               20                  25
<210> 1093
   <211> 9
   <212> PRT
   <213> Homo sapiens
<400> 1093
   Tyr Leu Thr Val Ala Ala Val Phe Arg
   1               5
<210> 1094
   <211> 445
   <212> PRT
   <213> Homo sapiens
<400> 1094
   Met Arg Glu Ile Val His Leu Gln Ala Gly Gln Cys Gly Asn Gln Ile
   1               5                   10                  15
   Gly Ala Lys Phe Trp Glu Val Ile Ser Asp Glu His Gly Ile Asp Pro
               20                  25                  30
   Thr Gly Thr Tyr His Gly Asp Ser Asp Leu Gln Leu Glu Arg Ile Asn
           35                  40                  45
   Val Tyr Tyr Asn Glu Ala Thr Gly Gly Lys Tyr Val Pro Arg Ala Val
       50                  55                  60
   Leu Val Asp Leu Glu Pro Gly Thr Met Asp Ser Val Arg Ser Gly Pro
   65                  70                  75                  80
   Phe Gly Gln Ile Phe Arg Pro Asp Asn Phe Val Phe Gly Gln Ser Gly
                   85                  90                  95
   Ala Gly Asn Asn Trp Ala Lys Gly His Tyr Thr Glu Gly Ala Glu Leu
               100                 105                 110
   Val Asp Ser Val Leu Asp Val Val Arg Lys Glu Ala Glu Ser Cys Asp
           115                 120                 125
   Cys Leu Gln Gly Phe Gln Leu Thr His Ser Leu Gly Gly Gly Thr Gly
       130                 135                 140
   Ser Gly Met Gly Thr Leu Leu Ile Ser Lys Ile Arg Glu Glu Tyr Pro
   145                 150                 155                 160
   Asp Arg Ile Met Asn Thr Phe Ser Val Val Pro Ser Pro Lys Val Ser
                   165                 170                 175
   Asp Thr Val Val Glu Pro Tyr Asn Ala Thr Leu Ser Val His Gln Leu
               180                 185                 190
   Val Glu Asn Thr Asp Glu Thr Tyr Cys Ile Asp Asn Glu Ala Leu Tyr
           195                 200                 205
   Asp Ile Cys Phe Arg Thr Leu Lys Leu Thr Thr Pro Thr Tyr Gly Asp
       210                 215                 220
   Leu Asn His Leu Val Ser Ala Thr Met Ser Gly Val Thr Thr Cys Leu
   225                 230                 235                 240
   Arg Phe Pro Gly Gln Leu Asn Ala Asp Leu Arg Lys Leu Ala Val Asn
                   245                 250                 255
   Met Val Pro Phe Pro Arg Leu His Phe Phe Met Pro Gly Phe Ala Pro
               260                 265                 270
   Leu Thr Ser Arg Gly Ser Gln Gln Tyr Arg Ala Leu Thr Val Pro Glu
           275                 280                 285
   Leu Thr Gln Gln Met Phe Asp Ala Lys Asn Met Met Ala Ala Cys Asp
       290                 295                 300
   Pro Arg His Gly Arg Tyr Leu Thr Val Ala Ala Val Phe Arg Gly Arg
   305                 310                 315                 320
   Met Ser Met Lys Glu Val Asp Glu Gln Met Leu Asn Val Gln Asn Lys
                   325                 330                 335
   Asn Ser Ser Tyr Phe Val Glu Trp Ile Pro Asn Asn Val Lys Thr Ala
   340 345 350
   Val Cys Asp Ile Pro Pro Arg Gly Leu Lys Met Ser Ala Thr Phe Ile
   355 360 365
   Gly Asn Ser Thr Ala Ile Gln Glu Leu Phe Lys Arg Ile Ser Glu Gln
   370 375 380
   Phe Thr Ala Met Phe Arg Arg Lys Ala Phe Leu His Trp Tyr Thr Gly
   385 390 395 400
   Glu Gly Met Asp Glu Met Glu Phe Thr Glu Ala Glu Ser Asn Met Asn
   405 410 415
   Asp Leu Val Ser Glu Tyr Gln Gln Tyr Gln Asp Ala Thr Ala Glu Glu
   420 425 430
   Glu Gly Glu Phe Glu Glu Glu Ala Glu Glu Glu Val Ala
   435 440 445
<210> 1095
   <211> 15
   <212> PRT
   <213> Homo sapiens
<400> 1095
   Asp Ala Ser Gly Ala Thr Phe Thr Trp Thr Pro Ser Ser Gly Lys
   1 5 10 15
<210> 1096
   <211> 24
   <212> PRT
   <213> Homo sapiens
<400> 1096
   Asp Ala Ser Gly Ala Thr Phe Thr Trp Thr Pro Ser Ser Gly Lys Ser
   1 5 10 15
   Ala Val Gln Gly Pro Pro Glu Arg
   20
<210> 1097
   <211> 15
   <212> PRT
   <213> Homo sapiens
<400> 1097
   Asp Val Leu Val Arg Trp Leu Gln Gly Ser Gln Glu Leu Pro Arg
   1 5 10 15
<210> 1098
   <211> 17
   <212> PRT
   <213> Homo sapiens
<400> 1098
   Asp Val Leu Val Arg Trp Leu Gln Gly Ser Gln Glu Leu Pro Arg Glu
   1               5                   10                  15
   Lys
<210> 1099
   <211> 9
   <212> PRT
   <213> Homo sapiens
<400> 1099
   Glu Lys Tyr Leu Thr Trp Ala Ser Arg
   1               5
<210> 1100
   <211> 26
   <212> PRT
   <213> Homo sapiens
<400> 1100
   Glu Lys Tyr Leu Thr Trp Ala Ser Arg Gln Glu Pro Ser Gln Gly Thr
   1               5                   10                  15
   Thr Thr Phe Ala Val Thr Ser Ile Leu Arg
               20                  25
<210> 1101
   <211> 20
   <212> PRT
   <213> Homo sapiens
<400> 1101
   Gly Asp Thr Phe Ser Cys Met Val Gly His Glu Ala Leu Pro Leu Ala
   1               5                   10                  15
   Phe Thr Gln Lys
               20
<210> 1102
   <211> 20
   <212> PRT
   <213> Homo sapiens
<400> 1102
   Gly Phe Ser Pro Lys Asp Val Leu Val Arg Trp Leu Gln Gly Ser Gln
   1 5 10 15
   Glu Leu Pro Arg
   20
<210> 1103
   <211> 25
   <212> PRT
   <213> Homo sapiens
<400> 1103
   His Tyr Thr Asn Pro Ser Gln Asp Val Thr Val Pro Cys Pro Val Pro
   1 5 10 15
   Pro Pro Pro Pro Cys Cys His Pro Arg
   20 25
<210> 1104
   <211> 21
   <212> PRT
   <213> Homo sapiens
<400> 1104
   Lys Gly Asp Thr Phe Ser Cys Met Val Gly His Glu Ala Leu Pro Leu
   1 5 10 15
   Ala Phe Thr Gln Lys
   20
<210> 1105
   <211> 30
   <212> PRT
   <213> Homo sapiens
<400> 1105
   Asn Phe Pro Pro Ser Gln Asp Ala Ser Gly Asp Leu Tyr Thr Thr Ser
   1 5 10 15
   Ser Gln Leu Thr Leu Pro Ala Thr Gln Cys Pro Asp Gly Lys
   20 25 30
<210> 1106
   <211> 17
   <212> PRT
   <213> Homo sapiens
<400> 1106
   Gln Glu Pro Ser Gln Gly Thr Thr Thr Phe Ala Val Thr Ser Ile Leu
   1 5 10 15
   Arg
<210> 1107
   <211> 9
   <212> PRT
   <213> Homo sapiens
<400> 1107
   Ser Ala Val Gln Gly Pro Pro Glu Arg
   1 5
<210> 1108
   <211> 32
   <212> PRT
   <213> Homo sapiens
<400> 1108
   Ser Gly Asn Thr Phe Arg Pro Glu Val His Leu Leu Pro Pro Pro Ser
   1 5 10 15
   Glu Glu Leu Ala Leu Asn Glu Leu Val Thr Leu Thr Cys Leu Ala Arg
   20 25 30
<210> 1109
   <211> 7
   <212> PRT
   <213> Homo sapiens
<400> 1109
   Ser Val Thr Cys His Val Lys
   1 5
<210> 1110
   <211> 10
   <212> PRT
   <213> Homo sapiens
<400> 1110
   Gly Phe Ser Pro Lys Asp Val Leu Val Arg
   1 5 10
<210> 1111
   <211> 13
   <212> PRT
   <213> Homo sapiens
<400> 1111
   Ala Gly Lys Gly Met His Gly Gly Val Pro Gly Gly Lys
   1 5 10
<210> 1112
   <211> 10
   <212> PRT
   <213> Homo sapiens
<400> 1112
   Gly Met His Gly Gly Val Pro Gly Gly Lys
   1 5 10
<210> 1113
   <211> 7
   <212> PRT
   <213> Homo sapiens
<400> 1113
   Gln Glu Leu Asn Pro Leu Lys
   1 5
<210> 1114
   <211> 10
   <212> PRT
   <213> Homo sapiens
<400> 1114
   Gln Phe Ile Glu Ser Glu Cys Ile Pro Arg
   1 5 10
<210> 1115
   <211> 85
   <212> PRT
   <213> Homo sapiens
<400> 1115
   Pro Ala Gln Glu Ala Gly Thr Ser Lys Pro Asn Glu Glu Ile Ser Gly
   1               5                   10                  15
   Pro Ala Glu Pro Ala Ser Pro Pro Glu Thr Thr Thr Thr Ala Gln Glu
               20                  25                  30
   Thr Ser Ala Ala Ala Val Gln Gly Thr Ala Lys Val Thr Ser Ser Arg
           35                  40                  45
   Gln Glu Leu Asn Pro Leu Lys Gln Ala Leu Ala Lys Ala Gly Lys Gly
       50                  55                  60
   Met His Gly Gly Val Pro Gly Gly Lys Gln Phe Ile Glu Ser Glu Cys
   65                  70                  75                  80
   Ile Pro Arg Trp Lys
   85
<210> 1116
   <211> 21
   <212> PRT
   <213> Homo sapiens
<400> 1116
   Val Phe Ser Asn Gly Ala Asp Leu Ser Gly Val Thr Glu Glu Ala Pro
   1               5                   10                  15
   Leu Lys Leu Ser Lys
               20
<210> 1117
   <211> 7
   <212> PRT
   <213> Homo sapiens
<400> 1117
   Ser Pro Leu Phe Met Gly Lys
   1               5
<210> 1118
   <211> 22
   <212> PRT
   <213> Homo sapiens
<400> 1118
   Gly Phe Tyr Pro Ser Asp Ile Ser Val Glu Trp Glu Ser Asn Gly Gln
   1               5                   10                  15
   Pro Glu Asn Asn Tyr Lys
               20
<210> 1119
   <211> 11
   <212> PRT
   <213> Homo sapiens
<400> 1119
   Asp Leu Ala Thr Val Tyr Val Asp Val Leu Lys
   1               5                   10
<210> 1120
   <211> 15
   <212> PRT
   <213> Homo sapiens
<400> 1120
   Ala Gly Val Glu Thr Thr Thr Pro Ser Lys Gln Ser Asn Asn Lys
   1               5                   10                  15
<210> 1121
   <211> 8
   <212> PRT
   <213> Homo sapiens
<400> 1121
   Ser Glu Gln Phe Ile Asn Leu Arg
   1               5
<210> 1122
   <211> 8
   <212> PRT
   <213> Homo sapiens
<400> 1122
   Leu Pro Ala Ser Phe Asp Ala Arg
   1               5
<210> 1123
   <211> 17
   <212> PRT
   <213> Homo sapiens
<400> 1123
   Thr Thr Ile Glu Lys Pro Val Trp Leu Gly Phe Leu Gly Pro Ile Ile
   1 5 10 15
   Lys
<210> 1124
   <211> 11
   <212> PRT
   <213> Homo sapiens
<400> 1124
   Asn Ser Lys Ile Glu Ile Ser Glu Leu Asn Arg
   1 5 10
<210> 1125
   <211> 16
   <212> PRT
   <213> Homo sapiens
<400> 1125
   Ile Pro Glu Pro Gly Cys Thr Lys Val Pro Glu Pro Gly Cys Thr Lys
   1 5 10 15
<210> 1126
   <211> 23
   <212> PRT
   <213> Homo sapiens
<400> 1126
   Glu Glu Pro Trp Val Asp Pro Asn Ser Pro Val Leu Leu Glu Asp Pro
   1 5 10 15
   Val Leu Cys Ala Leu Ala Lys
   20
<210> 1127
   <211> 11
   <212> PRT
   <213> Homo sapiens
<400> 1127
   Val Ala Phe Thr Gly Ser Thr Glu Val Gly Lys
   1 5 10
<210> 1128
   <211> 24
   <212> PRT
   <213> Homo sapiens
<400> 1128
   Asp Ala Ser Gly Val Thr Phe Thr Trp Thr Pro Ser Ser Gly Lys Ser
   1               5                   10                  15
   Ala Val Gln Gly Pro Pro Glu Arg
               20
<210> 1129
   <211> 732
   <212> PRT
   <213> Homo sapiens
<400> 1129
   Met Pro Glu Glu Thr Gln Thr Gln Asp Gln Pro Met Glu Glu Glu Glu
   1               5                   10                  15
   Val Glu Thr Phe Ala Phe Gln Ala Glu Ile Ala Gln Leu Met Ser Leu
               20                  25                  30
   Ile Ile Asn Thr Phe Tyr Ser Asn Lys Glu Ile Phe Leu Arg Glu Leu
           35                  40                  45
   Ile Ser Asn Ser Ser Asp Ala Leu Asp Lys Ile Arg Tyr Glu Ser Leu
       50                  55                  60
   Thr Asp Pro Ser Lys Leu Asp Ser Gly Lys Glu Leu His Ile Asn Leu
   65 70 75 80
   Ile Pro Asn Lys Gln Asp Arg Thr Leu Thr Ile Val Asp Thr Gly Ile
   85 90 95
   Gly Met Thr Lys Ala Asp Leu Ile Asn Asn Leu Gly Thr Ile Ala Lys
   100 105 110
   Ser Gly Thr Lys Ala Phe Met Glu Ala Leu Gln Ala Gly Ala Asp Ile
   115 120 125
   Ser Met Ile Gly Gln Phe Gly Val Gly Phe Tyr Ser Ala Tyr Leu Val
   130 135 140
   Ala Glu Lys Val Thr Val Ile Thr Lys His Asn Asp Asp Glu Gln Tyr
   145 150 155 160
   Ala Trp Glu Ser Ser Ala Gly Gly Ser Phe Thr Val Arg Thr Asp Thr
   165 170 175
   Gly Glu Pro Met Gly Arg Gly Thr Lys Val Ile Leu His Leu Lys Glu
   180 185 190
   Asp Gln Thr Glu Tyr Leu Glu Glu Arg Arg Ile Lys Glu Ile Val Lys
   195 200 205
   Lys His Ser Gln Phe Ile Gly Tyr Pro Ile Thr Leu Phe Val Glu Lys
   210 215 220
   Glu Arg Asp Lys Glu Val Ser Asp Asp Glu Ala Glu Glu Lys Glu Asp
   225 230 235 240
   Lys Glu Glu Glu Lys Glu Lys Glu Glu Lys Glu Ser Glu Asp Lys Pro
   245 250 255
   Glu Ile Glu Asp Val Gly Ser Asp Glu Glu Glu Glu Lys Lys Asp Gly
   260 265 270
   Asp Lys Lys Lys Lys Lys Lys Ile Lys Glu Lys Tyr Ile Asp Gln Glu
   275 280 285
   Glu Leu Asn Lys Thr Lys Pro Ile Trp Thr Arg Asn Pro Asp Asp Ile
   290 295 300
   Thr Asn Glu Glu Tyr Gly Glu Phe Tyr Lys Ser Leu Thr Asn Asp Trp
   305 310 315 320
   Glu Asp His Leu Ala Val Lys His Phe Ser Val Glu Gly Gln Leu Glu
   325 330 335
   Phe Arg Ala Leu Leu Phe Val Pro Arg Arg Ala Pro Phe Asp Leu Phe
   340 345 350
   Glu Asn Arg Lys Lys Lys Asn Asn Ile Lys Leu Tyr Val Arg Arg Val
   355 360 365
   Phe Ile Met Asp Asn Cys Glu Glu Leu Ile Pro Glu Tyr Leu Asn Phe
   370 375 380
   Ile Arg Gly Val Val Asp Ser Glu Asp Leu Pro Leu Asn Ile Ser Arg
   385 390 395 400
   Glu Met Leu Gln Gln Ser Lys Ile Leu Lys Val Ile Arg Lys Asn Leu
   405 410 415
   Val Lys Lys Cys Leu Glu Leu Phe Thr Glu Leu Ala Glu Asp Lys Glu
   420 425 430
   Asn Tyr Lys Lys Phe Tyr Glu Gln Phe Ser Lys Asn Ile Lys Leu Gly
   435 440 445
   Ile His Glu Asp Ser Gln Asn Arg Lys Lys Leu Ser Glu Leu Leu Arg
   450 455 460
   Tyr Tyr Thr Ser Ala Ser Gly Asp Glu Met Val Ser Leu Lys Asp Tyr
   465 470 475 480
   Cys Thr Arg Met Lys Glu Asn Gln Lys His Ile Tyr Tyr Ile Thr Gly
   485 490 495
   Glu Thr Lys Asp Gln Val Ala Asn Ser Ala Phe Val Glu Arg Leu Arg
   500 505 510
   Lys His Gly Leu Glu Val Ile Tyr Met Ile Glu Pro Ile Asp Glu Tyr
   515 520 525
   Cys Val Gln Gln Leu Lys Glu Phe Glu Gly Lys Thr Leu Val Ser Val
   530 535 540
   Thr Lys Glu Gly Leu Glu Leu Pro Glu Asp Glu Glu Glu Lys Lys Lys
   545 550 555 560
   Gln Glu Glu Lys Lys Thr Lys Phe Glu Asn Leu Cys Lys Ile Met Lys
   565 570 575
   Asp Ile Leu Glu Lys Lys Val Glu Lys Val Val Val Ser Asn Arg Leu
   580 585 590
   Val Thr Ser Pro Cys Cys Ile Val Thr Ser Thr Tyr Gly Trp Thr Ala
   595 600 605
   Asn Met Glu Arg Ile Met Lys Ala Gln Ala Leu Arg Asp Asn Ser Thr
   610 615 620
   Met Gly Tyr Met Ala Ala Lys Lys His Leu Glu Ile Asn Pro Asp His
   625 630 635 640
   Ser Ile Ile Glu Thr Leu Arg Gln Lys Ala Glu Ala Asp Lys Asn Asp
   645 650 655
   Lys Ser Val Lys Asp Leu Val Ile Leu Leu Tyr Glu Thr Ala Leu Leu
   660 665 670
   Ser Ser Gly Phe Ser Leu Glu Asp Pro Gln Thr His Ala Asn Arg Ile
   675 680 685
   Tyr Arg Met Ile Lys Leu Gly Leu Gly Ile Asp Glu Asp Asp Pro Thr
   690 695 700
   Ala Asp Asp Thr Ser Ala Ala Val Thr Glu Glu Met Pro Pro Leu Glu
   705 710 715 720
   Gly Asp Asp Asp Thr Ser Arg Met Glu Glu Val Asp
   725 730
<210> 1130
   <211> 724
   <212> PRT
   <213> Homo sapiens
<400> 1130
   Met Pro Glu Glu Val His His Gly Glu Glu Glu Val Glu Thr Phe Ala
   1 5 10 15
   Phe Gln Ala Glu Ile Ala Gln Leu Met Ser Leu Ile Ile Asn Thr Phe
   20 25 30
   Tyr Ser Asn Lys Glu Ile Phe Leu Arg Glu Leu Ile Ser Asn Ala Ser
   35 40 45
   Asp Ala Leu Asp Lys Ile Arg Tyr Glu Ser Leu Thr Asp Pro Ser Lys
   50 55 60
   Leu Asp Ser Gly Lys Glu Leu Lys Ile Asp Ile Ile Pro Asn Pro Gln
   65                  70                  75                  80
   Glu Arg Thr Leu Thr Leu Val Asp Thr Gly Ile Gly Met Thr Lys Ala
                   85                  90                  95
   Asp Leu Ile Asn Asn Leu Gly Thr Ile Ala Lys Ser Gly Thr Lys Ala
               100                 105                 110
   Phe Met Glu Ala Leu Gln Ala Gly Ala Asp Ile Ser Met Ile Gly Gln
           115                 120                 125
   Phe Gly Val Gly Phe Tyr Ser Ala Tyr Leu Val Ala Glu Lys Val Val
       130                 135                 140
   Val Ile Thr Lys His Asn Asp Asp Glu Gln Tyr Ala Trp Glu Ser Ser
   145                 150                 155                 160
   Ala Gly Gly Ser Phe Thr Val Arg Ala Asp His Gly Glu Pro Ile Gly
                   165                 170                 175
   Arg Gly Thr Lys Val Ile Leu His Leu Lys Glu Asp Gln Thr Glu Tyr
               180                 185                 190
   Leu Glu Glu Arg Arg Val Lys Glu Val Val Lys Lys His Ser Gln Phe
           195                 200                 205
   Ile Gly Tyr Pro Ile Thr Leu Tyr Leu Glu Lys Glu Arg Glu Lys Glu
       210                 215                 220
   Ile Ser Asp Asp Glu Ala Glu Glu Glu Lys Gly Glu Lys Glu Glu Glu
   225                 230                 235                 240
   Asp Lys Asp Asp Glu Glu Lys Pro Lys Ile Glu Asp Val Gly Ser Asp
                   245                 250                 255
   Glu Glu Asp Asp Ser Gly Lys Asp Lys Lys Lys Lys Thr Lys Lys Ile
               260                 265                 270
   Lys Glu Lys Tyr Ile Asp Gln Glu Glu Leu Asn Lys Thr Lys Pro Ile
           275                 280                 285
   Trp Thr Arg Asn Pro Asp Asp Ile Thr Gln Glu Glu Tyr Gly Glu Phe
       290                 295                 300
   Tyr Lys Ser Leu Thr Asn Asp Trp Glu Asp His Leu Ala Val Lys His
   305 310 315 320
   Phe Ser Val Glu Gly Gln Leu Glu Phe Arg Ala Leu Leu Phe Ile Pro
   325 330 335
   Arg Arg Ala Pro Phe Asp Leu Phe Glu Asn Lys Lys Lys Lys Asn Asn
   340 345 350
   Ile Lys Leu Tyr Val Arg Arg Val Phe Ile Met Asp Ser Cys Asp Glu
   355 360 365
   Leu Ile Pro Glu Tyr Leu Asn Phe Ile Arg Gly Val Val Asp Ser Glu
   370 375 380
   Asp Leu Pro Leu Asn Ile Ser Arg Glu Met Leu Gln Gln Ser Lys Ile
   385 390 395 400
   Leu Lys Val Ile Arg Lys Asn Ile Val Lys Lys Cys Leu Glu Leu Phe
   405 410 415
   Ser Glu Leu Ala Glu Asp Lys Glu Asn Tyr Lys Lys Phe Tyr Glu Ala
   420 425 430
   Phe Ser Lys Asn Leu Lys Leu Gly Ile His Glu Asp Ser Thr Asn Arg
   435 440 445
   Arg Arg Leu Ser Glu Leu Leu Arg Tyr His Thr Ser Gln Ser Gly Asp
   450 455 460
   Glu Met Thr Ser Leu Ser Glu Tyr Val Ser Arg Met Lys Glu Thr Gln
   465 470 475 480
   Lys Ser Ile Tyr Tyr Ile Thr Gly Glu Ser Lys Glu Gln Val Ala Asn
   485 490 495
   Ser Ala Phe Val Glu Arg Val Arg Lys Arg Gly Phe Glu Val Val Tyr
   500 505 510
   Met Thr Glu Pro Ile Asp Glu Tyr Cys Val Gln Gln Leu Lys Glu Phe
   515 520 525
   Asp Gly Lys Ser Leu Val Ser Val Thr Lys Glu Gly Leu Glu Leu Pro
   530 535 540
   Glu Asp Glu Glu Glu Lys Lys Lys Met Glu Glu Ser Lys Ala Lys Phe
   545 550 555 560
   Glu Asn Leu Cys Lys Leu Met Lys Glu Ile Leu Asp Lys Lys Val Glu
   565 570 575
   Lys Val Thr Ile Ser Asn Arg Leu Val Ser Ser Pro Cys Cys Ile Val
   580 585 590
   Thr Ser Thr Tyr Gly Trp Thr Ala Asn Met Glu Arg Ile Met Lys Ala
   595 600 605
   Gln Ala Leu Arg Asp Asn Ser Thr Met Gly Tyr Met Met Ala Lys Lys
   610 615 620
   His Leu Glu Ile Asn Pro Asp His Pro Ile Val Glu Thr Leu Arg Gln
   625 630 635 640
   Lys Ala Glu Ala Asp Lys Asn Asp Lys Ala Val Lys Asp Leu Val Val
   645 650 655
   Leu Leu Phe Glu Thr Ala Leu Leu Ser Ser Gly Phe Ser Leu Glu Asp
   660 665 670
   Pro Gln Thr His Ser Asn Arg Ile Tyr Arg Met Ile Lys Leu Gly Leu
   675 680 685
   Gly Ile Asp Glu Asp Glu Val Ala Ala Glu Glu Pro Asn Ala Ala Val
   690 695 700
   Pro Asp Glu Ile Pro Pro Leu Glu Gly Asp Glu Asp Ala Ser Arg Met
   705 710 715 720
   Glu Glu Val Asp
<210> 1131
   <211> 9
   <212> PRT
   <213> Homo sapiens
<400> 1131
   Ala Pro Phe Asp Leu Phe Glu Asn Arg
   1 5
<210> 1132
   <211> 17
   <212> PRT
   <213> Homo sapiens
<400> 1132
   Cys Leu Glu Leu Phe Thr Glu Leu Ala Glu Asp Lys Glu Asn Tyr Lys
   1 5 10 15
   Lys
<210> 1133
   <211> 27
   <212> PRT
   <213> Homo sapiens
<400> 1133
   Asp Leu Val Ile Leu Leu Tyr Glu Thr Ala Leu Leu Ser Ser Gly Phe
   1 5 10 15
   Ser Leu Glu Asp Pro Gln Thr His Ala Asn Arg
   20 25
<210> 1134
   <211> 11
   <212> PRT
   <213> Homo sapiens
<400> 1134
   Asp Gln Val Ala Asn Ser Ala Phe Val Glu Arg
   1 5 10
<210> 1135
   <211> 12
   <212> PRT
   <213> Homo sapiens
<400> 1135
   Glu Leu Ile Ser Asn Ser Ser Asp Ala Leu Asp Lys
   1 5 10
<210> 1136
   <211> 14
   <212> PRT
   <213> Homo sapiens
<400> 1136
   Gly Val Val Asp Ser Glu Asp Leu Pro Leu Asn Ile Ser Arg
   1 5 10
<210> 1137
   <211> 10
   <212> PRT
   <213> Homo sapiens
<400> 1137
   His Ile Tyr Tyr Ile Thr Gly Glu Thr Lys
   1 5 10
<210> 1138
   <211> 15
   <212> PRT
   <213> Homo sapiens
<400> 1138
   His Leu Glu Ile Asn Pro Asp His Ser Ile Ile Glu Thr Leu Arg
   1 5 10 15
<210> 1139
   <211> 15
   <212> PRT
   <213> Homo sapiens
<400> 1139
   His Ser Gln Phe Ile Gly Tyr Pro Ile Thr Leu Phe Val Glu Lys
   1 5 10 15
<210> 1140
   <211> 10
   <212> PRT
   <213> Homo sapiens
<400> 1140
   Leu Gly Ile His Glu Asp Ser Gln Asn Arg
   1 5 10
<210> 1141
   <211> 15
   <212> PRT
   <213> Homo sapiens
<400> 1141
   Asn Pro Asp Asp Ile Thr Asn Glu Glu Tyr Gly Glu Phe Tyr Lys
   1 5 10 15
<210> 1142
   <211> 13
   <212> PRT
   <213> Homo sapiens
<400> 1142
   Ser Leu Thr Asn Asp Trp Glu Asp His Leu Ala Val Lys
   1 5 10
<210> 1143
   <211> 16
   <212> PRT
   <213> Homo sapiens
<400> 1143
   Val Ile Leu His Leu Lys Glu Asp Gln Thr Glu Tyr Leu Glu Glu Arg
   1 5 10 15
<210> 1144
   <211> 9
   <212> PRT
   <213> Homo sapiens
<400> 1144
   Tyr Ile Asp Gln Glu Glu Leu Asn Lys
   1 5
<210> 1145
   <211> 11
   <212> PRT
   <213> Homo sapiens
<400> 1145
   Glu Gln Val Ala Asn Ser Ala Phe Val Glu Arg
   1 5 10
<210> 1146
   <211> 14
   <212> PRT
   <213> Homo sapiens
<400> 1146
   Gly Val Val Asp Ser Glu Asp Leu Pro Leu Asn Ile Ser Arg
   1 5 10
<210> 1147
   <211> 15
   <212> PRT
   <213> Homo sapiens
<400> 1147
   Asn Pro Asp Asp Ile Thr Gln Glu Glu Tyr Gly Glu Phe Tyr Lys
   1 5 10 15
<210> 1148
   <211> 10
   <212> PRT
   <213> Homo sapiens
<400> 1148
   Ser Ile Tyr Tyr Ile Thr Gly Glu Ser Lys
   1 5 10
<210> 1149
   <211> 13
   <212> PRT
   <213> Homo sapiens
<400> 1149
   Ser Leu Thr Asn Asp Trp Glu Asp His Leu Ala Val Lys
   1 5 10
<210> 1150
   <211> 16
   <212> PRT
   <213> Homo sapiens
<400> 1150
   Val Ile Leu His Leu Lys Glu Asp Gln Thr Glu Tyr Leu Glu Glu Arg
   1 5 10 15
<210> 1151
   <211> 9
   <212> PRT
   <213> Homo sapiens
<400> 1151
   Tyr Ile Asp Gln Glu Glu Leu Asn Lys
   1 5
<210> 1152
   <211> 17
   <212> PRT
   <213> Homo sapiens
<400> 1152
   Ala Asp Gly Ser Pro Val Lys Ala Gly Val Glu Thr Thr Lys Pro Ser
   1 5 10 15
   Lys
<210> 1153
   <211> 10
   <212> PRT
   <213> Homo sapiens
<400> 1153
   Ala Gly Val Glu Thr Thr Lys Pro Ser Lys
   1 5 10
<210> 1154
   <211> 19
   <212> PRT
   <213> Homo sapiens
<400> 1154
   Ala Asn Pro Thr Val Thr Leu Phe Pro Pro Ser Ser Glu Glu Leu Gln
   1 5 10 15
   Ala Asn Lys
<210> 1155
   <211> 20
   <212> PRT
   <213> Homo sapiens
<400> 1155
   Gln Ser Asn Asn Lys Tyr Ala Ala Ser Ser Tyr Leu Ser Leu Thr Pro
   1 5 10 15
   Glu Gln Trp Lys
   20
<210> 1156
   <211> 13
   <212> PRT
   <213> Homo sapiens
<400> 1156
   Arg Pro Ser Gly Val Pro Asp Arg Phe Ser Gly Ser Lys
   1 5 10
<210> 1157
   <211> 18
   <212> PRT
   <213> Homo sapiens
<400> 1157
   Ser His Arg Ser Tyr Ser Cys Gln Val Thr His Glu Gly Ser Thr Val
   1 5 10 15
   Glu Lys
<210> 1158
   <211> 15
   <212> PRT
   <213> Homo sapiens
<400> 1158
   Ser Tyr Ser Cys Gln Val Thr His Glu Gly Ser Thr Val Glu Lys
   1 5 10 15
<210> 1159
   <211> 23
   <212> PRT
   <213> Homo sapiens
<400> 1159
   Ser Tyr Ser Cys Gln Val Thr His Glu Gly Ser Thr Val Glu Lys Thr
   1 5 10 15
   Val Ala Pro Thr Glu Cys Ser
   20
<210> 1160
   <211> 8
   <212> PRT
   <213> Homo sapiens
<400> 1160
   Thr Val Ala Pro Thr Glu Cys Ser
   1 5
<210> 1161
   <211> 13
   <212> PRT
   <213> Homo sapiens
<400> 1161
   Ile Tyr Gly Asn Gln Asp Thr Ser Ser Gln Leu Lys Lys
   1 5 10
<210> 1162
   <211> 11
   <212> PRT
   <213> Homo sapiens
<400> 1162
   Glu Pro Gln Val Tyr Thr Leu Pro Pro Ser Arg
   1 5 10

## Description

### BACKGROUND

The present application encompasses proteins and peptide fragments of those proteins, which are produced by proteolytic digestion of the proteins, and which both proteins and peptide fragments are useful for diagnosing of cancer or for monitoring for the presence of cancer in an individual.

Screening mammograms typically have a sensitivity of 75% and specificity of around 98% resulting in a false positive rate of roughly 5% per mammogram (Brown, Houn, Sickles, & Kessler, 1995; Kolb, Lichy, & Newhouse, 2002; Luftner & Possinger, 2002). Breast tissue type, more specifically density, also greatly influences the performance of mammography. The degree of breast density is classified using the American College of Radiology Breast Imaging Reporting and Data System (BI-RADS). This system consists of four classifications 1-4; where category 1 is mostly fatty (<25% dense); category 2 is scattered fibroglandular densities (25-50% dense); category 3 is heterogeneously dense (51-75% dense) and category 4 is extremely dense (>75% dense) (Bigenwald, 2008; Klifa, 2010; Scheel, 2014).

For women with fatty breast tissue, mammography can be an effective screening tool, when patients are compliant with yearly screenings (Tabar, 2001; Pisano, 2005). However, as breast density increases, the effectiveness of mammography decreases leading to increased follow up imaging and, more importantly, missed cancer diagnosis. Mammographic sensitivity, for high-risk patients, has been shown to be as low as 31% for category 1, 27% for category 2, 20% for category 3, and 12.5% for category 4 (Bigenwald, 2008). Approximately fifty percent of the female population is in categories 3 and 4, which is considered dense breast tissue (Vachon, 2007). Currently, the best screening option for these women is MRI, which can be up to 10 times more expensive than mammography (Beignwald, 2008). Lack of good screening options is a serious problem as women with 75% or more dense tissue have four to six times greater risk of developing breast cancer than women with less dense tissue (Boyd, 2007).

Follow up imaging to evaluate false positives costs the US over 4 billion dollars with an additional 1.6 billion dollars spent for biopsies alone. In 2010 of the 1.6 million biopsies performed, as few as 16% (only 261,000) were found to have cancer (Grady, 2012). The answer to increasing the diagnostic parameters of imaging can be found in the pre- and post-image diagnostics that focus on genetic and proteomic information, more specifically, biomarkers (Armstrong, Handorf, Chen, & Bristol Demeter, 2013; Li, Zhang, Rosenzweig, Wang, & Chan, 2002).

Tissue and serum are commonly the most logical place for beginning biomarker research, however the large dynamic range of both mediums makes discovery quite difficult (Schiess, Wollscheid, & Aebersold, 2009). Han-Byoel Lee et al. and S S Cross et al. focus on the use of plasma samples. The answers may lie in less complex biological fluids, such as saliva and tears. The use of tears as diagnostic medium is not a novel application as the tear proteome has been extensively investigated previously (Böhm et al., 2012; 2011; Lebrecht, Boehm, Schmidt, Koelbl, & Grus, 2009a; Lebrecht et al., 2009b; Wu & Zhang, 2007). In this application a quantitative assay for the detection of a panel of tear-based biomarkers in response to cancer is disclosed. From this quantitative information, the framework for a Certified Laboratory Improvement Amendments (CLIA) protocol will be defined.

Böhm et al., Oncology Reports 28: 429-438, 2012, discloses an analysis of proteins in tear fluid from breast carcinoma patients and healthy women.

### SUMMARY

The claimed invention is defined by the appended claims. The invention provides a method of determining whether a subject has breast cancer. The method includes providing an ocular sample previously obtained from the subject; performing steps of detecting levels in the sample of at least three of the markers selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3), Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY). The subject has, or is likely to have, breast cancer if the levels of the at least three markers change as compared to the levels in a control sample from a subject lacking cancer, wherein the at least three markers comprise S100A8, S100A9 and LGBP, and wherein the levels of S100A8 are increased, the levels of S100A9 are increased, and the levels of LG3BP are decreased. The sample is an ocular sample, such as an isolated tear sample or ocular wash. An ocular sample indicates a tear sample encompassing secretions from the lacrimal gland and other tissues that connect with the lymphatic system.

### BRIEF DESCRIPTION OF THE DRAWINGS

Figure 1: ANOVA of spectral intensities for the following proteins identified in Experiment 1: (A) Ig Heavy Chain V-IV region HiL, (B) Ig Heavy Chain region V-III region BRO, (C) Ig Heavy Chain V-III region VH26, (D) Antileukoproteinase, (E) β2 Microglobulin, (F) Calmodulin like protein 5, (G) Lipocalin 1, (H) Cystatin B, (I) Galectin 3, (J) Zinc-α 2 glycoprotein.
Figure 2: ROC curves for cancer, benign, and control, from Experiment 1, using Ig Heavy Chain V-IV region HiL, Ig Heavy Chain region V-III region BRO, Ig Heavy Chain V-III region VH26, Antileukoproteinase, β2 Microglobulin, Calmodulin like protein 5, Lipocalin 1, Cystatin B, Galectin 3, Zinc-α 2 glycoprotein.
Figure 3: Mosaic plot of the distribution of breast density for 66 of the 75 samples tested in Experiment 1.

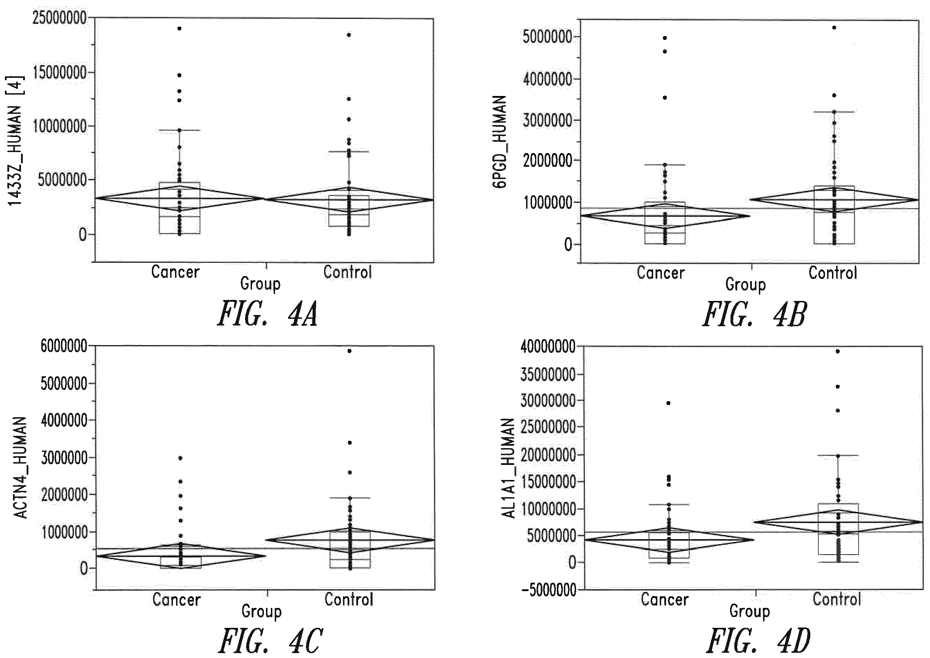

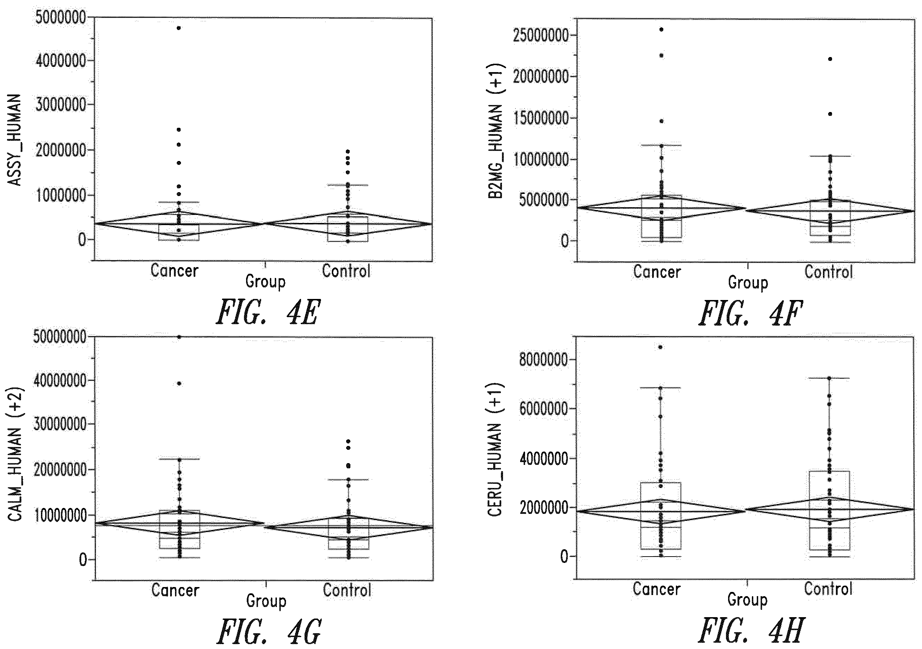

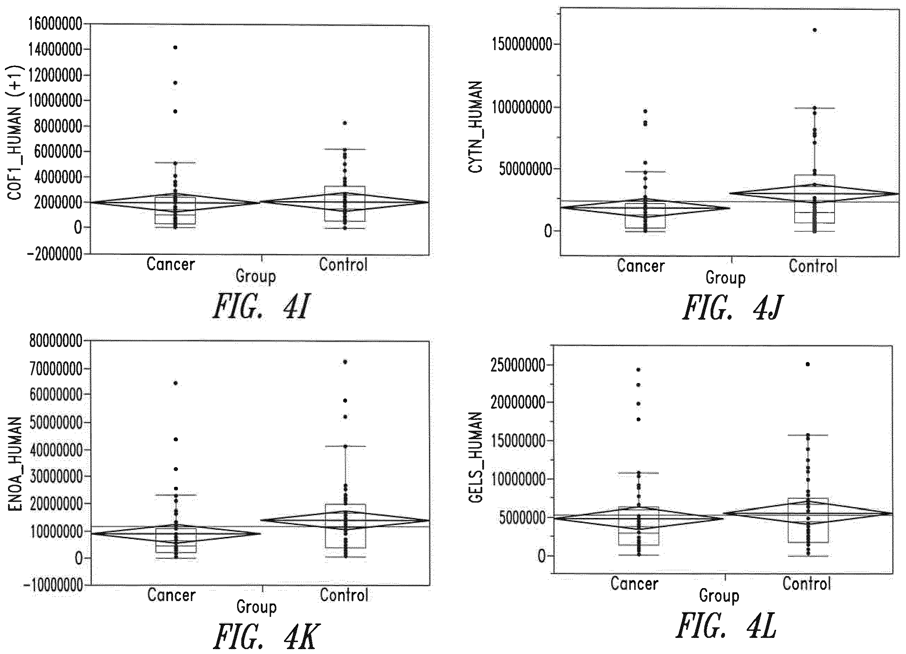

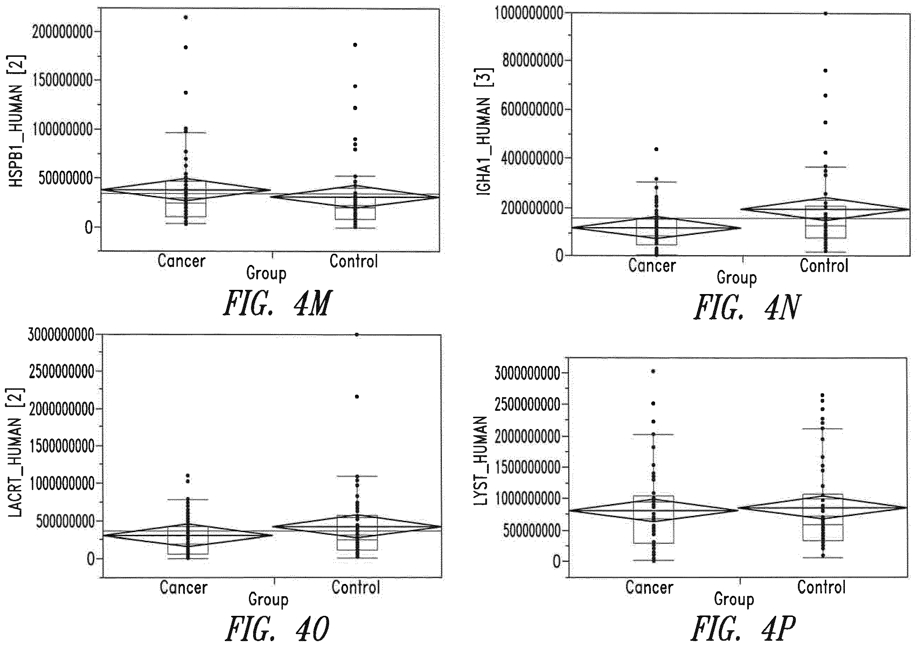

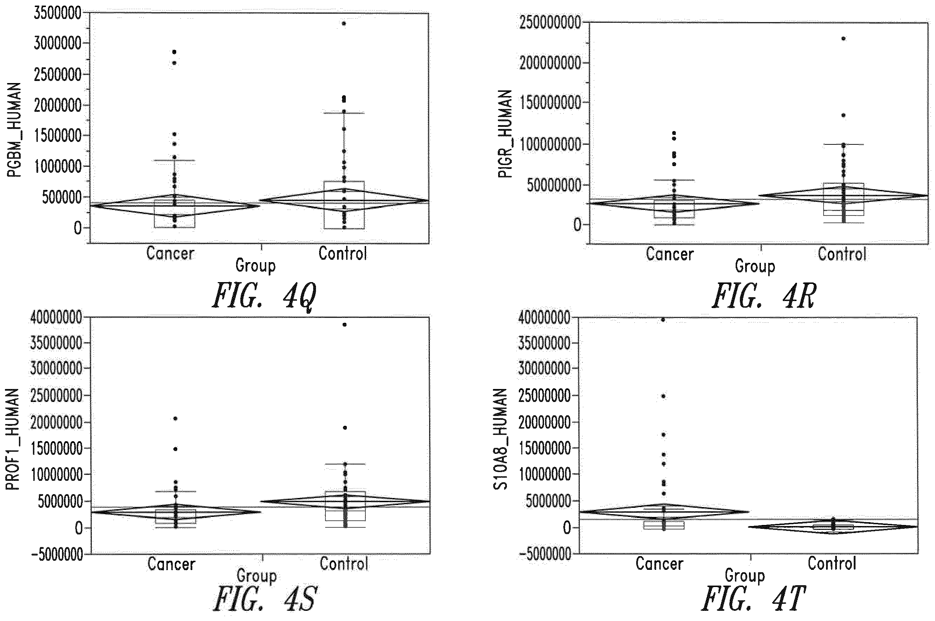

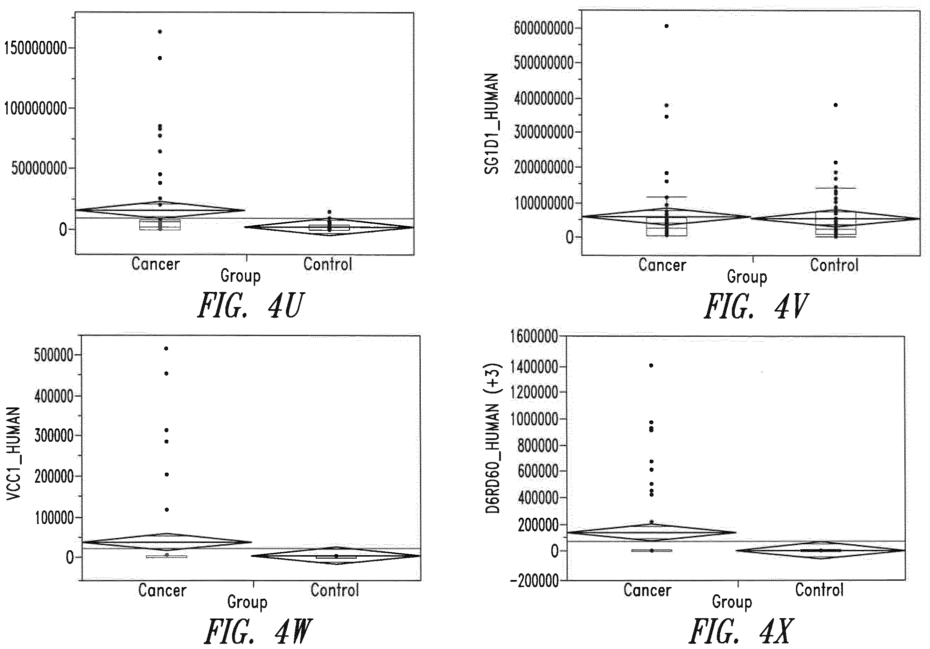

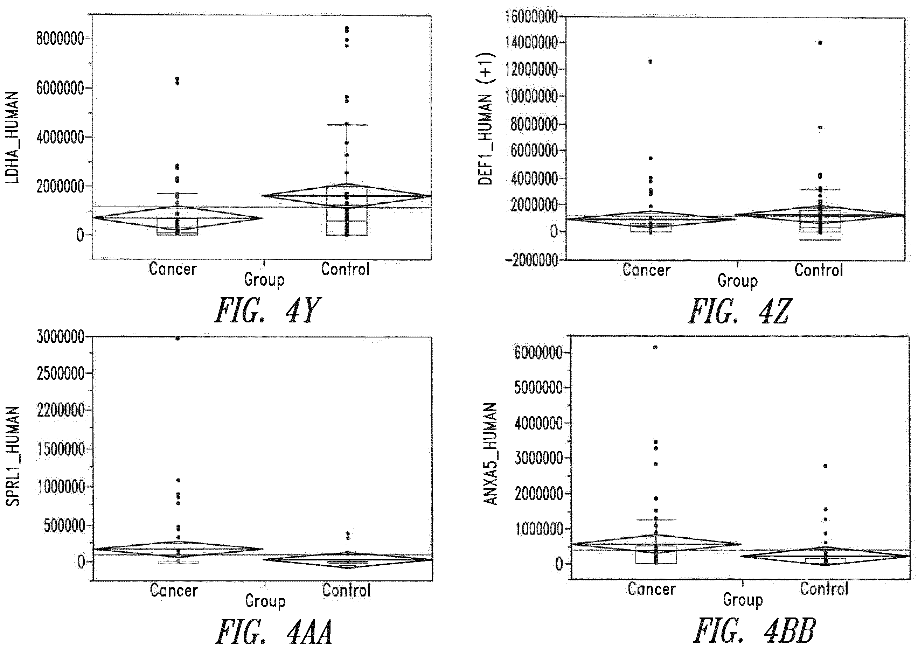

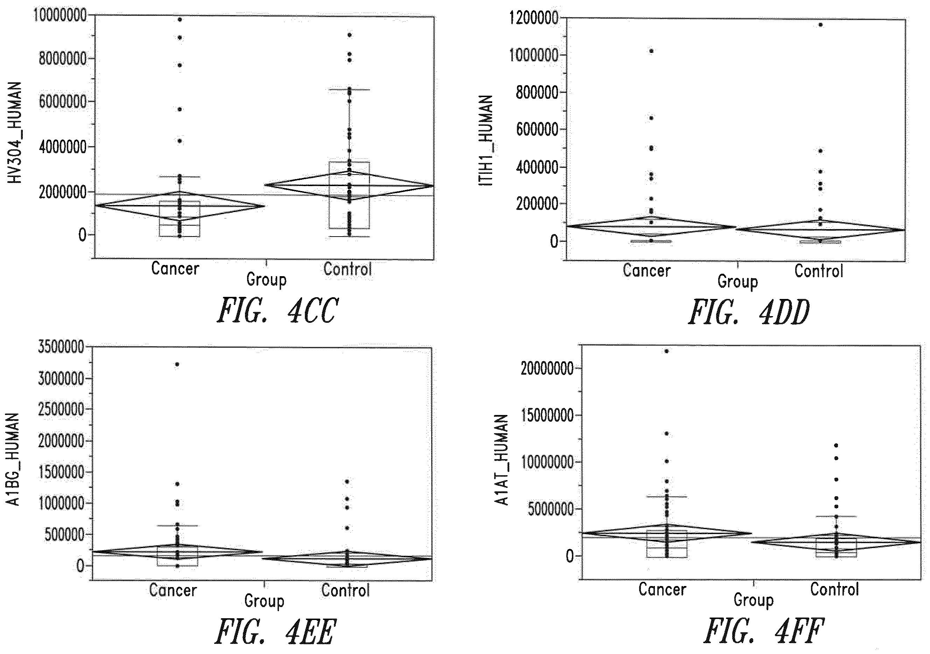

Figure 4: ANOVA of spectral intensities for proteins identified in Experiment 2. Proteins shown are: (A) 14-3-3 protein sigma (B) 6-phosphogluconate dehydrogenase, decarboxylating (C) Alpha-actinin-4 (D) Retinal Dehydrogenase (E) Argininosuccinate synthase (F) Beta-2-microglobulin (G) Calmodulin (H) Ceruloplasmin (I) Cofilin (J) Cystatin-SN (K) Enolase 1 (L) Gelsolin (M) Heat shock protein beta-1 (N) Ig alpha-1 chain C region (O) Lacritin (P) Lysozyme (Q) Basement membrane-specific heparin sulfate proteoglycan core protein (R) Polymeric immunoglobulin receptor (S) Profilin 1 (T) S100A8 (U) S100A9 (V) Secretoglobin family 1D member 1 (W) VEGF coregulated chemokine 1 (X) Histidine triad nucleotide-binding protein 1 (Y) Definsin 1 (Z) L-lactate dehydrogenase A chain (AA) SPARC-like protein 1 (BB) Annexin 5 (CC) Ig heavy chain V-III region TIL (DD) Inter-alpha-trypsin inhibitor heavy chain 1 (EE) Alpha-1B-glycoprotien (FF) Alpha-1-antitrypsin.
Figure 5: ANOVA comparing expression levels of S100A9, as determined by ELISA, in breast cancer and control samples.
Figure 6: ANOVA comparing expression levels of LG3BP, as determined by ELISA, in breast cancer and control samples.
Figure 7: ROC curve for S100A9
Figure 8: ROC curve for LG3BP
Figure 9: ROC curve of S100A9 and LG3BP combined.
Figure 10: ANOVA for S100A9 expression based on breast tissue category.
Figure 11: ANOVA of LG3BP expression based on breast tissue category.

### DETAILED DESCRIPTION

The invention is defined by the appended claims. Provided in the disclosure herein are proteins and peptide fragments obtained by trypsin digestion from ocular samples. The produced polypeptides are selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3), Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1(PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY). The trypsin sequences and full-length amino acid sequences of the proteins identified as being down regulated in cancer samples are provided in Appendix I.

The protein and peptides are shown in the Examples to either increase or decrease in biological samples in response to the presence of breast cancer as compared to controls. These proteins and peptides are biomarkers and will be used to determine the disease state of a patient or other subject.

Subjects include humans, domesticated animals such as cats, dogs, cows, pigs, or other animals susceptible to cancer. A "patient" indicates a subject who is diagnosed with a disease, or with cancer, or is being tested for having cancer. Thus the terms subject and patient can be used interchangeably herein. The subjects can be suspected of having cancer. The subject can be suspected of having cancer, including breast cancer, acoustic neuroma, acute lymphoblastic leukemia, acute myelogenous leukemia, adrenal tumors, AIDS-associated cancers, basal cell carcinoma, benign blood disorders, bladder cancer, bone cancer, brain tumors (metastatic and primary), breast cancer, cancer of unknown primary origin, cervical cancer, chronic lymphocytic leukemia, chronic myelogenous leukemia, colorectal cancer, esophageal cancer, gallbladder and bile duct cancers, gastrointestinal neuroendocrine tumors, GERD, Barrett's esophagus and achalasia, gestational trophoblastic disease, head and neck cancers, Kaposi sarcoma, kidney cancer, leukemias, liver cancer, liver metastases, low-grade glioma, lung cancer, lymphoma, male breast cancer, melanoma, Merkel cell carcinoma, mesothelioma, multiple myeloma, myelodysplastic syndrome, ovarian cancer, pancreatic cancer, pancreatic cysts, pituitary tumors, prostate cancer, pulmonary neuroendocrine tumors, rare blood disorder, skin cancer, soft tissue sarcoma, spine tumors, squamous cell carcinoma, stomach (gastric) cancer, testicular cancer (germ cell tumors), thymoma and other thymic tumors, tracheal diseases, uterine (endometrial) cancer, uterine sarcoma. The subjects can have an increased risk of developing breast cancer. For example, the subject can be at increased risk of cancer, or suspected of having cancer because of a positive mammography result, by detection of a lump in the breast, testing positive for a gene known to increase the risk of cancer such as BRCA, or already had a resection, biopsy, or other procedure to remove the cancer. The subject can be undergoing, or have previously undergone, treatment for cancer and methods and kits herein are used to monitor progression of treatment or alternatively to monitor for recurrence or spread of the cancer.

Also disclosed herein are methods and kits to collect ocular samples for use in determining the expression levels of the identified proteins or polypeptides in lacrimal secretions. The use of collection strips and tubes containing protease inhibitor or protein stabilizing agents is disclosed. Kits further contain buffers or reagents for the elution of breast cancer biomarkers from the collection strips that have been in contact with an eye to collect lacrimal secretions. The design of devices to collect proteins from the ocular cavity, as well as the packaging of this device with a container to house the collection device and elution buffers, is disclosed.

Methods disclosed herein encompass the use of these cancer biomarkers, singly or in multiples, in a CLIA based protocol utilizing a triple quadrupole LC-MS/MS platform, which will be carried out at a centralized laboratory testing facility. The ocular samples collected from individuals can be shipped to the testing facility in this embodiment. The identified proteins and their subsequent proteolytic fragments, as shown in Appendix I, are used for quantitative analysis of diagnostic peptides produced in the triple quad. A threshold value or a relative or actual value in terms of polypeptide concentration directly relating to the polypeptides selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3),, Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) can be defined or samples can be compared directly to non-cancerous controls. The quantitative information in report form can be provided to physicians to help in making decisions regarding the pathway of patient care. Physicians can base treatment decisions on these results and the final step can include administration of an appropriate anti-cancer therapeutic to the subject.

In an alternate embodiment, the polypeptides selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3), Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1, (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) can be detected by implementing binding agents, for example antibodies, peptoids, or coated surfaces, and reagents that accommodate a binding interaction specific to these proteins to produce a reaction that can be quantitated based on production of a detectable signal such as florescence, color change, or UV absorbance. Implementing these components in a cartridge with a partnering reading instrument, such as a point-of-care device that can be used at point of care is also provided. Binding agents for these proteins and polypeptides can also be used for detection in a lateral flow device. Thus, methods of detecting the level of protein expression in the samples using a binding partner such as an antibody can be used to detect the markers provided herein in an immunoassay.

The immunoassay typically includes contacting a test sample with an antibody or antigen that specifically binds to, or otherwise recognizes a biomarker, and detecting the presence of a complex of the antibody or antigen bound to the biomarker in the sample. The immunoassay procedure can be selected from a wide variety of immunoassay procedures known in the art involving recognition of antibody/antigen complexes, including enzyme-linked immunosorbent assays (ELISA), radioimmunoassay (RIA), and Western blots, and use of multiplex assays, including use of antibody arrays, wherein several desired antibodies are placed on a support, such as a glass bead or plate, and reacted or otherwise contacted with the test sample. Such assays are well-known to the skilled artisan.

The detection of the biomarkers described herein in a sample can be performed in a variety of ways. In one embodiment, the method provides the reverse-transcription of complementary DNAs from mRNAs obtained from the sample. Fluorescent dye-labeled complementary RNAs can be transcribed from complementary DNAs that are then hybridized to the arrays of oligonucleotide probes. The fluorescent color generated by hybridization can be read by machine, such as a SureScan microarray scanner (Agilent Technologies) and data obtained and processed using software, such as Agilent Feature Extraction Software (9.1). Such array-based methods include microarray analysis to develop a gene expression profile. As used herein, the term "gene expression profile" refers to the expression levels of mRNAs or proteins of a panel of genes in the subject. As used herein, the term "diagnostic panel" refers to a panel of genes, peptides or proteins with an expression level that can be relied on to diagnose or predict the status of the disease. Included in this panel are genes, peptides and proteins selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3), Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) as well as any combination thereof, as provided herein.

In other embodiments, complementary DNAs are reverse-transcribed from mRNAs obtained from the sample, amplified, and simultaneously quantified by real-time PCR, thereby enabling both detection and quantification (as absolute number of copies or relative amount when normalized to DNA input or additional normalizing genes) of a specific gene product in the complementary DNA sample as well as the original mRNA sample.

The methods herein described include detecting at least one biomarker. However, any number of biomarkers can be detected. It is preferred that at least two biomarkers are detected in the analysis. However, it is realized that three, four, or more, including all, of the biomarkers described herein can be utilized in the analysis. Thus, not only can one or more markers be detected, any number or combination of markers can be used in detection. In addition, other biomarkers not herein described can be combined with any of the presently disclosed biomarkers to aid in the diagnosis of cancer. Moreover, any combination of the above biomarkers can be detected.

The markers selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3),, Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) can increase or decrease at least 1.5 fold, 2 fold, 4 fold, 5 fold, 8 fold, 10 fold or more, relative to the level of the marker in the control sample. The control sample can be a sample from a subject that does not have cancer, a pooled sample from subjects that do not have cancer, or can be a control or baseline expression level known to be the average expression level of subjects without cancer.

Several terms are used throughout this disclosure and should be defined as commonly used in the art, or as specifically provided herein. As provided herein, mass spectrometry or MS refers to an analytical technique generating electrical or magnetic fields to determine mass-to-charge ratio of peptides and chemical compounds in order to identify or determine peptide sequence and chemical structures. LC-MS/MS spectrometry refers to an analytical technique combining the separation capabilities of high performance liquid chromatography (HPLC) with the mass analysis of mass spectrometry. Triple quadrupole mass spectrometry refers to a tandem mass spectrometer with three ionizing chambers (Q1, Q2, &Q3). This technique allows for target detection of molecules of interest. Ion pairs refers to a parent peptide detected in Q1 in its doubly or triply charged form and a resulting y or b ion as generated by Q2 and detected in Q3 of a triple quadrupole mass spectrometry instrument. SIS internal peptide refers to a synthesized isotopically-labeled peptide with the same sequence as the peptide to be monitored in Q1 and used as an internal standard for reference to quantify the peptide of interest. The -y ion refers to an ion generated from the c-terminal of a peptide fragment. The -b ion refers to an ion generated from the n-terminal of a peptide fragment. Quantitative Ion refers to the selected highest intensity y or b ion used to determine the quantity of its parent protein in a biological sample. Qualitative Ion refers to ion/ions chosen to ensure the integrity of the Qualitative Ion to selected protein of interest and labeled peptide to selected standards.

CLIA refers to Clinical Laboratory Improvements Amendments, which are federal regulatory standards that apply to all clinical laboratory testing preformed on humans in the United States, except clinical trials and basic research. *(CLIA related Federal Register and Code of Federal Regulation Announcements).* CLIA approved laboratory refers to a clinical lab that preforms laboratory testing on human specimens for diagnosis, prevention, or treatment of disease or impairment and is approved and monitored by a Food and Drug Administration (FDA) approved regulatory organization (CLIA Laws and Regulations, 2013). CLIA-waived test refers to a clinical laboratory test meeting specific criteria for risk, error, and complexity as defined by the FDA.

Point-of-care device refers to an instrument or cartridge available at the location of patient and physician care, which contains binding agents to a biomarker, or series of biomarkers of interest, and which can generate information on the presence, absence, and in some cases concentrations of detected biomarkers. Analyte refers to any measurable biomarker, which can be protein, peptide, macromolecule, metabolite, small molecule, or autoantibody. Biological fluid, as used herein, refers to tears, whole blood, serum, urine, and saliva. Biomarker refers to any substance (e.g. protein, peptide, metabolite, polynucleotide sequence) the concentration level of which changes in the body, for example increased or decreased, as a result of a disease or condition. Marker and biomarker can be used interchangeably used herein.

Lateral flow test refers to a device that measures the presence of an analyte in a biological fluid using porous paper of sintered polymer. ELISA refers to Enzyme-linked immunosorbent assay, which utilizes antibodies or antigens to detect the presence and concentration of an analyte of interest. Diagnostic panel refers to a group of molecules for example proteins or peptides, the combined concentrations of which are used to diagnose a disease state, for example cancer. A breast cancer marker refers to a molecule: for example protein, peptide, metabolite, polynucleotide sequence, the concentration level of which changes in the body: for example increased or decreased, as a result of the presence or absence of cancer.

In addition to being useful to diagnose cancer, and in particular breast cancer, in a subject, kits and methods provided herein can be used to monitor treatment or recurrence of cancer in an individual previously diagnosed with cancer. Thus if the levels of the markers selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3), Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) increases or decreases over time in the same subject after treatment, further chemotherapeutics targeting the cancer can be administered.

Methods and kits can also be used to monitor the effectiveness of a chemotherapeutic treatment. In this alternate embodiment, the levels of the biomarkers selected from the following and as listed in Table 1: Ig lambda chain V-IV region Hil (LV403), Ig heavy chain V-III BRO (HV305), Ig heavy chain V-III VH26 (HV303), β-2-microglobulin (B2MG), Lipocalin-1 (LCN1), Zinc-α-2-glycoprotein (ZA2G), Cystatin B (CYTB), Antileukoproteinase (SLP1), Galectin-3 (LEG3),, Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) can increase or decrease over time if the treatment regime is effective and either would not change or can increase or decrease over time if the treatment regime is not effective in a single subject, depending on which biomarker(s) are being examined in the subject.

Treating cancer includes reducing the number of cancer cells or the size of a tumor or mass in the subject, reducing progression of a cancer to a less aggressive form, reducing proliferation of cancer cells or reducing the speed of tumor growth, killing of cancer cells, reducing metastasis of cancer cells or reducing the likelihood of recurrence of a cancer in a subject. Treating a subject, as used herein, refers to any type of treatment that imparts a benefit to a subject afflicted with a disease or at risk of developing the disease, including improvement in the condition of the subject for example in one or more symptoms, delay in the progression of the disease, delay in the onset of symptoms, or delay in the progression of symptoms.

All percentages referring to amounts are by weight unless indicated otherwise.

### EXAMPLES

### Example 1:

### Participant Selection

Study participants were recruited from patients being seen at Breast Health and Surgery Centers AR, OK, TN and WA under Institutional Review Board approval. All patients were consented and provided a copy of the informed consent prior to sample collection or completing patient information sheet. Samples were collected by clinic staff (i.e. nurses and technicians) and/or Ascendant Dx staff. Inclusion/exclusion criteria used for patient selection were as follows:

### Inclusion criteria:

Individuals between the ages of 18-100 years of age,
Presenting for a routine check-up, or
Presenting for the evaluation of an abnormal exam or test, or
Presenting for the evaluation of palpable mass, or
Presenting with a mass pre or post biopsy as long as a portion of the mass is remaining, or
Have recently been diagnosed with breast cancer but have not undergone treatment of any kind.

### Exclusion criteria:

Individuals below the age of 18 or over 100
Experiencing a concurrent eye infection or trauma, or
Currently experiencing acute conjunctivitis, or
Have been diagnosed with breast cancer and have undergone treatment.

Control samples were collected from patients being seen for routine screening mammograms and did not receive a call back for additional procedures. Benign samples were collected at the time of biopsy and were included in the benign group once the pathology results were determined. Cancer samples were also collected at time of biopsy and included in the cancer group after pathology results were known, from patients having MRI's prior to surgery, and from patients undergoing sentinel node procedures prior to surgery.

Data collected from participants included the following: age, sex, race, currently taking birth control or on hormone replacement therapy, ophthalmological infections, current or recent chemotherapy treatments, family history of cancer, genetic testing (BRAC1/2) if available, cancer stage (I, II, III, IV), cancer type (Ductal Carcinoma In Situ, Invasive Ductal Carcinoma, Invasive Lobular Carcinoma, Lobular Carcinoma In Situ, and Unknown), hormone receptor status (ER+/-, PR+/-, HER2+/-), size of mass, tumor grade (I, II, III), breast density score (densities identified as category 1, 2, 3, or 4) and previous history of cancer.

### Example 2:

### Schirmer Strip Sample Collection Procedure

Institutional review board approval was obtained for the collection of tears using Schirmer strips (GuldenOpthalmics Elkins Park, PA). For collection, the rounded tip of the Schirmer strip was folded over at the 0 mm line forming a lip. The folded portion was placed in the lower eyelid of the participant and they were asked to close their eye and keep it in the closed position for a period of 5 minutes. After five minutes the strip was removed and placed in a sterile 1.5 mL pre-labeled snap top tube and placed at -20°C or -80°C depending on availability. Collection criteria stated that if the 35 mm mark was reached prior to the five minute time, the strip can be removed. Samples collected at participating clinics were retrieved by Ascendant Dx staff on a weekly basis and transferred on dry ice to Ascendant Dx's laboratory facility.

### Example 3:

### Schirmer Strip Sample Processing

Protein elution was carried out by first dicing the strips, using sterile tweezers and scissors, into clean sterile 1.5 mL snap top tube. 200 µL of 1X Phosphate-buffered saline (PBS) was added to the diced strip and the sample was incubated at 4°C with mild shaking for 3 hours. Following elution, the samples were spun briefly in a tabletop centrifuge to collect the strip fragments at the bottom of the tube, and the supernatant was transferred to a new clean 1.5 mL snap top tube. Total protein content was determined using standard bicinchoninic acid (BCA) assay, and the samples were stored at -80°C until further use.

Total protein content of each pool was determined using a BCA assay kit (Pierce) with a 1:20 (v/v) ratio of standard and unknown to working reagent and an incubation time of 30 min at 37°C. To ensure reliable total protein content calculation, a series of dilutions were made for each sample, for example 1:2, 1:4, 1:6, and all dilutions were plated in triplicate. A standard curve using diluted albumin (2 mg/ml, 1.5 mg/ml, 1m/ml, 0.75 mg/ml, 0.5 mg/ml, 0.25 mg/ml 0.125 mg/ml 0.025 mg/ml and Omg/ml) was generated and blank subtraction was applied to all standards and unknowns. The protein concentration for each unknown was calculated using a four-parameter fit of the standard curve. Concentrations were multiplied by the dilution factor and averaged to give an accurate total protein content calculation. Assays were considered valid if the coefficient of variation (%CV) was 15% or below.

### Example 4:

### Methods and Results for Label Free Quantitation by LC MS/MS

Experiment 1: In solution trypsin digestion followed by LC MS/MS was carried out on 25 breast cancer samples, 25 benign samples, and 25 control samples by the Proteomic Core at the University of Arkansas for Medical Sciences (UAMS). Solution digests were carried out on all 75 samples in 100 mM ammonium bicarbonate (Sigma-Aldrich), following reduction in 10 mM Tris[2-carboxyethyl]phosphine (Pierce) and alkylation in 50 mM iodoacetamide (Sigma-Aldrich), by addition of 100 ng porcine trypsin (Promega) and incubation at 37°C for 12-16 hours. Peptide products were then acidified in 0.1% formic acid (Fluka). Tryptic peptides were separated by reverse phase Jupiter Proteo resin (Phenomenex) on a 100 x 0.075 mm column using a nanoAcquity UPLC system (Waters). Peptides were eluted using an 80 min gradient from 97:3 to 35:65 buffer A:B ratio. [Buffer A = 0.1% formic acid, 0.05% acetonitrile; buffer B = 0.1% formic acid, 75% acetonitrile.] Eluted peptides were ionized by electrospray (1.8 kV) followed by MS/MS analysis using collision-induced dissociation on an LTQ Orbitrap Velos mass spectrometer (Thermo). MS data were acquired using the FTMS analyzer in profile mode at a resolution of 60,000 over a range of 375 to 1500 m/z. MS/MS data were acquired for the top 15 peaks from each MS scan using the ion trap analyzer in centroid mode and normal mass range with a normalized collision energy of 35.0. Proteins were identified from MS/MS spectra by database searching the Mascot search engine (Matrix Science) or MaxQuant quantitative proteomics software (Max Planck Institute). Mascot search results were compiled using Scaffold (Proteome Software).

The following criteria were set to select a group of proteins that can be indicative of altered breast physiology: 1) protein has a fold change of 1.5 or greater (in either positive or negative direction with respect to cancer). 2) fold change should be accompanied by p value of <0.05. 3) protein is present in 12 out of the 25 cancer samples. Using these criteria, the list of over 500 was reduced to the following proteins: Alpha-1-antitrypsin (A1AT), Antileukoproteinase (SLP1), Cofilin (COF1), Antithrombin-III (ANT3), Beta-2-microglobulin (B2MG), Protein-glutamine-gamma-glutamyltransferase (B4DIT7), Uncharacterized protein (B8ZZQ6), Calmodulin-like protein 5 (CALL5), Cystatin-B (CYTB), Neutrophil defensing-1 (DEF1), Destrin (DEST), Kaliocin-1 (E7ER44), Cluster of Rab GDP dissociation inhibitor beta (E7EU23), Elongation factor 1-alpha (EF1A1), Ezrin (EZRI), Heme-binding protein 2 (HEB2), Heat Shock cognate 71 (HSP7C), Heat shock protein beta 1 (HSPB1), Ig Heavy chain V-III (HV303), Cluster of Ig Heavy chain V-III region BRO (HV305), Lipocalin-1 (LCN1), Galectin-3 (LEG3), Ig lambda chain V-IV (LV403), Isoform 2 of Ig mu chain c region (P01871), Cluster of isoform 2 heat shock protein (P07900-2), Cluster of 14-3-3 protein zeta delta (P63104), Isoform 3 of Perilipin-3 (PLIN3), Proteasome activator complex subunit 1 (Q06323), UMP-CMP kinase (Q5T0D2), Protein S100-A4 (S10A4), S100-A8 (S10A8), Protein S100-A9 (S10A9), Protein S100-A11 (S10AB), Submaxillary gland androgen-regulated protein (SMR3B), Zinc-alpha-2-glycoprotien (ZA2G), Zymogen granule protein 16 homolog B (ZG16B) .

Experiment 2: In solution trypsin digestion followed by LC MS/MS was repeated using fifty samples in each group (breast cancer, benign, control). Identical methods as described for Experiment 1 were used for Experiment 2. The increase in samples size caused some of the trends observed in Experiment 1 to disappear, while making trends emerging in Experiment 1 more prominent in Experiment 2. From this data the list of 500 proteins described in Experiment 1 was further refined down to 103 proteins of interest. This list includes: Histidine triad nucleotide-binding protein 1 (D6RD60), S100A9 (S10A9), S100A8 (S10A8), Galectin-3-binding protein (LG3BP), Cluster of Ig alpha-1 chain C region (IGHA1), Cluster of Ig kappa chain V-III region HAH (KV312), VEGF co-regulated chemokine 1 (VCC1), L-lactate dehydrogenase A chain (LDHA), Aldo-keto reductase family 1 member C (AKR1C1), Rootletin (B1AKD8), L-lactate dehydrogenase B chain (LDHB), Retinal dehydrogenase 1 (AL1A1), Uncharacterized Protein (B4E1Z4), Alpha-1-antichymotrypsin (AACT), Superoxide dismutase [Cu-Zn] (SODC), SPARC-like protein 1 (SPRL1), Ig heavy chain V-III region TIL (HV304), Keratin (K1C9), Cystatin-SN (CYTN), Alpha-actinin-4 (ACTN4), Ig lambda-3 chain C regions (Fragment) (IGLC3), Immunoglobulin lambda-like polypeptide 5 (IGLL5), Alcohol dehydrogenase 1C (ADHIG), Malate dehydrogenase, mitochondrial (MDHM), Calmodulin-like protein 5 (CALL5), Alpha-1-antitrypsin (A1AT), Alpha-1B-glycoprotein (A1BG), Leucine-rich alpha-2-glycoprotein (A2GL), Small ubiquitin-related modifier 3 (A8MU27), Anterior gradient protein 2 homolog (AGR2), Profilin-1 (PROF1), Cluster of Ig lambda chain V-III region LOI (LV302), Prothrombin (E9PIT3), Hemopexin (HEMO), Ig gamma-2 chain C region (IGHG2), Ubiquitin-40S ribosomal protein S27a (RPS27A), Afamin (AFAM), Apolipoprotein A-I (APOA1), Apolipoprotein A-IV (APOA4), Flavin reductase (NADPH)(BLVRB), Prosaposin (PSAP), Lacritin (LACRT), 60S acidic ribosomal protein P1 (RLA1), Inter-alpha-trypsin inhibitor heavy chain H2 (ITIH2), Mucin-like protein 1 (MUCL1), S100 A6 (S100A6), Na(+)/H(+) exchange regulatory cofactor NHE-RF1 (NHRF1), Thioredoxin domain-containing protein 17 (I3L0K2), Lymphocyte-specific protein (LSP1), Cluster of Haptoglobin (H3BS21), Myosin regulatory light chain 12A (J2QRS3), Ribonuclease inhibitor (RINI), Alpha-enolase (ENOA), Cluster of Ig kappa chain V-I region EU (KV106), Alcohol dehydrogenase class 4 mu/sigma chain (ADH7), Protein AMBP (AMBP), Angiotensinogen (ANGT), Antithrombin-III (ANT3), Apolipoprotein A-II (APOA2), Calpastatin (B7Z574), Brain acid soluble protein 1 (BASP1), Alpha-2-HS-glycoprotein (C9JV77), Calreticulin (CALR), Calpain-1 catalytic subunit (CAN1), Cell division control protein 42 homolog (CDC42), Complement C3 (CO3), Coronin-1A (COR1A), Programmed cell death 6-interacting protein (DCD), Definsin 1 (DEF1), F-box only protein 50 (FBX50), Gamma-glutamylcyclotransferase (GGCT), Glutathione reductase, mitochondrial (GSHR), Keratin, type II cytoskeletal 1 (K2C1), UMP-CMP kinase (KCY), Mesothelin (MSLN), N-acetylmuramoyl-L-alanine amidase (PGRP2), Nicotinate phosphoribosyltransferase (PNCB), Inter-alpha-trypsin inhibitor heavy chain H1 (ITIH1), Ribonuclease T2 (RNASET2), Superoxide dismutase [Mn], mitochondrial (SODM), Small proline-rich protein 3 (SPRR3), Src substrate cortactin (SRC8), Cluster of Tubulin beta-4B chain (TBB4B), Tropomyosin alpha-3 chain (TPM3), Serotransferrin (TRFE), Glutathione S-transferase P (THIO), Vitronectin (VTNC), Vitamin D Binding protein (Q6LDC6), Inter-alpha-trypsin inhibitor heavy chain H4 (ITIH4), Metalloprotease inhibitor (TIMP1), Heat Shock protein 90 (HSP90), Cathepsin B (CATB), Ceruloplasmin (CERU), Calprotectin, 14-3-3 sigma (1433S), alpha-2-hs-glycoprotein (FETUA), alpha-2-macroglobulin (A2MG), Transthyretin (TTHY) (Appendix 1). The complete list of proteins identified was exported from Scaffold software for analysis using JMPpro11 statistical software package.

### Example 5:

### Statistical Analysis using JMPpro11 software

Experiment 1: The data was organized by using Microsoft Excel® spreadsheet software with spectral intensities of each of the previously identified proteins for all 75 samples. Also included in the spreadsheet were sample status (cancer, benign, control) cancer type, tumor size grouping (1=0.1mm-1cm, 2=1.1-2.9 cm, 3=3cm or greater) and tumor grade status. This spreadsheet was uploaded into the JMPpro11 software package for analysis.

Binary linear regression was applied between cancer and control samples. Six proteins were identified as significant based on their sigmoidal curve and p-value, they were Ig lambda chain V-IV (LV403), Ig heavy chain V-III region BRO (HV305), Ig heavy chain V-III VH26 (HV303), Lipocallin-1 (LCN1), Beta-2-microglobulin (B2MG), Zinc-alpha-2-glycoprotein (ZA2G). All six proteins appeared to be down-regulated with respect to cancer. To determine if the down regulation of these proteins was specific to breast cancer a binary linear regression was repeated between benign and control. This comparison indicated the six previously identified proteins were also down-regulated in benign samples and an additional four proteins were identified as significant between benign and control; Antileukoproteinase (SLP1), Calmodulin-like-protein 5 (CALL5), Cystatin B (CYTB), and Galectin-3 (LEG3).

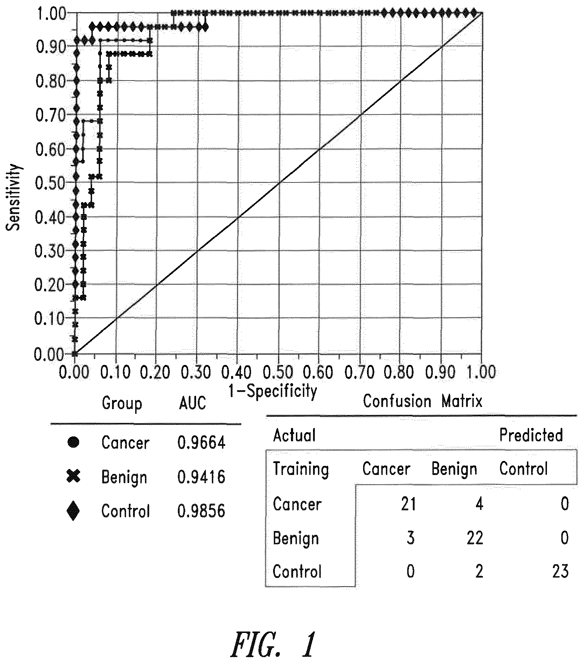

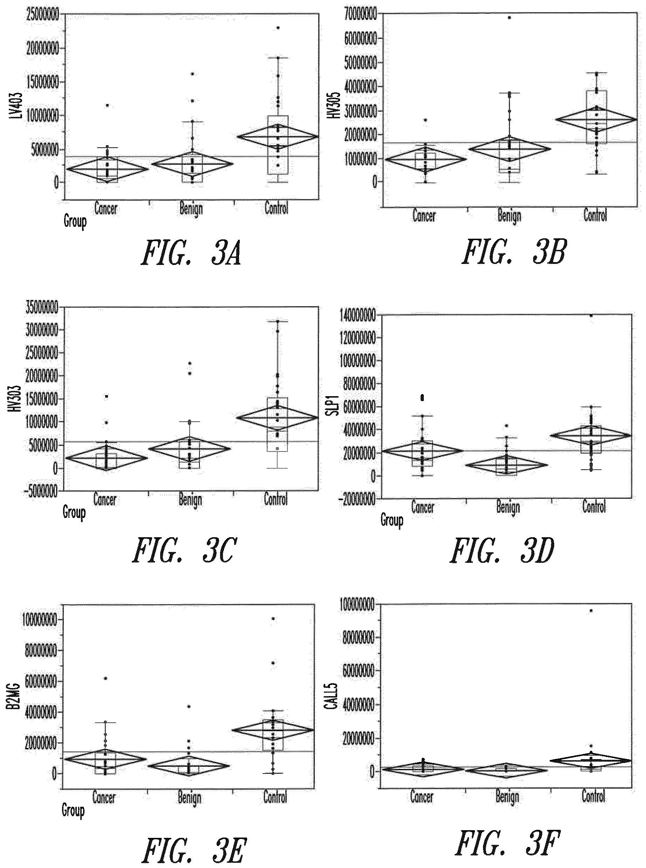

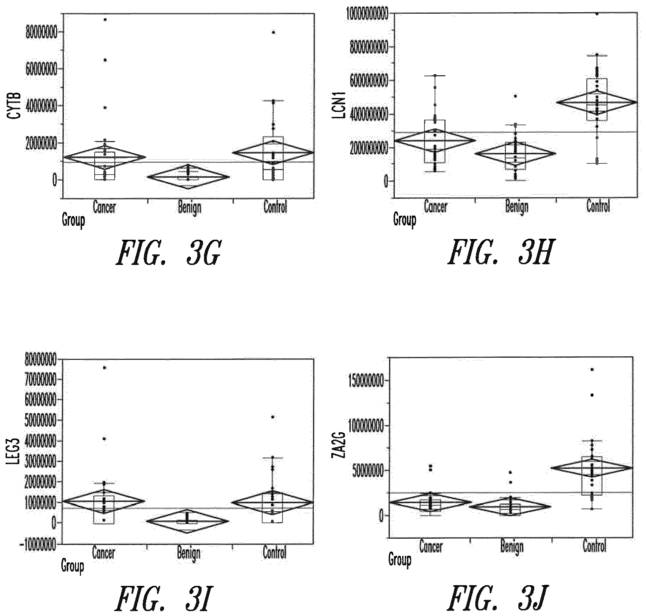

Nominal logistic regression was carried out using all three groups and all ten proteins to determine degree of differentiation between all groups. Degree of misclassification was for the group of ten proteins was 12%. Receiver operator curves for each category produced AUC values of 0.96 for cancer, 0.94 for benign, and 0.98 for control (Figure 1). One way ANOVA analysis was preformed to ensure a shifting mean value for the three categories of samples by each protein (Figure 3).

Experiment 2: One-way ANOVA analysis was preformed using spectral intensities recorded for all proteins identified from mass spectrometry. Tryptic peptide products were analyzed for all proteins. Proteins were compared in three ways: 1) control vs cancer, 2) cancer vs benign, 3) benign vs control. An alpha level of 0.05 was used as an indicator of significant expression change between the groups. Variations in spectral intensities of tryptic fragments were compared. A series of proteins was identified where expression level increased in breast cancer samples with respect to control. Proteins were also identified where expression decreased in breast cancer samples with respect to control. To ensure experimental validity, it was established that proteins were present in whose expression remained constant across all groups tested (Figure 4).

The initial list of 500 proteins was reduced to 103 proteins of interest. Within this group of proteins are proteins with increased expression with respect to breast cancer as well as proteins with decreased expression with respect to breast cancer (Figure 4). The diagnostic capability of whole protein sequence, tryptic peptides, and sequence anomalies, which can be present as a result of disease, are all of interest.

### Example 6:

### ELISA Analysis

A series of proteins with an increase in expression level in cancer samples relative to control samples were observed by mass spectrometry, and several candidates were selected for further validated using ELISA. S100A9 was selected as a representative example of increased expression in tears of breast cancer patients relative to control. LG3BP is shown as a representative of proteins with a decreased expression level in breast cancer samples relative to control samples. Standard ELISA protocol was used to evaluate the expression level of in tears. Data for S100A9 and LG3BP was obtained using kits purchased as DuoSets from R&D Systems (Minneapolis, MN). ELISA procedures were carried out according to instructions provided by R&D systems. Briefly, 100 µL per well of capture antibody, diluted to the recommended working concentration (GAL3BP 1.0 ug/ml; S100A9 0.5 ug/ml) in 1XPBS, was added to a high binding 96 well plate and incubated overnight (∼16hrs) at room temperature. The plate was rinsed with 1XPBS with 0.05% Tween four times, and blotted against clean absorbent paper to remove any traces of liquid following each rinse. Rinse cycles were carried out after each step of the assay. Blocking buffer (1XPBS with 1% BSA) was added to all wells (-200 µL/well) and the plates were incubated for two hours at room temperature. Tear samples were diluted using 1XPBS with 1%BSA at dilutions of 1:10 and 1:50 for S100A9 and 1:100 & 1:500 for Galectin 3 Binding Protein. Dilutions known to fall within the linear region of the standard curve for each assay were selected based on results from previous optimization assays. Samples and standards were tested in duplicate using 100 µL/well and incubated at room temperature for 2 hours. Detection antibody was diluted to the recommended working concentration (GAL3BP 2ug/ml and S100A9 1ug/ml) 100 µL was added to each well, and the plate was allowed to incubate for 2 hours at room temperature. Streptavidin-HRP solution was added to each well (100 µL/well) and allowed to incubate for 15 min at room temperature. Visualization was achieved by addition of TMB substrate solution. After 15 minutes, 50 µL of 1M H₂SO₄ was added to each well, and the absorbance at 450 nm was determined. ELISA data was analyzed using Prism version 6.0 available from GraphPad.

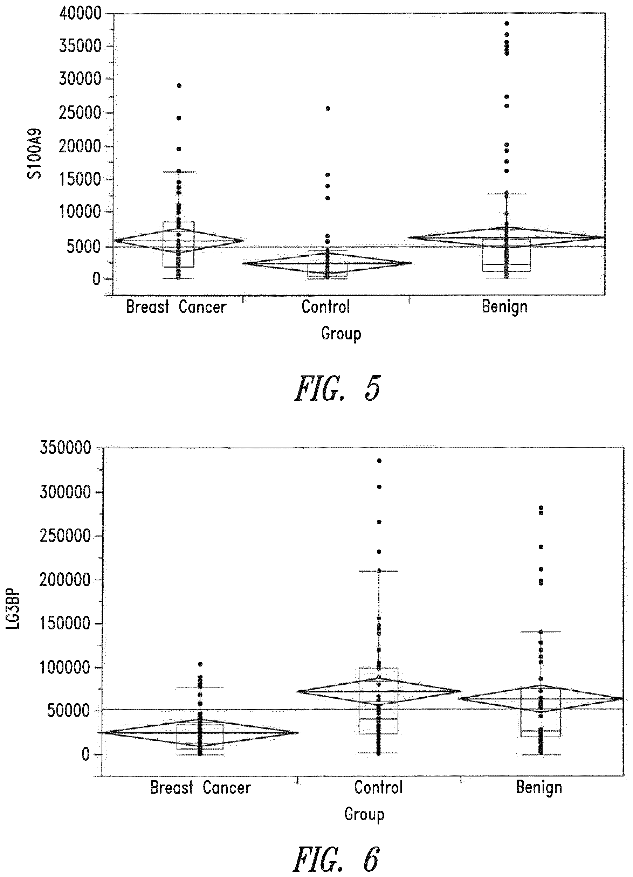

*Statistical analysis of ELISA data*: Concentrations of each protein, as calculated by Prism software, were exported into JMP Pro11 for statistical evaluation. Numerous candidates were selected to be investigated using ELISA assays on Control, Cancer or Benign samples. Results of two proteins (S100A9 and Galectin-3-Binding Protein) are shown (Figure 5 and Figure 6, respectively) to provide representative example of increased and decreased protein expression in breast cancer tear samples with respect to control. ANOVA of S100A9 for 63 breast cancer samples, 79 control samples, and 92 benign samples resulted in a p-value of 0.0005 when all three groups were evaluated. Group means for S100A9 were: breast cancer=5673.02 pg/ml; control=2130.18 pg/ml; benign=6179.10 pg/ml. S100A9 expression is increased by 2.6 fold in cancer samples compared to control samples and 2.9 fold increase in benign samples compared to control. ANOVA of Galectin-3 Binding Protein for 66 breast cancer samples, 55 control samples, and 54 benign samples resulted in a p-value of <0.0001 when all three groups were evaluated. Group means for LG3BP were: breast cancer=24448.1 pg/ml; control=70242.2 pg/ml; benign=62329.7 pg/ml. LG3BP has a 2.8 fold increase in control samples compared to cancer samples and a 2.5 fold increase in benign samples compared to cancer.

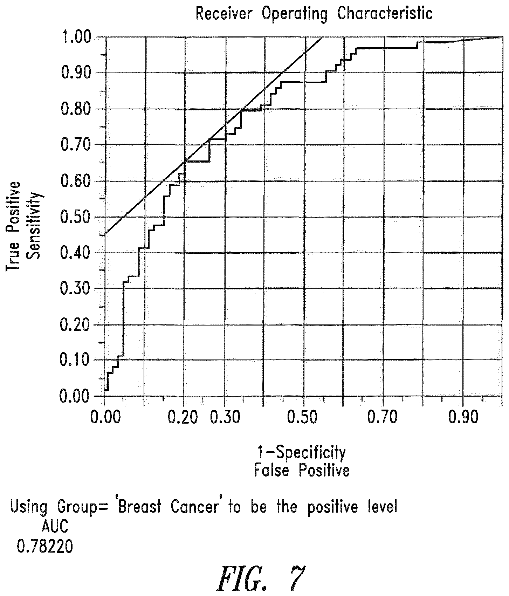

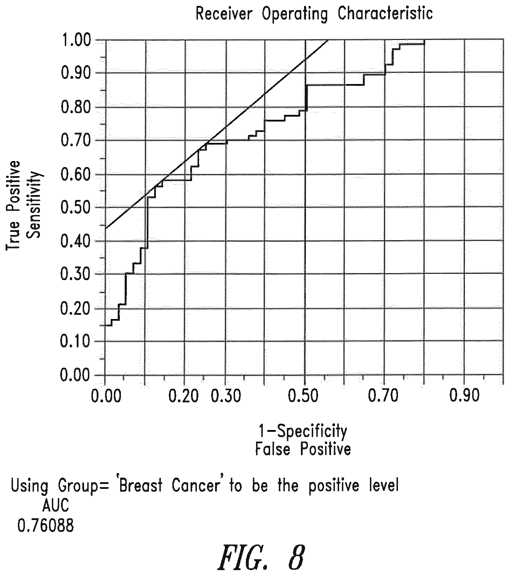

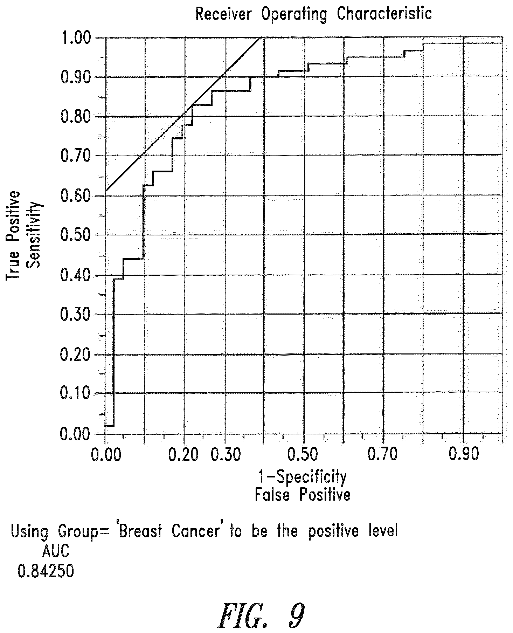

Nominal logistic regression analysis for breast cancer and control samples, was conducted using the two representative proteins. The generated ROC curve for S100A9 has an AUC of 0.78220 (Figure 7), and LG3BP has an AUC of 0.76088 (Figure 8). The analysis was repeated using two proteins and an AUC of 0.84250 was obtained (Figure 9).

*Sample population characteristics:* Clinical data such as breast density, cancer type, and tumor size were obtained on as many samples as possible.

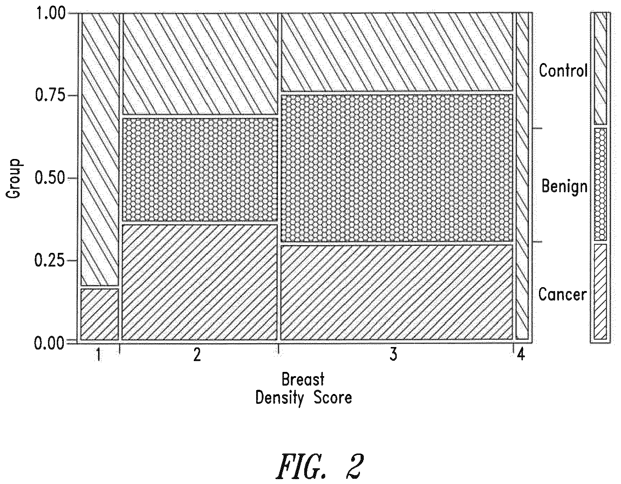

Experiment 1: Out of the 75 samples tested, breast density scores were obtained for 41 of the samples. The break down of the sample population by breast density was 14% category 1, 36.6% category 2, 43.9% category 3, 4.7% category 4 (Figure 2). Eighteen of the twenty-five cancer samples were collected from patients diagnosed with Intra ductal carcinoma (IDC). Two patients were diagnosed with ductal carcinoma In Situ (DCIS), one patient was diagnosed with lobular carcinoma In Situ (LCIS), and one patient was diagnosed with intra lobular carcinoma (ILC).

For classification a scale was designed where a classification of "small" was given to tumors < 20 mm, a classification of "medium" was assigned to tumors between 21 mm-99 mm, and a classification of "large" was assigned to tumors >1cm. Seventeen of the samples collected from breast cancer patients were classified as large tumors, two samples were classified as medium, and six were classified as small. Pathology information of receptor type was obtained for eight of the cancer samples. One sample was ER-/HER2-, one sample was ER-/PR-, two samples were ER+/HER2-, three samples were ER+/HER2+, and one sample was ER+/PR-.

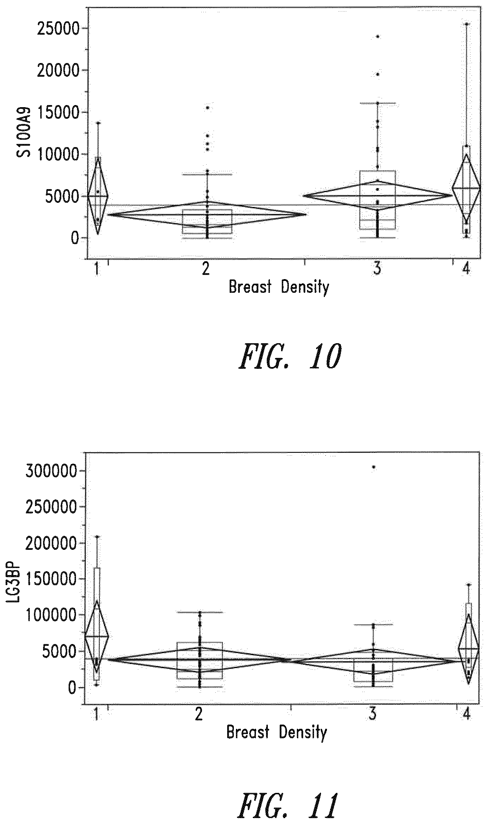

Experiment 2: The sample pool analyzed in this study consisted of 5.9% category 1 breast tissue; 50% category 2, 37.8% category 3, and 5.4% category 4. ANOVA analysis was conducted on several proteins evaluated by ELISA to determine if breast tissue type affects protein expression levels. Representative data is provided for two proteins studied (Figure 10 and Figure 11). Using an alpha level of 0.05, no statistically significant difference was observed for the means of the four types of breast tissue for each of the proteins evaluated in the ELISAs shown in Figures 10 and 11.

Samples were collected from a variety of breast cancer types resulting in 17 samples from patients diagnosed with DCIS, 44 samples from patients diagnosed with IDC, 4 samples from patients with both DCIS & IDC, and 5 samples from patients with ILC. Tumor size varied tremendously. Using that classification system described for Experiment 1, for the samples where tumor size was provided, 33 samples from patients with large tumors were tested, 9 samples from patients with medium tumor size, and 12 samples from patients with small tumor size. Of the 38 samples where receptor type was provided 1 sample was ER-, 2 samples were ER-/HER2-, 2 samples were triple negative (ER-/HER2-/PR-), 1 sample was ER-/HER2+, 11 samples were ER+, ER+/HER2 16, and 5 samples were ER+/HER2+. To date no distinguishing trend has been observed based on tumor size, cancer type, or receptor status, for the proteins that have been studied.

### References:

Armstrong, K., Handorf, E. A., Chen, J., & Bristol Demeter, M. N. (2013). Breast cancer risk prediction and mammography biopsy decisions: a model-based study. American Journal of Preventive Medicine, 44(1), 15-22. doi:10.1016/j.amepre.2012.10.002
Bigenwald, R.Z., Warner, E., Gunasekara, A., Hill, K.A., Causer, P.A., Messner, S.J., Eisen, A., Plewes, D.B., Narod, S.A., Zhang, L., Yaffe, M.J. (2008) Is Mammography Adequate for Screening Women with Inherited BRCA Mutations and Low Breast Density?. Cancer Epidemiology Biomarkers Prevention. 17(3); 707-711.
Böhm, D., Keller, K., Pieter, J., Boehm, N., Wolters, D., Siggelkow, W., et al. (2012). Comparison of tear protein levels in breast cancer patients and healthy controls using a de novo proteomic approach. Oncology Reports, 28(2), 429-438. doi:10.3892/or.2012.1849
Böhm, D., Keller, K., Wehrwein, N., Lebrecht, A., Schmidt, M., Kölbl, H., & Grus, F.-H. (2011). Serum proteome profiling of primary breast cancer indicates a specific biomarker profile. Oncology Reports, 26(5), 1051-1056. doi:10.3892/or.2011.1420
Boyd, N.F, Guo, H., Martin, L.J., Sun, L., Stone, J., Fishell, E., Jong, R.A., Hislop, G., Chiarelli, A., Minkin, S., Yaffee, M.J., (2007) Mammographic Density and the Risk and Detection of Breast Cancer. The New England Journal of Medicine. 356(3): 227-236).
Brown, M. L., Houn, F., Sickles, E. A., & Kessler, L. G. (1995). Screening Mammography in Community Practice: Positive Predictive. American Journal of Radiology, 165, 1373-1377.
CLIA Laws and Regulations, 2013, http://wwwn.cdc.gov/CLIA/Regulatory/default.aspx.
Grady, D. (2012). Study of Breast Biopsies Finds Surgery Used Too Extensively. New York Times, 1-4.
Han-Byoel Lee et al. (2015) Development and Validation of a Novel Plasma Protein Signature for Breast Cancer Diagnosis by Using Multiple Reaction Monitoring-based Mass Spectrometry HAN-. Anticancer Research, 35:1, 6271-6280
Klifa, C., Carballido-Gamio, J., Wilmes, L., Laprie, A., Shepherd, J., Gibbs, J., Fan, B., Noworolski, S., Hylton, N. (2010) Magnetic resonance imaging for secondary assessment of breast density in a high-risk cohort. Magnetic Resonance Imaging. 28;8-15.
Kolb, T., Lichy, J., & Newhouse, J. (2002). Comparison of the performance of screening mammography, physical examination, and breast US and evaluation of factors that influence them: an analysis of 27,825 patient evaluations. Radiology, 225(1), 165-175.
Lebrecht, A., Boehm, D., Schmidt, M., Koelbl, H., & Grus, F. H. (2009a). Surface-enhanced Laser Desorption/Ionisation Time-of-flight Mass Spectrometry to Detect Breast Cancer Markers in Tears and Serum. Cancer Genomics & Proteomics, 6(2), 75-83.
Lebrecht, A., Boehm, D., Schmidt, M., Koelbl, H., Schwirz, R. L., & Grus, F. H. (2009b). Diagnosis of breast cancer by tear proteomic pattern. Cancer Genomics & Proteomics, 6(3), 177-182.
Li, J., Zhang, Z., Rosenzweig, J., Wang, Y., & Chan, D. (2002). Proteomics and bioinformatics approaches for identification of serum biomarkers to detect breast cancer. Clin Chem, 48(8), 1296-1304.
Luftner, D., & Possinger, K. (2002). Nuclear matrix proteins as biomarkers for breast cancer. Expert Rev Mol Diagn, 2(1), 23-31. doi:ERM020106 [pii] 10.1586/14737159.2.1.23
Pisano, E.D., Gatsonis, C., Hendrick E., Yaffe, M., Baum, J.K., Acharyya, S., Conant, E.F. Fajardo, L.L., Bassett, L., D'Orsi, C. Jong, R., Rebner., M. (2005). Diagnostic Performance of Digital versus Film Mammography for Breast-Cancer Screening. The New England Journal of Medicine. 17(353). 1773-1783.
Scheel, J.R., Lee, J.M., Sprague, B.L., Lee, C.I., Lehman, C.D. (2015) Screening ultrasound as an adjunct to mammography in women with mammographically dense breasts. Gynecology. 212(1);9-17.
Schiess, R., Wollscheid, B., & Aebersold, R. (2009). Targeted proteomic strategy for clinical biomarker discovery. Molecular Oncology, 3(1), 33-44. doi:10.1016/j.molonc.2008.12.001
   S S Cross et al. (2005) Expression of S100 proteins in normal human tissues and common cancers using tissue microarrays: S100A6, S100A8, S100A9 and S100A11 are all overexpressed in common cancers. Histopathology, 46:3, 256-269
Tabar, L., Vitak, B., Chen, T.H., Yen, A.M., Cohen, A., Tot, T., Chiu, S., Chen, S. Fann, J. Rosell, J., Fohlin, H., Smith, R. A. , Duffy, S.W., (2011) Swedish Two-County Trial: Impact of Mammographic Screening on Breast Cancer Mortality during 3 Decades. Radiology, 3(260), 658-663.
Vachon, C.M., van Gils, C. H., Sellers, T.A., Ghosh, K., Pruthi, S., Brandt, K.R., Pankratz, V.S., (2007) Mammographic density, breast cancer risk and risk prediction. Breast Cancer Research 9:2017 (doi:10.1186/bcr1829).
Wu, K., & Zhang, Y. (2007). Clinical application of tear proteomics: Present and future prospects. Proteomics. Clinical Applications, 1(9), 972-982. doi:10. 1002/prca.200700125

**Table 1.**

| **Protein Name** | **Gene** | **Protein Name** | **Gene** |
|---|---|---|---|
| Ig lambda chain V-IV region Hil | LV403 | 60S acidic ribosomal protein P1 | RLA1 |
| Ig heavy chain V-III BRO | HV305 | Inter-alpha-trypsin inhibitor heavy chain H2 | ITIH2 |
| Ig heavy chain V-III VH26 | HV303 | Mucin-like protein 1 | MUCL1 |
| Beta-2-microglobulin | B2MG | S100 A6 | S100A6 |
| Lipocalin-1 | LCN1 | Na(+)/H(+) exchange regulatory cofactor NHE-RF1 | NHRF1 |
| Zinc-α-2-glycoprotein | ZA2G | Thioredoxin domain-containing protein 17 | I3L0K2 |
| Cystatin B | CYTB | Lymphocyte-specific protein | LSP1 |
| Antileukoproteinase | SLP1 | Cluster of Haptoglobin | H3BS21 |
| Galectin-3 | LEG3 | Myosin regulatory light chain 12A | J2QRS3 |
| Histidine triad nucleotide-binding protein 1 | D6RD60 | Ribonuclease inhibitor | RINI |
| S100A9 | S10A9 | Alpha-enolase | ENOA |
| S100A8 | S10A8 | Cluster of Ig kappa chain V-I region EU | KV106 |
| Galectin-3-binding protein | LG3BP | Alcohol dehydrogenase class 4 mu/sigma chain | ADH7 |
| Cluster of Ig alpha-1 chain C region | IGHA1 | Protein AMBP | AMBP |
| Cluster of Ig kappa chain V-III region HAH | KV312 | Angiotensinogen | ANGT |
| VEGF co-regulated chemokine 1 | VCC1 | Antithrombin-III | ANT3 |
| L-lactate dehydrogenase A chain | LDHA | Apolipoprotein A-II | APOA2 |
| Aldo-keto reductase family 1 member C | AKR1C1 | Calpastatin | B7Z574 |
| Rootletin | B1AKD8 | Brain acid soluble protein 1 | BASP1 |
| L-lactate dehydrogenase B chain | LDHB | Alpha-2-HS-glycoprotein | C9JV77 |
| Retinal dehydrogenase 1 | AL1A1 | Calreticulin | CALR |
| Uncharacterized Protein | B4E1Z4 | Calpain-1 catalytic subunit | CAN1 |
| Alpha-1-antichymotrypsin | AACT | Cell division control protein 42 homolog | CDC42 |
| Superoxide dismutase [Cu-Zn] | SODC | Complement C3 | CO3 |
| SPARC-like protein 1 | SPRL1 | Coronin-1A | COR1A |
| Ig heavy chain V-III region TIL | HV304 | Programmed cell death 6-interacting protein | DCD |
| Keratin | K1C9 | Definsin 1 | DEF1 |
| Cystatin-SN | CYTN | F-box only protein 50 | FBX50 |
| Alpha-actinin-4 | ACTN4 | Gamma-glutamylcyclotransferase | GGCT |
| Ig lambda-3 chain C regions (Fragment) | IGLC3 | Glutathione reductase, mitochondrial | GSHR |
| Immunoglobulin lambda-like polypeptide 5 | IGLL5 | Keratin, type II cytoskeletal 1 | K2C1 |
| Alcohol dehydrogenase 1C | ADHIG | UMP-CMP kinase | KCY |
| Malate dehydrogenase, mitochondrial | MDHM | Mesothelin | MSLN |
| Calmodulin-like protein 5 | CALLS | N-acetylmuramoyl-L-alanine amidase | PGRP2 |
| Alpha-1-antitrypsin | A1AT | Nicotinate phosphoribosyltransferase | PNCB |
| Alpha-lB-glycoprotein | A1BG | Inter-alpha-trypsin inhibitor heavy chain H1 | ITIH1 |
| Leucine-rich alpha-2-glycoprotein | A2GL | Ribonuclease T2 | RNASET2 |
| Small ubiquitin-related modifier 3 | A8MU27 | Superoxide dismutase [Mn], mitochondrial | SODM |
| Anterior gradient protein 2 homolog | AGR2 | Small proline-rich protein 3 | SPRR3 |
| Profilin-1 | PROF1 | Src substrate cortactin | SRC8 |
| Cluster of Ig lambda chain V-III region LOI | LV302 | Cluster of Tubulin beta-4B chain | TBB4B |
| Prothrombin | E9PIT3 | Tropomyosin alpha-3 chain | TPM3 |
| Hemopexin | HEMO | Serotransferrin | TRFE |
| Ig gamma-2 chain C region | IGHG2 | Glutathione S-transferase P | THIO |
| Ubiquitin-40S ribosomal protein S27a | RPS27A | Vitronectin | VTNC |
| Afamin | AFAM | Vitamin D Binding protein | Q6LDC6 |
| Apolipoprotein A-I | APOA1 | Inter-alpha-trypsin inhibitor heavy chain H4 | ITIH4 |
| Apolipoprotein A-IV | APOA4 | Metalloprotease inhibitor | TIMP1 |
| Flavin reductase (NADPH) | BLVRB | Heat Shock protein 90 | HSP90 |
| Prosaposin | PSAP | Cathepsin B | CATB |
| Lacritin | LACRT | Ceruloplasmin | CERU |
| 14-3-3 sigma | 1433S | Calprotectin | |
| alpha-2-hs-glycoprotein | FETUA | alpha-2-macroglobulin | A2MG |
| | | Transthyretin | TTHY |

### APPENDIX I

| **Protein Name** | **UniProt** | **IPI** | **Gene Name** |
|---|---|---|---|
| Lipocalin 1 | P10325 | | LCN1_Human |

| **Trypsin Fragments** | | | |
|---|---|---|---|
| **1.** QSETCSPGSD | **2.** VTMLISGR | | **3.** AVLEKTDEPGK |
| **4.** AVLEKTDEPGKYTADGGK | **5.** TDEPGKYTADGGK | | **6.** HVAYIIR |
| **7.** DPKNNLEALEDFEK | **8.** SHVKDHYIFYCEGELHGKPVR | | **9.** DHYIFYCEGELHGKPVR |
| **10.** SHVKDHYIFYCEGELHGK | **11.** LVGRDPKNNLEALEDFEK | | **12.** GLSTESILIPR |
| **13.** NNLEALEDFEKAAGAR | **14.** YTADGGKHVAYIIR | | **15.** AAGARGLSTESILIPR |
| **16.** TDEPGKYTADGGKHVAYIIR | **17.** AAGARGLSTESILIPRQSETCSPGSD | | **18.** DHYIFYCEGELHGK |
| **19.** NNLEALEDFEK | **20.** AMTVDREFPEMNLESVTPMTLTTLEGGN | | **21.** GLSTESILIPRQSETCSPGSD |

### Proteins selected through Experiment 1:

| **Protein Name** | **UniProt** | **IPI** | **Gene Name** |
|---|---|---|---|
| Zinc-α-2-glycoprotein | **P25311** | | **ZA2G_Human** |

| **Trypsin Fragments** | | | |
|---|---|---|---|
| **1**. AGEVQEPELR | **2.** AYLEEECPATLR | | 3. CLAYDFYPGK |
| 4. EIPAWVPFDPAAQITK | 5. HVEDVPAFQALGSLNDLQFFR | | 6. LKCLAYDFYPGK |
| 7. NILDRQDPPSVVVTSHQAPGEK | 8. QDPPSVVVTSHQAPGEK | | 9. QKWEAEPVYVQR |
| 10. WEAEPVYVQR | 11. YSKNILDRQDPPSVVVTSHQAPGEK | | 12. YSLTYIYTGLSK |
| 13. YYYDGKDYIEFNK | 14. AYLEEECPATLRK | | 15. AKAYLEEECPATLRK |
| 16. QVEGMEDWKQDSQLQK | 17. CLAYDFYPGKIDVHWTR | | |

| | | | |
|---|---|---|---|
| **Protein Name** | **UniProt** | **IPI** | **Gene Name** |

| **Protein Name** | **UniProt** | **IPI** | **Gene Name** |
|---|---|---|---|
| Ig heavy chain V-III region BRO | **P01766** | | **HV305_Human** |

| **Trypsin Fragments** | | | |
|---|---|---|---|
| **1. EVQLVESGGGLVQPGGSLR** | **2. AEDTAVYYCAR** | | **3.** |

| **Protein Name** | **UniProt** | **IPI** | **Gene Name** |
|---|---|---|---|
| Beta-2-microglobulin | **P61769** | | **B2MG_Human** |

| **Trypsin Fragments** | | | |
|---|---|---|---|
| **1. IQVYSRHPAENGK** | **2. SNFLNCYVSGFHPSDIEVDLLK** | | **3. IEKVEHSDLSFSK** |
| **4. VEHSDLSFSK** | **5. VNHVTLSQPK** | | **6. DEYACRVNHVTLSQPK** |

| | | | |
|---|---|---|---|
| Antileukoproteinase | **P03972** | | **SLP1_Human** |

| **Trypsin Fragments** | | | |
|---|---|---|---|
| **1. AGVCPPKKSAQCLR** | **2. CCPDTCGIKCLDPVDTPNPTR** | | **3. CLDPVDTPNPTR** |
| **4. YKKPECQSDWQCPGK** | **5. SCVSPVKA** | | **6. KPECQSDWQCPGK** |

| **Protein Name** | **UniProt** | **IPI** | **Gene Name** |
|---|---|---|---|
| Ig Heavy Chain V-III VH26 | **P01764** | | **HV303_Human** |

| **Trypsin Fragments** | | | |
|---|---|---|---|
| **1. EVQLLESGGGLVQPGGSLR** | **2. AEDTAVYYCAK** | | **3.** |

| **Protein Name** | **UniProt** | **IPI** | **Gene Name** |
|---|---|---|---|
| Cystatin-B | **P04080** | | **CYTB_Human** |

| **Trypsin Fragments** | | | |
|---|---|---|---|
| **1. AVSFSQVVAGTNFIK** | **2. SQVVAGTNYFIK** | | **3. VFQSLPHENKPLETLSNYQTNK** |
| **4. VHVGDEDFVHLR** | | | |

| **Protein Name** | **UniProt** | **IPI** | **Gene Name** |
|---|---|---|---|
| Ig Lambda Chain V-IV region HiL | **P01717** | | **LV403_Human** |

| **Trypsin Fragments** | | | |
|---|---|---|---|
| **1. SYELTQPPSVSVSPGQTAR** | **2.** | | **3.** |

| **Protein Name** | **UniProt** | **IPI** | **Gene Name** |
|---|---|---|---|
| Galectin-3 | **P17931** | | **LEG3_Human** |

| **Trypsin Fragments** | | | |
|---|---|---|---|
| **1. IQVLVEPDHFK** | **2. QSVFPFESGKPFK** | | **3. VIVCNTKLDNNWGR** |
| **4. IALDFQR** | **5. VAVNDAHLLQYNHR** | | **6. GNDVAFHFNPR** |
| **7. MLITILGTVKPNANR** | **8. RVIVCNTKLDNNWGR** | | **9. KLNEISK** |

### Proteins selected from Experiment 2:

| **Protein Name** | **UniProt** | **IPI** | **Gene Name** |
|---|---|---|---|
| Calmodulin-like protein 5 | **Q9NZT1** | | **CALL5_Human** |

| **Trypsin Fragments** | | | |
|---|---|---|---|
| **1. AGELTPEEEAQYKK** | **2. KAFSAVDTDGNGTINAQELGAALK** | | **3. AFSAVDTDGNGTINAQELGAALK** |
| **4. AGLEDLQVAFR** | **5. EADVDQDGRVNYEEFAR** | | **6. NLSEAQLR** |

## Claims

1. A method of determining whether a subject has breast cancer comprising:
providing an ocular sample previously obtained from the subject; performing steps of detecting levels in the sample of at least three of the markers from the list of markers provided in Table 1; and determining the subject has, or is likely to have, breast cancer if the levels of the at least three markers change as compared to the levels in a control sample lacking cancer, wherein the at least three markers comprise S100A8, S100A9 and LG3BP, and wherein the levels of S100A8 are increased, the levels of S100A9 are increased and the levels of LG3BP are decreased.

2. The method of claim 1, wherein the subject is a human.

3. The method of any one of the preceding claims, wherein the LG3BP marker is decreased at least, 1.5 fold, 2 fold, 4 fold or more relative to the level of the marker in the control sample.

4. The method of any one of the preceding claims, wherein the S100A8 and S100A9 markers are increased at least, 1.5 fold, 2 fold, 4 fold or more relative to the level of the marker in the control sample.

5. The method of any one of the preceding claims, wherein the level of the marker is detected by liquid chromatography-mass spectroscopy (LC-MS).

6. The method of any one of the preceding claims, wherein the level of the marker is detected by an antibody-based detection method.

7. The method of any one of the preceding claims, wherein the level of the marker is detected by multiplex protein detection method.

8. The method of any one of the preceding claims, wherein the level of the marker is detected by an mRNA detection method.

9. The method of any one of the preceding claims, wherein the subject is suspected of having cancer.

10. The method of any one of the preceding claims, wherein the subject is at increased risk for developing a cancer.

11. The method of any one of the preceding claims, wherein the subject has a palpable lump suspected of being cancerous.

12. The method of any one of the preceding claims, wherein the breast density of the subject is category 1, category 2, category 3 or category 4.

13. The method of any one of the preceding claims, wherein the cancer is detected as a stage of breast cancer.

## Patentansprüche

1. Verfahren zur Bestimmung, ob ein Subjekt Brustkrebs hat, umfassend:
Bereitstellen einer Augenprobe, die zuvor vom Subjekt erhalten wurden; Durchführen von Schritten zum Nachweis in der Probe von Spiegeln von mindestens drei der Marker aus der Liste von Markern, die in Tabelle 1 bereitgestellt sind;
und Bestimmen, dass das Subjekt Brustkrebs hat oder wahrscheinlich Brustkrebs hat, wenn die Spiegel der mindestens drei Marker sich verändern, verglichen mit den Spiegeln einer Kontrollprobe, bei der Krebs fehlt, wobei die mindestens drei Marker S100A8, S100A9 und LG3BP umfassen und wobei die Spiegel von S100A8 erhöht sind, die Spiegel von S100A9 erhöht sind und die Spiegel von LG3BP verringert sind.

2. Verfahren nach Anspruch 1, wobei das Subjekt ein Mensch ist.

3. Verfahren nach einem der vorhergehenden Ansprüche, wobei der LG3BP-Marker um mindestens das 1,5-Fache, 2-Fache, 4-Fache oder mehr relativ zum Spiegel des Markers in der Kontrollprobe verringert ist.

4. Verfahren nach einem der vorhergehenden Ansprüche, wobei die S100A8- und S100A9-Marker um mindestens das 1,5-Fache, 2-Fache, 4-Fache oder mehr relativ zum Spiegel des Markers in der Kontrollprobe erhöht sind.

5. Verfahren nach einem der vorhergehenden Ansprüche, wobei der Spiegel des Markers mittels Flüssigchromatographie mit Massenspektrometrie-Kopplung (LC-MS) nachgewiesen wird.

6. Verfahren nach einem der vorhergehenden Ansprüche, wobei der Spiegel des Markers mittels eines Antikörperbasierten Nachweisverfahrens nachgewiesen wird.

7. Verfahren nach einem der vorhergehenden Ansprüche, wobei der Spiegel des Markers mittels eines Multiplex-Proteinnachweisverfahrens nachgewiesen wird.

8. Verfahren nach einem der vorhergehenden Ansprüche, wobei der Spiegel des Markers mittels eines mRNA-Nachweisverfahrens nachgewiesen wird.

9. Verfahren nach einem der vorhergehenden Ansprüche, wobei bei dem Subjekt Verdacht auf Krebs besteht.

10. Verfahren nach einem der vorhergehenden Ansprüche, wobei bei dem Subjekt das erhöhte Risiko besteht, an Krebs zu erkranken.

11. Verfahren nach einem der vorhergehenden Ansprüche, wobei das Subjekt einen ertastbaren Knoten aufweist, bei dem der Verdacht besteht, dass er kanzerös ist.

12. Verfahren nach einem der vorhergehenden Ansprüche, wobei die Brustdichte des Subjekts Kategorie 1, Kategorie 2, Kategorie 3 oder Kategorie 4 ist.

13. Verfahren nach einem der vorhergehenden Ansprüche, wobei der Krebs als ein Stadium von Brustkrebs nachgewiesen wird.

## Revendications

1. Méthode permettant de déterminer si un sujet a un cancer du sein consistant à : utiliser un échantillon oculaire préalablement obtenu à partir du sujet ; effectuer des étapes de détection de taux dans l'échantillon d'au moins trois des marqueurs provenant de la liste de marqueurs fournie dans le tableau 1 ; et déterminer si le sujet a, ou est susceptible d'avoir, un cancer du sein si les taux des au moins trois marqueurs changent par comparaison avec les taux dans un échantillon témoin sans cancer, dans laquelle les au moins trois marqueurs comprennent S100A8, S100A9 et LG3BP et dans laquelle les taux de S100A8 sont accrus, les taux de S100A9 sont accrus et les taux de LG3BP sont réduits.

2. Méthode selon la revendication 1, dans laquelle le sujet est un être humain.

3. Méthode selon l'une quelconque des revendications précédentes, dans laquelle le marqueur LG3BP est réduit d'un facteur d'au moins 1, 5, 2, 4 ou plus par rapport au taux du marqueur dans l'échantillon témoin.

4. Méthode selon l'une quelconque des revendications précédentes, dans laquelle les marqueurs S100A8 et S100A9 sont accrus d'un facteur d'au moins 1, 5, 2, 4 ou plus par rapport au taux du marqueur dans l'échantillon témoin.

5. Méthode selon l'une quelconque des revendications précédentes, dans laquelle le taux du marqueur est détecté par chromatographie liquide-spectrométrie de masse (LC-MS).

6. Méthode selon l'une quelconque des revendications précédentes, dans laquelle le taux du marqueur est détecté par une méthode de détection à base d'anticorps.

7. Méthode selon l'une quelconque des revendications précédentes, dans laquelle le taux du marqueur est détecté par une méthode de détection de protéines en multiplex.

8. Méthode selon l'une quelconque des revendications précédentes, dans laquelle le taux du marqueur est détecté par une méthode de détection d'ARNm.

9. Méthode selon l'une quelconque des revendications précédentes, dans laquelle le sujet est supposé avoir un cancer.

10. Méthode selon l'une quelconque des revendications précédentes, dans laquelle le sujet présente un risque accru de développer un cancer.

11. Méthode selon l'une quelconque des revendications précédentes, dans laquelle le sujet a une grosseur palpable supposée être cancéreuse.

12. Méthode selon l'une quelconque des revendications précédentes, dans laquelle la densité des seins du sujet est de catégorie 1, de catégorie 2, de catégorie 3 ou de catégorie 4.

13. Méthode selon l'une quelconque des revendications précédentes, dans laquelle le cancer est détecté sous forme d'un stade de cancer du sein.
